(19) Europäisches Patentamt
European Patent Office
Office européen des brevets

(11) Publication number: **0 367 514**
**A2**

# (12) EUROPEAN PATENT APPLICATION

(21) Application number: 89311130.2

(51) Int. Cl.[5] **C07K 5/00 , A61K 37/64**

(22) Date of filing: 27.10.89

The application is published incomplete as filed (Article 93 (2) EPC). The points in the originally filed description (pages 90,91 and 93) at which the omission obviously occurs has been left blank.

(30) Priority: 27.10.88 US 263385
20.09.89 US 409894

(43) Date of publication of application:
09.05.90 Bulletin 90/19

(84) Designated Contracting States:
ES GR

(71) Applicant: **UNIVERSITY OF KENTUCKY RESEARCH FOUNDATION**
**Administration Building University of Kentucky**
**Lexington Kentucky 40506-0032(US)**

(72) Inventor: **Digenis, George A.**
**2133 Woodmont Drive**
**Lexington, Kentucky 40502(US)**
Inventor: **Agha, Bushra J.**
**57 New Holland Village**
**Nanuet, New York 10954(US)**
Inventor: **Khori, Charles Dr.**
**550 Brickell Avenue, Suite 502**
**Miami, Florida 33131(US)**

(74) Representative: **Overbury, Richard Douglas et al**
**HASELTINE LAKE & CO Hazlitt House 28**
**Southampton Buildings Chancery Lane**
**London WC2A 1AY(GB)**

## (54) Human leukocyte elastase inhibitors and methods for producing and using same.

(57) Compounds selected from the group consisting of a compound of the formula

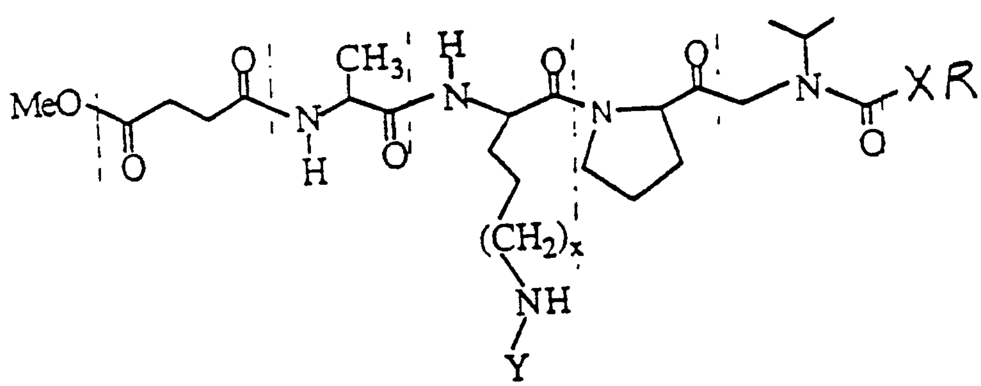

(I)

and a compound of the formula

EP 0 367 514 A2

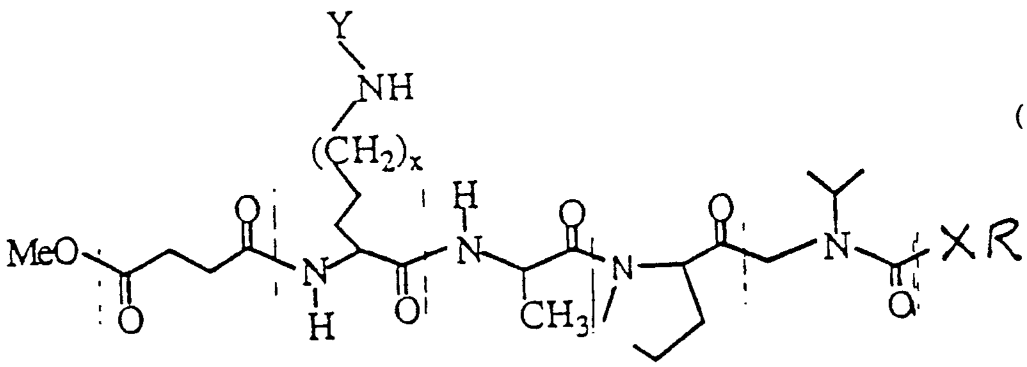

(II)

wherein
x is 1 or 2,
Y is carbobenzoxy or benzoyl, and
XR is

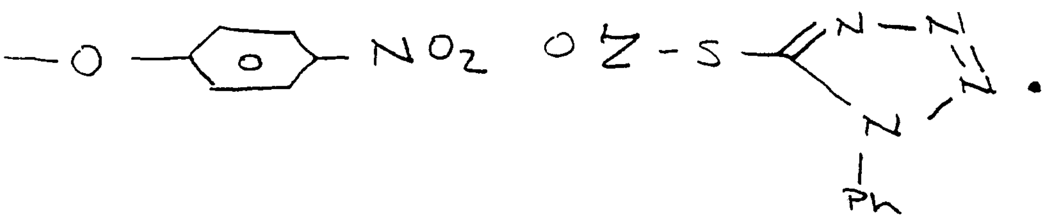

have use as elastase enzyme inhibitors. Particularly potent are the L-proline diastereomers.

Elastase Enzyme inhibitory compositions comprise a carrier and an elastase enzyme inhibiting amount of the compounds of the invention.

A method of selectively inhibiting the enzyme elastase in an animal or a human in need of such treatment comprises administering to the animal or human an enzyme elastase inhibiting amount of one of the compounds of the invention or a composition thereof.

A method of reducing corneal scarring or fibroblast proliferation comprises applying to an area of a subject's eye afflicted with the condition a corneal scar- fibroblast proliferation-reducing amount of a free or polymer-bound HLE inhibitory agent under conditions and for a period of time effective to attain the desired effect. A method of reducing neovascularization of corneal scar tissue comprises applying to an area of a subject's eye afflicted with the condition a neovascularization-inhibitory amount of a free or polymer-bound HLE inhibitory agent under conditions and for a period of time effective to attain the desired effect.

# HUMAN LEUKOCYTE ELASTASE INHIBITORS AND METHODS OF PRODUCING AND USING SAME

## Technical Field

This invention relates generally to the production of human leukocyte elastase inhibitors and more particularly to novel peptidyl carbamate inhibitors of the enzyme elastase and to novel synthetic routes to synthesize the peptidyl carbamates of the invention and to methods of inhibiting the enzyme elastase with the compounds of the invention. This invention also relates to methods of treating corneal scarring or fibroblast proliferation by applying to the ocular area a human leukocyte elastase (HLE) inhibitory agent in a prescribed amount.

## Background Art

Proteinases from polymorphonuclear leukocytes and macrophages, especially elastases (human leukocyte elastase and cathepsin G), appear to be responsible for the chronic tissue destruction associated with inflammation, arthritis and emphysema. During infection or inflammation, the normal lung is protected from proteolytic digestion by the protease inhibitor, alpha$_1$-antitrypsin. The protective mechanism appears to be nonoperative in individuals with an alpha$_1$-antitrypsin deficiency due to genetic or other causes. Synthetic elastase inhibitors capable of replacing alpha$_1$-antitrypsin may therefore be useful in the treatment of pulmonary emphysema and related diseases.

Several types of elastase inhibitors have been reported in the literature. These include peptide chloromethyl ketones as described by P.M. Tuhy and J.C. Powers, "Inhibition of Human Leukocyte Elastase by Peptide Chloromethyl Ketones", FEBS Letters, 50, 359-61 (1975); J.C. Powers, B. F. Gupton, A. D. Harley, N. Nishino and R. Whitley, "Specificity of Porcine Pancreatic Elastase, Human Leukocyte Elastase and Cathepsin G. Inhibition with Peptide Chloromethyl Ketones", Biochem. Biophys. Acta. 485, 156-66 (1977); azapeptides, C. P. Dorn, M. Zimmerman, S. S. Yang, E. C. Yurewicz, B. M. Ashe, R. Frankshun and H. Jones, "Proteinase Inhibitors. 1. Inhibitors of Elastase", J. Med. Chem., 20, 1464-68 (1977); J. C. Powers and B. F. Gupton, "Reaction of Serine Proteases with Aza-amino Acid and Aza-peptide Derivatives", Meth. Enzymol., 46, 208-16 (1977); sulfonyl fluorides, T. Yoshimura, L. N. Barker and J. C. Powers, "Specificity and Reactivity of Human Leukocyte Elastase, Porcine Pancreatic Elastase, Human Granulocyte Cathepsin G, and Bovine Pancreatic Elastase, Human Granulocyte Cathepsin G, and Bovine pancreatic Chymotrypsin with Arylsulfonyl Fluorides. Discovery of a new series of potent and specific irreversible Elastase Inhibitors", J. Biol. Chem. 257, 5077-84 (1982); heterocyclic acylating agents, M. Zimmerman, H. Morman, D. Mulvey, H. Jones, R. Frankshum and B. M. Ashe, "Inhibition of Elastase and Other Serine Proteases by Heterocyclic Acylating Agents", J. Biol. Chem. 25, 9848-51 (1980); B. M. Ashe, R. L. Clark, H. Jones and M. Zimmerman, "Selective Inhibition of Human Leukocyte Elastase and Bovine a$_1$-Chymotrypsin by Novel Heterocycles", J. Biol. Chem. 256: 11603-6(1981); imidazole N-carboxamides, W. C. Groutas, R. C. Badger, T. D. Ocain, D. Felder, J. Frankson and M. Theodorakis, Biochem. Biophys. Res. Commun., 95, 1890 (1980); and p-nitrophenyl-N alkyl carbamates, R. E. Scofield, R. P. Werner and F. Wold, "p-Nitrophenyl Carbamates as Active-Site-Specific Reagents for Serine Proteases", Biochemistry, 16, 2492 (1977).

Although some peptide chloromethyl ketones have been shown to be effective in preventing elastase induced emphysema in animal models, A. Jaoff and R. Dearing, "Prevention of Elastase Induced Experimental Emphysema by Oral Administration of Synthetic Elastase Inhibitor", Am. J. Respir. Dis. 121, 1025-3 (1980). However, there is considerable question whether such reactive agents can be used for treating emphysema in humans. This is not surprising since the alkylating moieties in these inhibitors might render them toxic when used on a continuous basis. To be suitable for human use, an enzyme inhibitor has to show a high degree of selectivity and must have minimal toxic side effects. As a result, most drugs are molecules that reversibly bind to specific enzymes or receptor sites. Examples are the carbamate esters physostigmine and neostigmine which have been clinically used as inhibitors of acetyl choline esterases (A. G. Gilman, L. S. Goodman, and A. Gilman, "The Pharmacological Basis of Therapeutics", p. 101, MacMillan Publishing Co. (1980)).

U. S. Patent 4,643,991, Tsuji K. et al, B.B.R.C. 122(2):571 (1984) and Digenis, G. A. et al, J. Med. Chem. 29:1468 (1986) describe peptide elastase inhibitors which are specific and active-site directed and are not subject to the disadvantages associated with other prior art compounds for this purpose.

A series of peptide elastase inhibitors were disclosed in U.S. Patent 4,643,991 to Digenis et al. Another

group of polymer-bound elastase inhibitors was disclosed in U.S. Application Serial No. 242,294 by Digenis et al filed on September 9, 1988.

There still remains a need in the art for compounds which are superior specific, active-site directed inhibitors of the enzyme elastase without the concomitant detrimental features of other prior art compounds.

Various conditions of the eye are known to be associated with corneal scarring and fibroblast proliferation, amongst them ocular coagulation and burns, mechanical and chemical injury, ocular infections such as kerato-conjunctivitis, and other ocular conditions. Some of these conditions are known to arise postoperatively after surgical treatment of other ocular conditions. This undesirable tissue growth is easily neovascularized and therefore becomes permanently established and irrigated. Tissue scarring or fibroblast proliferation is a condition which is difficult to treat. Presently, it is treated by subjecting the ocular area to further surgery or by using steroids, topically or by injection. However, steroids do increase side effects such as infection, cataract and glaucoma. Other non-steroidal agents like indomethcin have very little anti-scarring effects. (Williamson J. et al., British J. of Ophthalmology 53:361 (1969); Babel, Histologie Der Crtisonkatarakt, p.327. Bergmann, Munich (1973)).

Even after further surgery the proliferation of fibroblastic tissue continues to occur and further scar tissue appears. Thus, in addition to surgery being an extremely invasive procedure, the results attained thereof are not entirely satisfactory.

Accordingly, there remains a need in the art for methods of reducing and/or preventing the formation of corneal scar tissue or fibroblast proliferation which are not subject to the disadvantages of methods known in the art for this purpose.

## Summary of the Invention

This invention relates to a compound of the formula selected from the group consisting of
a compound of the formula

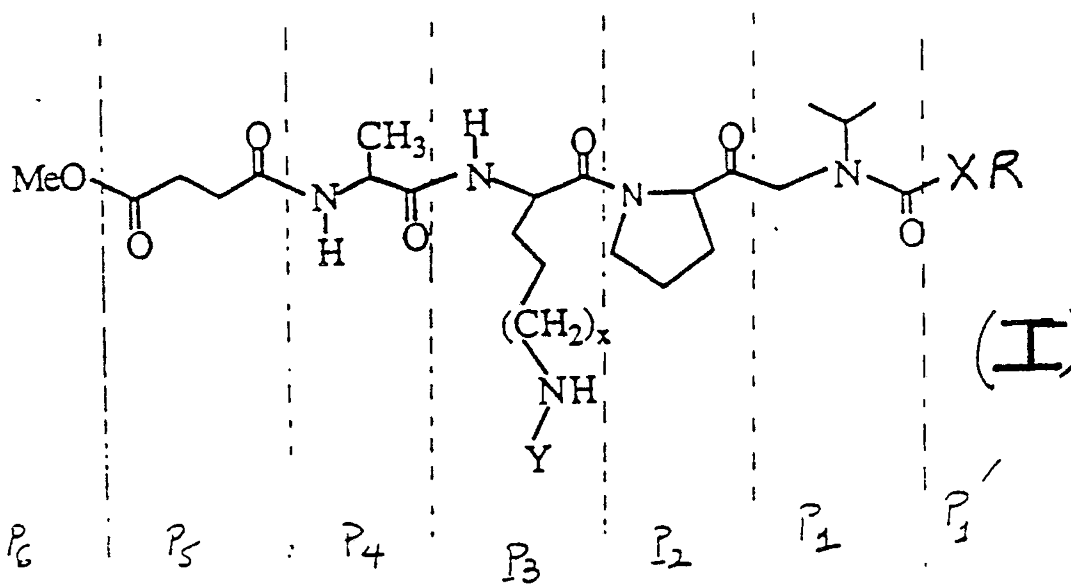

(I)

and
a compound of the formula

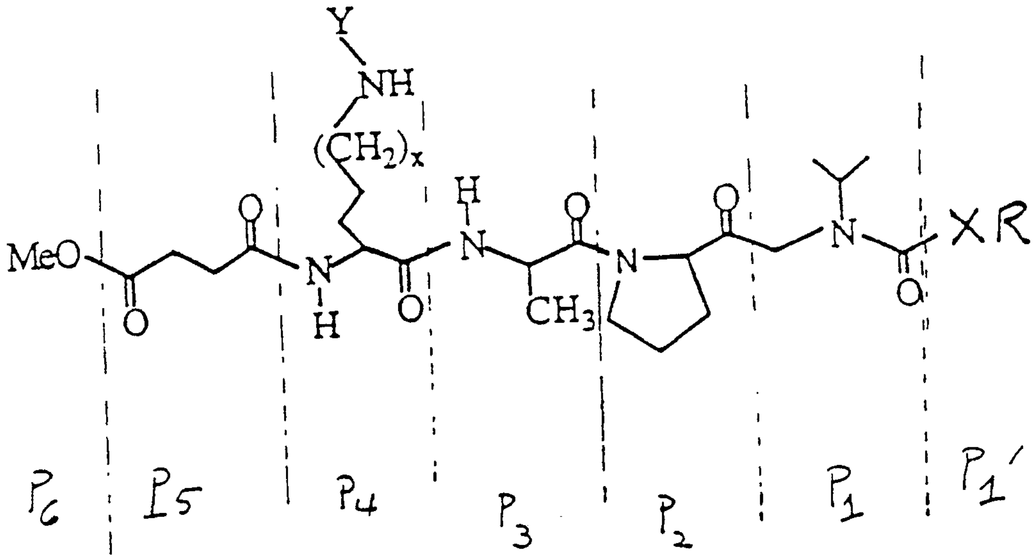

(II)

wherein
x is 1 or 2,
Y is carbobenzoxy or benzoyl, and

XR =

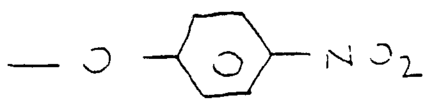

or

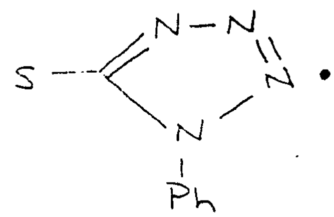

This invention also provides an enzyme elastase inhibitory composition comprising an enzyme elastase inhibitory amount of the compound of the invention, and a carrier.

Also part of the invention is a method of inhibiting the activity of the enzyme elastase comprising adding to an enzyme solution an enzyme elastase inhibitory amount of the compound of the invention.

This invention also relates to a method of selectively inhibiting the activity of the enzyme elastase in the presence of an enzyme selected from the group consisting of trypsin and chymotrypsin comprising adding to an elastase enzyme solution an enzyme elastase inhibitory amount of the compound of the invention.

Still part of this invention is a method of selectively inhibiting the enzyme elastase in an animal or human in need of such treatment comprising administering to said animal or human an enzyme elastase inhibiting amount of the compound of the invention.

This invention also relates to a method of reducing corneal scarring or fibroblast proliferation comprising applying to an area of a subject's eye afflicted with the condition a corneal scar- reducing or fibroblast proliferation-reducing amount of a human leukocyte elastase (HLE) inhibitory agent under conditions and for a period of time effective to attain the desired effect.

Also part of this invention is a method of reducing neovascularization of corneal scar tissue comprising applying to an area of a subject's eye afflicted with the condition a neovascularization-inhibitory amount of an HLE inhibitory agent under conditions and for a period of time effective to attain the desired effect.

A more complete appreciation of the invention and many of the attendant advantages thereof will be readily perceived as the same becomes better understood by reference to the following detailed description when considered in connection with the accompanying figures.

## Brief Description of Drawings

Fig. 1 depicts the inhibition of human leukocyte elastase (HLE) by various peptidyl carbamate

derivatives and $alpha_1$-proteinase inhibitor ($alpha_1$-PI) using MeO-Suc-Ala-Ala-Pro-VAL-NA as a substrate, where the concentration of the substrate is 1.62 x $10^{-4}$ M and the concentration of the enzyme is 3.4 x $10^{-3}$ M.

Fig. 2 depicts the cumulative molecular weight distribution of the polymer-bound compound described in Example 69 (polymer IV).

Fig. 3 depicts the absorption spectrum of the polymer bound compound of Example 69 (polymer IV).

Fig. 4 shows a gel permeation chromatography (GPC) analysis of the reaction mixture of the compound and the polymer of Example 69 (compound III) at various times of the reaction.

Fig. 5 depicts the binding of the elastase inhibitory peptide to the polymer (compound III) of Example 69.

Fig. 6 is a print of a histological microscopic photograph of a corneal tissue as described in Table 6. This photograph shows a pathology section of an untreated corneal burn. Excessive inflammation and fibroblastic activity (thin arrows) can be seen. Severe neovascular formation is marked with thick short arrows (H&E Stain x200).

Fig. 7 is a print of a histological microscopic photograph of a corneal tissue described in Table 6. This photograph shows a pathology section of a corneal burn treated with the inhibitor of this invention. As can be seen, the degree of inflammation, scarring and neovascular formation is minimal and has been greatly reduced when compared with Fig. 6, the control eye (H&E Stain x 200).

Figures 8, 10 and 12 are pathology sections of rabbit corneas that were thermally burned and treated for two weeks with polyvinyl alcohol. Severe inflammatory reaction is seen including formation of polymorphonuclear neutrophils, eosinophils, fibroblasts, and granulation tissue (arrows ) (Hematoxylin and eosin x 100).

Figures 9, 11 and 13 show pathology sections of rabbit corneas that were thermally burned and treated with the inhibitor of this invention for two weeks. As can be seen, the inflammatory reaction is much reduced in comparison to the control eyes (untreated) shown in Figures 8, 10 and 12. Inflammation, scarring and neovascularization are much reduced when compared with the control eyes (Hematoxylin and eosin x 100).

Other objects, advantages and features of the present invention will become apparent to those skilled in the art from the following discussion.

## Best Mode for Carrying out the Invention

### A. Peptidyl Carbamate Inhibitors of the Enzyme Elastase.

The first part of this invention arose from the desire of providing novel, more potent and selective peptidyl carbamate inhibitors of the enzyme elastase. Thus, the present genus of peptidyl carbamate inhibitors incorporates derivatized lysine and ornithine residues into the P3 or P4 positions of the dipeptides, i.e., the compounds of formulas I and II. The present compounds resemble the desmosine cross-linking units in mature elastin, which is the natural substrate of the human leukocyte elastase enzyme.

Thus, this invention provides certain novel substituted peptidyl carbamate compounds, pharmaceutical compositions containing these compounds, and methods for using these pharmaceutical compositions in the selective inhibition of the enzyme elastase without affecting similar serine dependent proteases, e.g., trypsin and chymotrypsin.

It is known from the art that proteases from polymorphonuclear leukocytes and macrophages, especially elastases (human leukocyte HL elastase and cathepsin G) appear to be responsible for the chronic tissue destruction associated with inflammation, arthritis and emphysema. During infection or inflammation, the normal lung is protected from proteolytic digestion by the protease inhibitor, al-antitrypsin. This protective mechanism appears to be non-operative in individuals with an al-antitrypsin deficiency due to genetic or other causes. Synthetic elastase inhibitors capable of replacing al-antitrypsin are therefore useful in the treatment of pulmonary emphysema and related diseases.

According to the present invention, a class of compounds containing the carbamate functionality and oligopeptides have been found to be superior active-site directed inhibitors of the enzyme elastase in animals and humans. This class of compounds, therefore, provides an opportunity to incorporate chemical moieties of increased affinity towards the enzyme, and greater capability for the transfer of the acylating moiety to the active site of the enzyme. The nature of the acylating moiety may be varied to optimize the duration of the enzymatic inactivation.

The mechanism of the invention appears to take advantage of the fact that these carbamate esters will react with proteases and esterases at the carbonyl carbon by losing the alkoxy portion and transferring the carbamylating moiety to the active site of the enzyme. Acylation will then lead to recovery of enzymatic activity.

The present invention provides a series of carbamate compounds which are active in accordance with the above proposals as elastase enzyme inhibitors. These novel compounds are carbamates substituted by oligopeptides which are selected from the group consisting of

a compound of the formula

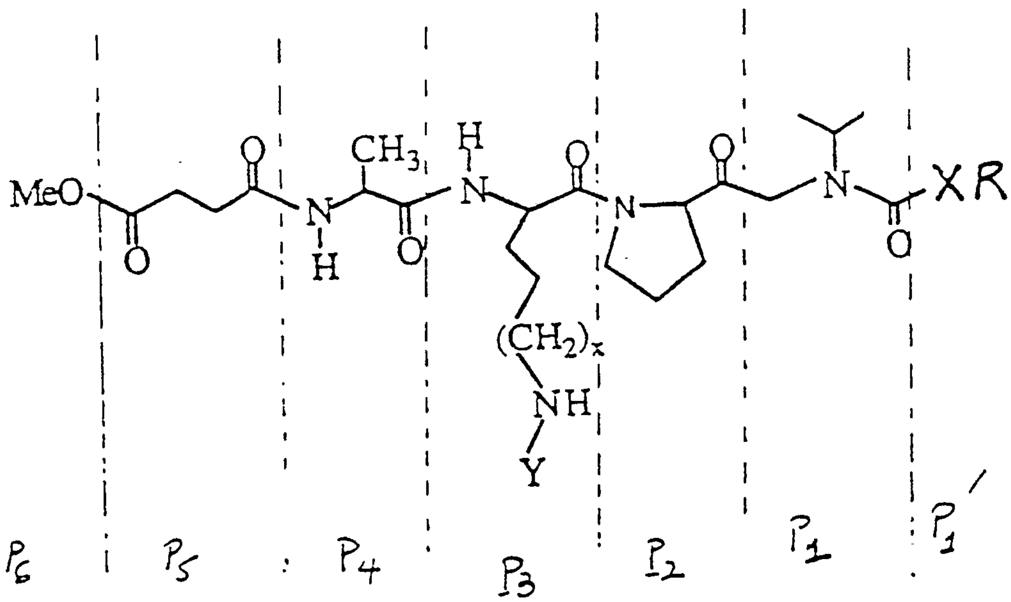

(I)

and

a compound of the formula

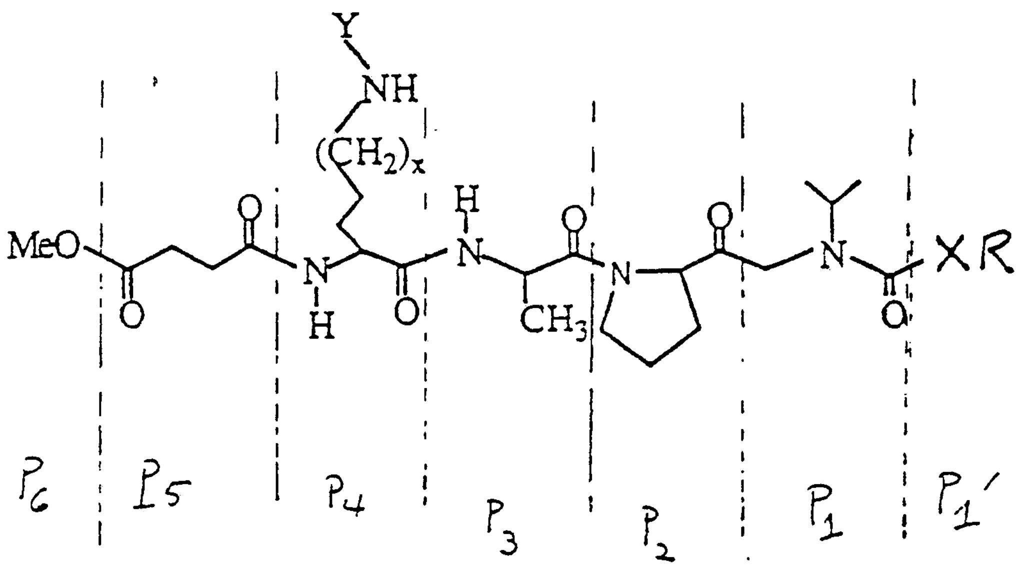

(II)

wherein

x is 1 or 2;

Y is carbobenzoxy or benzoyl; and

XR =

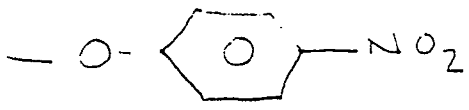

or

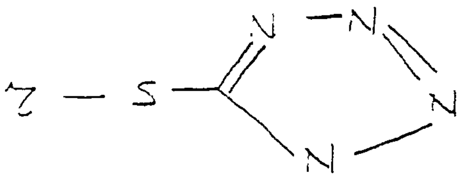

In a more preferred and detailed embodiment the compound of the invention is selected from the group consisting of

1. p-Nitrophenyl N-[(Methoxysuccinyl)-L-alanyl-L-alanyl-L-prolylmethyl]-N-isopropylcarbamate,
2. Methyl succinimide succinate,
3. t-Butyl-Methoxysuccinyl-L-alanine ester,
4. Methoxysuccinyl-L-alanine,
5. $N_\alpha$-Methoxysuccinyl-L-alanyl-N-benzoyl-L-lysine,
6. $N_\alpha$-Methoxysuccinyl-L-alanyl-$N_\epsilon$-carbobenzoxy-L-lysine phenacyl ester,
7. $N_\alpha$-Methoxysuccinyl-L-alanyl-$N_\epsilon$-carbobenzoxy-L-lysine,
8. $N_\alpha$-Methoxysuccinyl-L-alanyl-$N_\delta$-carbobenzoxy-L-ornithine phenacyl ester
9. $N_\alpha$-Methoxysuccinyl-L-alanyl-$N_\delta$-carbobenzoxy-L-ornithine,
10. $N_\alpha$-Methoxysuccinyl-$N_\delta$-carbobenzoxy-L-ornithine,
11. $N_\alpha$-Methoxysuccinyl-$N_\delta$-carbobenzoxy-L-ornithyl-L-alanine t-butyl ester,
12. $N_\alpha$-Methoxysuccinyl-$N_\delta$-carbobenzoyl-L-ornithyl-L-alanine,
13. $N_\alpha$-Methoxysuccinyl-$N_\epsilon$-carbobenzoxy-L-lysine,
14. $N_\alpha$-Methoxysuccinyl-$N_\epsilon$-carbobenzoxy-L-lysyl-L-alanine t-butyl ester
15. $N_\alpha$-Methoxysuccinyl-$N_\epsilon$ carbobenzoxy-L-lysyl-alanine,
16. $N_\alpha$-Methoxysuccinyl-$N_\epsilon$-benzoyl-L-lysine,
17. $N_\alpha$-Methoxysuccinyl-$N_\epsilon$-benzoyl-L-lysyl-L-alanine t-butyl ester,
18. $N_\alpha$-Methoxysuccinyl-$N_\epsilon$-benzoyl-L-lysyl-L-alanine,
19. N-Boc-L-prolyl chloromethyl ketone,
20. N-[(N-Boc-L-prolyl)methyl]isopropylamine,
21. N-[(N-Boc-L-prolyl)methyl]-N-isopropylcarbamate,
22. p-Nitrophenyl N-(L-prolylmethyl)-N-isopropyl-carbamate hydrochloride,
23. $N_\alpha$-Methoxysuccinyl-L-alanyl-$N_\epsilon$-carbobenzoxy-L-lysyl-D-proline phenacyl ester,
24. $N_\alpha$-Methoxysuccinyl-L-alanyl-$N_\epsilon$-carbobenzoxy-L-lysyl-L-proline phenacyl ester,
25. $N_\alpha$-Methoxysuccinyl-L-alanyl-$N_\epsilon$-carbobenzoxy-L-lysyl-D-proline,
26. $N_\alpha$-Methoxysuccinyl-L-alanyl-$N_\epsilon$-carbobenzoxy-L-lysyl-L-proline,
27. $N_\alpha$-Methoxysuccinyl-L-alanyl-$N_\epsilon$-carbobenzoxy-L-lysyl-D-prolyl chloromethyl ketone,
28. $N_\alpha$-Methoxysuccinyl-L-alanyl-$N_\epsilon$-carbobenzoxy-L-lysyl-L-prolyl chloromethyl ketone,
29. N-[Methoxysuccinyl-L-alanyl-($N_\epsilon$-carbobenzoxy)-L-lysyl-D prolylmethyl]-N-isopropylamine,
30. N-[Methoxysuccinyl-L-alanyl-($N_\epsilon$-carbobenzoxy-L-lysyl-L-prolylmethyl]-N-isopropylamine,
31. $N_\alpha$-t-Boc-$N_\epsilon$-carbobenzoxy-L-lysine,
32. $N_\alpha$-t-Boc-$N_\epsilon$-carbobenzoxy-L-lysine phenacyl ester,
33. $N_\epsilon$-Carbobenzoxy-L-lysine phenacyl ester hydrochloride,
34. $N_\alpha$-t-Boc-$N_\delta$-carbobenzoyl-L-ornithine,
35. $N_\alpha$-t-Boc-$N_\delta$-carbobenzoxy-L-ornithine phenacyl ester,
36. $N_\delta$-Carbobenzoxy-L-ornithine phenacyl ester hydrochloride,
37. N-t-Boc-D-proline,
38. N-t-Boc-L-proline,
39. N-t-Boc-D-proline phenacyl ester,
40. N-t-Boc-L-proline phenacyl ester,
41. D-Proline phenacyl ester hydrochloride,
42. L-Proline phenacyl ester hydrochloride,
43. $N_\epsilon$-Benzoyl-L-lysine,

44a. p-Nitrophenyl-N-[Methoxysuccinyl-($N_\epsilon$-carbobenzoxy)-L-lysyl-L-alanyl-L-prolylmethyl]-N-isopropylcarbamate,

44b. p-Nitrophenyl N-[Methoxysuccinyl-($N_\epsilon$-carbobenzoxy)-L-lysyl-L-alanyl-D-prolylmethyl]-N-isopropylcarbamate,

45a. p-Nitrophenyl N-[Methoxysuccinyl-($N_\epsilon$-benzoyl)-L-lysyl-L-alanyl-L-prolylmethyl]-N-isopropylcarbamate,

45b. p-Nitrophenyl N-[Methoxysuccinyl-($N_\epsilon$-benzoyl)-L-lysyl-L-alanyl-D-prolylmethyl]-N-isopropylcarbamate,

46a. p-Nitrophenyl-N-[Methoxysuccinyl-($N_\delta$-carbobenzoxy)-L-ornithyl-L-alanyl-L-prolylmethyl]-N-isopropylcarbamate,

46b. p-Nitrophenyl-($N_\delta$-carbobenzoxy)-L-ornithyl-L-alanyl-D-prolylmethyl]-N-isopropylcarbamate,

47a. p-Nitrophenyl N-[Methoxysuccinyl-($N_\delta$-benzoyl)-L-ornithyl-L-alanyl-L-prolylmethyl]-N-isopropylcarbamate,

47b. p-Nitrophenyl N-[Methoxysuccinyl-($N_\delta$-benzoyl)-L-ornithyl-L-alanyl-D-prolylmethyl]-N-isopropylcarbamate,

48a. p-Nitrophenyl N-[Methoxysuccinyl-L-alanyl-($N_\epsilon$-carbobenzoxy)-L-lysyl-L-prolylmethyl]-N-isopropylcarbamate,

48b. p-Nitrophenyl N-[Methoxysuccinyl-L-alanyl-($N_\epsilon$-carbobenzoxy)-L lysyl-D-prolylmethyl]-N-isopropylcarbamate,

49a. p-Nitrophenyl N-[Methoxysuccinyl-L-alanyl-($N_\epsilon$-benzoyl)-L-lysyl-L-prolylmethyl]-N-isopropylcarbamate,

49b. p-Nitrophenyl N-[Methoxysuccinyl-L-alanyl-($N_\epsilon$-benzoyl)-L-lysyl-D-prolylmethyl]-N-isopropylcarbamate,

50a. p-Nitrophenyl N-[Methoxysuccinyl-L-alanyl-$N_\delta$-carbobenzoxy)-L-ornithyl-L-prolylmethyl]-N-isopropylcarbamate,

50b. p-Nitrophenyl N-[Methoxysuccinyl-L-alanyl-($N_\delta$-carbobenzoxy)-L-ornithyl-D-prolylmethyl]-N-isopropylcarbamate ,

51a. p-Nitrophenyl N-[Methoxysuccinyl-L-alanyl-($N_\delta$-benzoyl)-L-ornithyl-L-prolylmethyl]-N-isopropylcarbamate,

51b. p-Nitrophenyl N-[Methoxysuccinyl-L-alanyl-($N_\delta$-benzoyl)-L-ornithyl-D-prolylmethyl]-N-isopropylcarbamate,

52. S-(1-phenyl-5-tetrazol) chloroformate,

53. S-(1-phenyl-5-tetrazoyl)-N-[(N-Boc-L-prolyl)methyl]-N-isopropyl-thiocarbamate,

54. S-(1-phenyl-5-tetrazoyl-N-prolymethyl)-N-isopropyl-thio carbamate hydrochloride,

PC5. S-(1-phenyl-5-tetrazoyl)-N-[methoxysuccinyl-alanyl-($N_\epsilon$-Carbobenzoxyl) lysyl prolyl methyl]-N-isopropyl-thio carbamate, and

PC6. S-(1-phenyl-5-tetrazoyl)-N-[methoxysuccinyl-(N -carbobenzoyl)ornithylalanyl(prolylmethyl)-N-isopropylthio carbamate .

In another particularly preferred embodiment of the invention the elastase enzyme inhibitors of this invention are selected from the group consisting of

1. p-Nitrophenyl N-[(Methoxysuccinyl)-L-alanyl-L-alanyl-L-prolylmethyl]-N-isopropylcarbamate,
2. Methyl succinimide succinate,
3. t-Butyl-Methoxysuccinyl-L-analine ester,
4. Methoxysuccinyl-L-alanine,
5. $N_\alpha$-Methoxysuccinyl-L-alanyl-N-benzoyl-L-lysine,
6. $N_\alpha$-Methoxysuccinyl-L-alanyl-$N_\epsilon$-carbobenzoxy-L-lysine phenacyl ester,
7. $N_\alpha$-Methoxysuccinyl-L-alanyl-$N_\epsilon$-carbobenzoxy-L-lysine,
8. $N_\alpha$-Methoxysuccinyl-L-alanyl-$N_\delta$-carbobenzoxy-L-ornithine phenacyl ester
9. $N_\alpha$-Methoxysuccinyl-L-alanyl-$N_\delta$-carbobenzoxy-L-ornithine,
10. $N_\alpha$-Methoxysuccinyl-$N_\delta$-carbobenzoxy-L-ornithine,
11. $N_\alpha$-Methoxysuccinyl-$N_\delta$-carbobenzoxy-L-ornithyl-L-alanine t-butyl ester,
12. $N_\alpha$-Methoxysuccinyl-$N_\delta$-carbobenzoyl-L-ornithyl-L-alanine,
13. $N_\alpha$-Methoxysuccinyl-$N_\epsilon$-carbobenzoxy-L-lysine,
14. $N_\alpha$-Methoxysuccinyl-$N_\epsilon$-carbobenzoxy-L-lysyl-L-alanine t-butyl ester
15. $N_\alpha$-Methoxysuccinyl-$N_\epsilon$ carbobenzoxy-L-lysyl-alanine,
16. $N_\alpha$-Methoxysuccinyl-$N_\epsilon$-benzoyl-L-lysine,
17. $N_\alpha$-Methoxysuccinyl-$N_\epsilon$-benzoyl-L-lysyl-L-alanine t-butyl ester,
18. $N_\alpha$-Methoxysuccinyl-$N_\epsilon$-benzoyl-L-lysyl-L-alanine,
19. N-Boc-L-prolyl chloromethyl ketone,
20. N-[(N-Boc-L-prolyl)methyl]isopropylamine,
21. N-[(N-Boc-L-prolyl)methyl]-N-isopropylcarbamate,
22. p-Nitrophenyl N-(L-prolylmethyl)-N-isopropyl-carbamate hydrochloride,
23. $N_\alpha$-Methoxysuccinyl-L-alanyl-$N_\epsilon$-carbobenzoxy-L-lysyl-D-proline phenacyl ester,

24. $N_\alpha$-Methoxysuccinyl-L-alanyl-$N_\epsilon$-carbobenzoxy-L-lysyl-L-proline phenacyl ester.
25. $N_\alpha$-Methoxysuccinyl-L-alanyl-$N_\epsilon$-carbobenzoxy-L-lysyl-D-proline.
26. $N_\alpha$-Methoxysuccinyl-L-alanyl-$N_\epsilon$-carbobenzoxy-L-lysyl-L-proline.
27. $N_\alpha$-Methoxysuccinyl-L-alanyl-$N_\epsilon$-carbobenzoxy-L-lysyl-D-prolyl chloromethyl ketone.

In yet another preferred embodiment the enzyme elastase inhibitors of this invention are selected from the group consisting of

28. $N_\alpha$-Methoxysuccinyl-L-alanyl-$N_\epsilon$-carbobenzoxy-L-lysyl-L-prolyl chloromethyl ketone,
29. N-[Methoxysuccinyl-L-alanyl-($N_\epsilon$-carbobenzoxy-L-lysyl-D prolylmethyl]-N-isopropylamine,
30. N-[Methoxysuccinyl-L-alanyl-($N_\epsilon$-carbobenzoxy)-L-lysyl-L-prolylmethyl]-N-isopropylamine,
31. $N_\alpha$-t-Boc-$N_\epsilon$-carbobenzoxy-L-lysine,
32. $N_\alpha$-t-Boc-$N_\epsilon$-carbobenzoxy-L-lysine phenacyl ester,
33. $N_\epsilon$-Carbobenzoxy-L-lysine phenacyl ester hydrochloride,
34. $N_\alpha$-t-Boc-$N_\delta$-carbobenzoyl-L-ornithine,
35. $N_\alpha$-t-Boc-$N_\delta$-carbobenzoyl-L-ornithine phenacyl ester,
36. $N_\delta$-Carbobenzoxy-L-ornithine phenacyl ester hydrochloride,
37. N-t-Boc-D-proline,
38. N-t-Boc-L-proline,
39. N-t-Boc-D-proline phenacyl ester,
40. N-t-Boc-L-proline phenacyl ester,
41. D-Proline phenacyl ester hydrochloride,
42. L-Proline phenacyl ester hydrochloride,
43. $N_\epsilon$-Benzoyl-L-lysine,,

44a. p-Nitrophenyl-N-[Methoxysuccinyl-($N_\epsilon$-carbobenzoxy)-L-lysyl-L-alanyl-L-prolylmethyl]-N-isopropylcarbamate,

44b. p-Nitrophenyl N-[Methoxysuccinyl-($N_\epsilon$-carbobenzoxy)-L-lysyl-L-alanyl-D-prolylmethyl]-N-isopropylcarbamate,

45a. p-Nitrophenyl N-[Methoxysuccinyl-($N_\epsilon$-benzoyl)-L-lysyl-L-alanyl-L-prolylmethyl]-N-isopropylcarbamate,

45b. p-Nitrophenyl N-[Methoxysuccinyl-($N_\epsilon$-benzoyl)-L-lysyl-L-alanyl-D-prolylmethyl]-N-isopropylcarbamate,

46a. p-Nitrophenyl-N-[Methoxysuccinyl-($N_\delta$-carbobenzoxy)-L-ornithyl-L-alanyl-L-prolylmethyl]-N-isopropylcarbamate,

46b. p-Nitrophenyl-($N_\delta$-carbobenzoxy)-L-ornithyl-L-alanyl-D-prolylmethyl]-N-isopropylcarbamate,

47a. p-Nitrophenyl N-[Methoxysuccinyl-($N_\delta$-benzoyl)-L-ornithyl-L-alanyl-L-prolylmethyl]-N-isopropylcarbamate,

47b. p-Nitrophenyl N-[Methoxysuccinyl-($N_\delta$-benzoyl)-L-ornithyl-L-alanyl-D-prolylmethyl]-N-isoproppylcarbamate, and

48a. p-Nitrophenyl N-[Methoxysuccinyl-L-alanyl-($N_\epsilon$-carbobenzoxy)-L-lysyl-L-prolylmethyl]-N-isopropylcarbamate,

In still another preferred embodiment of the invention the elastase enzyme inhibitor is selected from the group consisting of

48b. p-Nitrophenyl N-[Methoxysuccinyl-L-alanyl-($N_\epsilon$-carbobenzoxy)-L lysyl-D-prolylmethyl]-N-isopropylcarbamate,

49a. p-Nitrophenyl N-[Methoxysuccinyl-L-alanyl-($N_\epsilon$-benzoyl)-L-lysyl-L-prolylmethyl]-N-isopropylcarbamate,

49b. p-Nitrophenyl N-[Methoxysuccinyl-L-alanyl-($N_\epsilon$-benzoyl)-L-lysyl-D-prolylmethyl]-N-isopropylcarbamate,

50a. p-Nitrophenyl N-[Methoxysuccinyl-L-alanyl-$N_\delta$-carbobenzoxy)-L-ornithyl-L-prolylmethyl]-N-isopropylcarbamate,

50b. p-Nitrophenyl N-[Methoxysuccinyl-L-alanyl-($N_\delta$-carbobenzoxy)-L-ornithyl-D-prolylmethyl]-N-isopropylcarbamate ,

51a. p-Nitrophenyl N-[Methoxysuccinyl-L-alanyl-($N_\delta$-benzoyl)-L-ornithyl-L-prolylmethyl]-N-isopropylcarbamate,

51b. p-Nitrophenyl N-[Methoxysuccinyl-L-alanyl-($N_\delta$-benzoyl)-L-ornithyl-D-prolylmethyl]-N-isopropylcarbamate,

52. S-(1-phenyl-5-tetrazol) chloroformate,
53. S-(1-phenyl-5-tetrazoyl)-N-[(N-Boc-L-prolyl)methyl]-N-isopropyl-thiocarbamate,
54. S-(1-phenyl-5-tetrazoyl-N-prolymethyl)-N-isopropyl-thio carbamate hydrochloride,

PC5. S-(1-phenyl-5-tetrazoyl)-N-[methoxysuccinyl-alanyl-($N_\epsilon$-Carbobenzoxyl) lysyl prolyl methyl]-N-isopropyl-thio carbamate, and

PC6. S-(1-phenyl-5-tetrazoyl)-N-[methoxysuccinyl-(N -carbobenzoyl)ornithylalanyl(prolylmethyl)-N-isopropylthio carbamate .

Of the above compounds still more preferred are those having the formula

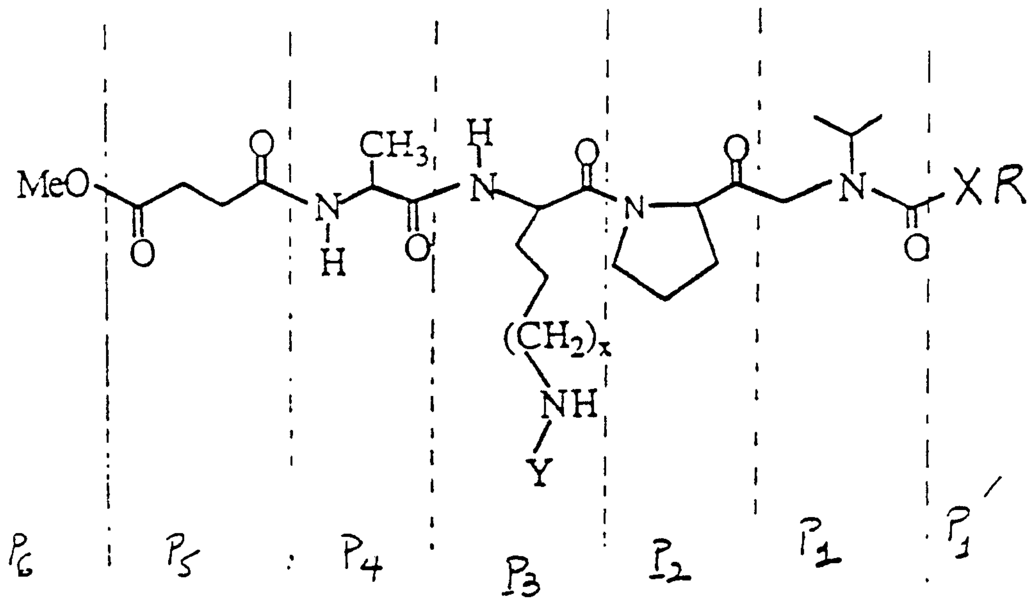

(I)

wherein

x is 1 or 2;

Y is carbobenzoxy or benzoyl; and

XR is

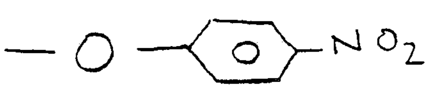

or

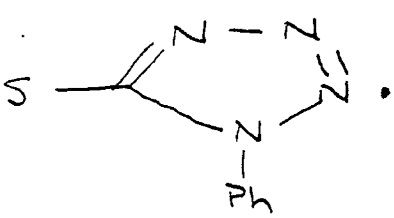

.

Also a more preferred group of compounds of the invention are those having the formula

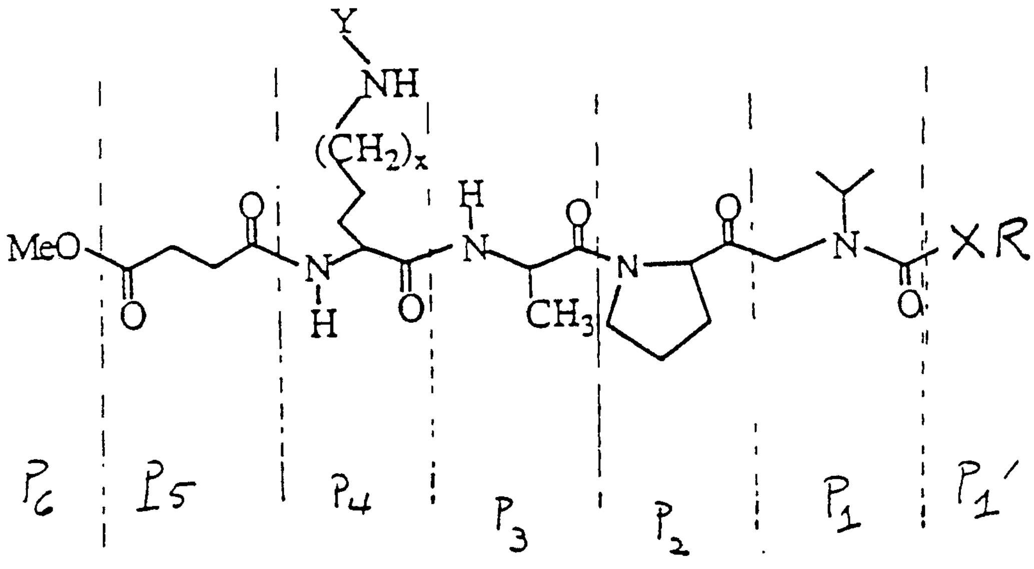

(II)

wherein x is 1 or 2;
X is carbobenzoxy or benzoyl; and

XR is

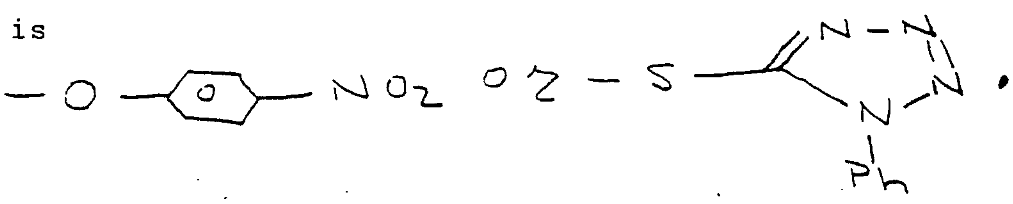

Two synthetic routes are provided herein to produce the p-nitrophenyl peptidyl carbamates of the invention. One of the synthetic routes is designed to incorporate D or L proline into the $P_2$ position of the molecule in a stereospecific manner. Derivatives incorporating D-proline, however, possess reduced elastase enzyme inhibitory properties when compared to their diastereomers which incorporate L-proline at the P2 position.

The peptidyl carbamate inhibitors of the invention selectively inhibit the enzyme elastase, e.g., human leukocyte elastase (HLE) and porcine pancreatic elastase (PPE), with inhibitor dissociation constants ranging from $3x10^{-6}$M to $2x10^{-9}$M.

All the peptidyl carbamate inhibitors of the invention have been found to selectively inhibit the enzyme elastase without inhibiting other enzymes such as trypsin or chymotrypsin, amont other enzymes.

The peptidyl carbamate inhibitors of the invention are desmosine-like derivatives incorporating L-lysine or L-ornithine residues at the $P_3$ or $P_4$ regions of their structures. These features simulate the protruding chains of desmosine cross-linking units in mature elastin.

The present compounds are synthesized in general by methods which are improvements over the method described in U.,S. patent 4,643,991 to Digenis et al, the entire content of which is incorporated herein by reference. The synthetic approach of this invention involves the coupling of the $P_6$-$P_3$ moieties of the inhibitor molecules with the $P_2$-$P_1$ moieties as depicted in Scheme 1 herebelow.

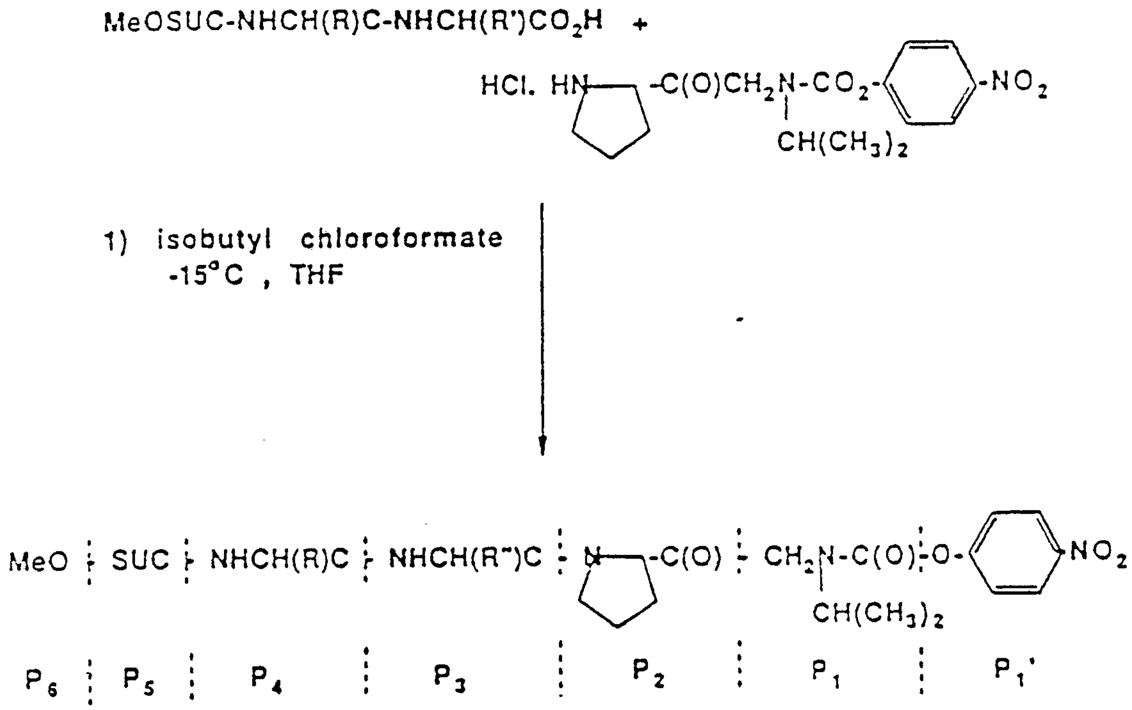


Scheme 1

General synthesis of desmosine-like peptidyl carbamates. Coupling of $P_6$ - $P_3$ moiety with the $P_2$ - $P_1$' moiety

This coupling reaction is conducted in a solvent, preferably a polar solvent at low temperature. The conditions for this process are standard in the art and will be known to an artisan.

The synthesis of methoxysuccinyl-alanine aminoacid dipeptides of the invention is depicted in Scheme 2 herebelow

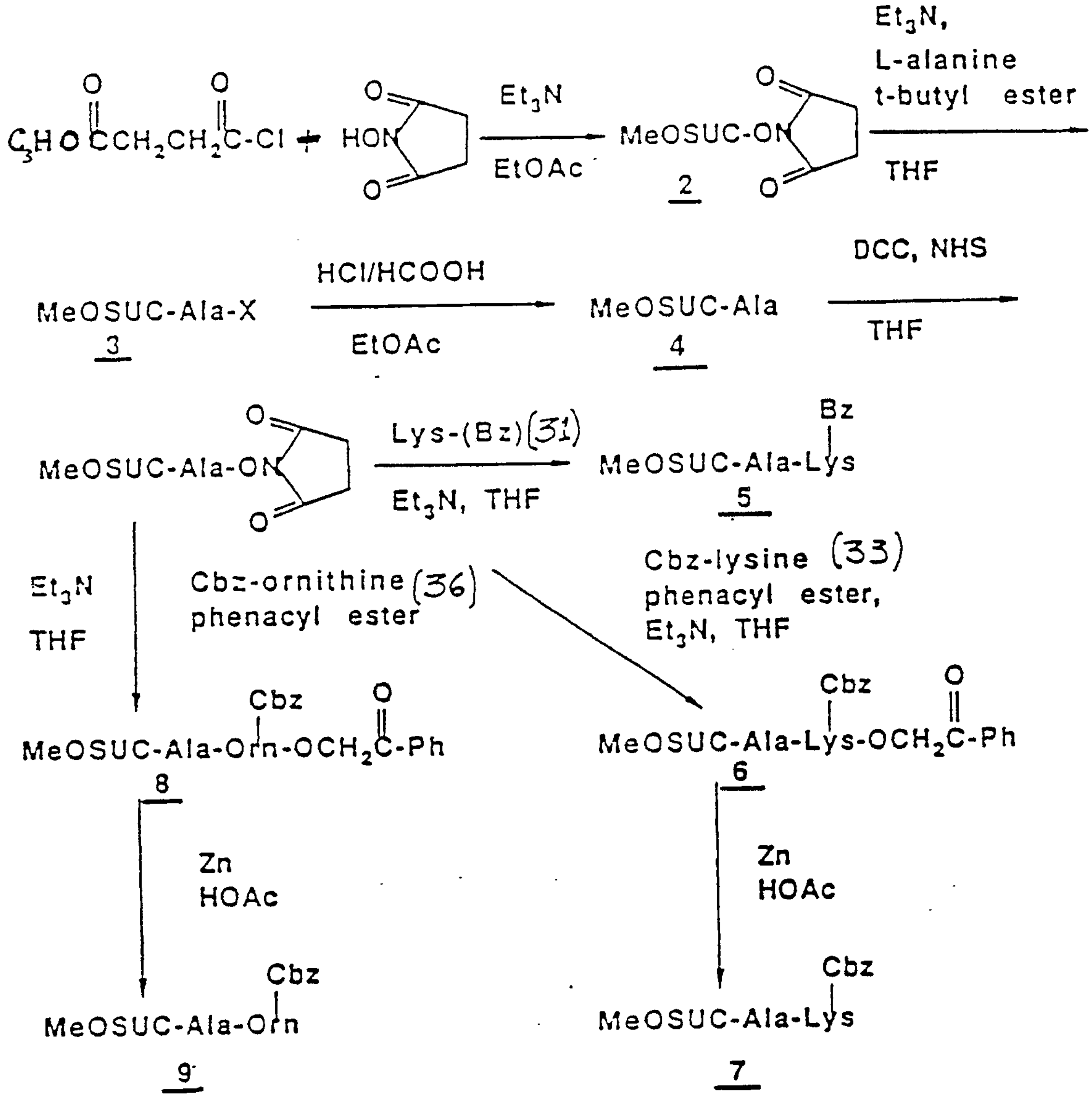


Scheme 2

Synthesis of the MeOSUC-Ala-Amino acid dipeptides.

The synthesis of methoxysuccinyl-alanine aminoacid dipeptides in which the aminoacid residue can be ornithine (residue 9) or lysine (residue 7) with carbobenzoxy (Cbz) or benzoyl (Bz) groups at their terminal amine function (N__) is shown in Scheme 2. These reactions are conducted under standard conditions which are known to an artisan.

The synthesis of methoxysuccinyl-aminoacid-alanine dipeptides is depicted in Scheme 3 herebelow.

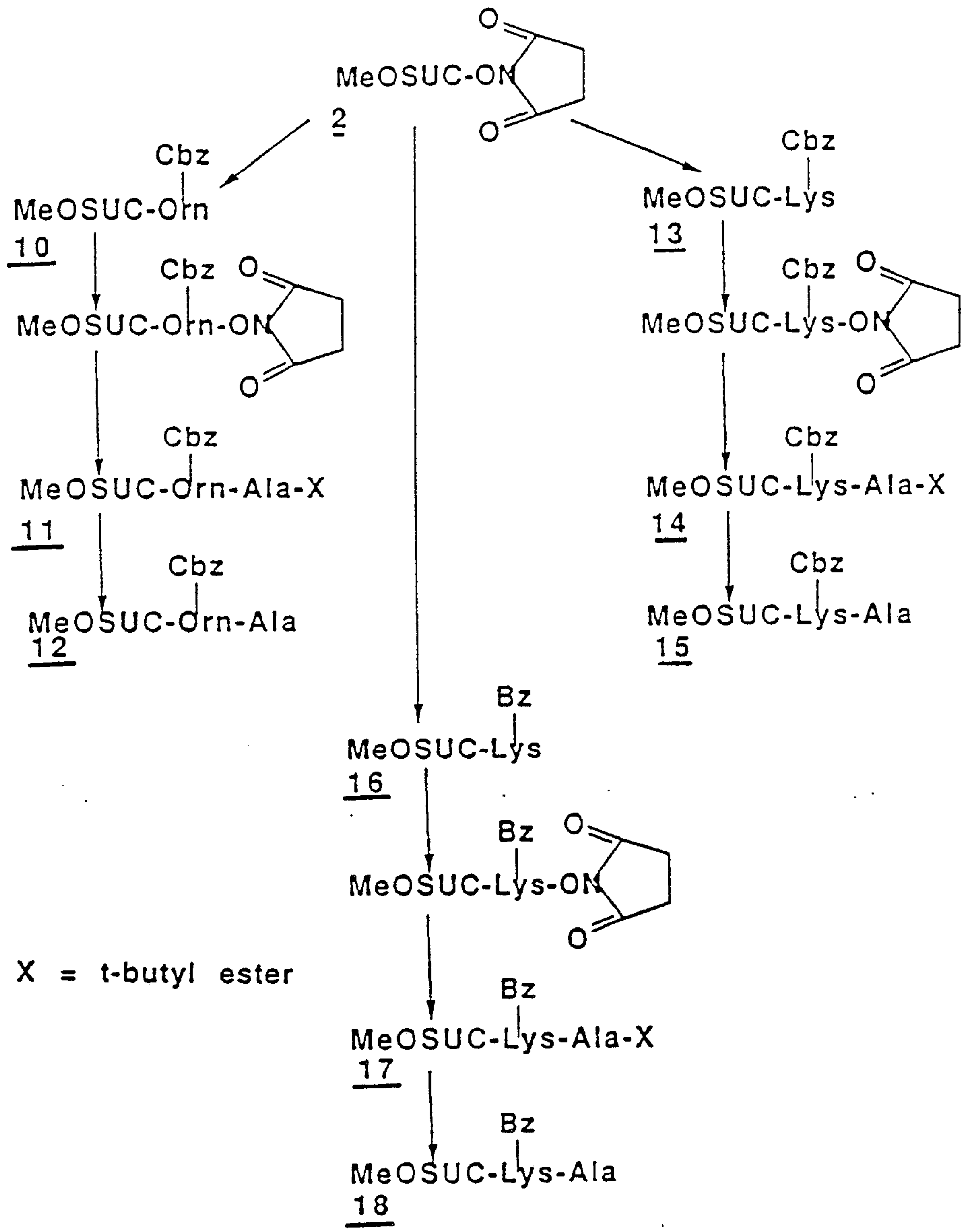

2, 10, 11, 12, 13, 14, 15, 16, 17, 18

X = t-butyl ester

Scheme 3

Synthesis of the MeOSUC-Amino acid-Ala dipeptides

As previously indicated the aminoacid residue could be ornithine or lysine with carbobenzoxy or benzoyl at their terminal amino functions ($N_\epsilon$). Examples of these compounds are represented by the structures of Compounds 12, 15 and 18 which are products obtained in accordance with the procedure described in Scheme 3.

The coupling of other inhibitor compounds similar to compounds 7 and 9 shown in Scheme 2 with

intermediate compound 22 appearing in Scheme 4 results in the synthesis of the compounds 48-51 shown in Table 2 below.

Table 2

Inhibition of HLE by Desmosine-like Peptidyl Carbamates; Variations in $P_3$

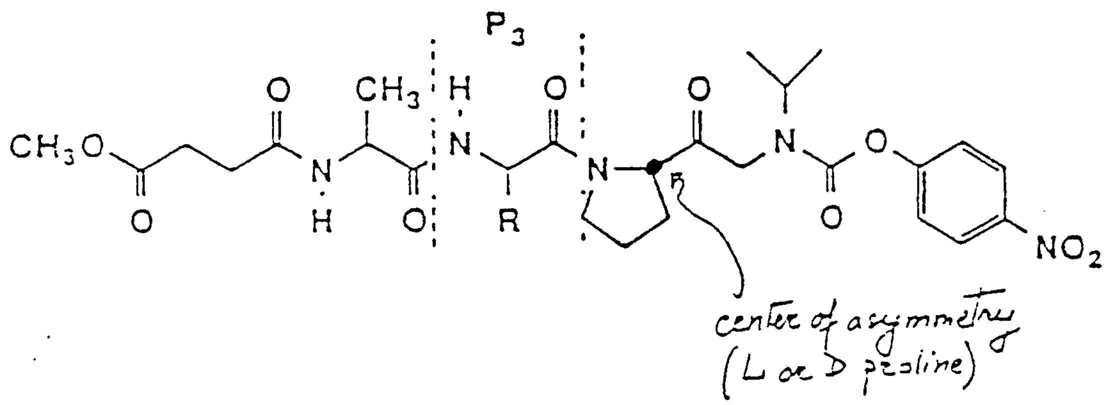


| Cpd. | $P_3$[a] | Isomer[b] | | $K_i$ values (μM) Dixon[c] | Replot[d] |
|---|---|---|---|---|---|
| 48 | $N_\epsilon$-Cbz-Lys | a) | L | 1.10 | 0.22 |
| | | b) | D | 20.40 | 11.33 |
| 49 | $N_\epsilon$-Bz-Lys | a) | L | 1.60 | 0.31 |
| | | b) | D | 34.00 | 38.50 |
| 50 | $N_\delta$-Cbz-Orn | a) | L | 0.75 | 1.05 |
| | | b) | D | 22.20 | 19.25 |
| 51 | $N_\delta$-Bz-Orn | a) | L | 5.33 | 2.14 |
| | | b) | D | 104.00 | 100.00 |

[a]Cbz = carbobenzoxy; Bz = benzoyl; Lys = L-lysine; Orn = L-ornithine.
[b]L & D refer to the configuration at the prolyl α-carbon.
[c]Lines contained linear regression analysis correlations of > 0.950.
[d]$K_i$ values determined from Lineweaver-Burk slope replots. Slopes were obtained from lines with linear regression analysis correlations of > 0.990.

Table 2 provides Ki values for 2 diastereomers of each compound (a and b). In all cases the diastereomers incorporate L proline, where L or D refers to the configuration at the propyl alpha-carbon, exhibit greater inhibitory activity against the enzyme elastase than their corresponding diastereomers derived from D-proline.

The coupling of the intermediate compounds such as compounds 12, 15 and 18 shown in Scheme 3 with compound 22 shown in Scheme 4 herebelow results in the synthesis of compounds 44-47 shown in Table 1 herebelow.

Table 1

Inhibition of HLE by Desmosine-like Peptidyl Carbamates; Variations in $P_4$

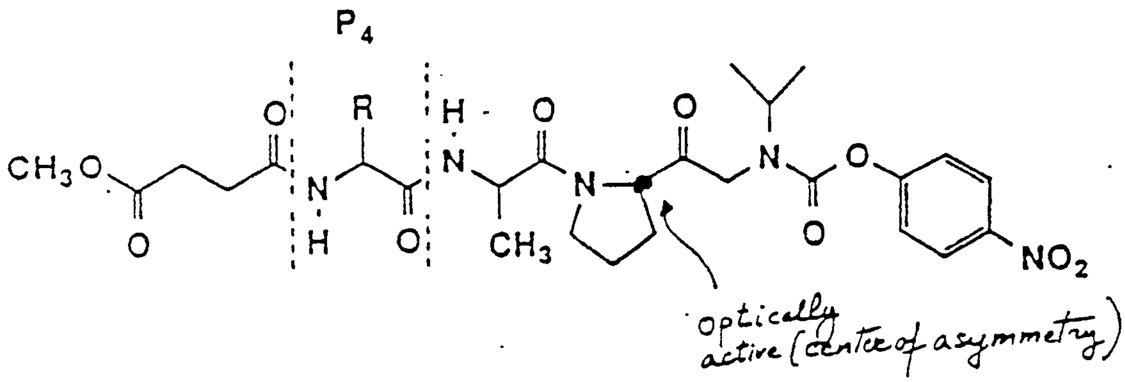


| Cpd. | $P_4$[a] | Isomer | $K_i$ value (μM) Dixon[c] | Replot[d] |
|---|---|---|---|---|
| 44 | $N_\epsilon$-Cbz-Lys | a) L | 0.56 | 0.47 |
| | | b) D | 7.30 | 7.63 |
| 45 | $N_\epsilon$-Bz-Lys | a) L | 0.73 | 0.38 |
| | | b) D | 3.70 | 3.13 |
| 46 | $N_\delta$-Cbz-Orn | a) L | 0.43 | 0.36 |
| | | b) D | 8.80 | 7.95 |
| 47 | $N_\delta$-Bz-Orn | a) L | 0.65 | 0.19 |
| | | b) D | 10.00 | 17.70 |

[a]Cbz = carbobenzoxy; Bz = benzoyl; Lys = L-lysine; Orn = L-ornithine.

[b]L & D refer to the configuration at the prolyl α-carbon.

[c]Lines contained linear regression analysis correlations of >0.950.

[d]$K_i$ values determined from Lineweaver-Burk slope replots. Slopes were obtained from lines with linear regression analysis correlations of >0.990.

Scheme 4 is shown herebelow and provides the synthesis of P3-P1 moieties as their stable hydrochloride salts (compound 22).

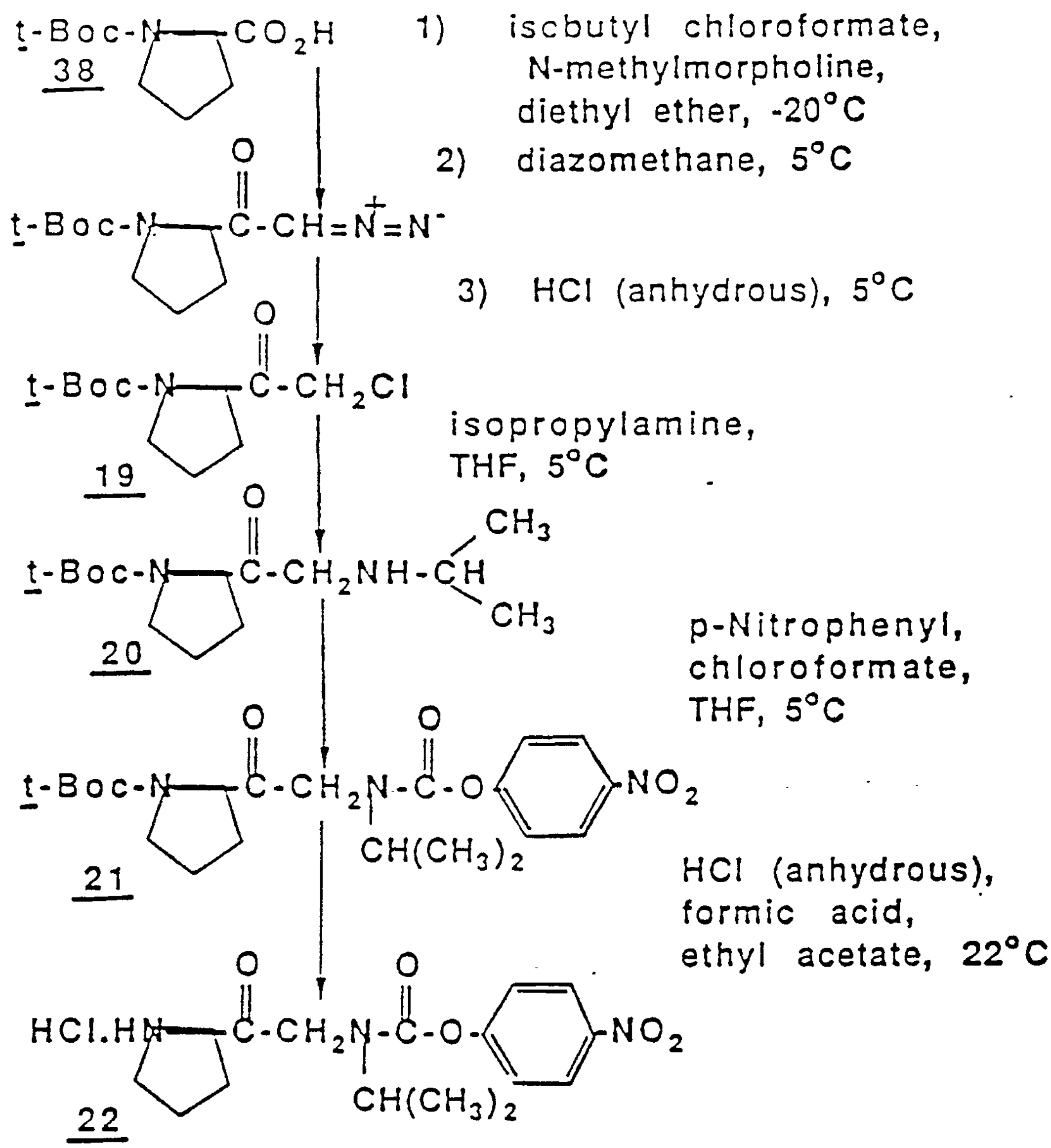


Scheme 4

Synthesis of $P_3$ - $P_1$' moiety as the stable hydrochloride salt (22).

These reactions are also conducted under conditions which are standard in the art and would be known to an artisan.

As already indicated above here also the diastereomers incorporating L proline designated as a) are

shown to have a greater inhibitory capacity against the enzyme elastase, e.g. HLE, than those compounds derived from D-proline as shown in Table 1 above.

In all cases compound 22 is synthesized from compound 38 by a synthetic route which is shown in Scheme 4 hereabove.

The stereo specific synthesis of compounds 44-51 follows a synthetic procedure which is depicted in Scheme 5 herebelow.

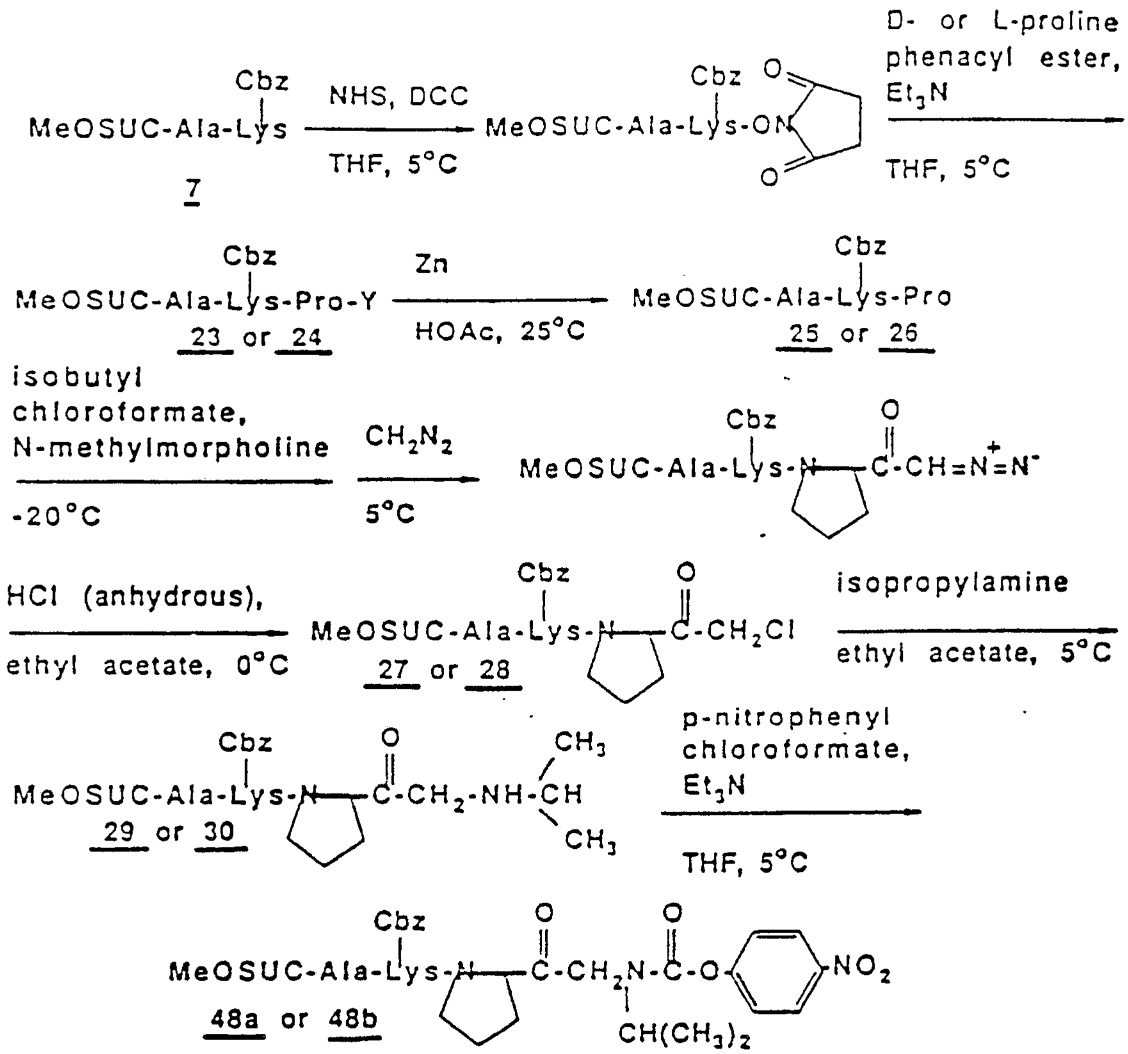


Y = phenacyl ester

Scheme 5

Stereospecific synthesis of desmosine-like peptidyl carbamates.

As in the previous cases the conditions for conducting the various steps in this method are standard in the art and would be known to an artisan.

The peptidyl carbamate inhibitor of Formula 46 may be synthesized from all L aminoacids (L-L isomer). When tested against human leukocyte elastase enzyme it is evidenced that its inhibitory activity may be enhanced by utilizing a 50:50 mixture of the two diastereomers which result from the incorporation of D or L proline into the structure of compound 46.

Table 3 below shows the effect of the stereo chemistry at $P_2$ on the elastase enzyme inhibitory activity of compound 46 of this invention.

Table 3

Effect of Stereochemistry at $P_2$ on the HLE Inhibitory Activity

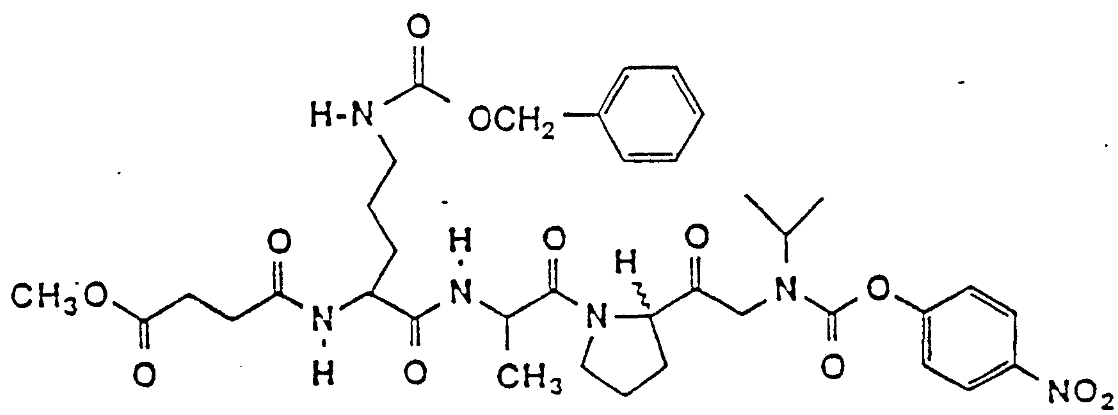

| Compound (46) | $K_i$ value[a] (μM) | $k_{obs}$[b,c] ($s^{-1}$).($M^{-1}s^{-1}$) | $k_{obs}/[I]$[c] |
|---|---|---|---|
| L-L-L | 0.65 | 0.376 | 8237 |
| L-L-D | 7.95 | 0.021 | 461 |
| L-L-DL[d] | 0.42 | 0.437 | 9587 |

[a] $K_i$ value was determined by steady state kinetics.

[b] $[I] = 7.6 \times 10^{-7}M$, $[E] = 7.6 \times 10^{-8}M$.

[c] First and second order rate constants were determined by pre-steady state inhibition kinetics.

[d] 50:50 mixture of two inhibitors, each contributing $3.8 \times 10^{-7}M$.

Extremely highly potent inhibitors of the enzyme elastase are obtained by replacing the p-nitrophenyl functionality at $P_1$ of Formulas I and II by N-phenylthiotetrazole. This results in compounds PC5 and PC6 which were shown to have Ki values of 2.0x10-8M to 3x10-9M respectively.

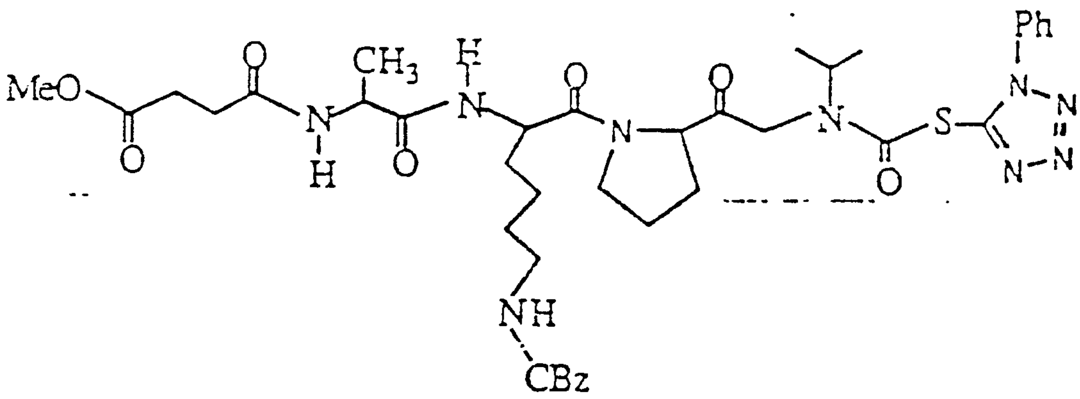

PC5

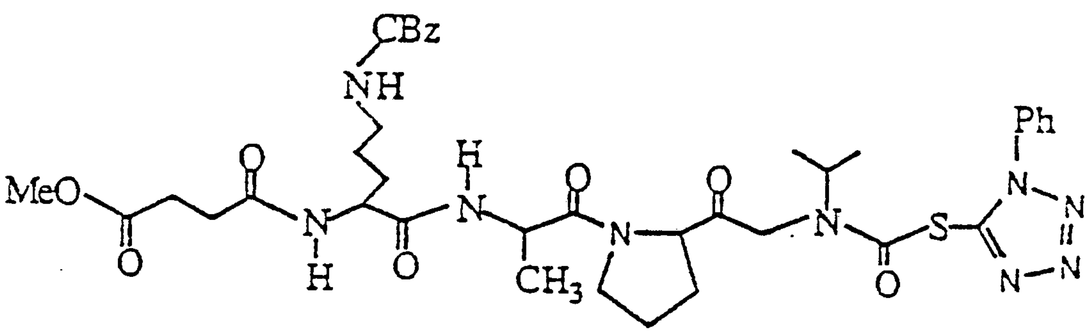

PC6

The coupling of the intermediate 7 with the compound 54 results in the elastase enzyme inhibitory compound PC5. The coupling of compound 12 with compound 54 results in the elastase enzyme inhibitory compound PC6. The synthesis of these compounds are depicted in Scheme 6 below.

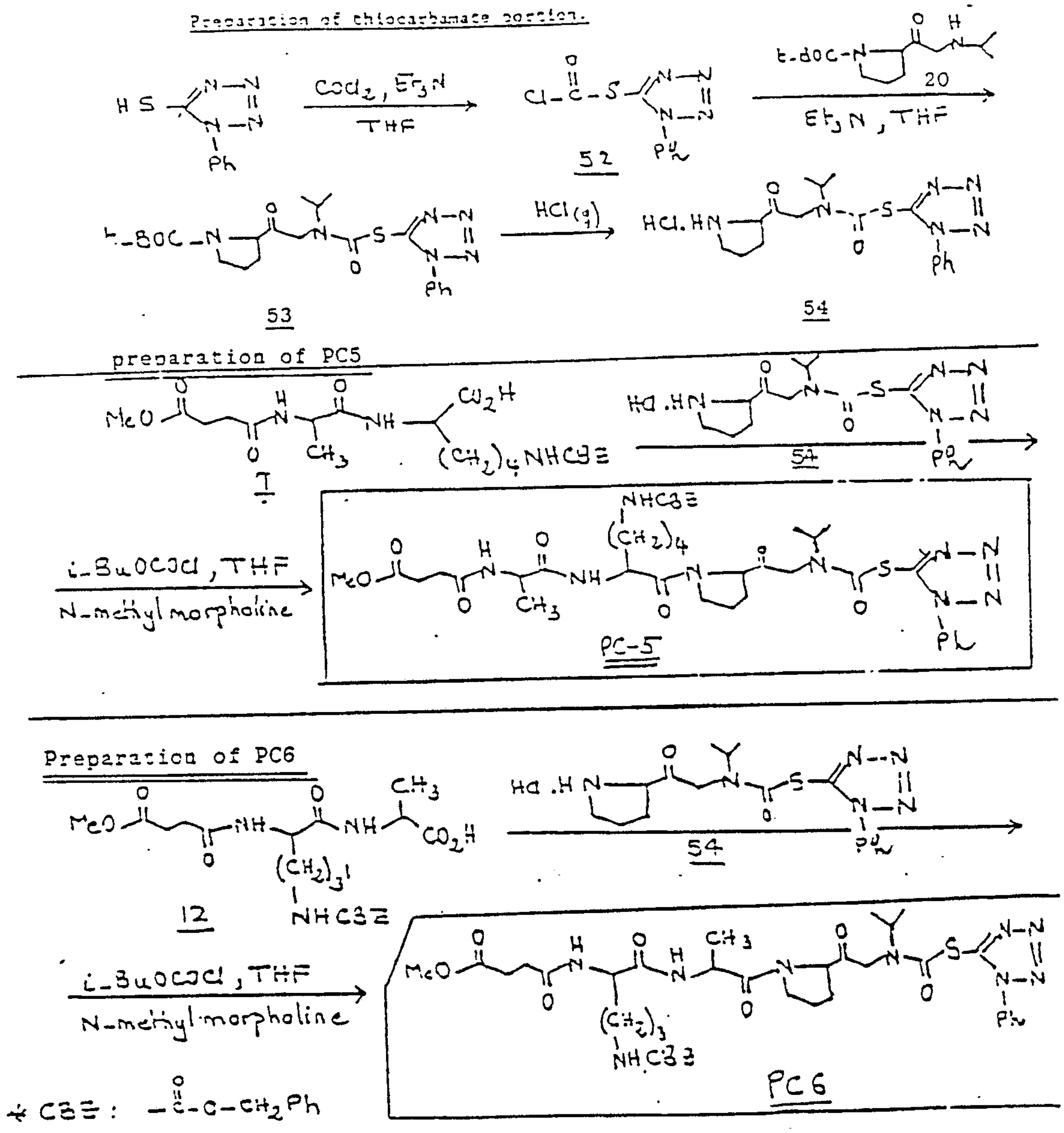


Scheme 6

Synthesis of PC5 and PC6

The conditions for conducting the various steps encompassed by these methods are known in the art and to an artisan in the field.

As pointed out above, the compounds of the invention may be employed as specific active site directed inhibitors sof the enzyme, elastase. For this purpose, the compounds are preferably combined with a pharmaceutically acceptable carrier for administration by injection or in the oral form. Conventional adjuvant and carriers may be employed in combination with about 0.001 to 2.0 weight percent of the active compound. The compounds may be administered to animals or humans at about 10 mg/kg, preferably an average amount of about 6 mg/kg.

The following examples illustrate preferred embodiments of the invention but the invention is not considered to be limited thereto. In the examples and throughout this specification, parts are by weight unless otherwise indicated.

In synthesis of the compounds of the invention, melting points were determined on a Thomas-Hoover Uni-Melt apparatus and are uncorrected. $^1$H NMR spectra were obtained using a Varian EM-360 (60 MH$_2$O

or EM-390 (90 MHz) spectrometer. Infrared (IR) spectra were recorded on a Perkin-Elmer 567 spectrophotometer. Microanalyses were performed by Atlantic Microlab, Inc., Atlanta, Ga. or by Micro Analysis, Inc., Wilmington, De.

Reactions were routinely followed by thin layer chromatography (TLC) using Whatman MK6F silica gel plates. Spots were detected by UV (254 nm), iodine or HBr-Ninhydrin spraying. Column chromatography was carried out using Silica Gel 60 from E. Merck, Darmstadt, Germany. All compounds were identified by spectral data and elemental analysis.

Having now generally described this invention, the same will be better understood by reference to certain specific examples, which are included herein for purposes of illustration only and are not intended to be limiting of the invention or any embodiment thereof, unless so specified.

## EXPERIMENTAL

### Ex. 1: Methyl succinimide succinate (2)

2

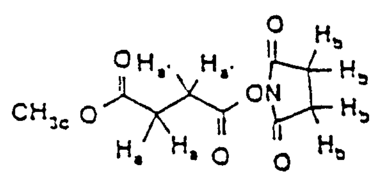

This compound was synthesized by a modified procedure of Digenis et al.[39]

A solution of 3-carbomethoxypropionyl chloride (5g, 33.2mmoles) and N-hydroxysuccinimide (3.8g, 33.2mmoles) in ethyl acetate (60 mL) was cooled to 5° C. While stirring, triethylamine (4.3g, 33.2mmoles) was slowly added to the cooled solution over a 15 min interval. The mixture was allowed to equilibrate to room temperature (22° C) and react for an additional 3h. The formed triethylamine salt was filtered, washed with ethyl acetate and the filtrate evaporated under vacuum. The powder was recrystallized from ethyl acetate/hexane to give 5.5g (24.2mmoles) (72% yield) of white needles mp 84-86° C (lit.[39] mp 84-86° C). $^1$H-NMR ($CDCl_3$)$\delta$ 2.44(2$H_a$,app.t, J=8Hz); 2.64(2$H_a$,app.t, J=8Hz); 2.86(4$H_b$,s); 3.66(3$H_c$,s)ppm. IR (Nujol) 1840, 1800, 1775, 1725cm$^{-1}$.

### Ex. 2 : t-Butyl Methoxysuccinylalanine ester (3)

3

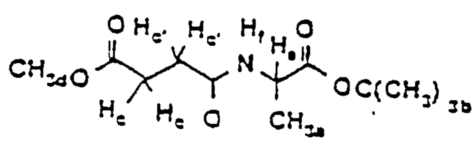

To an ice cooled suspension of the activated ester 2(3.6g, 16mmoles) and L-alanine t-butyl ester (2.3g, 16mmoles) in THF (50mL), triethylamine (2.0g, 16mmoles) in THF (1mL) was added dropwise. The progress of the reaction was monitored by TLC (10% methanol in chloroform) and stirred at 5° C for 3.5h. The precipitate was filtered under vacuum and washed with ethyl acetate. The residual oil, containing the product, was chromatographed using 40g of silica gel column (2 x 50cm). Impurities were eliminated by first passing 50mL of methylene chloride and subsequently compound 3 was eluted with 2% methanol in methylene chloride. Upon evaporation of the eluent solvent under reduced pressure the product was obtained. The latter was then crystallized from ethyl acetate/petroleum ether to give 2.9g (11.3mmoles) (71% yield) of a crystalline powder mp 91-92° C. $^1$H-NMR ($CDCl_3$) $\delta$ 1.36(3$H_a$,d, J=8Hz); 1.46(9$H_b$,s); 2.44-(2$H_c$,app.t, J=8Hz); 2.64(2$H_c'$,app.t,J=8Hz); 3.66(3$H_d$,s); 4.20-4.60(1$H_e$,m); 6.85(1$H_f$,m, rotamer of amide -NH)ppm. IR ($CHCl_3$) 3400, 1815, 1785, 1740cm$^{-1}$

Ex. 3: Methoxysuccinylalanine (4)

4

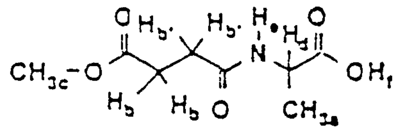

The t-butyl ester 3 was hydrolyzed by either of the following methods in good yield.

Procedure A:

Formic acid (98%) (1.5mL) was added to an ice cooled solution of 3 (1.0g, 3.9mmoles) dissolved in ethyl acetate (10mL). Hydrogen chloride gas was slowly bubbled through the cooled solution in two short (30s) intervals 10 min apart. The solution was allowed to warm to room temperature and stirred for 2h. The volatile liquids were evaporated in vacuo. The residue, containing the product, was chromatographed on 15g of silica gel column (1 x 25cm). The product 4 was eluted with 4% methanol in methylene chloride. Upon evaporation of the eluent solvent under reduced pressure 0.77g (3.8mmoles) (98% yield) of a transparent oil was obtained.

Procedure B:

A solution of 3 (1.0g, 3.9mmoles) in glacial acetic acid (5mL) at room temperature was slowly diluted with 30% HBr in acetic acid (5mL) and stirred for 30 min. The reaction was stopped by the addition of ice-water (15mL) and the fine suspension was extracted with methylene chloride (5 x 25mL). The organic layer was washed with brine, dried (5g of $MgSO_4$), and the solvent was azeotropically removed under vaccum using n-heptane. The product was chromatographically purified as described in Procedure A, to give 0.77g (3.8mmoles) (98% Yield) of a transparent oil. $^1$H-NMR ($CDCl_3$) $\delta$ 1.36(3$H_a$,d,J=8Hz); 2.56-(4$H_{b,b'}$,app.t,J=8Hz);3.66(3$H_c$,s);4.20-4.60(1$H_d$,m); 6.56(1$H_e$,m, rotamer of amide-NH); 9.50(1$H_f$,s)ppm. IR ($CHCl_3$) 3280. 1735, 1690, 1630, 1540$cm^{-1}$.

Ex. 4: $N_\alpha$-Methoxysuccinylalanyl-$N_\epsilon$-benzoyllysine (5)

5

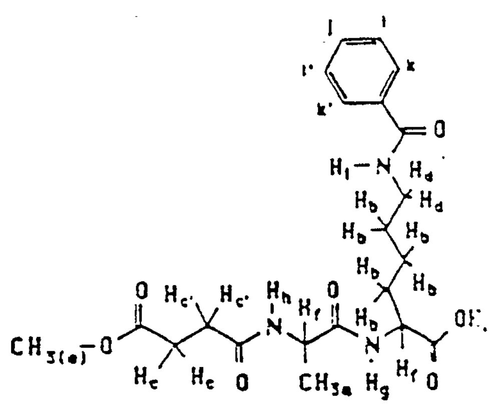

This compound was prepared from $N_\epsilon$-benzoyl-L-lysine (not the phenacyl ester) according to an analogous procedure to that described for 6, with the following changes: the solvent was changed to DMF, the reaction time was extended to 24h and the eluting solvent was changed to 8% methanol in chloroform. Upon evaporation of the eluent solvent under reduced pressure 0.37g (0.85mmoles) (47% yield) of a transparent oil was obtained. $^1$H-NMR ($CDCl_3$) $\delta$ 1.36(3$H_a$,d,J=8Hz); 1.50-1.86(6$H_b$,m); 2.56(4$H_{c,c'}$,app. t,J=8Hz); 3.23(2$H_d$,m); 3.66(3$H_8$,s); 4.00-4.70(2$H_f$,m); 5.80(1$H_g$,m, rotamer of amide -NH); 7.23(1$H_h$m, rotamer of amide -NH); 7.34(2$H_{i,i'}$,app. dd, $J_{ik}$ = 8Hz, $J_{ii'}$ = 2Hz); 7.43(1$H_j$,app. dd, $J_{ji}$=8Hz, $J_{jk}$=2Hz);

7.86($2H_{k,k'}$,app. dd,$J_{kl}$ = 8Hz,$J_{kl}$ = 2Hz); 8.10($1H_{l'}$,m, rotamer of amide -NH); 9.56($1H_m$,s)ppm. IR ($CHCl_3$) 3330, 1730, 1645, 1600, 1540$cm^{-1}$.

Ex. 5 : $N_\alpha$-Methoxysuccinylalanyl-$N_\epsilon$-carbobenzoxylsine phenacyl ester (6)

6

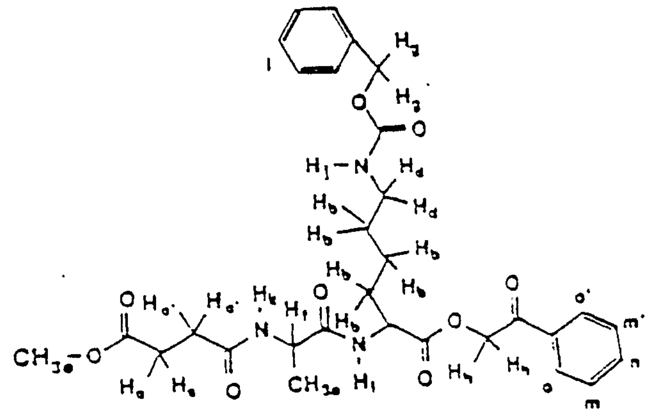

Methoxysuccinylalanine (4) (0.37g, 1.8mmoles) and N-hydroxysuccinimide (0.2g, 1.8mmoles) were mixed in THF (3mL) and cooled to 5° C. A concentrated solution of N,N'-dicyclohexylcarbodiimide (0.4g, 1.8mmoles) in THF was added dropwise to the cooled solution. The suspension was stirred for 14h at 5° C, and the precipitated urea formed was filtered under vacuum. The filtrate was cooled to 5° C and used in the next reaction without further purification.

To a mixture of $N_\epsilon$-carbobenzoxylysine phenacyl ester hydrochloride (35) (0.7g, 1.7mmoles) and the above N-hydroxysuccinimide ester in cooled THF (7mL), triethylamine (0.17g, 1.7mmoles) in THF (0.5mL) was added dropwise. A solution was observed for a short period of time before a precipitate was formed. The progress of the reaction was monitored by TLC (10% methanol in chloroform). Upon completion of the reaction (4h), the formed triethylamine salt was filtered, washed with ethyl acetate and the filtrate evaporated under vacuum. The residue, containing the product, was chromatographed on 15g of silica gel column (1 x 25cm). Impurities were eliminated by first passing 50mL of methylene chloride and subsequently the compound was eluted with 2% methanol in methylene chloride. Upon evaporation of the elution solvent under reduced pressure a hygroscopic product was obtained. The latter was then recrystallized from ethyl acetate/diethylether to give 0.8g (1.3mmoles) (84% yield) of a crystalline powder, mp 114-116° C. $^1$H-NMR ($CDCl_3$) $\delta$ 1.36($3H_a$,d, J = 8Hz); 1.50-2.20($6H_b$,m); 2.44($2H_c$,app. t,J = 8Hz); 2.64($2H_{c'}$,app.t, J = 8Hz); 3.23($2H_d$,m); 3.66($3H_e$,s); 4.30-4.90($2H_f$m); 5.10($2H_g$,s); 5.46($2H_h$,s); 5.80($1H_i$,m, rotamer of amide -NH); 6.56($1H_j$,m, rotamer of amide -NH); 7.10($1H_k$,m, rotamer of amide -NH); 7.40($5H_l$,s); 7.63($3H_{m,m',n}$,app. dd, $J_{mo}$ = 8Hz, $J_{mm'}$ = 2Hz); 8.06($2H_{o,o'}$,app. dd, $J_{om}$ = 8Hz, $J_{on}$ = 2Hz)ppm. IR ($CHCl_3$) 3330, 1755, 1730, 1645, 1600, 1540$cm^{-1}$.

Ex. 6 : $N_\alpha$-Methoxysuccinylalanyl-$N_\epsilon$-carbobenzoxylysine (7)

7

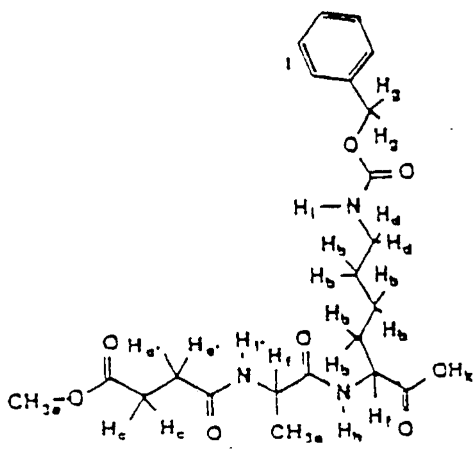

Small portions of zinc metal (total 2g) were added over a 1h period to a solution of phenacyl ester 6-(0.6g, 1.1mmoles) in glacial acetic acid (10mL). The reaction was completed within one additional hour of stirring at room temperature. The suspension was diluted with 20% methanol in chloroform (50mL), filtered under vacuum and the precipitate washed with 50mL of 20% methanol in chloroform. The filtrate, containing the product, was evaporated under reduced pressure. The residue was partially dissolved in 5% methanol in chloroform and filtered to remove the zinc oxide. The second filtrate was evaporated under reduced vacuum to an impure oil. The latter was dissolved in methylene chloride and chromatographed on 10g of silica gel column (1 x 25cm). Impurities were eliminated by first passing 100mL of 2% methanol in methylene chloride and subsequently compound 7 was eluted with 5% methanol in methylene chloride. Upon evaporation of the eluent solvent under reduced pressure an oil with a tendency to foam under vacuum was obtained. The product was crystallized from ethyl acetate/hexane to give 0.37g (0.74mmole)-(74% yield) of a crystalline white powder, mp 116-118 °C. $^1$H-NMR ($CDCl_3$) $\delta$ 1.36($3H_a$,d, J=8Hz); 1.40-2.20($6H_b$,m); 2.56(4Hc,c′,app. t, J=8Hz); 3.23($2H_d$,m); 3.66($3H_e$,s); 4.30-4.90($2H_f$,m); 5.10($2H_g$,s); 5.60-($1H_h$,m, rotamer of amide -NH); 6.80-7.20($2H_{i,j}$′,m, rotamer of amide -NH); 7.34($5H_j$,s); 9.60($1H_k$,s)ppm. IR ($CHCl_3$) 3330, 1730, 1710, 1590, 1540cm$^{-1}$.

Ex. 7 : Nα-Methoxysuccinylalanyl-Nδ-carbobenzoxyornithine phenacyl ester (8)

8

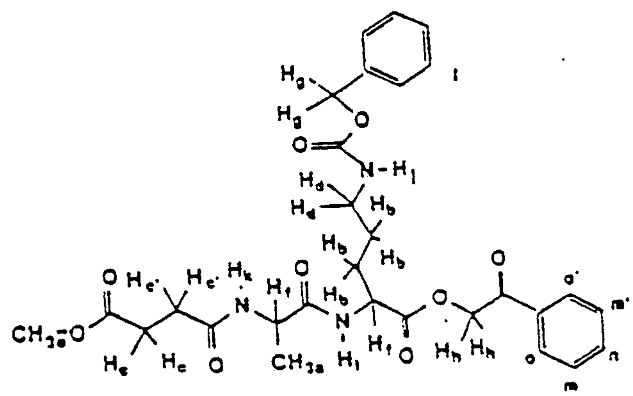

The title compound was prepared from Nδ-carbobenzoxy-L-ornithine phenacyl ester hydrochloride following an analogous procedure to that described for 6. The product was crystallized from ethyl acetate/diethyl ether to give 0.46g (0.8 mmole) (46% yield) of a crystalline powder mp 112-113 °C. $^1$H-NMR ($CDCl_3$) $\delta$ 1.36($3H_a$,d,J=8Hz); 1.40-2.20($4H_b$,m); 2.44($2H_c$,app. t,J=8Hz); 2.64($2H_c$′,app. t,J=8Hz); 3.18-($2H_d$,m); 3.66($3H_e$s); 4.36-5.00($2H_f$,m); 5.10($2H_g$,s); 5.46($2H_h$,s); 5.80($1H_i$,m, rotamer of amide -NH); 6.56-($1H_j$,m, rotamer of amide -NH); 7.10($1H_k$,m, rotamer of amide -NH); 7.34($5H_l$,s); 7.63($3H_{m,m}$′,n,app. dd, $J_{mo}$=8Hz, $J_{mm}$′=2Hz); 8.06($2H_{o,o}$′,app. dd, $J_{om}$=8Hz), $J_{on}$=2Hz)ppm. IR ($CHCl_3$) 3330, 1755, 1730, 1645, 1600, 1540cm$^{-1}$.

Ex. 8: $N_\alpha$-Methoxysuccinylalanyl-$N_\delta$-carbobenzoxyornithine (9)

9

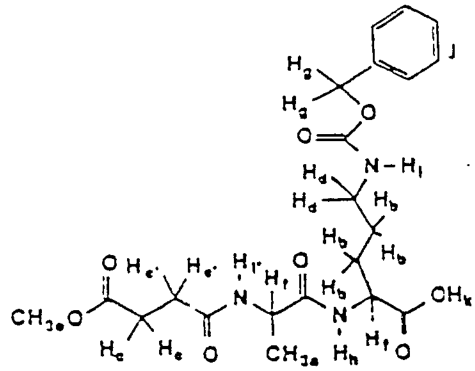

This compound was prepared from 8 according to an analogous procedure to that described for 7. Upon evaporation of the eluent solvent under reduced pressure 0.3g (0.75mmole)(68% yield) of a transparent oil was obtained. $^1$H-NMR (CDCl3) $\delta$ 1.36($3H_a$,d, J=8Hz); 1.40-1.90($4H_b$,m); 2.56($4H_{c,c'}$,app. t, J=8Hz); 3.23($2H_d$,m); 3.66($3H_e$,s); 4.40-4.80($2H_f$,m); 5.10($2H_g$,s); 6.80-7.10($2H_j$,m, rotamer of amide -NH); 7.34($5H_j$,s); 10.10($1H_k$,s)ppm. IR ($CHCl_3$) 3330, 1730, 1710, 1590, 1540cm$^{-1}$.

Ex. 9: $N_\alpha$-Methoxysuccinyl-$N_\delta$-carbobenzoxyornithine (10)

10

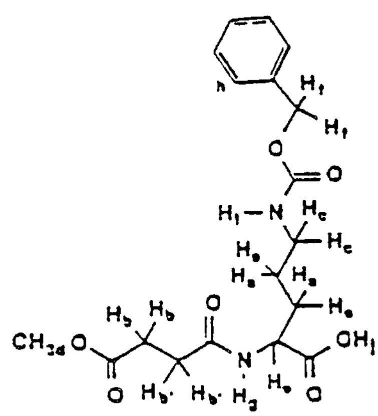

A suspension of 2 (0.86g,3.8mmoles) and $N_\delta$-carbobenzoxy-L-ornithine (1g, 3.8mmoles) in DMF were stirred during and after the addition of triethylamine (0.38g, 3.8mmoles) at room temperature. The progress of the reaction was monitored by TLC (10% acetic acid in ethyl acetate), upon completion of the reaction (24h), the formed triethylamine salt was filtered and the filtrate coevaporated with toluene under vacuum. The residue, containing the product, was dissolved in a 50:50 mixture of ethyl acetate/0.001M aqueous HCl (30mL). The organic layer was washed with water (30mL) and brine (30mL); dried (5g of $MgSO_4$) and evaporated under reduced pressure. The residue was chromatographed on 30g of silica gel column (2 x 5cm). The desired product was eluted with 4% methanol in chloroform. Upon evaporation of the eluent solvent under reduced pressure, 1.4g (3.6mmoles) (96.7% yield) of an oil was obtained. The phenacyl ester derivative was recrystallized from ethyl acetate/hexane to give a crystalline powder mp 112-113°C. $^1$H-NMR ($CDCl_3$) $\delta$ 1.50-2.00($4H_a$,m); 2.44($2H_{b'}$app. t, J=8Hz); 2.64($2H_{b'}$,app. t, J=8Hz); 3.23($2H_c$,m); 3.66($3H_d$,s); 4.30-4.80($1H_c$,m); 5.10($2H_f$,s); 7.20($1H_g$,m, rotamer of amide -NH); 7.40($5H_h$,s); 8.13($1H_i$,m, rotamer of amide -NH); 9.46($1H_j$,s)ppm. IR ($CHCl_3$) 3320, 1720, 1700, 1660, 1540cm$^{-1}$.

Ex. 10: $N_\alpha$-Methoxysuccinyl-$N_\delta$-carbobenzoxyornithylalanine t-butyl ester (11)

11

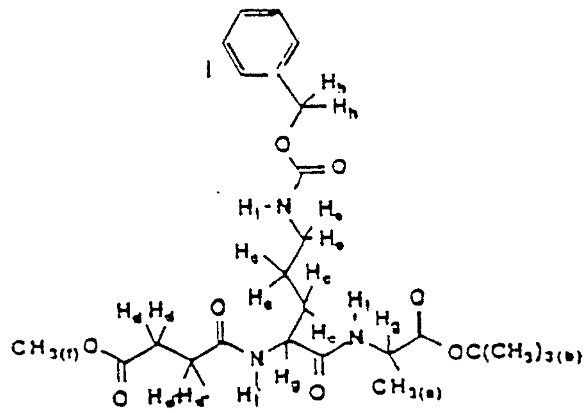

This compound was prepared from 10 according to an analogous procedure to that described for 14. The product (11 crystallized from ethyl acetate/hexane to give 1.7g (3.3mmoles) (87% yield) of a white crystalline powder mp 133-135° C. 'H-NMR ($CDCl_3$) δ 1.36($3H_a$,dd, J=8Hz); 1.46($9H_b$,s); 1.50-2.20($4H_c$,m); 2.44($2H_d$,app. t, J=8Hz); 2.64($2H_d'$,app. t,J=8Hz); 3.23($2H_e$,m); 3.66($3H_f$,s); 4.00-4.52($2H_g$,m); 5.10($2H_h$,s); 6.80-7.20($3H_i$,m, rotamer of amide -NH); 7.40($5H_j$,s)ppm. IR ($CHCl_3$) 3330. 1735, 1725, 1590, 1540cm$^{-1}$.

Ex. 11: $N_\alpha$-Methoxysuccinyl-$N_\delta$-carbobenzoxyornithyalanine (12)

12

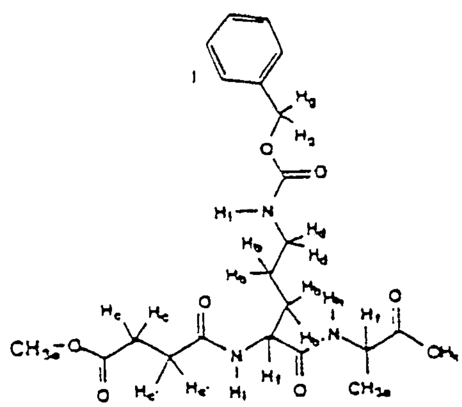

This compound was prepared from 11 according to analogous procedure to that described for 15. The product (12) was crystallized from ethyl acetate/hexane to give 0.2 (0.5mmole) (48% yield) of a crystalline powder, mp 147-148° C. 'H-HMR ($CDCl_3$) δ 1.36($3H_a$,d, J=8Hz); 1.50-2.20($4H_b$,m); 2.44($2H_c$,app. t, J=8Hz); 2.64($2H_c'$,app. t, J=8Hz); 3.23($2H_d$,m); 3.66($3H_8$,s); 4.00-4.52($2H_f$,m); 5.10($2H_g$s); 6.56($1H_h$,m, rotamer of amide -NH); 6.80-7.20($2H_i$,m, rotamer of amide -NH); 7.34($5H_j$,s); 9.36($1H_k$,s)ppm. IR ($CHCl_3$) 3330, 1730, 1645, 1590, 1540cm$^{-1}$.

Ex. 12: $N_\alpha$-Methoxysuccinyl-$N_\epsilon$-carbobenzoxylysine (13)

13

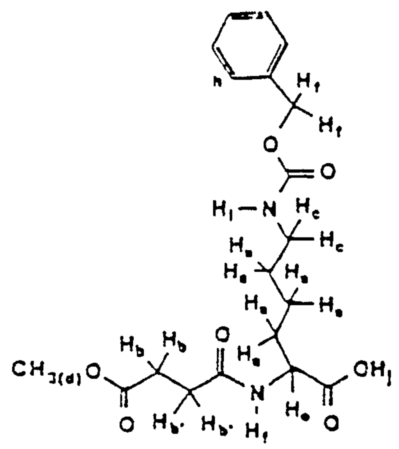

This compound was prepared from $N_\epsilon$-carbobenzoxy-L-lysine according to an analogous procedure to that described for 10. The product was crystallized from chloroform hexane to give 1.35 (3.42mmoles) (90% yield) of a crystalline powder, mp 85-86 °C. $^1$H-NMR ($CDCl_3$) $\delta$ 1.50-2.00($6H_a$,m); 2.44($2H_b$,app. t, J = 8Hz); 2.64($2H_b'$,app. t, J = 8Hz); 3.23($2H_c$,m); 3.66($3H_d$,s); 4.30-4.80($1H_g$,m); 5.10($2H_f$,s); 7.20($1H_g$,m, rotamer of amide -NH); 7.40($5H_h$,s); 8.13($1H_i$,m, rotamer of amide -NH); 9.46($1H_j$,s)ppm. IR ($CHCl_3$) 3320, 1720, 1705, 1660, 1535cm$^{-1}$. Elemental analysis cal'd for $C_{19}H_{25}N_2O_7$. C,57.86;H,6.64;N,7.10. Found: C.57.87;H,6.67;N,7.09.

Ex. 13:$N_\alpha$-Methoxysuccinyl-$N_\epsilon$-carbobenzoxylysylalanine t-butyl ester (14)

14

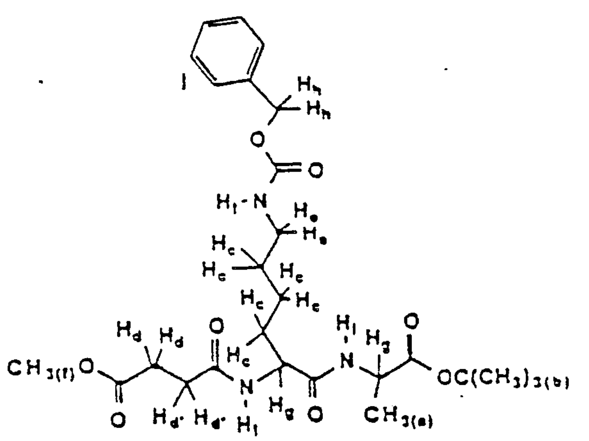

The carboxylic acid function of 13 (3.8mmoles) was activated with N hydroxysuccinimide (0.4g, 3.8mmoles) using N,N′-dicyclohexylcarbodiimide (0.8g 3.8mmoles) in THF at 0 °C. The cold reaction was stirred for one hour and stored in the refrigerator overnight (14h). The precipitated urea was filtered under vacuum and washed with a small amount of THF.

L-Alanine t-butyl ester (0.7g,3.8mmoles) was added as a solid to an ice-cooled THF (7mL) solution of the activated ester (above). Triethylamine (0.38g, 3.8mmoles) in THF (0.5mL) was added dropwise (10 min) to the fine suspension. The formed precipitate was filtered after 1h and the filtrate evaporated under reduced pressure. The residue, containing the product, was chromatographed on 20g silica gel column (1 x 50cm). Impurities were eliminated by first passing 50mL methylene chloride and subsequently the compound was eluted with 3% methanol in methylene chloride. Upon evaporation of the eluent solvent under reduced pressure an oil was obtained. The latter was crystallized from ethyl acetate/hexane to give 1.67g (3.1mmoles) (82% yield from 11 of white crystalline powder, mp 155-157 °C. $^1$H-NMR ($CDCl_3$) $\delta$ 1.36-($3H_a$,dd, J = 8Hz); 1.46($9H_b$,s); 1.50-2.20($6H_c$,m); 2.44($2H_d$,app. t, J = 8Hz); 2.64($2H_d'$,app. t, J = 8Hz); 3.23-($2H_e$,m); 3.66($3H_f$,s); 4.00-4.25($2H_g$,m); 5.10($2H_h$,s); 6.80-7.20($3H_i$,m, rotamer of amide -NH); 7.40($5H_j$,s)ppm. IR($CHCl_3$) 3330, 1735, 1725, 1590, 1540cm$^{-1}$, Elemental analysis cal'd for $C_{25}H_{37}N_3O_9$; C,61.08;H,7.59;N,8.55. Found: C,61:08;H,7.60;N,8.53.

Ex. 14: $N_\alpha$-Methoxysuccinyl-$N_\epsilon$-carbobenzoxylysylalanine (15)

15

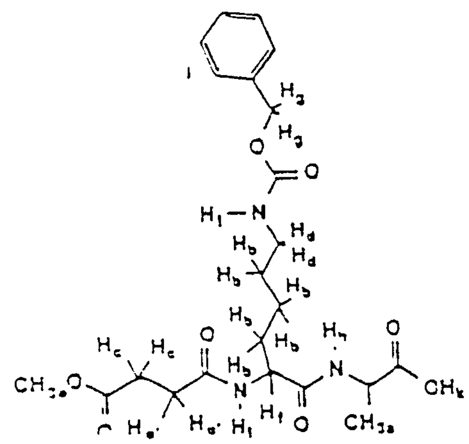

Formic acid (98%) (0.75mL) was added to an ice-cooled solution of the t-butyl ester 14 (1mmole) in ethyl acetate (5mL). Hydrogen chloride gas was slowly bubbled through the cooled solution inn two short 30s intervals 10 min apart. The solution was allowed to warm to room temperature and was stirred until starting material had totally disappeared, as observed from TLC (10% methanol in chloroform)(3h). The formic acid and t-butanol were azeotroped with n-heptane in vacuo. The product was crystallized from ethyl acetate.heptane to give 0.4g (0.9mmole) (88% yield) of a crystalline powder, mp 192-194 °C. $^1$H-NMR ($CDCl_3$) δ 1.36(3$H_a$,d, J=8Hz); 1.50-2.20(6$H_b$,m); 2.44(2$H_c$,app. t, J=8Hz); 2.64(2$H_c'$,app. t. J=8Hz); 3.23-(2$H_d$,m); 3.66(3$H_e$,s); 4.00-4.52(2$H_f$,m); 5.10(2$H_g$,s); 6.56(1$H_h$,m, rotamer of amide -NH); 6.80-7.20(2$H_i$,m, rotamer of amide -NH); 7.34(5$H_j$,s); 9.36(1$H_k$,s)ppm. IR ($CHCl_3$) 3330, 1730, 1645, 1590, 1540cm$^{-1}$. Elemental Analysis cal'd. for $C_{22}H_{31}N_3O_8$: C,56.76;H,6.71;N,9.03. Found: C,56.56:H,6.77;N,8.97.

Ex. 15: $N_\alpha$-Methoxysuccinyl-$N_\epsilon$-benzoyllysine(16)

16

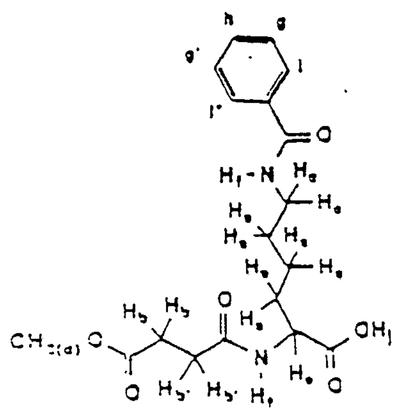

This compound was prepared from $N_\epsilon$-benzoxy-L-lysine (43) according to an analogous procedure to that described for 10. Upon evaporation of the eluent solvent 1.2g (3.3mmoles) (88% yield) of a transparent oil was obtained. $^1$H-NMR ($CDCl_3$) δ 1.50-1.90(6$H_a$,m); 2.70(2$H_b$,app. t, J=8Hz); 2.90(2$H_b'$,app. t, J=8Hz); 3.33(2$H_c$,m); 3.66(3$H_d$,s); 4.10-4.80(1$H_e$,m); 6.90(2$H_f$,m, rotamer of amide -NH); 7.34(2$H_{g,g'}$,app. dd, $J_{gi}$=8Hz, $J_{gg'}$=2Hz); 7.43(1$H_h$,app. dd, $J_{hg}$=8Hz, $J_{hi}$=2Hz); 7.86(2$H_{i,i'}$,app. dd, $J_{ig}$=8Hz, $J_{ih}$=2Hz); 8.90-(1$H_i$,s)ppm. IR ($CHCl_3$) 3520, 3360, 1840, 1790, 1735, 1650, 710, 640cm$^{-1}$.

Ex. 16:$N_\alpha$-Methoxysuccinyl-$N_\epsilon$-benzoyllysylalanine t-butyl ester (17)

17

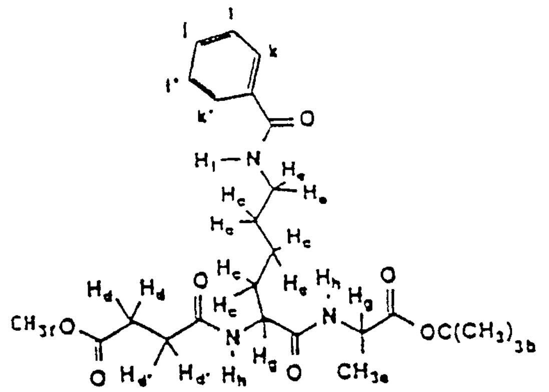

This compound was prepared from 16 according to an analogous procedure to that described for 14. The product (17) was crystallized from ethyl acetate-hexane to give 0.9g (1.8mmoles) (47% yield) of a white crystalline powder, mp 156-158° C. $^1$H-NMR ($CDCl_3$) δ 1.36($3H_a$,d, J=8Hz); 1.46($9H_b$,s); 1.50-2.20($6H_c$,m); 2.44($2H_d$,app. t, J=8Hz); 2.64($2H_d'$,app. t, J=8Hz); 3.23($2H_e$,m); 3.66($3H_f$,s); 4.10-4.70($2H_g$,m); 6.60-7.10-($2H_h$,m, rotamer of amide -NH); 7.34 ($1H_{i,i'}$,app. dd. $J_{ij}$=8Hz,$J_{ii'}$=2Hz); 7.42($1H_j$,app. dd, $J_{ji}$=8Hz, $J_{jk}$=2Hz); 8.10($1H_l$,m,rotamer of amide -NH)ppm. IR ($CHCl_3$) 3330, 1730, 1630, 1540, 1460, 1380cm$^{-1}$.

Ex. 17: $N_\alpha$-Methoxysuccinyl-$N_\epsilon$-benzoyllsylalanine (18)

18

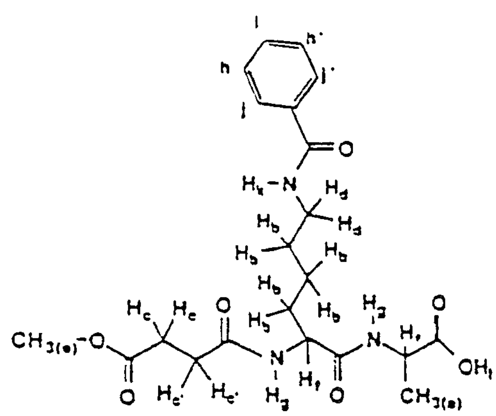

This compound was prepared from 17 according analogous procedure to that described for 15. The product (18 was crystallized from ethyl acetate/hexane to give 0.4g (1mmole) (96% yield) of a crystalline powder, mp 193-194° C. $^1$H-NMR ($CDCl_3$) δ 1.36($3H_a$,d, J=8Hz); 1.50-2.20($6H_b$,m); 2.44($2H_c$,app. t, J=8Hz); 2.64($2H_c'$,app. t, J=8Hz); 3.23($2H_d$,m); 3.66($3H_e$,s); 4.10-4.70($2H_f$,m); 6.60-7.10($2H_g$,m, rotamer of amide -NH); 7.34($2H_{h,h'}$,app. dd, $J_{hi}$=8Hz, $J_{hh'}$,=2Hz); 7.43($1H_i$,app. dd, $J_{ih}$=8Hz, $J_{ij}$=2Hz); 7.83 ($2H_{j,j'}$,app. dd, $J_{jh}$=8Hz, $J_{ji}$=2Hz); 8.10($1H_k$,m, rotamer of amide -NH); 9.60($1H_l$,s)ppm. IR ($CHCl_3$) 3330, 1730, 1630, 1590, 1540, 1460, 1380cm$^{-1}$.

Ex. 18: Preparation of an ether alcoholic solution of diazomethane, with a Diazald distillation kit

Ethanol, 95% (8mL) was added to a solution of potassium hydroxide (1.79g) in water (2.7mL) in a 50mL distilling flask fitted with a dropping funnel and an efficient condenser set downward for distillation. The condenser was connected to two receiving flasks in series, the second containing 20mL of diethyl ether. The inlet tube of the second receiver was dipped below the surface of the ether. Both receivers were cooled to 0° C. The flask containing the alkali solution was heated in a water bath to 65° C, and a solution of Diazald (N-methyl-N-nitroso-p-toluenesulfonamide (7.2g, 33.0mmole) in ether (70mL) was added through the dropping funnel over about 30 min. The rate of distillation was approximately equal to the rate of addition. When the dropping funnel was empty, another 20mL of ether was added slowly and the distillation was

continued until the distilling ether was colorless. The combined ethereal distillate contained about 1g (33.0mmoles) of diazomethane based on the amount of Diazald used. Extreme care is warranted during and after the preparation of diazomethane. Care must be taken to avoid possible explosions by thoroughly checking the glassware for cracks and scratches, heating the alkali solution with a water bath (not to exceed 75° C) and keeping the generated diazomethane solutions at or below 5° C.

Ex. 19: N-t-Boc-L-prolyl chloromethyl ketone (19)

19

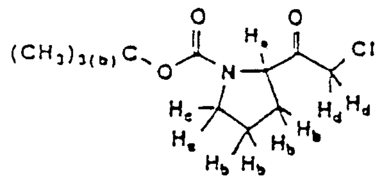

The procedure of Digenis et al.[39] was followed for the preparation of compound 19.

Isobutylchloroformate (2.1g, 15.3mmoles) was added to a solution of triethylamine (1.55g, 15.3mmoles) and t-Boc-L-proline (3.3gm, 15.3mmoles) in diethyl ether (30mL) was cooled to -15° C. The reaction mixture was stirred at this temperature for 10 min, at which time a cooled solution (0° C) of diazomethane (1g, 32.4mmoles) in diethyl ether (200mL) was added. The vessel, equipped with a calcium sulfate drying tube, was stirred at 0° C in the hood overnight (14h). The organic layer was washed subsequently with saturated bicarbonate solution (30mL) water (30mL) and brine (30mL). After drying with 15g of $MgSO_4$, the solvent was evaporated under reduced pressure to yield a yellow oil.

Hydrogen chloride gas was bubbled through an ice cooled solution of the yellow oil (N-t-Boc-L-prolyl azomethyl ketone) in diethyl ether (50mL) for 60s. The reaction was stopped after 10 min by diluting with cooled diethyl ether (50mL) and evaporated under vacuum. The residue, containing the product, was chromatographed on 50g of silica column (3 x 75cm). Impurities were eliminated by first passing 100mL of 10% hexane in chloroform and subsequently the compound was eluted with chloroform. Upon evaporation of the eluent solvent under reduced pressure a transparent oil was obtained. The latter (19) was then crystallized from chloroform/hexane to give 3.1g (12.5mmoles)(82% yield) of colorless crystals m.p. 47-49° C. $^1$H-NMR ($CDCl_3$) δ 1.46(9$H_a$,s); 1.86-2.16(4$H_b$,m); 3.33-3.76(2$H_c$,m); 4.36(2$H_d$,s); 4.40-4.80(1$H_c$,m)-ppm. IR (Nujol) 1740, 1690$cm^{-1}$. Elemental Analysis Cal'd for $C_{11}H_{18}ClNO_3$: C,53.33;H,7.32;N,5.65. Found,C,53.46;H,7.35;N,5.56.

The NMR, IR and Elemental analysis were found to be identical to those reported by Digenis et al.[39].

Ex. 20: N-[(N-t-Boc-L-propyl)methyl]isopropylamine (20)

20

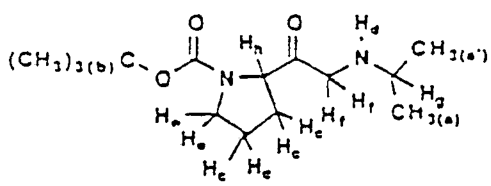

This compound was synthesized by a modified procedure of Digenis et al.[39].

Isopropylamine (7.4g, 125.0mmoles) was slowly added to a cooled solution(0° C) of 19 (3.1g, 12.5mmoles) in diethyl ether and stirred overnight at room temperature (14h). The salt formed was filtered and the filtrate evaporated under vacuum. The oil was chromatographed on 30g of silica gel column (2 x 25cm). Impurities were eliminated by first passing 50mL of 1% methanol in chloroform and subsequently the compound was eluted with 5% methanol in chloroform. Upon evaporation of the eluent solvent under reduced pressure 3.1gm (11.4mmoles) (90.8% yield) of an oil was obtained. $^1$H-NMR($CDCl_3$) δ 0.93-1.31 (6$H_a$,app. d, J=7Hz); 1.46(9$H_b$,s); 1.86-2.16(4$H_c$,m); 2.83(1$H_d$,m); 3.30-3.73(5$H_{e,f,g}$,m); 4.30(1$H_h$,app. t, J=8Hz)ppm. IR ($CHCl_3$) 3330, 1695$cm^{-1}$.

The NMR and IR of this compound were found to be identical to those reported by Digenis et al.[39].

Ex. 21: p-Nitrophenyl N-[(N-Boc-L-prolyl)methyl]-N-isopropyl-carbamate (21)

21

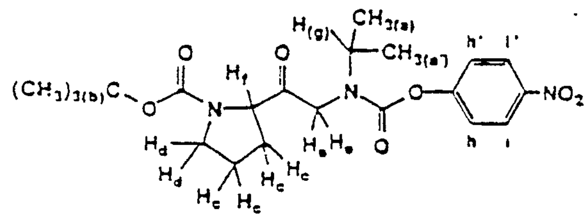

The procedure of Digenis et al.[39] was followed for the preparation of compound 21.

p-Nitrophenylchloroformate (2.75g, 13.6mmoles) was added as a powder to an ice-cooled solution of 20 (3.1g, 11.4mmoles) in THF (10mL) and triethylamine (1.15g, 11.4mmoles) at 5° C. The mixture was stirred at 5° C for 2h, the formed triethylamine salt filtered and the filtrate evaporated under vacuum. The crude oil was dissolved in ethyl acetate and washed with water, 10% aqueous citric acid (30mL), water (30mL) and brine (30mL); dried (10g of $MgSO_4$) and evaporated under reduced pressure. The residue, containing the product, was dissolved in a small amount of chloroform and chromatographed on 40g of silica gel column (2 x 50cm). The impurities were eliminated by first passing chloroform and subsequently the compound was eluted with 50:1 chloroform in ethyl acetate. Upon evaporation of the eluent solvent under reduced pressure 4.6g (10.7mmoles) (94% yield) of a transparent oil was obtained. $^1$H-NMR($CDCl_3$) $\delta$ 1.13(3$H_a$,app. d, J=8Hz); 1.20(3$H_a'$,add. d, J=8Hz); 1.46(9$H_b$,s); 1.86-2.16(4$H_c$,m); 3.56-3.84(2$H_d$,m); 4.20,4.32(2$H_e$, center of dd, overlapping with another set, J=20Hz, rotamers of the $CH_{2(e)}$ geminal system); 4.50(2$H_f$,app. t, J=8Hz); 4.53-4.80(4$H_g$,m); 7.22(1$H_h$,app. d, J=10Hz); 7.26(1$H_h'$app. d, J=10Hz); 8.22(1$H_i$,app. d, J=10Hz); 8.24-(1$H_i'$.app. d, J=10Hz). IR ($CHCl_3$) 1740, 1710, 1690, 1595, 1520cm$^{-1}$.

The NMR and IR for this compound were found to be identical to those reported by Digenis et al.[39]

Ex. 22: p-Nitrophenyl-N-(L-Prolylmethyl)-N-isopropylcarbamate Hydrochloride (22)

22

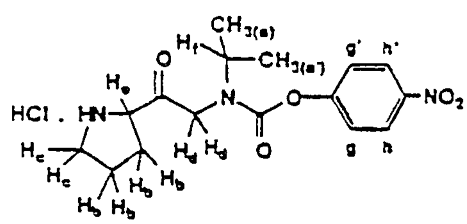

The procedure of Digenis et al. was followed for the preparation compound 22.

Three additions of hydrogen chloride gas (30s each) were introduced at 10 min intervals to an ice-cooled ethyl acetate (20mL) solution of 21 (1.6g, 5.6mmoles) and formic acid (98%) (2.4mL). The solution was stirred at 5° C for 1h, then allowed to equilibrate to room temperature (1h). The product was collected by vacuum filtration and washed with ethyl acetate. A second crop was collected after concentrating the solution. The latter was crystallized from ethanol/diethyl ether to give 1.46g (3.8mmoles) (68% yield) of a tan powder, mp 179-182° C (lit.[39] mp 190-193° C). $^1$H-NMR (DMSO-$d_6$) $\delta$ 1.13(3$H_a$,app. d, J=8Hz); 1.20-(3$H_a'$,app. d, J=8Hz); 1.70-2.30(4$H_b$,m); 3.20-3.56(2$H_c$,m); 4.20(2$H_d$,m); 4.50(2$H_{e,f}$,m); 7.22(1$H_g$,app. d, J=10Hz); 7.26(1$H_g'$,app. d, J=10Hz); 8.22(1$H_h$,app. d, J=10Hz); 8.24(1$H_h'$app. d, J=10Hz)ppm. IR (Nujol) 1740, 1720, 1610, 1595, 1520cm$^{-1}$.

Ex. 23: p-Nitrophenyl N-[Methoxysuccinyl-($N_\epsilon$-carbobenzoxy)lysylalanylprolylmethyl]-N-isopropylcarbamate (44b, the LLD diastereomer)

44b

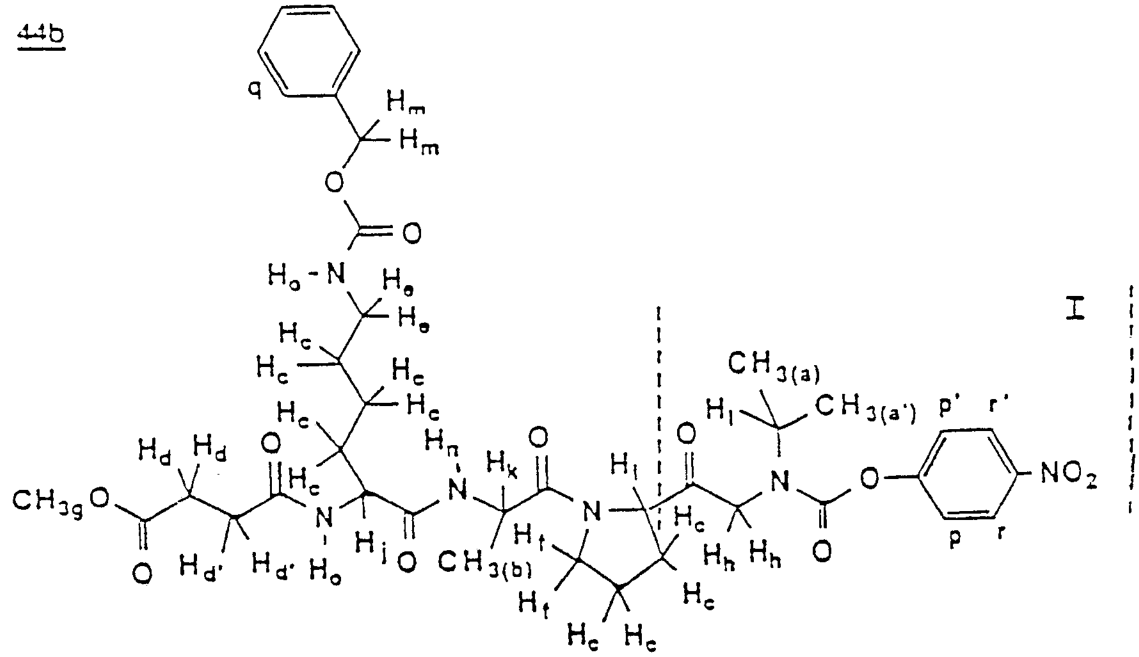

To a cooled solution (-50 °C) of 15 (0.85 mmoles) and N-methylmorpholine (0.85mmoles) in THF (5mL) isobutylchloroformate (0.13g, 0.94mmoles) in acetonitrile (3mL) was added. After 10 min, 22 (0.4g, 1mmole) was added as a solid and N-methylmorpholine (0.1g, 1mmole) as an acetonitrile solution (2mL). The reaction mixture was allowed to warm to 5 °C in 30min and stirred (3h). The reaction mixture was filtered, the filtrate was evaporated under vacuum and the residue was redissolved in methylene chloride (25mL). The organic solvent was subsequently washed with water (25mL), 10% aqueous citric acid (25mL), water (25mL) and brine (25mL); dried (5g of $MgSO_4$) and evaporated under reduced pressure. The residue, containing the product, was chromatographed on 10g silica gel column (1 x 25cm). The impurities were eliminated by first passing 25mL of methylene chloride and 25mL of 2% methanol in methylene chloride. Subsequently the compound was eluted with 8% methanol in methylene chloride. Upon evaporation of the eluent solvent under reduced pressure an amorphous powder was obtained. The amorphous powder containing 44a & 44b was dissolved in chloroform and applied to a preparative TLC plate (100mg/plate) and developed two to three times with 4% methanol in ethyl acetate. The two bands were separately scraped from the plate and extracted with 20% methanol in chloroform (3 X 25mL). The eluent solvent was evaporated under reduced pressure to give an amorphous powder. The lower band was pure 44a. The desired product (44b) isolated as 0.23g (0.3mmoles)(35% yield) of a white amorphous powder, mp 46-47 °C, was obtained from the upper TLC band. $^1$H-NMR ($CDCl_3$) δ 1.13(3$H_a$,app.d, J=7Hz, rotamer of I); 1.20(3$H_a'$,app.d, J=7Hz, rotamer of I); 1.36(3$H_b$,d, J=8Hz); 1.40-2.22(10$H_c$,m); 2.44(2$H_d$,app.t, J=8Hz); 2.64(2$H_d'$,app.t, J=8Hz); 3.18(2$H_e$,m); 3.33(2$H_f$,app.t, J=8Hz); 3.66(2$H_g$,s); 4.30(2$H_h$, center of 2 sets of dd, J=20Hz, rotamer of the $CH_{2(h)}$ geminal system); 4.53(1$H_i$,app.t, J=8Hz); 4.63(1$H_j$,app.t, J=8Hz); 4.80-(2$H_{k,l}$,m); 5.10(2$H_m$,s); 5.42(1$H_n$,m); 6.70-7.10(2$H_o$,m, rotamer of amide -NH); 7.24(1$H_p$,app.d, J=10Hz, rotamer of I); 7.28(1$H_p'$,app.d, J=10Hz, rotamers of I); 7.34(5$H_q$,app.s); 8.20(1$H_r$,app.d, J = 10Hz, rotamer of I); 8.25(1$H_r'$,app.d, J = 10Hz, rotamer of I)ppm. IR ($CHCl_3$) 3310, 1730, 1650, 1520, 735, 700cm$^{-1}$. Elemental analysis cal'd for $C_{38}H_{50}N_6O_{12}$: C,58.30;H,6.44;N, 10.74. Found: C,58.19;H,6.50;N,10.64.

Ex. 24: p-Nitrophenyl N-[Methoxysuccinyl-($N_\epsilon$-carbobenzoxy)lysylalanylprolylmethyl]-N-isopropylcarbamate (44a, the LLL diasetereomer)

44a

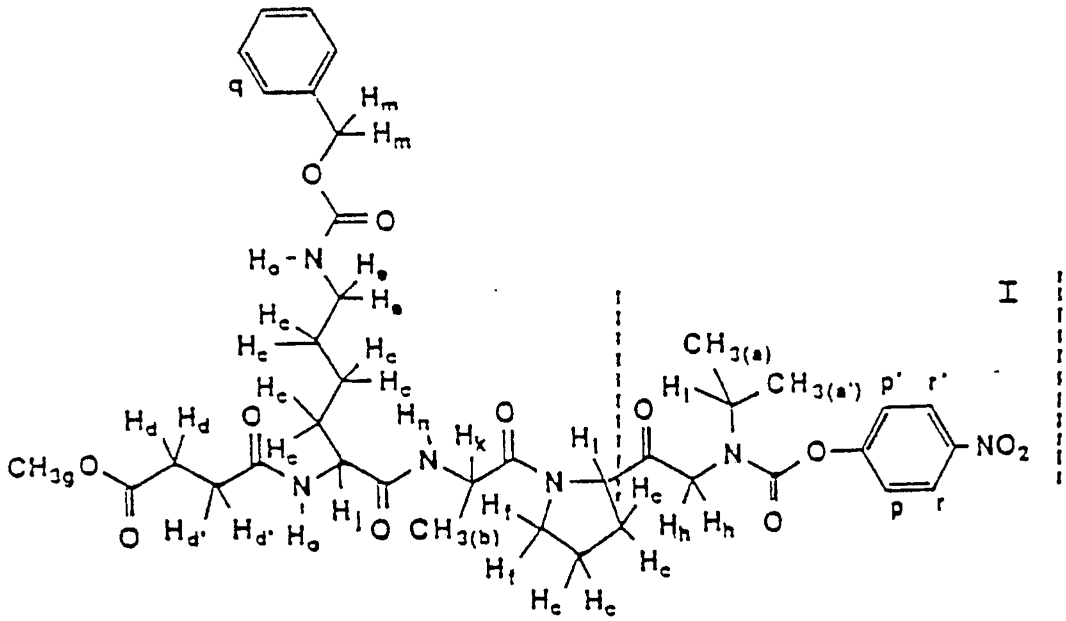

The title compound was prepared from 15 and 22 following a procedure analogous to that described for 44b. Tha amorphous powder containing 44a and 44b was dissolved in chloroform and applied to a preparative TLC plate (100mg plate) and developed two or three times with 4% methanol in ethyl acetate. The lower band contained 44a while the upper band 44b. The two bands were separately scraped from the plate, extracted with 20% methanol in chloroform (3 x.25mL) and eluent solvent evaporated under reduced pressure. The desired product (44a) isolated as 0.33g (0.43mmoles) (50% yield) of a white amorphous powder, mp 50-51 °C, was obtained from the lower TLC band. $^1$H-NMR ($CDCl_3$) δ 1.06-1.28(6$H_{a,a'}$,m, rotamer of I); 1.32(3$H_b$,d, J=8Hz); 1.38-2.32(10$H_c$,m); 2.46(2$H_d$,app.t, J=8Hz); 2.64(2$H_{d'}$,app.t, J=8Hz); 3.20(2$H_e$,m); 3.36(2$H_f$,m); 3.66(3$H_g$,s); 4.20,4.32(2$H_h$, center of a set of dd, overlapping with another set, J=20Hz, rotamers of the $CH_{2(h)}$ geminal system); 4.53(1$H_i$,app.t, J=8Hz); 4.63(1$H_j$,app.t, J=8Hz); 4.80-(2$H_{k,l}$,m); 5.10(2$H_l$,s); 5.42 (1$H_m$,m); 6.36-7.08(2$H_n$,m, rotamers of amide -NH); 7.35(5$H_p$,app.s); 8.20-(1$H_q$,app.d, J=10Hz, rotamers of I); 8.25(1$H_{q'}$,app.d, J=10Hz, rotamers of I) ppm. IR ($CHCl_3$) 3310, 1730, 1650, 1520, 735, 700cm$^{-1}$. Elemental Analysis cal'd for $C_{38}H_{50}N_6O_{12}$: C,58.30;H,6.44;N, 10.74. Found: C,58.47;H,6.48;N, 10.67.

Ex. 25: p-Nitrophenyl N-[Methoxysuccinyl-(N$_\epsilon$-benzoyl)lysylalanylprolylmethyl]-N-isopropylcarbamate (45b, the LLD diastereomer)

45b

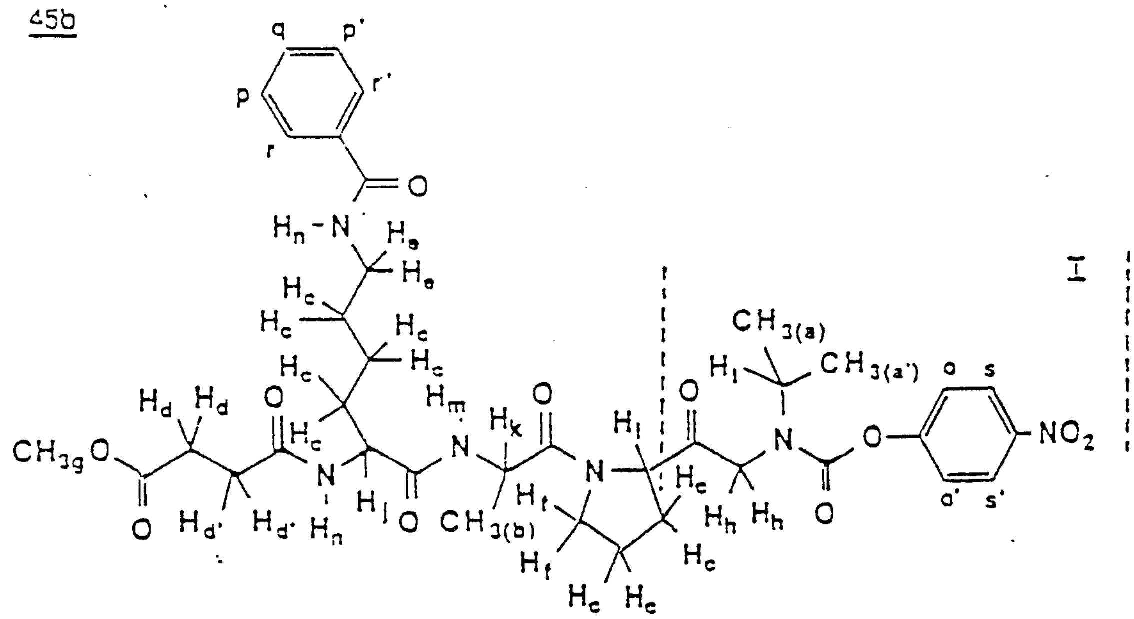

The title compound was prepared from 18 and 22 following a procedure analogous to that described for 44b. The amorphous powder containing 45a & 45b was dissolved in chloroform and applied to a preparative TLC plate (100mg/ml) and developed two or three times with 15% isopropanol in chloroform. The lower band contained 45a while the upper band contained 45b. The two bands were separately scraped from the plate, extracted with 20% methanol in chloroform (3 x 25mL) and eluent solvent evaporated under reduced pressure. The desired product (45b) isolated as 0.26g (0.34mmoles) (40% yield) of a white amorphous powder mp 63-64°C was obtained from the upper TLC band. $^1$H-NMR ($CDCl_3$) δ 1.13($3H_a$,app.d,J=7Hz, rotamers of I); 1.20($3H_a'$,app.d, J=7Hz, rotamers of I); 1.36($3H_b$,d, J=8Hz); 1.40-2.22($10H_c$,m); 2.44-($2H_d$,app.t, J=8Hz); 2.64($2H_d'$,app.t, J=8Hz); 3.18($2H_e$,m); 3.33($2H_f$,app.t, J=8Hz); 3.66($2H_g$,s); 4.30($2H_h$, center of 2 sets of dd, J=20Hz, rotamers of the $CH_{2(h)}$ geminal system); 4.53($1H_i$,app.t, J=8Hz); 4.63-($1H_j$,app.t, J=8Hz); 4.80($2H_{k,l}$,m); 5.42($1H_m$,m); 6.70-7.10($2H_n$,m, rotamers of amide -NH); 7.24($1H_o$,app.d, J=10Hz, rotamers of I); 7.28($1H_o'$,app.d, J=10Hz, rotamers of I); 7.34($2H_{p,p}'$,app.dd, $J_{qp}$=8Hz, $J_{qr}$=2Hz); 7.43 ($1H_q$,app.dd, $J_{qp}$=8Hz, $J_{qr}$=2Hz); 7.83($2H_{r,r}'$,app.dd, $J_{rp}$=8Hz, $J_{rq}$=2Hz); 8.20($1H_s$,app.d, J=10Hz, rotamers of I); 8.25($1H_s'$,app.d,J=10Hz, rotamers of I) ppm. IR ($CHCl_3$) 3400, 1730, 1645, 1525, 1440, 1345, 1215, 750, 665cm$^{-1}$. Elemental Analysis cal'd for $C_{37}H_{48}N_6O_{11}$: C,58.90;H,6.37;N,11.14. Found: C,58.59;H,6.76;N,10.24.

Ex. 26: p-Nitrophenyl N-[Methoxysuccinyl-($N_\epsilon$-benzoyl)lysylalanylprolylmethyl]-N-isopropylcarbamate (45a, the LLL diastereomer)

45a

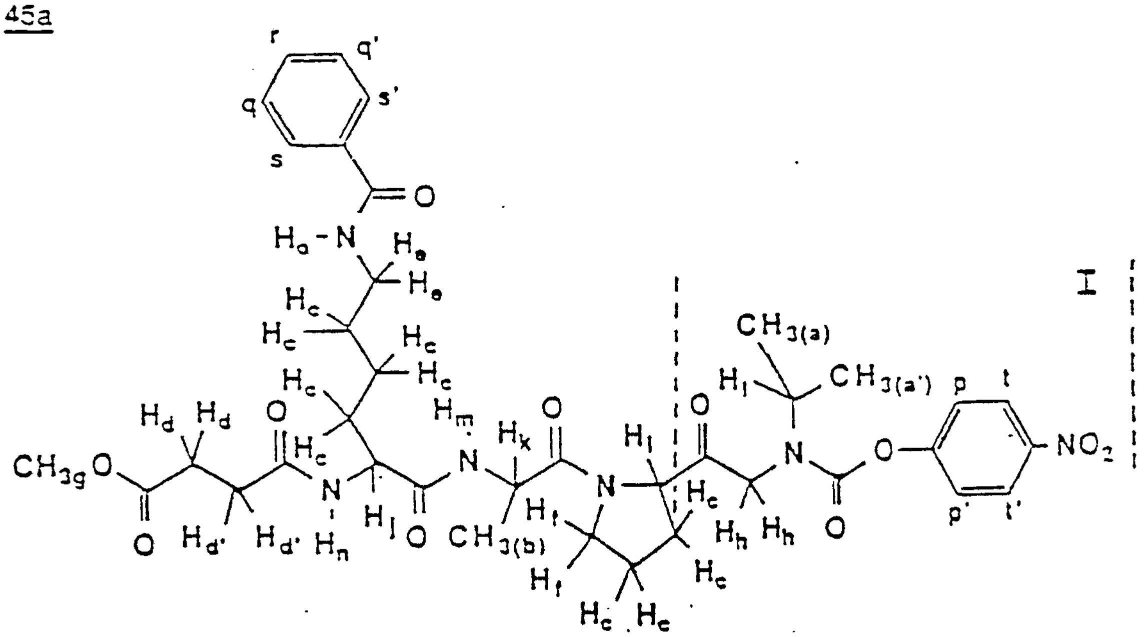

The title compound was prepared from 18 and 22 followed a procedure analogous to that described for 44b. The amorphous powder containing 45a and 45b was dissolved in chloroform and applied to a preparative TLC plate (100mg/plate) and developed two or three times with 15% isopropanol in chloroform. The lower band contained 45a while the upper band contained 45b. The two bands were separately scraped from the plate, extracted with 20% methanol in chloroform (3 x 25mL) and eluent solvent evaporated under reduced pressure. The desired product (45a) isolated as 0.26g (0.35mmoles) (41% yield) of a white powder, mp 45-46 °C, was obtained from the lower TLC band. $^1$H-NMR($CDCl_3$) δ 1.06-1.28($6H_{a,a'}$,m, rotamers of I); 1.32($3H_b$,d, J = 8Hz); 1.38-2.32($10H_c$,m); 2.46($2H_d$,app.t, J = 8Hz); 2.64($2H_{d'}$,app.t, J = 8Hz); 3.20($2H_e$,m); 3.53-3.88($2H_f$,m); 3.66($3H_g$,s); 4.20,4.32($2H_h$, center of a set of dd, overlapping with an other set, J = 20Hz, rotamers of $CH_{2(h)}$ geminal system); 4.53($1H_i$,app.t, J = 8Hz); 4.63($1H_j$,app.t, J = 8Hz); 4.80($2H_{k,l}$m); 5.42-($1H_m$,m); 6.70-7.10($2H_{n,o}$,m, rotamers of amide -NH); 7.24($1H_p$,app.d, J = 10Hz, rotamers of I); 7.28-($1H_{p'}$,app.d, J = 10Hz, rotamers of I); 7.34($2H_{q,q'}$,app.dd, $J_{qr}$ = 8Hz, $J_{qq'}$ = 2Hz); 7.43($1H_r$,app.dd, $J_{rq}$ = 8Hz, $J_{rs}$ = 2Hz); 7.83($2H_{s,s'}$,app.dd, $J_{sq}$ = 8Hz, $J_{sr}$ = 2Hz); 8.20($1H_t$,app.d, J = 10Hz, rotamers of I); 8.25($1H_{t'}$,app.d, J = 10Hz, rotamers of I). IR ($CHCl_3$) 3400, 1730, 1645, 1525, 1440, 1345, 1215, 750, 665cm$^{-1}$. Elemental Analysis cal'd for $C_{37}H_{48}N_6O_{11}$: C,58.90;H,6.37;N,11.14. Found: C,59.18;H,6.76;N,11.24.

Ex. 27: p-Nitrophenyl N-[Methoxysuccinyl-(Nδ-carbobenzoxy) ornithylalanylprolylmethyl]-N-isopropylcarbamate (46b, the LLD diasetereomer)

46b

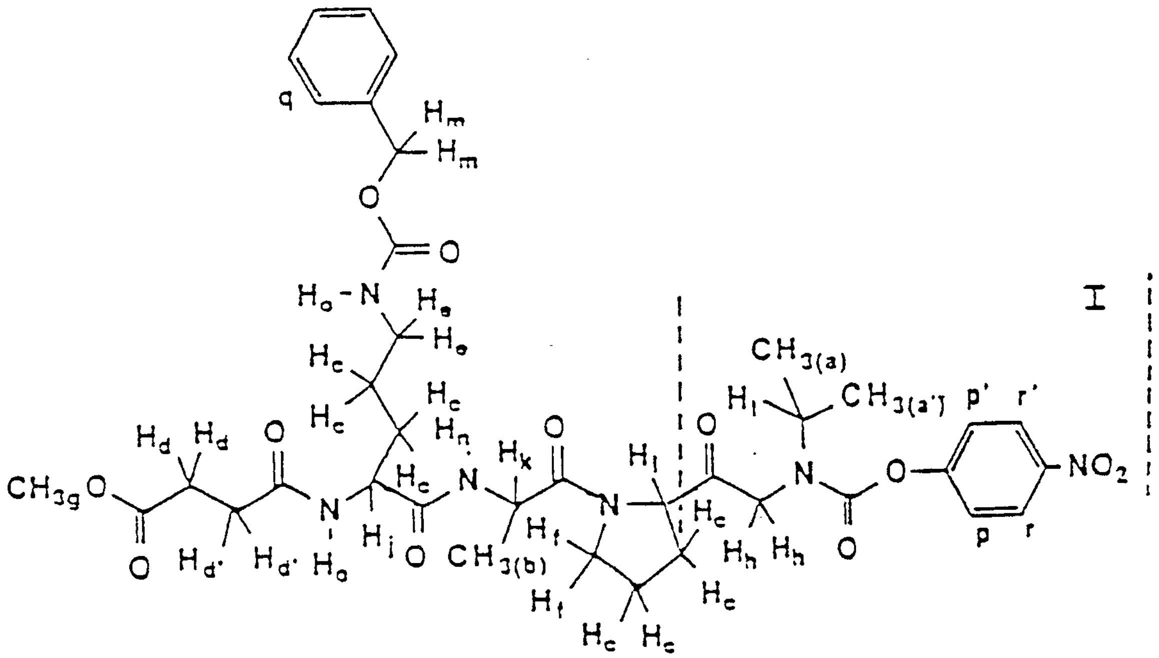

The title compound was prepared from 12 and 22 following a procedure analogous to that described for 44b. The amorphous powder containing 46a and 46b was dissolved in chloroform and applied to a preparative TLC plate (100mg/plate) and developed two or three times with 10% isopropanol in chloroform. The lower band contained 46a while the upper band contained 46b. The two bands were separately scraped from the plate, extracted with 20% methanol in chloroform (3 x 25mL) and eluent solvent evaporated under reduced pressure. The desired product (46b) isolated as 0.08g (0.13mmoles) (15% yield) of a white amorphous powder, mp 50-51 °C, was obtained from the upper TLC band. $^1$H-NMR ($CDCl_3$) $\delta$ 1.13-($3H_a$,app.d, J=7Hz, rotamers of I); 1.20($3H_{a'}$,app.d, J=7Hz, rotamers of I); 1.36($3H_b$,d, J=8Hz); 1.40-2.22-($8H_c$,m); 2.44($2H_d$,app.t, J=8Hz); 2.64($2H_{d'}$,app.t, J=8Hz); 3.18($2H_e$,m); 3.33($2H_f$,app.t, J=8Hz); 3.66($2H_g$,s); 4.30($2H_h$, center of 2 sets of dd, J=20Hz, rotamers of the $CH_{2(h)}$ geminal system); 4.53($1H_i$,app.t, J=8Hz); 4.63($1H_j$,app.t, J=8Hz); 4.80($2H_{k,j}$,m); 5.10($2H_m$,s); 5.42($1H_n$,m); 6.70-7.10($2H_o$,m, rotamers of amide -NH); 7.24($1H_p$,app.d, J=10Hz, rotamers of I); 7.28($1H_{p'}$,app.d, J=10Hz, rotamers of I); 7.34($5H_q$,app.s); 8.20-($1H_r$,app.d, J=10Hz, rotamers of I); 8.25($1H_{r'}$,app.d, J=10Hz, rotamers of I)ppm. IR ($CHCl_3$) 3310, 1730, 1650, 1520, 735, 700cm$^{-1}$. Elemental Analysis cal'd for $C_{37}H_{48}N_6O_{12}$: C,57.80;H,6.25;N,10.94. Found: C,57.42;H,6.36;N,11.82.

Ex. 28: p-Nitrophenyl N-[Methoxysuccinyl-(Nδ-carbobenzoxy)ornithylalanylprolylmethyl]-N-isopropylcarbamate (46a, the LLL diastereomer)

46a

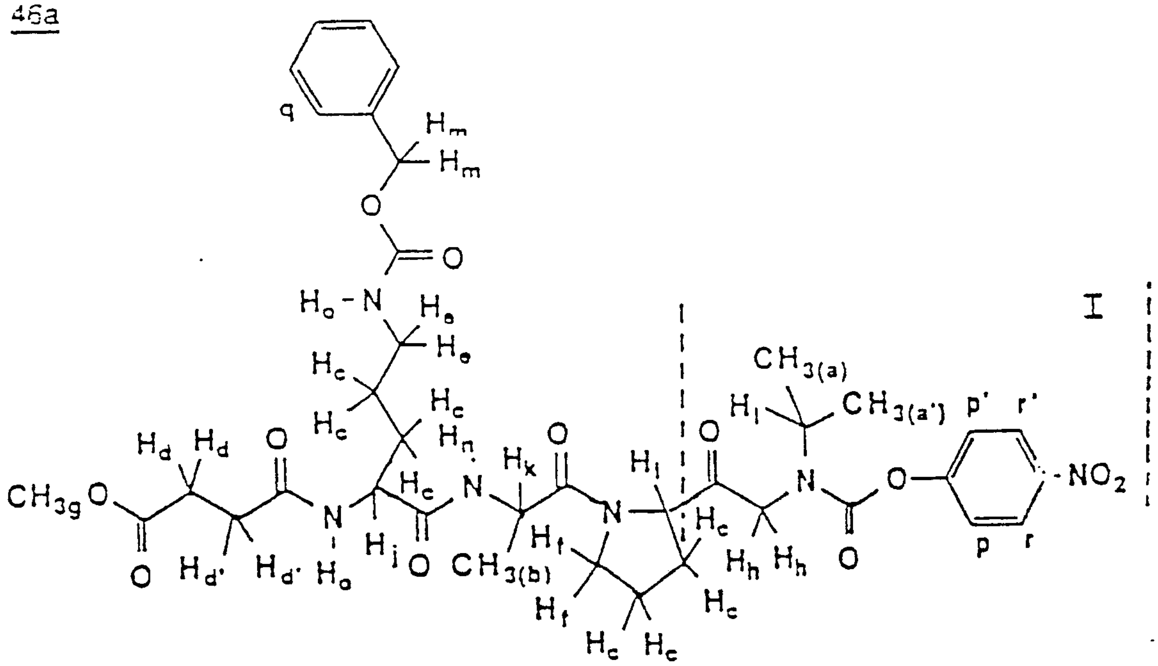

The title compound was prepared from 12 and 22 following a procedure analogous to that described for 44b. The amorphous powder containing 46a and 46b was dissolved in chloroform and applied to a preparative TLC plate (100mg.plate) and developed two or three times with 10% isopropanol in chloroform. The lower band contained 46a while the upper band contained 46b. The two bands were separately scraped from the plate, extracted with 20% methanol in chloroform (3 x 25mL) and eluent solvent evaporated under reduced pressure. The desired product (46a) isolated as 0.31g (0.41mmoles) (48% yield) of a white amorphous powder, mp 55-56 °C, was obtained from the lower TLC band. 'H-NMR ($CDCl_3$) δ 1.13-($3H_a$,app.d, J=7Hz, rotamer of I); 1.20($3H_a'$,app.d, J=7Hz, rotamer of I); 1.36($3H_b$,d, J=8Hz); 1.40-2.22-($8H_c$,m); 2.44($2H_d$,app.t, J=8Hz); 2.64($2H_d'$,app.t, J=8Hz); 3.18($2H_e$,m); 3.33($2H_f$,app.t, J=8Hz); 3.66($2H_g$,s); 4.30($2H_h$, center of 2 sets of dd, J=20Hz, rotamers of the $CH_{2(h)}$ geminal system); 4.53($1H_i$,app.t, J=8Hz); 4.63($1H_j$,app.t, J=8Hz); 4.80($2H_{k,j}$,m); 5.10($2H_m$,s); 5.42($1H_n$,m); 6.70-7.10($2H_o$,m, rotamers of amide -NH); 7.24($1H_p$,app.d, J=10Hz, rotamers of I); 7.28($1H_p'$,app.d, J=10Hz, rotamers of I); 7.34($5H_q$,app.s); 8.20-($1H_r$,app.d, J=10Hz, rotamers of I); 8.25($1H_r'$,app.d, J=10Hz, rotamers of I)ppm. IR ($CHCl_3$) 3310, 1730, 1650, 1520, 735, 700cm$^{-1}$. Elemental Analysis cal'd for $C_{37}H_{48}N_8O_{12}$: C,57.80;H,6.25;N, 10.94. Found: C,57.81;H,6.36;N,10.68.

Ex. 29: p-Nitrophenyl N-[Methoxysuccinyl-(Nδ-benzoyl)ornithylalanylprolylmethyl]-N-isopropylcarbamate (47b, the LLD diasetereomer)

47b

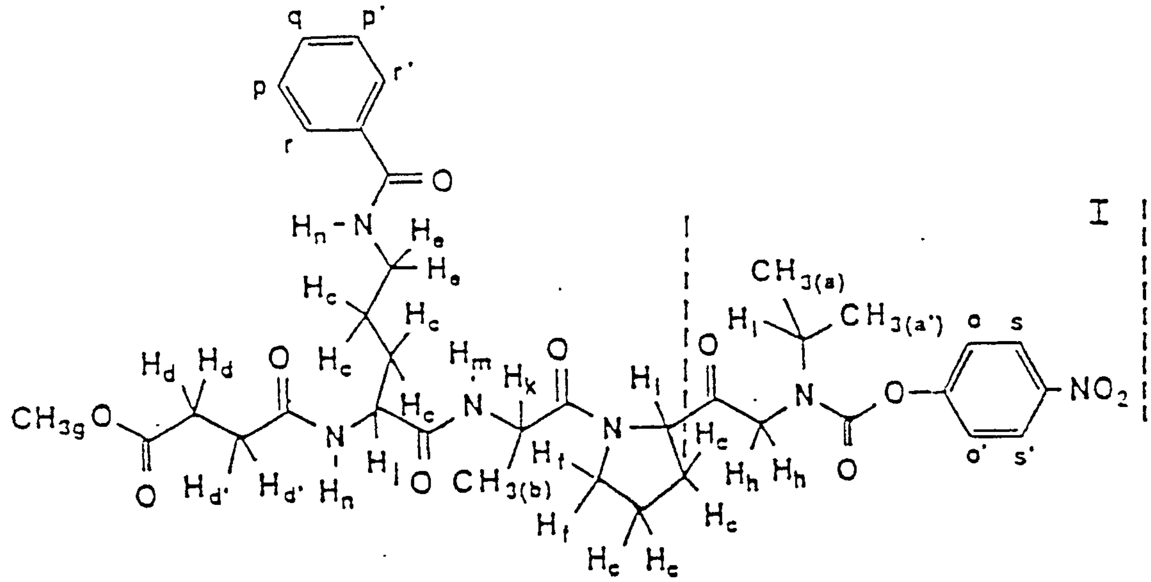

A solution of the diastereomers 46a & 46b (0.16g, 0.2mmole) in glacial acetic acid (0.3mL) was diluted with 30% hydrogen bromide in acetic acid (0.3mL) and stirred at room temperature for 90 min. The reaction was stopped by the addition of dry diethyl ether (10mL) and the suspension decanted 4 times to produce a hygroscopic brown powder. The brown powder was dried under a flow of nitrogen and dissolved in acetonitrile (2mL).

Carbonyldiimidazole (0.90g, 0.6mmole) was added to a stirred solution of benzoic acid (0.07g, 0.6mmole) in acetonitrile (1mL), and stirring continued for 10 min at room temperature. This solution was then added dropwise to a solution N-methylmorpholine (0.08g, 0.8mmole) and the hydrobromide salt described above. The mixture was stirred for 24h at room temperature. The reaction mixture was evaporated under vacuum and the residue was dissolved in methylene chloride (20mL). The solution was washed with water (20mL), 10% aqueous citric acid solution (20mL), water (20mL), brine (20mL), dried (2g of $MgSO_4$) and evaporated under reduced pressure. The residue, containing 47a and 47b, was applied to a preparative TLC plate and developed two or three times with 15% isopropanol in chloroform. The lower band contained 47a while the upper band contained 47b. The two bands were separately scraped from the plate, extracted with 20% methanol in chloroform (3 x 25mL) and, eluent solvent evaporated under reduced pressure. The desired product (47b) isolated as 0.04g (0.05mmoles) (25% yield) of a white amorphous powder, mp 65-66 ° C, was obtained from the upper TLC band. $^1$H-NMR ($CDCl_3$) δ 1.13(3$H_a$,app.d, J = 7Hz, rotamers of I); 1.20(3$H_a{'}$,app.d, J = 7Hz, rotamers of I); 1.36(3$H_b$,d, J = 8Hz); 1.40-2.22(8$H_c$,m); 2.44-(2$H_d$,app.t, J = 8Hz); 2.64(2$H_d{'}$,app.t, J = 8Hz); 3.18(2$H_e$,m); 3.33(2$H_f$,app.t, J = 8Hz); 3.66(2$H_g$,s); 4.30(2$H_h$, center of 2 sets of dd, J = 20Hz, rotamers of the $CH_{2(h)}$ geminal system); 4.53(1$H_i$,app.t,J = 8Hz); 4.63-(1$H_j$,app.t, J = 8Hz); 4.80(2$H_{k,l}$,m); 5.42(1$H_m$,m); 6.70-7.10(2$H_n$,m, rotamers of amide -NH); 7.24(1$H_o$,app.d, J = 10Hz, rotamers of I); 7.28(1$H_o{'}$,app.d, J = 10Hz, rotamers of I); 7.34(2$H_{p,p}{'}$,app.dd, $J_{qp}$ = 8Hz, $J_{qr}$ = 2Hz); 7.43(1$H_q$,app.dd, $J_{qp}$ = 8Hz, $J_{qr}$ = 2Hz); 7.83(2$H_{r,r}{'}$,app.dd, $J_{rp}$ = 8Hz, $J_{rq}$ = 2Hz); 8.20(1$H_s$,app.d, J = 10Hz, rotamers of I); 8.25(1$H_s{'}$,app.d, J = 10Hz, rotamers of I)ppm. IR ($CHCl_3$) 3400, 1730, 1645, 1525, 1440, 1345, 1215, 750, 665cm$^{-1}$. Elemental Analysis cal'd for $C_{36}H_{46}N_6O_{11}$: C,58.50;H,6.20;N,11.38. Found: C,58.53;H,6.37;N,11.31.

Ex. 30: p-Nitrophenyl N-[Methoxysuccinyl-(Nδ-benzoyl)ornithylalanylprolylmethyl]-N-isopropylcarbamate (47a, the LLL-diasetereomer)

47a

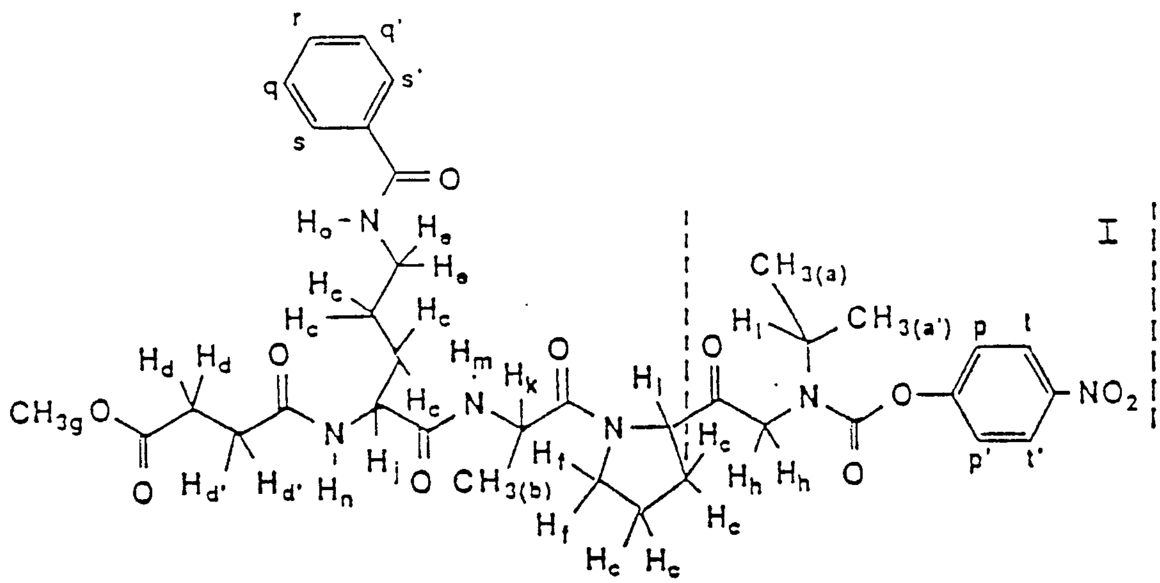

The title compound was prepared from 46a and 46b following a procedure analogous to that described for 47b. The residue, containing 47a and 47b, was applied to a preparative TLC plate and developed two or three times with 15% isopropanol in chloroform. The lower band contained 47a while the upper band contained 47b. The two bands were separately scraped from the plate, extracted with 20% methanol in chloroform (3 x 25mL) and eluent solvent evaporated under reduced pressure. The desired product (47a) isolated as 0.06g (0.09mmoles) (43% yield) of a white amorphous powder, mp 55-56 °C, was obtained from the lower TLC band. $^1$H-NMR($CDCl_3$) $\delta$ 1.06-1.28(6$H_{a,a'}$,m, rotamer of I); 1.32(3$H_b$,d, J=8Hz); 1.38-2.32-(8$H_c$,m); 2.46(2$H_d$,app.t, J=8Hz); 2.64(2$H_{d'}$,app.t, J=8Hz); 3.20(2$H_e$,m); 3.53-3.88(2$H_f$,m); 3.66(3$H_g$,s); 4.20,4.32(2$H_h$, center of a set of dd, overlapping with an other set, J=20Hz, rotamers of $CH_{2(h)}$ geminal system); 4.53(1$H_i$,app.t, J=8Hz); 4.63(1$H_j$,app.t, J=8Hz); 4.80(2$H_{k,l}$,m); 5.42(1$H_m$,m); 6.70-7.10(2$H_{n,o}$,m, rotamers of amide -NH); 7.24(1$H_p$,app.d, J=10Hz, rotamers of I); 7.28(1$H_{p'}$,app.d, J=10Hz, rotamers of I); 7.34(2$H_{q,q'}$,app.dd, $J_{qr}$=8Hz, $J_{qq'}$=2Hz); 7.43(1$H_r$,app.dd, $J_{rq}$=8Hz, $J_{rs}$=2Hz); 7.83(2$H_{s,s'}$,app.dd, $J_{sq}$=8Hz, $J_{sr}$=2Hz); 8.20(1$H_t$,app.d, J=10Hz, rotamers of I); 8.25(1$H_{t'}$,app.d, J=10Hz, rotamers of I) ppm. IR ($CHCl_3$) 3400, 1730, 1645, 1525, 1440, 1345, 1215, 750, 665$cm^{-1}$. Elemental Analysis cal'd for $C_{36}H_{46}N_6O_{11}$: C,58.50; H,6.20;N,11.38. Found: C,58.54; H,6.36;N,11.18.

Ex. 31: p-Nitrophenyl N-[Methoxysuccinylalanyl-($N_\epsilon$-carbobenzoxy)lysylprolylmethyl]-N-isopropylcarbamate (48b, the LLD diastereomer)

48b

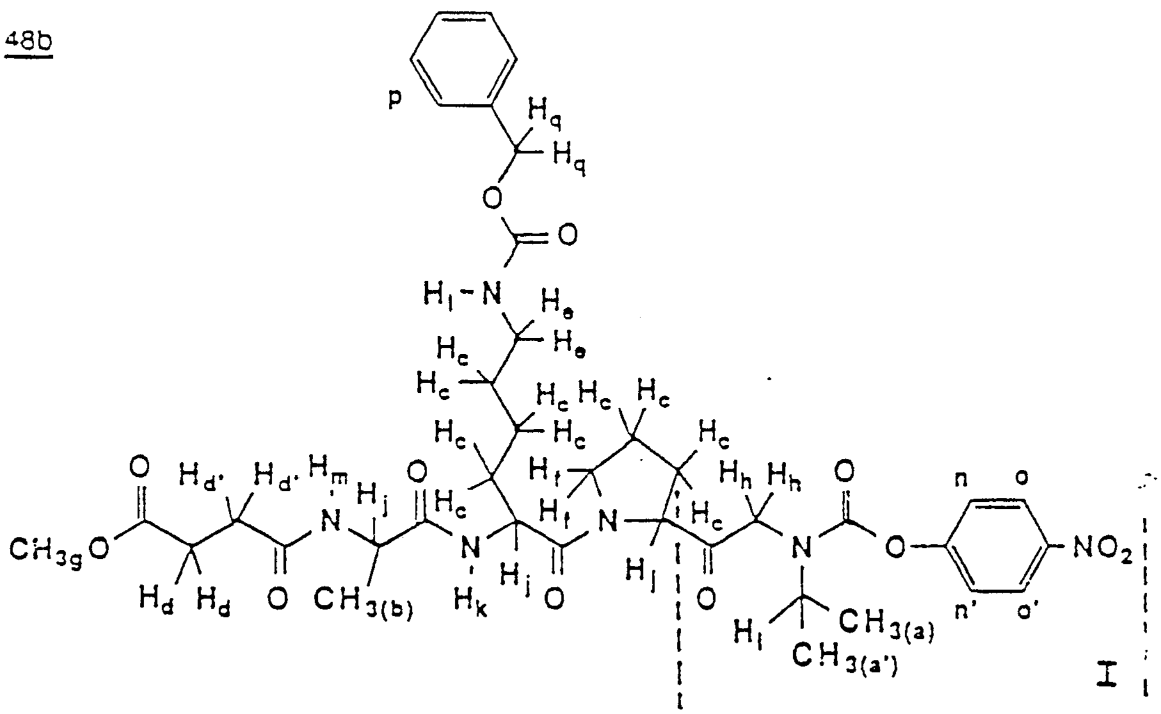

The title compound was prepared from 7 and 22 following a procedure analogous to that described for 44b. The amorphous powder contining 48a and 48b was dissolved in chloroform and applied to a preparative TLC plate (100mg/plate) and developed two or three times with 4% methanol in ethyl acetate. The upper band contained 48a while the lower band contained 48b. The two bands were separately scraped from the plate, extracted with 20% methanol in chloroform (3 x 25mL) and eluent solvent evaporated under reduced pressure. The desired product (48b) isolated as 0.24g (0.31mmoles) (36% yield) of a white amorphous powder, mp 44-45° C, was obtained from the upper TLC band. $^1$H-NMR ($CDCl_3$) δ 1.13(3$H_a$′, app.d, J=7Hz, rotamer of I); 1.20(3$H_a$′, app.d, J=7Hz, rotamer of I); 1.36(3$H_b$,d, J=8Hz); 1.40-2.22-(10$H_c$,m); 2.44(2$H_d$, app.t, J=8Hz); 2.64(2$H_d$′, app.t, J=8Hz); 3.18(2$H_e$,m); 3.56-3.84(2$H_f$,m); 3.66(3$H_g$,s); 4,23(2$H_h$, center of 2 sets of dd, J=20Hz, rotamers of the $CH_{2(h)}$ geminal system); 4,34-4.58(4$H_{i,j}$,m); 4.60-4.64(1$H_k$,m); 5.10(2$H_q$,s); 6.34-7.16(2$H_{l,m}$,m, rotamer of amide -NH); 7.22(1$H_n$, app.d, J=10Hz, rotamers of I); 7.26(1$H_n$′, app.d, J=10Hz, rotamers of I); 8.22(1$H_o$, app.d, J=10Hz, rotamers of I); 8.24(1$H_o$′,app.d, J=10Hz, rotamers of I); 7.34(5$H_p$,app.s)ppm. IR ($CHCl_3$) 3305, 1720, 1645, 1520, 735, 700cm$^{-1}$. Elemental Analysis cal'd for $C_{38}H_{50}N_6O_{12}$: C,58.30;H,6.40; N,10.70. Found: C,58.49;H,6.47;N,10.63.

Ex. 32: p-Nitrophenyl N-[Methoxysuccinyl-(Nδ-carbobenzoxy)lysylprolylmethyl)-N-isopropylcarbamate (48a, the LLL diastereomer)

48a

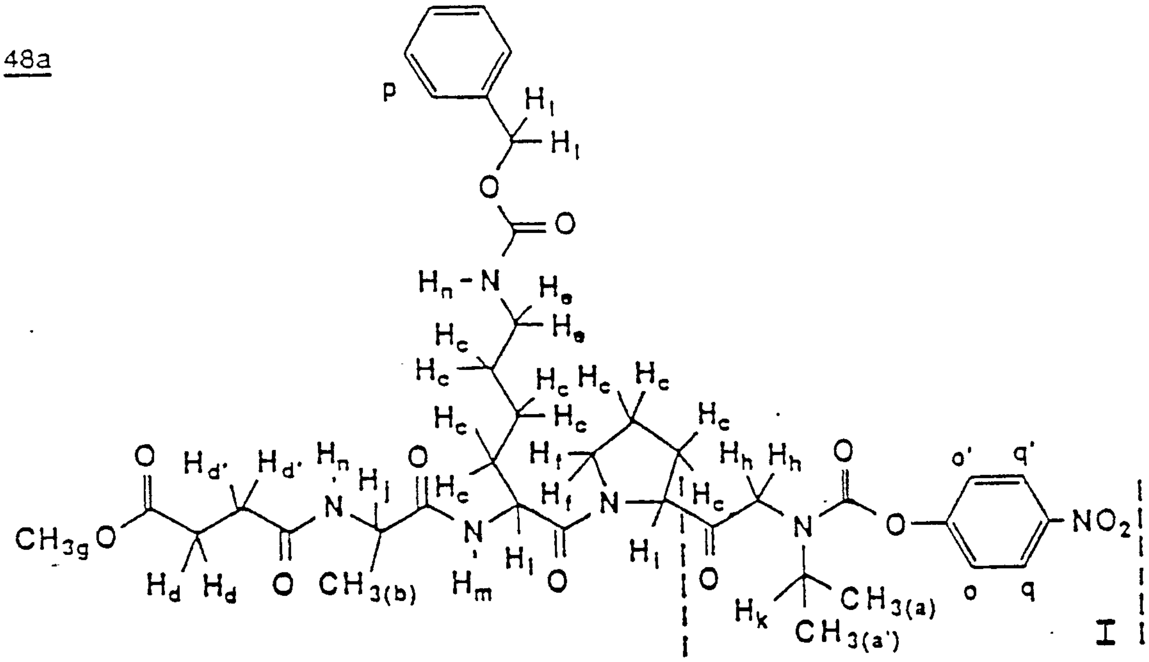

The title compound was prepared from 7 and 22 following a procedure analogous to that described for 44b. The amorphous powder containing 48a and 48b was dissolved in chloroform and applied to a preparative TLC plate (100mg/plate) and developed two or three times with 4% methanol in chloroform. The upper band contained 48a while the lower band contained 48b. The two bands were separately scraped from the plate, extracted with 20% methanol in chloroform (3 x 25mL) and eluent solvent evaporated under reduced pressure. The desired product (48a) isolated as 0.36 (0.46mmoles) (54% yield) of a white amorphous powder, mp56-57° C, was obtained from the upper TLC band. $^1$H-NMR ($CDCl_3$) $\delta$ 1.06-1.28-($6H_{a,a'}$,m, rotamers of I); 1.32($3H_b$,d, J=8Hz); 1.38-2.32($10H_c$,m); 2.46($2H_d$,app.t, J=8Hz); 2.64($2H_{d'}$,app.t, J=8Hz); 3.20($2H_e$,m); 3.52-3.88($2H_f$,m); 3.66($3H_g$,s); 4.20,4.32($2H_h$, center of a set of dd, overlapping with another set, J=20Hz, rotamers of the $CH_{2(h)}$ geminal system); 4.50($2H_i$, app.t, J=8Hz); 4.50-4.80($2H_{j,k}$,m); 5.10($2H_l$,s); 5.42($1H_m$,m); 6.36-7.08($2H_n$,m, rotamers of amide -NH); 7.24($1H_o$,app.d, J=10Hz, rotamers of I); 7.28($1H_{o'}$,app.d, J=10Hz, rotamers of I); 7.35($5H_p$, app.s); 8.20($1H_q$, app. d, J=10Hz, rotamers of I); 8.25-($1H_{q'}$, app.d, J=10Hz, rotamers of I) ppm. IR ($CHCl_3$) 3305, 1720, 1645, 1520, 735, 700cm$^{-1}$. Elemental Analysis cal'd for $C_{38}H_{50}N_6O_{12}$: C,58.30; H,6.40;N,10.70. Found: C,58.12; H,6.55; N,10.64.

Ex. 33: p-Nitrophenyl N-[Methoxysuccinyl)alanyl-($N_\epsilon$-benzoyl)lysylprolylmethyl]-N-isopropylcarbamate (49b, the LLD diasetereomer)

49b

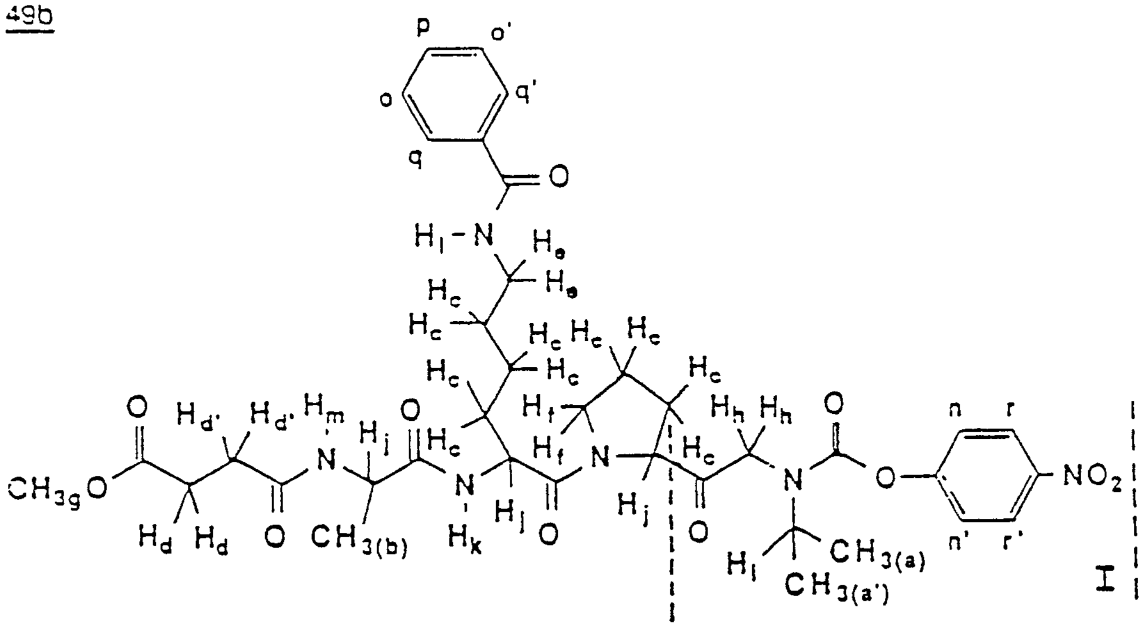

The title compound was prepared from 5 and 22 following a procedure analogous to that described for 44b. The amorphous powder containing 49a and 49b was dissolved in chloroform and applied to a preparative TLC plate (100mg.plate) and developed two or three times with 15% isopropanol in chloroform. The upper band contained 49a while the lower band contained 49b. The two bands were separately scraped from the plate, extracted with 20% methanol in chloroform (3 x 25mL) and eluent solvent evaporated under reduced pressure. The desired product (49b) isolated as 0.29g (0.39mmoles) (46% yield) of a white amorphous powder, mp 54-55° C, was obtained from the lower TLC band. $^1$H-NMR ($CDCl_3$) $\delta$ 1.13(3$H_a'$, app.d, J=7Hz, rotamer of I); 1.20(3$H_a'$, app.d, J=7Hz, rotamer of I); 1.36(3$H_b$,d, J=8Hz); 1.40-2.22-(10$H_c$,m); 2.44(2$H_d$, app.t, J=8Hz); 2.64(2$H_{d'}$, app.t, J=8Hz); 3.18(2$H_e$,m); 3.56-3.84(2$H_f$,m); 3.66(3$H_g$,s); 4.23(2$H_h$, center of 2 sets of dd, J=20Hz, rotamers of the $CH_{2(h)}$ geminal system); 4,34-4.58(4$H_{i,j}$,m); 4.60-4.64(1$H_k$,m); 6.34-7.16(2$H_{l,m}$,m, rotamer of amide -NH); 7.22(1$H_n$, app.d, J=10Hz, rotamers of I); 7.26(1$H_n'$, app.d, J=10Hz, rotamers of I); 7.34(2$H_{o,o}'$,app.dd, $J_{oq}$=8Hz, $J_{oo'}$=2Hz); 7.43(1$H_p$,app.d, $J_{po}$=8Hz); 7.83-(2$H_{q,q}'$,app.dd, $J_{qo}$=8Hz, $J_{qp}$=2Hz); 8.22(1$H_r$, app.d, J=10Hz, rotamers of I); 8.24(1$H_r'$,app.d, J=10Hz, rotamers of I) ppm. IR ($CHCl_3$) 3340, 1735, 1645, 1520, 1435, 1345, 1260, 735, 700$cm^{-1}$. Elemental Analysis cal'd for $C_{37}H_{48}N_6O_{11}$: C,58.92;H,6.37;N,11.14. Found: C,58.56; H,6.47; N,11.30.

Ex. 34: p-Nitrophenyl N-[Methoxysuccinyl)alanyl-(N$\epsilon$-benzoyl)lysylprolylmethyl]-N-isopropylcarbamate (49a, the LLL diastereomer)

49a

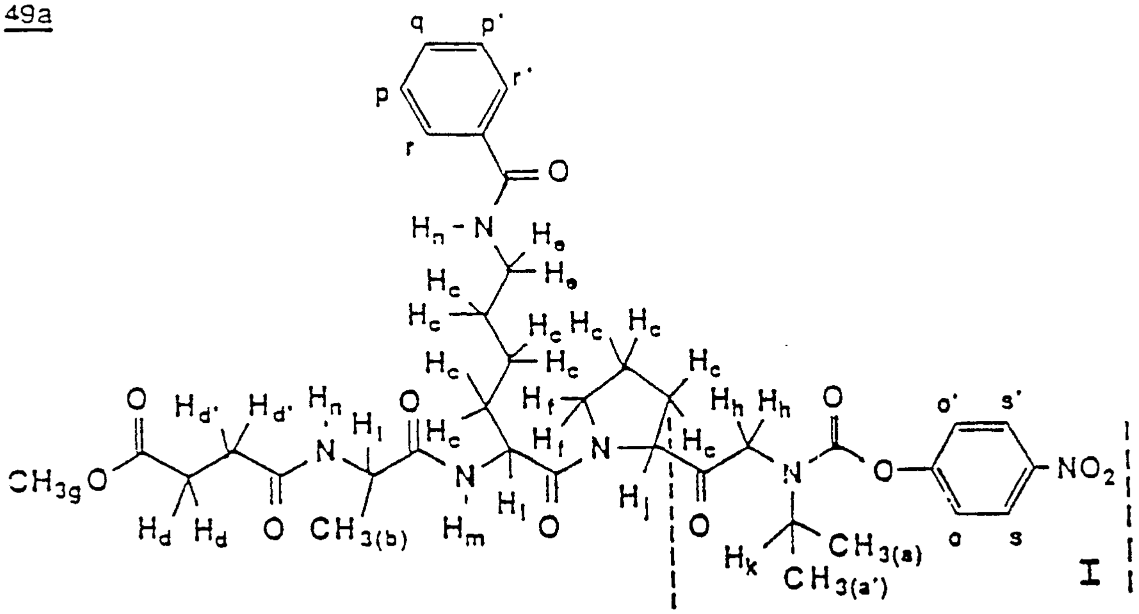

The title compound was prepared from 5 and 22 following a procedure analogous to that described for 44b. The amorphous powder containing 49a and 49b was dissolved in chloroform and applied to a preparative TLC plate (100mg/plate) and developed two or three times with 15% isopropanol in chloroform. The upper band contained 49a while the lower band contained 49b. The two bands were separately scraped from the plate, extracted with 20% methanol in chloroform (3 x 25mL) and eluent solvent evaporated under reduced pressure. The desired product (49a) isolated as 0.29g (0.38mmoles) (45% yield) of a white amorphous powder, mp 61-62° C, was obtained from the upper TLC band. $^1$H-NMR ($CDCl_3$) δ 1.06-1.28-($6H_{a,a'}$,m, rotamers of I); 1.32($3H_b$,d, J=8Hz); 1.38-2.32($10H_c$m); 2.46($2H_d$,app.t, J=8Hz); 2.64($2H_d'$,app.t, J=8Hz); 3.20($2H_e$,m); 3.52-3.88($2H_f$,m); 3.66($3H_g$,s); 4.20,4.32($2H_h$, center of a set of dd, overlapping with another set, J=20Hz, rotamers of the $CH_{2(h)}$ geminal system); 4.50($2H_{i,j}$, app.t, J=8Hz); 4.50-4.80($2H_{k,l}$,m); 5.42($1H_m$,m); 6.36-7.08($2H_n$,m, rotamers of amide -NH); 7.24($1H_o$,app.d, J=10Hz, rotamers of I); 7.28-($1H_o'$,app.d, J=10Hz, rotamers of I); 7.34($2H_{p,p}'$,app.dd, $J_{pr}$=8Hz,$J_{pp}'$=2Hz); 7.43($1H_q$,app.d, $J_{qp}$=8Hz, $J_{qr}$=2Hz); 7.83($2H_{r,r}'$,app.dd, $J_{rp}$=8Hz, $J_{rq}$=2Hz); 8.20($1H_s$, app. d, J=10Hz, rotamers of I); 8.25($1H_s'$,app.d, J=10Hz, rotamers of I) ppm. IR ($CHCl_3$) 3340, 1730, 1645, 1520, 1435, 1345, 1260, 735, 700cm$^{-1}$. Elemental Analysis cal'd for $C_{37}H_{48}N_6O_{11}$: C,58.92; H,6.37; N,11.14. Found: C,58.80; H,6.46; N,11.28.

Ex. 35: p-Nitrophenyl N-[Methoxysuccinylalanyl-(Nδ-carbobenzoxy)ornithylprolylmethyl]-N-isopropylcarbamate (50b, the LLD diastereomer)

50b

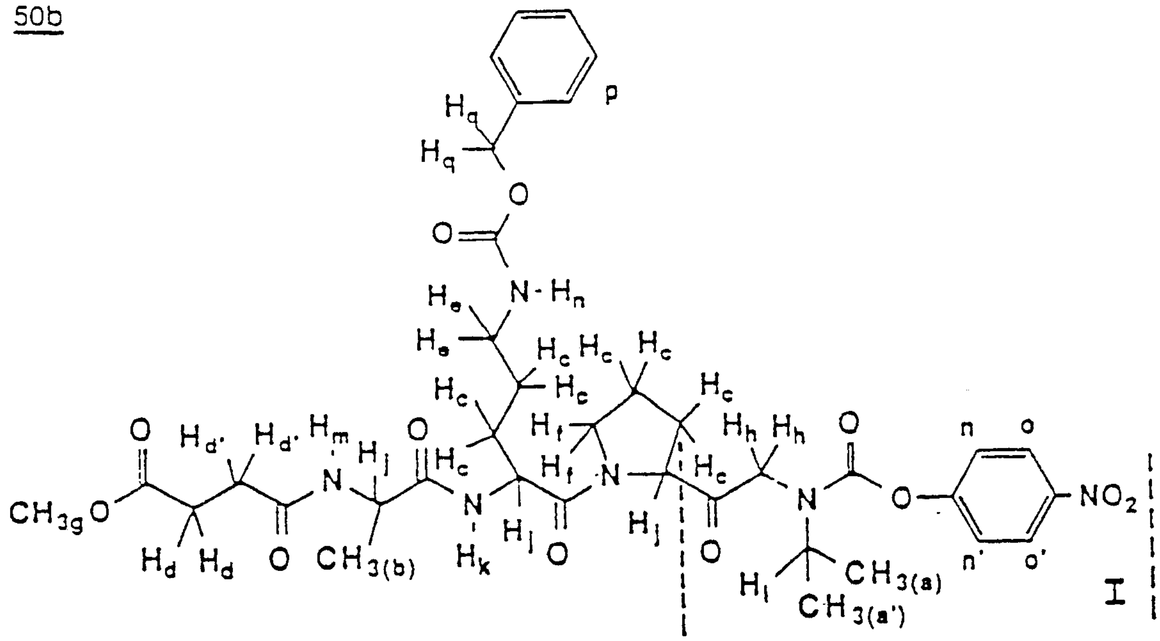

The title compound was prepared from 9 and 22 following a procedure analogous to that described for 44b. The amorphous powder containing 50a and 50b was dissolved in chloroform and applied to a preparative TLC plate (100mg/plate) and developed two or three times with 4% methanol in ethyl acetate. The upper band contained 50a while the lower band 50b. The two bands were separately scraped from the plate, extracted with 20% methanol in chloroform (3 x 25mL) and eluent solvent evaporated under reduced pressure. The desired product (50b) isolated as 0.1g (0.13mmoles) (15% yield) of a white amorphous powder, mp65-68° C, was obtained from the lower TLC band. $^1$H-NMR ($CDCl_3$) $\delta$ 1.13($3H_a{}'$, app.d, J = 7Hz, rotamer of I); 1.20($3H_a{}'$, app.d, J = 7Hz, rotamer of I); 1.36($3H_b$,d, J = 8Hz); 1.40-2.22($8H_c$,m); 2.44($2H_d$, app.t, J = 8Hz); 2.64($2H_d{}'$, app.t, J = 8Hz); 3.18($2H_e$,m); 3.56-3.84($2H_f$,m); 3.66($3H_g$,s); 4,23($2H_h$, center of 2 sets of dd, J = 20Hz, rotamers of the $CH_{2(h)}$ geminal system); 4,34-4.58($4H_{i,j}$,m); 4.60-4.64($1H_k$,m); 5.10($2H_q$,s); 6.34-7.16($2H_{l,m}$,m, rotamer of amide -NH); 7.22($1H_n$, app.d, J = 10Hz, rotamers of I); 7.26($1H_n{}'$, app.d, J = 10Hz, rotamers of I); 8.22($1H_o$, app.d, J = 10Hz, rotamers of I); 8.24($1H_o{}'$,app.d, J = 10Hz, rotamers of I); 7.34-($5H_p$,app.s) ppm. IR ($CHCl_3$) 3305, 1720, 1645, 1520, 735, 700cm$^{-1}$. Elemental Analysis cal'd for $C_{37}G_{48}N_6O_{12}$: C,57.80; H,6.25; N,10.94. Found: C,58.12; H,6.55; N,10.64.

Ex. 36: p-Nitrophenyl N-[Methoxysuccinylalanyl-(N$\delta$-carbobenzoxy)ornithylprolylmethyl]-N-isopropylcarbamate (50a, the LLL diastereomer)

50a

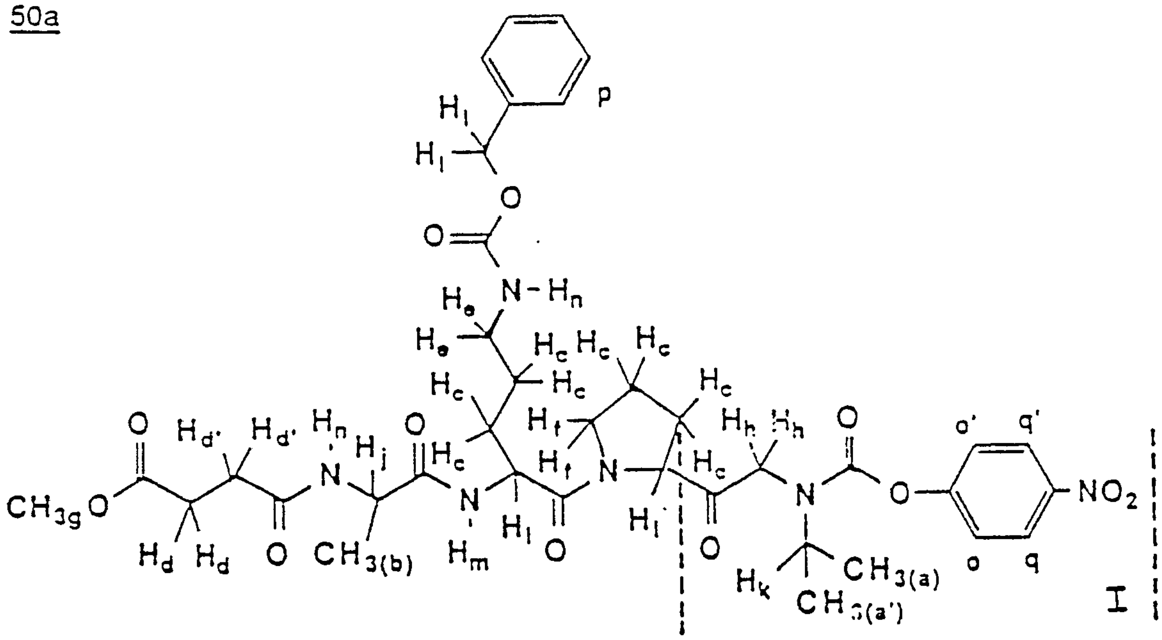

The title compound was prepared from 9 and 22 following a procedure analogous to that described for 44b. The amcrphous powder containing 50a and 50b was dissolved in chloroform and applied to a preparative TLC plate (100mg/plate) and developed two or three times with 4% methanol in ethyl acetate. The upper band contained 50a while the lower band contained 50b. The two bands were separately scraped from the plate, extracted with 20% methanol in chloroform (3 x 25mL) and eluent solvent evaporated under reduced pressure. The desired product (50a) isolated as 0.11g (0.14mmoles) (16% yield) of a white amorphous powder, mp55-56° C, was obtained from the upper TLC band. $^1$H-NMR ($CDCl_3$) $\delta$ 1.06-1.28-($6H_{a,a}'$,m, rotamers of I); 1.32($3H_b$,d, J=8Hz); 1.38-2.32($8H_c$,m); 2.46($2H_d$,app.t, J=8Hz); 2.64($2H_d'$,app.t, J=8Hz); 3.20($2H_e$,m); 3.52-3.88($2H_f$,m); 3.66($3H_g$,s) 4.20,4.32($2H_h$, center of a set of dd, overlapping with another set, J=20Hz, rotamers of the $CH_{2(h)}$ geminal system); 4.50($2H_i$, app.t,J=8Hz); 4.50-4.80($2H_{j,k}$,m); 5.10($2H_l$,s); 5.42($1H_m$,m); 6.36-7.08($2H_n$,m, rotamers of amide -NH); 7.24($1H_o$,app.d, J=10Hz, rotamers of I); 7.28($1H_o'$,app.d, J=10Hz, rotamers of I); 7.35($5H_p$, app.s); 8.20($1H_q$, app. d, J=10Hz, rotamers of I); 8.25-($1H_q'$, app.d, J=10Hz, rotamers of I) ppm. IR ($CHCl_3$) 3305, 1720, 1645, 1520, 735, 700. Elemental Analysis cal'd for $C_{37}G_{48}N_8O_{12}$: C,57.80; H,6.25; N,10.94. Found: C,57.68: H,6.39; N,10.89.

Ex. 37: p-Nitrophenyl N-[Methoxysuccinylalanyl-(Nδ-benzoyl)ornithylprolylmethyl]-N-isopropylcarbamate (51b, the LLD diastereomer)

51b

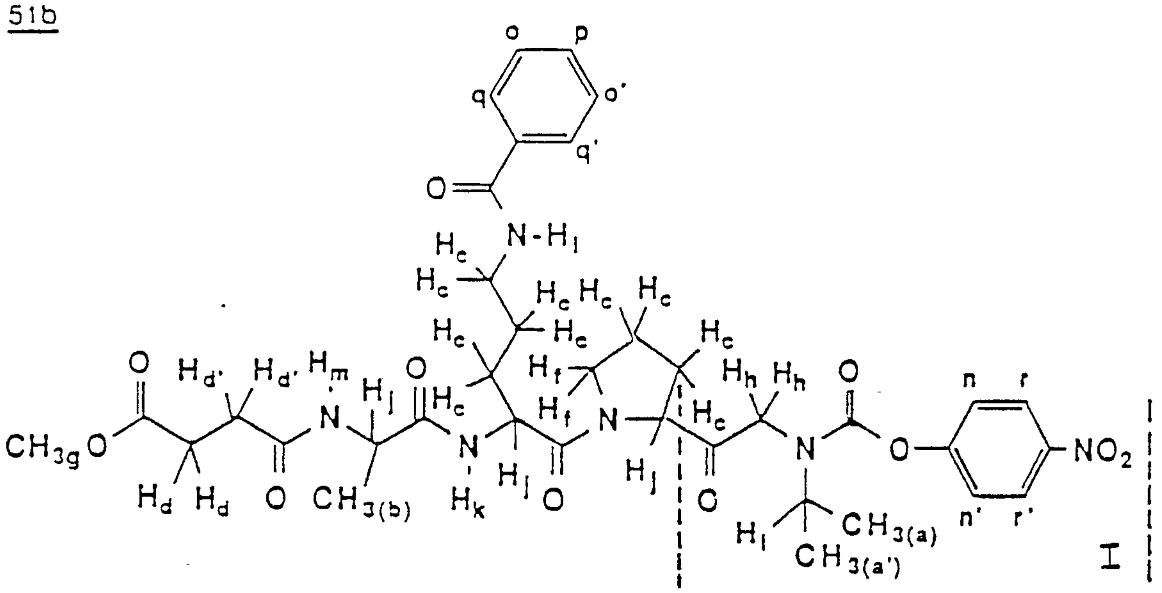

The title compound was prepared from 50a and 50b following a procedure analogous to that described for 47b. The residue, containing 51a and 51b, was applied to a preparative TLC plate and developed two or three times with 4% methanol in ethyl acetate. The upper band contained 51a while the lower band contained 51b. The two bands were separately scraped from the plate, extracted with 20% methanol in chloroform (3 x 25L) and eluent solvent evaporated under reduced pressure. The desired product (51b) isolated as 0.04g (0.05mmoles) (26% yield) of a white amorphous powder, mp 54-55° C, was obtained from the lower TLC band. $^1$H-NMR ($CDCl_3$) δ 1.13(3$H_{a}'$, app.d, J = 7Hz, rotamer of I); 1.20(3$H_{a}'$, app.d, J = 7Hz, rotamer of I); 1.36(3$H_b$,d, J = 8Hz); 1.40-2.22(8$H_c$,m); 2.44(2$H_d$, app.t, J = 8Hz); 2.64(2$H_{d}'$, app.t, J = 8Hz); 3.18(2$H_e$,m); 3.56-3.84(2$H_f$,m); 3.66(3$H_g$,s); 4,23(2$H_h$, center of 2 sets of dd, J = 20Hz, rotamers of the $CH_{2(h)}$ geminal systèm); 4.34-4.58(4$H_{i,j}$,m); 4.60-4.64(1$H_k$,m); 6.34-7.16(2$H_{l,m}$,m, rotamer of amide -NH); 7.22(1$H_n$, app.d, J = 10Hz, rotamers of I); 7.26(1$H_{n}'$, app.d, J = 10Hz, rotamers of I); 7.34(2$H_{o,o}'$,app.dd, $J_{oq}$ = 8Hz, $J_{oo'}$ = 2Hz); 7.43(1$H_p$,app.d, $J_{po}$ = 8Hz); 7.83(2$H_{q,q}'$,app.dd, $J_{qo}$ = 8Hz, $J_{qp}$ = 2Hz); 8.22(1$H_r$, app.d, J = 10Hz, rotamers of I); 8.24(1$H_{r}'$,app.d, J = 10Hz, rotamers of I) ppm. IR ($CHCl_3$) 3340, 1730, 1645, 1520, 1435, 1345, 1260, 735, 700cm$^{-1}$. Elemental Analysis cal'd for $C_{36}H_{46}N_6O_{11}$: C,58.50;H,6.20; N,11.38. Found: C,58.43; H,6.29 N,11.24.

Ex. 38: p-Nitrophenyl N-[Methoxysuccinylalanyl-(Nδ-benzoyl)ornithylprolylmethyl]-N-isopropylcarbamate (51a, the LLL diastereomer)

51a

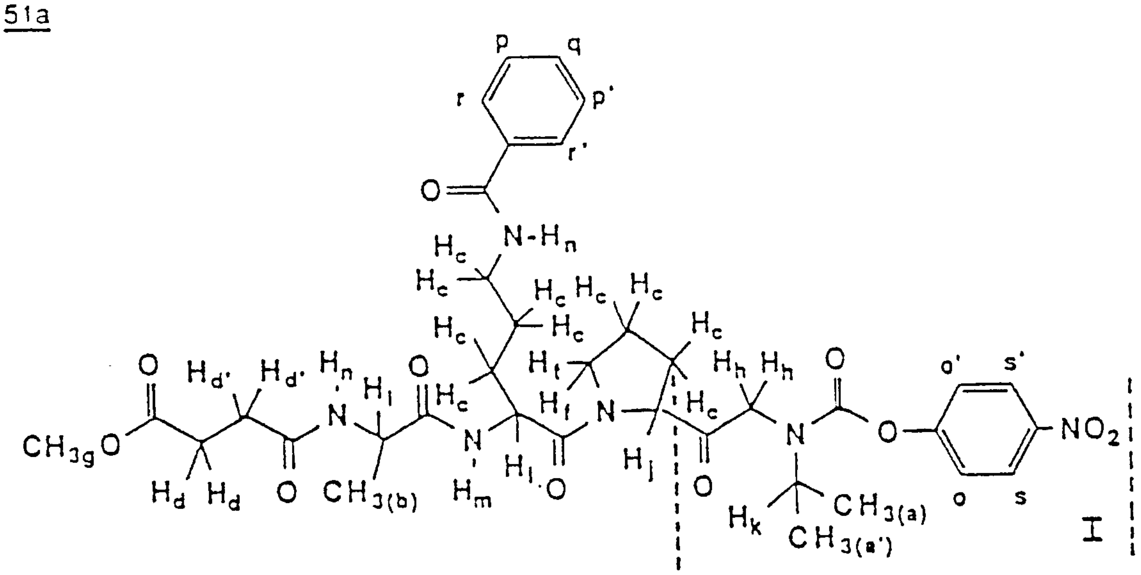

The title compound was prepared from 50a and 50b following a procedure analogous to that described for 47b. The residue containing 51a and 51b, was applied to a preparative TLC plate and developed two or three times with 4% methanol in ethyl acetate. The upper band contained 51a while the lower band contained 51b. The two bands were separately scraped from the plate, extracted with 20% methanol in chloroform (3 x 25mL) and eluent solvent evaporated under reduced pressure. The desired product (51a) isolated as 0.05g (0.06mmoles) (32% yield) of a white amorphous powder, mp 55-56 °C, was obtained from the upper TLC band. ¹H-NMR ($CDCl_3$) δ 1.06-1.28(6$H_{a,a'}$,m, rotamers of I); 1.32(3$H_b$,d, J=8Hz); 1.38-2.32-(8$H_c$,m); 2.46(2$H_d$,app.t, J=8Hz); 2.64(2$H_{d'}$,app.t,J=8Hz); 3.20(2$H_e$,m); 3.52-3.88(2$H_f$,m); 3.66(3$H_g$,s); 4.20,4.32(2$H_h$, center of a set of dd, overlapping with another set, J=20Hz, rotamers of the $CH_{2(h)}$ geminal system); 4.50(2$H_{i,j}$, app.t, J=8Hz); 4.50-4.80(2$H_{k,l}$,m); 5.42(1$H_m$,m); 6.36-7.08(2$H_n$,m, rotamers of amide -NH); 7.24(1$H_o$,app.d, J=10Hz, rotamers of I); 7.28(1$H_{o'}$,app.d, J=10Hz, rotamers of I); 7.34(2$H_{p,p'}$,app.dd, $J_{pr}$=8Hz, $J_{pp'}$=2Hz); 7.43(1$H_q$,app.d, $J_{qr}$=2Hz); 7.83(2$H_{r,r'}$,app.dd, $J_{rp}$=8Hz, $J_{rq}$=2Hz); 8.20(1$H_s$, app. d, J=10Hz, rotamers of I); 8.25 (1$H_{s'}$,app.d, J=10Hz, rotamers of I) ppm. IR ($CHCl_3$) 3340, 1730, 1645, 1520, 1435, 1345, 1260, 735, 700cm⁻¹. Elemental Analysis cal'd for $C_{36}H_{46}H_6O_{11}$: C,58.50; H,6.20; N,11.38. Found C,58.38; H,6.36; N,11.38.

2. Stereospecific synthesis of desmosine-like peptidyl carbamate 48a & 48b

Ex. 39: $N_\alpha$-Methoxysuccinylalanyl-$N_\epsilon$-carbobenzoxylysylproline phenacyl ester (23, the LLD diastereomer)

23

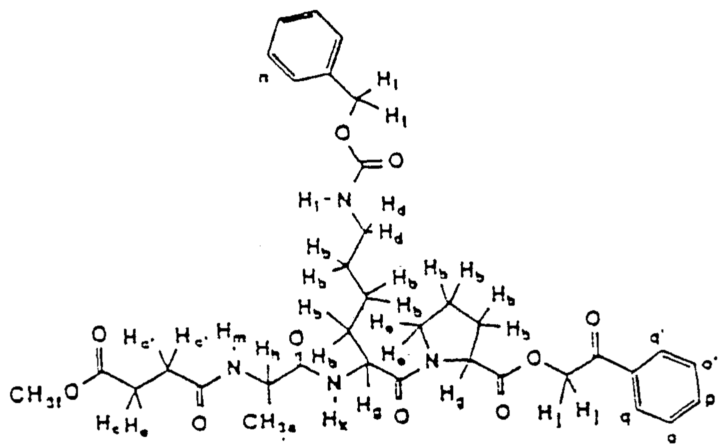

Procedure 1:

A concentrated solution of N,N′-dicyclohexylcarbodiimide (0.17g, 0.8mmole) in THF was added dropwise to an ice cooled solution containing 7 (0.4g, 0.8mmole) and N-hydroxysuccinimide (0.1g, 0.8mmole) in THF (4mL). The solution was stirred at 5° C for 14h and the precipitated urea formed was filtered under vacuum. The filtrate was immediately used in the next reaction without further work-up.

A mixture of the above cooled (0° C) solution and D-proline phenacyl ester hydrochloride (41) (0.22g, 0.8mmole) was stirred and triethylamine (0.08g, 0.8mmole) in THF (0.5mL) was added dropwise. Upon completion of the reaction (5h), the precipitate was filtered, washed with ethyl acetate and the filtrate evaporated under vacuum. The residue, containing the product, was chromatographed on 20g of silica gel column (1 x 50cm). Impurities were eliminated by first passing methylene chloride and subsequently the compound was eluted with 4% methanol in methylene chloride. Upon evaporation of the eluant solvent under reduced pressure an oil was obtained. The latter was crystallized form ethyl acetate/hexane to give a white crystalline powder. Incorporation of 41 into the product gave 0.5g (0.77mmole) (96% yield) of crystalline powder, mp 114-116° C.

Procedure 2:

Isobutylchloroformate (0.43g, 3.2mmoles) in acetonitrile (4mL) was added to a dry-ice/carbon tetrachloride cooled solution of 7 (1.3g, 2.9mmoles) and N-methylmorpholine (0.3g, 2.9mmoles) in THF (10mL). After 10 min, D-proline phenacyl ester hydrochloride (41) (0.9g, 1.5mmoles) as a solid and N-methylmorpholine (0.35g, 3.5mmoles) in acetonitrile (6mL) were added to the reaction mixture maintained at -15° C. The reaction mixture was allowed to warm to 5° C in 30 min, was filtered after 90min, and the filtrate was evaporated in vacuo. The residue was redissolved in methylene chloride (20mL) and washed with water (20mL), 10% aqueous citric acid (20mL), water (20mL) and brine (20mL); dried (2g of $MgSO_4$) and evaporated in vacuo. The residue, containing the product, was purified similar to the methodology in procedure 1. The transparent oil was crystallized from ethyl acetate/hexane. The incorporation of 41 gave 0.85g (1.25mmoles) (43% yield) of white crystalline powder (23), mp 114-116° C. $^1$H-NMR ($CDCl_3$) $\delta$ 1.36-(3$H_a$,d,J=8Hz); 1.40-2.22(10$H_b$,m); 2.44(2$H_c$,app. t,J=8Hz); 2.64(2$H_{c'}$,app. t,J=8Hz); 3.18(2$H_d$,m); 3.56-(2$H_e$,m); 3.66(3$H_f$,s); 4.50(2$H_g$,app. t,J=8Hz); 4.53-4.80(1$H_h$,m); 5.10(2$H_i$,s); 5.43(2$H_j$,s); 5.76(1$H_k$,m, rotamer of amide -NH); 6.70-7.30(2$H_{l,m}$,m, rotamer of amide -NH); 7.36(5$H_n$,s); 7.60(3$H_{o,o',p}$,app. dd, $J_{oq}$=8Hz,$J_{oo'}$=2Hz); 8.06(2$H_{q,q'}$,app. dd, $J_{qo}$=8Hz,$J_{qp}$=2Hz)ppm. IR ($CHCl_3$) 3280, 1725, 1680, 1630, 725, 635$cm^{-1}$.

Ex. 40: $N_\alpha$-Methoxysuccinylalanyl-$N_\epsilon$-carbobenzoxylysylproline phenacyl ester (24, the LLL diastereomer)

24

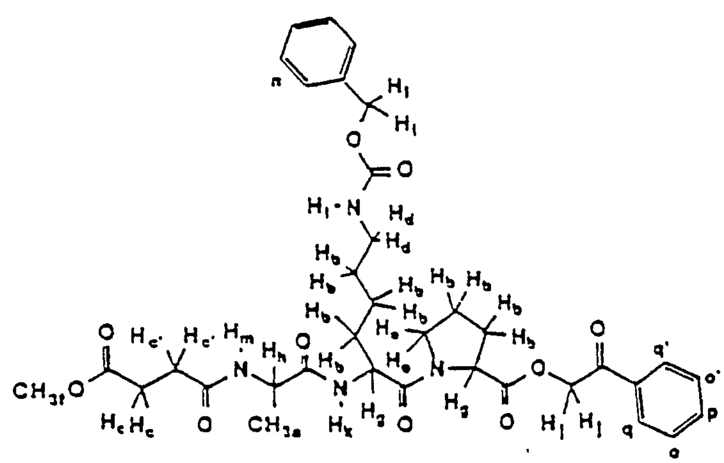

The title compound was prepared by an analogous procedure to that described in the preparation of compound 23. L-Proline phenacyl ester hydrochloride 42 was substituted in place of the D-enantiomer to form the LLL diastereomer product 24. Following procedure 1, the incorporation of 42 into 24 gave rise to 0.4g (0.54mmole) (74% yield) of crystalline powder mp 116-118° C. The LLL diastereomer 24 demonstrated a lower Rf value than the LLD diastereomer 23 on silica gel TLC plates eluted with 4% ethyl acetate in methylene chloride and 5% methanol in methylene chloride. Procedure 2, produced 1.3g (1.94mmoles) (66.8% yield) of white crystalline powder (24), mp 116-118° C, with similar Rf values. 'H-NMR ($CDCl_3$) $\delta$ 1.36($3H_a$,d,J=8Hz); 1.40-2.22($10H_b$,m); 2.44($2H_c$,app. t,J=8Hz); 2.64($2H_c$[$6H_c'$,app. t,J=8Hz); 3.18($2H_d$,m); 3.56($2H_e$,m); 3.66($3H_f$,s); 4.34-4.58($3H_{g,h}$,m); 4.60-4.64($1H_k$,m); 5.10($2H_i$,s); 5.43($2H_j$,s); 6.70-7.30($2H_{l,m}$,m, rotamer of amide -NH); 7.36($5H_n$,s); 7.60($3H_{o,o'}$,p,app. dd, $J_{oq}$=8Hz,$J_{oo'}$=2Hz); 8.06($2H_{qq'}$,app. dd, $J_{qo}$=8Hz,$J_{qp}$=2Hz).

Ex. 41: $N_\alpha$-Methoxysuccinylalanyl-$N_\epsilon$-carbobenzoxylysylproline (25, the LLD diastereomer)

25

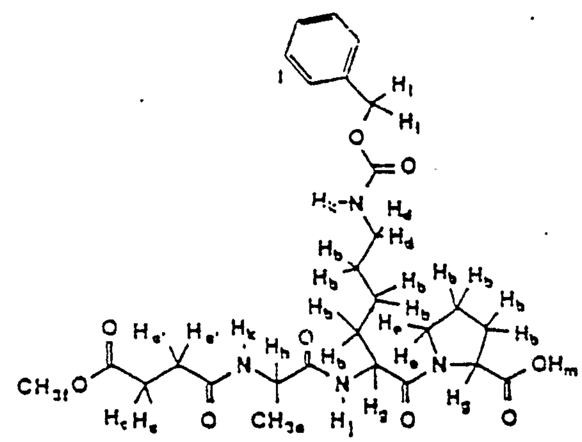

Small portions of zinc metal (total 2g) were added over a 1h period to the phenacyl ester (23) (0.5g, 0.8mmole) dissolved in glacial acetic acid (10mL). After 3h, the suspension was diluted with 20% methanol in chloroform (50mL), filtered under vacuum and evaporated to a crude solid. Work-up was performed in a similar manner to that described in the preparation of 7. The chromatographically pure oil required two crystallizations from chloroform/petroleum ether to yield a white solid. Hydrolysis of the phenacyl ester of 23 produced 0.26g (0.46mmole) (57% yield) of a white powder (25) mp 119-120° C. 'H-NMR ($CDCl_3$) $\delta$ 1.36($3H_a$,d,J=8Hz); 1.40-2.22($10H_b$,m); 2.44($2H_c$,app. t,J=8Hz); 2.64($2H_c'$,app. t,J=8Hz); 3.18($2H_d$,m); 3.56-($2H_e$,m); 3.66($3H_f$,s); 4.50($2H_g$,app. t,J=8Hz); 4.83($1H_h$,m); 5.10($2H_i$,s); 5.60($1H_j$,m, rotamer of amide -NH); 6.70-7.26($2H_k$,m, rotamer of amide -NH); 7.36($5H_l$,s); 10.20($1H_m$,s)ppm. IR ($CHCl_3$) 3400, 1740, 1680, 1630cm$^{-1}$.

Ex. 42: $N_\alpha$-Methoxysuccinylalanyl-$N_\epsilon$-carbobenzoxylysylproline (26, the LLL diastereomer)

(see 25 for structure)

The title compound was prepared by an analogous procedure to that described in the preparation of compound 25. Hydrolysis of the phenacyl ester of 24 produced 0.44g (0.78mmole) (98% yield) of a white powder (26), mp 119-120° C. The LLL diastereomer 26 demonstrated a lower Rf value than the LLD diastereomer 25 on silica gel TLC plates eluted with 4% methanol in ethyl acetate. 'H-NMR ($CDCl_3$) δ 1.36-(3H$_a$,d,J=8Hz); 1.40-2.22(10H$_b$,m); 2.44(2H$_c$,app. t,J=8Hz); 2.64(2H$_c$',app. t,J=8Hz); 3.18(2H$_d$,m); 3.56-(2H$_e$,m); 3.66(3H$_f$,s); 4.34-4.58(3H$_{g,h}$,m); 4.60-4.64(1H$_l$,m, rotamer of amide -NH); 5.10(2H$_i$,s); 6.70-7.26-(2H$_k$,m, rotamer of amide -NH); 7.36(5H$_l$,s); 10.20(1H$_m$,s).

Ex. 43: $N_\alpha$-Methoxysuccinylalanyl-$N_\epsilon$-carbobenzoxylysylprolyl chloromethyl ketone (27, the LLD diastereomer)

27

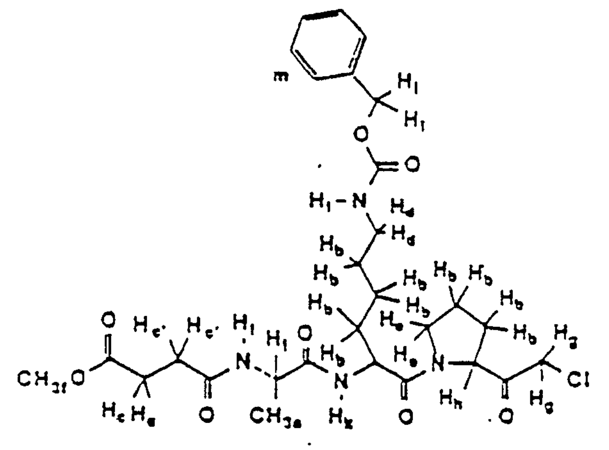

Isobutylchloroformate (0.11g, 0.8mmole) in THF (2mL) was added to a cooled solution of 25 (0.45g, 0.8mmole) and N-methylmorpholine (0.08g, 0.8mmole) in THF (4.5mL) and the mixture was stirred for 10 min at -15° C. A cold solution (0° C) of diazomethane (cal'd. 0.13gm, 3.2mmoles) in diethyl ether was added and the mixture stirred at -10° C for 30 min, then at 5° C for 90 min. The reaction mixture was then diluted with ethyl acetate (40mL), and washed with saturated aqueous sodium bicarbonate (2 x 30mL), water (30mL) and brine (30mL); dried (5g of $MgSO_4$) and evaporated in vacuo to give a yellow oil. $^1$H-NMR for the α-azomethyl ketone intermediate ($CDCl_3$) δ 0.86-2.23(13H,m), 2.56(4H,t,J=7Hz), 3.03-3.40(2H,m), 3.46-4.00-(5H,m), 4.40-4.83(3H,m), 5.13(2H,s), 5.40-5.80(2H,m), 6.53-7.20(2H,m), 7.40(5H,s). IR ($CHCl_3$) 3280,2215,1735,1635,1535cm$^{-1}$.

Hydrogen chloride gas was bubbled through an ice-cooled solution of the α-azomethylketone intermediate (0.8mmole) in ethyl acetate (10mL) for 30s. Subsequent stirring for 10 min at 5° C, bubbling of nitrogen gas through the reaction mixture to remove excess hydrogen chloride, diluting with cold ethyl acetate,and evaporating in vacuo, gave rise to a yellow oil. The disappearance of the UV-bright TLC spot corresponding to the α-azomethylketone and appearance of the low UV absorbing TLC spot (with a lower RF value) corresponding to the α-chloromethylketone was followed using silica gel TLC plates eluted with 4% ethyl acetate in methylene chloride. The residue, containing the product, was chromatographed on 5g of silica gel column (1 x 25cm). Impurities were eliminated by first passing 25mL of methylene chloride and subsequently the compound was eluted with 3% methanol in methylene chloride. Upon evaporation of the eluent solvent under reduced pressure a transparent oil was obtained. The latter was then crystallized from chloroform/petroleum ether to give 0.25g (0.4mmole) (54% yield) of crystalline powder, mp 46-48° C. $^1$H-NMR ($CDCl_3$) δ 1.36(3H$_a$,d,J=8Hz); 1.40-2.22(10H$_b$,m); 2.44(2H$_c$,app. t,J=8Hz); 2.64(2H$_c$',app. t,J=8Hz); 3.20(2H$_d$,m); 3.56(2H$_e$,m); 3.66(3H$_f$,s); 4.20,4.32(2H$_g$, center of a set of dd, overlapping with another set, J=20Hz, rotamers of the $CH_{2(g)}$ geminal system); 4.50(2H$_h$,app. t,J=8Hz); 4.83(1H$_i$,m); 5.10(2H$_j$,s); 5.60-(1H$_k$,m, rotamer of amide -NH); 6.70-7.26(2H$_l$,m, rotamers of amide -NH); 7.36(5H$_m$,s)ppm. IR ($CHCl_3$) 3300, 1725, 1625, 1635, 1535cm$^{-1}$.

Ex. 44: $N_\alpha$-Methoxysuccinylalanyl-$N_\epsilon$-carbobenzoxylysylprolyl chloromethyl ketone (28. the LLL diastereomer)

see 27 for structure)

The title compound was prepared by an analogous procedure to that described in the preparation of compound 27. The presence of impurities in the IR and NMR for the α-azomethylketone intermediate produced during the reaction did not demonstrate clear differences between the LLD and LLL diastereomers. The Rf value of the LLD and LLL diastereomers were significantly different when eluted twice on silica gel plates with 4% acetate in methylene chloride or methylene chloride. The α-chloromethylketone 28 was crystallized from chloroform petroleum ether to give 0.25g (0.4mmole) (54% yield) of crystalline powder, mp 58-60° C. 'H-NMR ($CDCl_3$) δ 1.36($3H_a$,d,J=8Hz); 1.40-2.22($10H_b$,m); 2.44($2H_c$,app. t,J=8Hz); 2.64($2H_c'$,app. t,J=8Hz); 3.20($2H_d$,m); 3.56($2H_e$,m); 3.66($3H_f$,s); 4.23($2H_g$, center of 2 sets of dd,J=20Hz, rotamers of the $CH_{2(g)}$ geminal system); 4.34-4.58($3H_{h,i}$,m); 4.60-4.64($1H_k$,m); 5.10($2H_l$,s); 6.70-7.26($2H_l$,m, rotamers of amide -NH); 7.36($5H_m$,s)ppm.

Ex. 45: N-Methoxysuccinylalanyl-$N_\epsilon$-carbobenzoxy)lysylprolylmethyl]-N-isopropylamine (29, the LLD diastereomer)

29

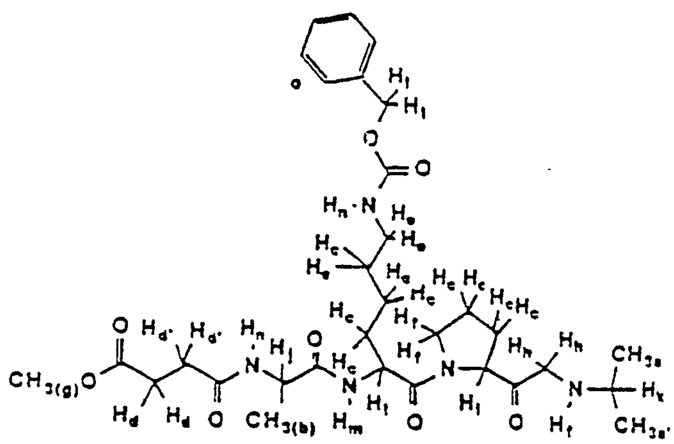

Isopropylamine (1.4g, 24.4mmoles) was added to a cold solution (0° C) of 27 (1.45g, 2.44mmoles) in THF (5mL). The reaction mixture was stirred at 0° C for 12h, filtered under vacuum and the filtrate evaporated to a crude oil. The residue, containing the product, was redissolved in a small amount of methylene chloride, and chromatographed on 25g of silica gel column (1 x 50cm). The product was eluted with 7% methanol in methylene chloride. The eluent solvent was evaporated under reduced pressure. The reaction produced 0.31g (0.5mmole) (21% yield) of a yellow oil. $^1$H-NMR ($CDCl_3$) δ 1.06-1.28($6H_{a,a'}$,m, rotamers of isopropylamine); 1.32($3H_b$,d, J=8Hz); 1.38-2.32($10H_c$,m); 2.46($2H_d$,app. t, J=8Hz); 2.64-($2H_d'$,app. t, J=8Hz); 3.20($2H_e$,m); 3.52.3.88($3H_f$,m); 3.66($3H_g$,s); 4.20,4.32($2H_h$, center of a set of dd, overlapping with another set, J=20Hz, rotamers of the $CH_{2(h)}$ geminal system); 4.50($2H_i$,app.t, J=8Hz); 4.50-4.80($2H_{j,k}$,m); 5.10($2H_l$,s); 5,42($1H_m$,m, rotamer of amide -NH); 6.36-7.08($2H_n$,m, rotamers of amide -NH); 7.35($5H_o$,s)ppm.

Ex. 46: $N_\alpha$-Methoxysuccinylalanyl-$N_\epsilon$-carbobenzoxy)lysylprolylmethyl]-N-isopropylamine (30, the LLL diastereomer)

(see 29 for structure)

The title compound was prepared by an analogous procedure to that described in the preparation of compound 29. The reaction produced 0.31g (0.5mmole) (21% yield) of a yellow oil. $^1$H-NMR ($CDCl_3$) δ 1.06-1.28($6H_{a,a'}$,m, rotamers of isopropylamine); 1.32($3H_b$,d, J=8Hz); 1.38-2.32($10H_c$,m); 2.46($2H_d$,app.t, J=8Hz); 2.64($2H_d'$,app. t, J=8Hz); 3.20($2H_e$,m); 3.52.3.88($3H_f$,m); 3.66($3H_g$,s); 4.23 ($2H_h$, center of 2 sets of dd, J=20Hz, rotamers of the $CH_{2(h)}$ geminal system); 4.34-4.58($3H_{h,i}$,m); 4.60-4.64($1H_k$,m); 5.10($2H_l$,s); 5,42-($1H_m$,m, rotamer of amide -NH); 6.36-7.08($2H_n$,m, rotamers of amide -NH); 7.35($5H_o$,s)ppm.

Ex. 47: p-Nitrophenyl N-[Methoxylsuccinylalanyl-($N_\epsilon$-carbobenzoxy)lysylprolylmethyl]- N-isopropylcarbamate (48b, the LLD diastereomer)

(see page 45 for structure)

An ice cooled solution of 29 (0.3g, 0.5mmole), N-methylmorpholine (0.07g, 0.7mmole) and 4-nitrophenyl chloroformate (0.15g, 0.75mmole) in THF (3mL) was stirred for 2h at 0° C. The reaction mixture was diluted with methylene chloride (15mL) and washed subsequently with water (15mL), 10% aqueous citric acid (15mL), water (15mL) and brine (15mL); dried (2g of $MgSO_4$) and evaporated in vacuo to an oil. The oil was chromatographed on 5g of silica gel column (1 x 25cm). The impurities were eliminated by first passing 10mL of methylene chloride and 20mL of 2% methanol in methylene chloride. Subsequently the compound was eluted with 4% methanol in methylene chloride. Upon evaporation of the eluent solvent under pressure an amorphous solid was obtained. The product was further purified by preparative TLC (100mg/plate). The TLC plates were developed twice using 4% methanol in ethyl acetate in order to see if two bands were present. The one band observed was scraped from the plate and the product was extracted with 20% methanol in chloroform. An amorphous white solid was obtained by evaporating the extraction solvent under reduced pressure. The incorporation of D-proline into the synthetic scheme gave rise to 89.9mg (115.0 moles) (23% yield) of amorphous solid (48b), mp 44-45° C. $^1$H-NMR ($CDCl_3$) (see 48b above). Elemental analysis cal'd. for $C_{38}H_{50}N_6O_{12}$: C,58.3;H,6.44;N,10.73. Found: C,58.49; H,6.47; N,10.63.

Ex. 48: p-Nitrophenyl N-[Methoxysuccinylalanyl-($N_\epsilon$-carbobenzoxy)lysylprolylmethyl]-N-isopropylcarbamate (48a, the LLL diastereomer)

(see page 46 for structure)

The title compound was prepared by an analogous procedure to that described in the preparation of compound 48b. The reaction pathway which incorporated L-proline into the final peptidyl carbamate 48a gave rise to 153.9mg (196.0 moles) (39% yield) of amorphous solid (48a), mp 56-57° C. $^1$H-NMR ($CDCl_3$) (see 48a above). Elemental analysis cal'd. for $C_{38}H_{50}N_6O_{12}$: C,58.30; H,6.44;N ,10.73. Found: C,58.12:H,6.55;N,10.64%.

## 3. Synthesis of protected amino acids

Ex. 49: $N_\alpha$-t-Boc-$N_\epsilon$-carbobenzoxylysine (31)

31

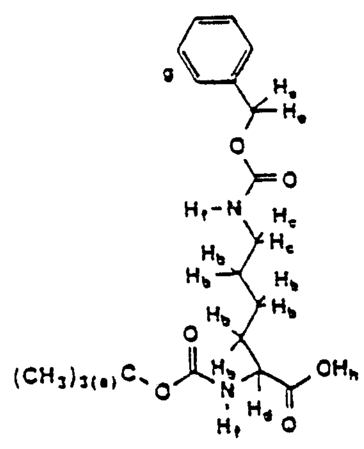

This compound was synthesized by a modified procedure of Itoh et al.[91].

t-BOC-ON (2.2g, 8.9mmoles) was added as a solid to a solution of triethylamine (0.9g, 8.9mmoles) and $N_\epsilon$-carbobenzoxy-L-lysine (2.5g, 8.9mmoles) in DMF (20mL) at room temperature and the components allowed to react for 24 h. The resulting precipitate was filtered and the filtrate evaporated under vacuum. The crude oil was dissolved in methylene chloride and chromatographed on 50g of silica gel column (3 x

50cm). Impurities were eliminated by first passing 100mL of methylene chloride and subsequently the compound was eluted with 10% methanol in methylene chloride. Upon evaporation of the eluent solvent under reduced pressure 3.25g (8.6mmoles) (96% yield) of an oil was obtained. ¹H-NMR ($CDCl_3$) δ 1.46-($9H_a$,s); 1.50-2.20($6H_b$,m); 3.23($2H_c$,m); 4.00-4.30($1H_d$,m); 5.10($2H_e$,s); 6.56-7.10($2H_f$,m, rotamers of carbamate -NH); 7.36($5H_g$,s); 9.80($1H_h$,s)ppm.

Ex. 50: $N_\alpha$-t-Boc-$N_\epsilon$-carbobenzoxy-L-lysine phenacyl ester (32)

32

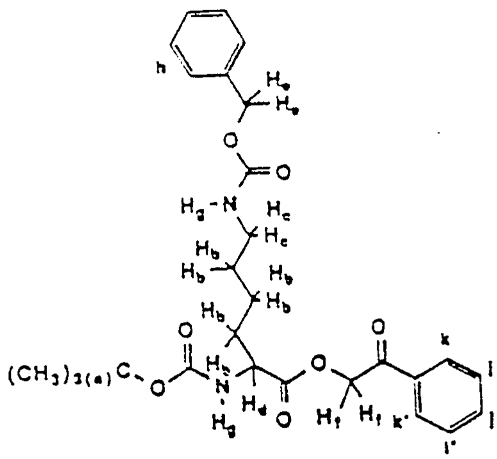

This compound was synthesized by a modified procedure of Hendrickson et al.[85]

2-Bromoacetophenone (1.8g, 8.9mmoles) was added as a solid to a solution of triethylamine (0.9g, 8.9mmoles) and 31 (3.4g, 8.9mmoles) in DMF (25mL). The progress of the reaction was monitored by TLC (10% methanol in chloroform). Upon completion of the reaction (1.5h), the reaction was stopped by the addition of water (30mL) and decanted. The residue, containing the product, was dissolved in chloroform (40mL) and washed with water (30mL) and brine (30mL); dried (10g of $MgSO_4$) and evaporated under reduced pressure. The resulting oil was chromatographed on 30g of silica gel column (2 x 50cm). Impurities were eliminated by first passing 50mL of methylene chloride and subsequently the compound was eluted with 3% methanol in methylene chloride. Upon evaporation of the eluent solvent under reduced pressure 3.6g (7.2mmoles) (81% yield) of a transparent oil was obtained. ¹H-NMR ($CDCl_3$) δ 1.46($9H_a$,s); 1.50-2.20($6H_b$,m); 3.23($2H_c$,m); 4.00-4.30($1H_d$,m); 5.10($2H_e$,s); 5.56($2H_f$,s); 6.56-7.10($2H_g$,m, rotamers of carbamate -NH); 7.36($5H_h$,s); 7.63($3H_{i,i'}$,j,app. dd, $J_{ik}$ = 8Hz,$J_{ii'}$ = 2Hz); 8.06($2H_{k,k'}$,app. dd,$J_{ki}$ = 8Hz,$J_{kj}$ = 2Hz)ppm.

Ex. 51: $N_\epsilon$-Carbobenzoxy-L-lysine phenacyl ester hydrochloride (33)

33

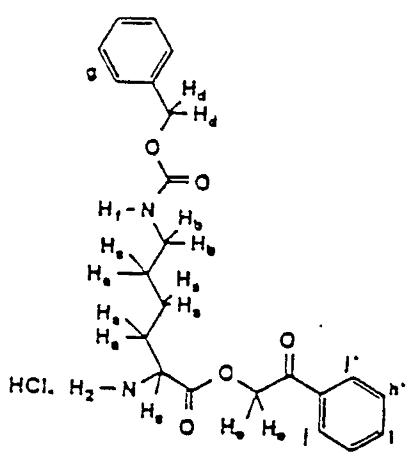

Formic acid (98%) (6mL) was added to a 10% solution of 32 (3.3g, 6.9mmoles) in ethyl acetate (50mL) cooled to 5° C. Hydrogen chloride gas was slowly bubbled through the cooled solution in three 30s intervals, 10 min apart. The solution was stirred at 5° C for 30min and was then allowed to equilibrate to room temperature. The progress of the reaction was monitored by TLC (10% methanol in chloroform). Upon completion of the reaction (3h at 22° C), the suspension was filtered. The latter was recrystallized from absolute ethanol hexane to give 2.2g (5.1mmoles) (73% yield) of a crystalline powder, mp 149-151° C. $^1$H-NMR (DMSO-$d_6$) $\delta$ 1.50-2.26(6$H_a$,m); 3.23(2$H_b$,m); 4.03-4.43(1$H_c$,m); 5.10(2$H_d$,s); 5.80(2$H_e$,s); 7.10(1$H_f$,m); 7.36(5$H_g$,s); 7.63(3$H_{h,h'}$,i,app. dd,$J_{hi}$ = 8Hz,$J_{hi}$ = 2Hz); 8.06(2$H_{i,i'}$,app. dd,$J_{ih}$ = 8Hz,$J_{ii}$ = 2Hz); 8.86(2$H_k$,m)ppm. IR (Nujol) 3350, 1750, 1700, 1680, 1585, 1535$cm^{-1}$.

Ex. 52: $N_\alpha$-t-Boc-$N_\delta$-carbobenzoxy-L-ornithine (34)

34

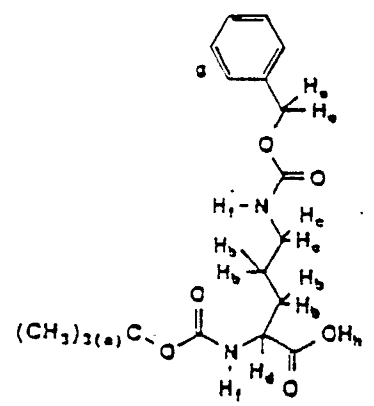

The title compound was synthesized and purified by an analogous procedure to that described in the preparation of compound 31. The residue, containing product, was chromatographed to give 1.34g (3.6mmoles) (97% yield) of a transparent oil. $^1$H-NMR ($CDCl_3$) $\delta$ 1.46(9$H_a$,s); 1.50-2.10(4$H_b$,m); 3.23(2$H_c$,m); 4.00-4.40(1$H_d$,m); 5.10(2$H_e$,s); 6.83-7.10(2$H_f$,m,rotamer of carbamate -NH); 7.40(5$H_g$,s); 9.80(1$H_h$,s)ppm.

Ex. 53: $N_\alpha$-t-Boc-$N_\delta$-carbobenzoxy-L-ornithine phenacyl ester (35)

35

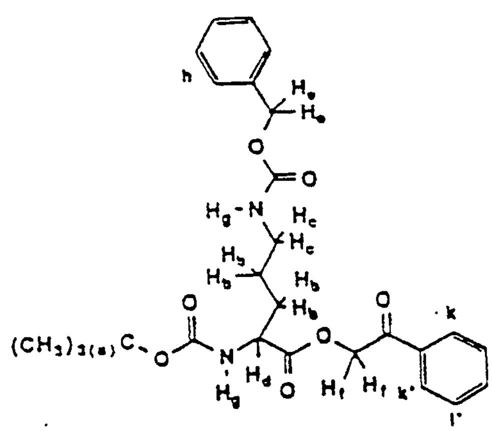

The title compound was synthesized and purified by an analogous procedure to that described in the preparation of compound 32. The reaction produced 3.7g (7.7mmoles) (86% yield) of a transparent oil. $^1$H-NMR ($CDCl_3$) $\delta$ 1.46(9$H_a$,s); 1.50-2.20(4$H_b$,m); 3.23(2$H_c$,m); 4.00-4.30(1$H_d$,m); 5.10(2$H_e$,s); 5.56(2$H_f$,s); 6.56-7.10(2$H_g$,m, rotamers of carbamate -NH); 7.36(5$H_h$,s); 7.63(3$H_{i,i'}$,j,app. dd, $J_{ik}$ = 8Hz,$J_{ii'}$ = 2Hz); 8.06-(2$H_{k,k'}$,app. dd,$J_{ki}$ = 8Hz,$J_{kj}$ = 2Hz)ppm.

Ex. 54: Nδ-Carbobenzoxy-L-ornithine phenacyl ester hydrochloride (36)

36

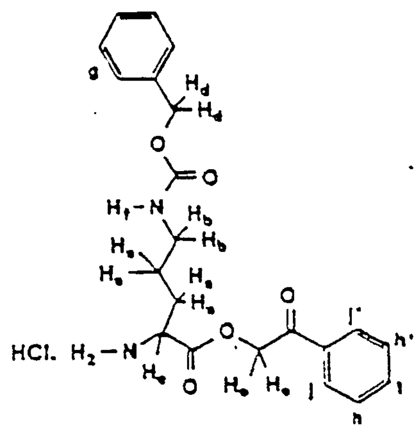

The title compound was prepared by an analogous procedure to that described in the preparation of compound 33 The resulting white powder was recrystallized from ethanol hexane to give 2.4g (5.7mmoles) (76% yield) of a crystalline powder mp 166-167 °C. $^1$H-NMR (DMSO-$d_6$) δ 1.50-2.26(4$H_a$,m); 3.23(2$H_b$,m); 4.03-4.43(1$H_c$,m); 5.10(2$H_d$,s); 5.80(2$H_e$,s); 7.10(1$H_f$,m); 7.36(5$H_g$,s); 7.63(3$H_{h,h'}$,i, app. dd,$J_{hj}$=8Hz,$J_{hi}$=2Hz); 8.06(2$H_{i,j}$',app. dd,$J_{jh}$=8Hz,$J_{ji}$=2Hz); 8.86(2$H_k$,m)ppm. IR (Nujol) 3370, 1770, 1700, 1530cm$^{-1}$.

Ex. 55: N-t-Boc-D-proline (37)

37

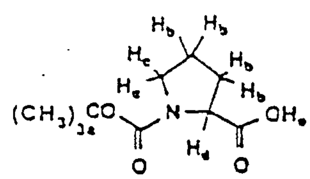

The procedure of Itoh et al.[91] was followed for the preparation of compound 37.

Triethylamine (0.2g, 2mmoles) was slowly added to a solution of (D-proline (0.23g, 2mmoles) and t-BOC-ON (0.5g, 2mmoles) in DMF (3mL) at room temperature and the mixture stirred for 36h. The DMF was coevaporated with toluene under vacuum. Aqueous hydrochloric acid (1 x $10^{-4}$M) (20mL) and ethyl acetate (20mL) were added to the flask to dissolve the residue. The organic layer was washed with water (3 x 20mL) and brine (2 x 20mL); dried (5g of $MgSO_4$) and evaporated under reduced pressure. The residue, containing the product, was chromatographed on 5g of silica gel column (1 x 25cm). Upon evaporation under reduced pressure a transparent oil was obtained. The oil was crystallized from ethyl acetate/diethyl ether to give 0.4g (2mmoles) (99% yield) of a crystalline powder, mp 133-134 °C (lit.[91] mp 132-133 °C). $^1$H-NMR ($CDCl_3$) δ 1.46(9$H_a$,s); 1.86-2.40(4$H_b$,m); 3.43-3.80(2$H_c$,m); 4.26-4.66(1$H_d$,m); 11.43(1$H_e$,s). IR (Nujol) 1740, 1635cm$^{-1}$.

Ex. 56: N-t-Boc-L-proline (38)

(see 37 for structure)

The title compound was prepared by an analogous procedure to that described in the preparation of compound 37. L-proline was substituted in place of the D-enantiomer to produce compound 38. The oil was crystallized from ethyl acetate/diethyl ether to give 0.4g (2.0mmoles) (99% yield) of a crystalline powder, mp 133-134 °C (lit.[91] mp 132-133 °C).

Ex. 57: N-t-Boc-D-proline phenacyl ester (39)

39

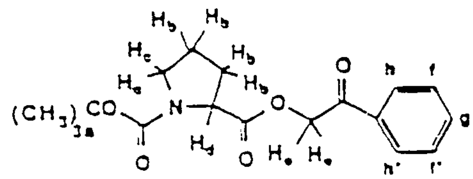

This compound was synthesized by a modified procedure of Hendrickson et al.[85].

2-Bromoacetophenone (0.4g, 2mmoles) was added slowly to an ice-cooled solution of t-Boc-D-proline (0.4g, 2mmoles) and triethylamine (0.2g, 2mmoles) in THF (4mL). The progress of the reaction was monitored by TLC (5% methanol in chloroform). Upon completion of the reaction (1.5h), the formed triethylamine salt was filtered, washed with ethyl acetate (10mL) and the filtrate evaporated under vacuum. The residue, containing the product, was chromatographed on 5g of silica gel column (1 x 25cm). Impurities were eliminated by first passing 25mL of methylene chloride and subsequently the compound was eluted with 3% methanol in methylene chloride. Upon evaporation of the eluent solvent under reduced pressure a white solid was obtained. The latter was recrystalized from ethyl acetate/hexane. The incorporation of 37 into the product gave 0.58g (1.75mmoles) (87.5% yield) of crystalline powder (39), mp 78-79°C. $^1$H-NMR ($CDCl_3$) δ 1.46(9$H_a$,s); 1.86-2.40(4$H_b$,m); 3.20-3.60(2$H_c$,m); 4.30(1$H_d$,m); 5.80(2$H_e$,s); 7.63(3$H_{f,f'}$,g,app. dd, $J_{fh}$ = 8Hz, $J_{ff'}$ = 2Hz); 8.06(2$H_{h,h'}$,app. dd, $J_{hf}$ = 8Hz, $J_{hg}$ = 2Hz)ppm.

Ex. 58: N-t-Boc-L-proline phenacyl ester (40)

(see 39 for structure)

The tilte compound was prepared by an analogous procedure to that described in the preparation of compound 39. t-Boc-L-proline (38) was substituted into the reaction to produce 0.59g (1.78mmoles) (88.8% yield) of crystalline powder (40), mp 76-78°C.

Ex. 59: D-Proline phenacyl ester hydrochloride (41)

41

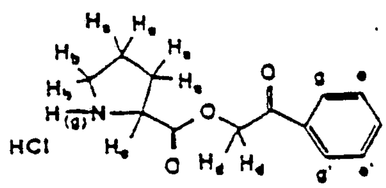

Formic acid (98%) (0.6mL) was added to a cooled solution (0°C) of t-Boc-D-proline phenacyl ester (0.6g, 1.75mmoles) in ethyl acetate (10mL). Hydrogen chloride gas was slowly bubbled through the above solution in two 30s intervals, 10 min apart. The reaction mixture was stirred for 30 min at 0-5°C, then allowed to equilibrate to room temperature and stirred until the reaction was completed (approximately 1h). The suspension was diluted with ethyl acetate and filtered under vacuum. The product was washed with ethyl acetate, air dried and recrystallized from absolute ethanol/diethyl ether. The use of 39 (the D isomer) in this reaction gave 0.3g (1.1mmoles) (63.5% yield) of crystalline powder (41), mp 154-156°C. $^1$H-NMR (DMSO-$d_6$) δ 1.86-2.76(4$H_a$,m); 3.16-3.63(2$H_b$,m); 4.40-4.86(1$H_c$,m); 5.80(2$H_d$,s); 7.63(3$H_{e,e'}$,f,app. dd, $J_{eg}$ = 8Hz,$J_{ee'}$ = 2Hz); 8.06(2$H_{g,g'}$,app. dd, $J_{ge}$ = 8Hz, $J_{gf}$ = 2Hz); 9.30(1$H_g$,m)ppm. IR (Nujol) 1755, 1600, 1500cm$^{-1}$.

Ex. 60: L-Proline phenacyl ester hydrochloride (42)

(see 41 for structure)

The title compound was prepared by an analogous procedure to that described in the preparation of compound 41. t-Boc-L-proline phenacyl ester was substituted into the reaction to produce 0.4g (1.54mmoles) (88% yield) of crystalline powder (42), mp 144-156°C.

Ex. 61: $N_\epsilon$-Benzoyl-L-lysine (43)

43

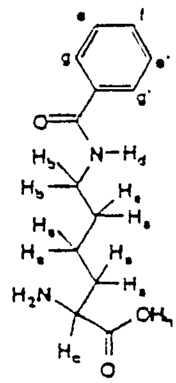

This compound was synthesized by a modified procedure of Kurtz et al.[33].

Copper (II) carbonate, basic (16.3g, 74.0mmoles) was added to a hot (80 °C) solution of L-lysine (15g, 82.0mmoles) in distilled water (250mL). Excess copper carbonate was removed by gravity filtration while the mixture was still warm. The epsilon amino group of L-lysine was benzoylated by the dropwise addition of benzoyl chloride (14g, 99.0mmoles) in THF (35mL) to the above solution after being cooled to 5 °C. Sodium bicarbonate was added in small portions (total, 14g, 167.0mmoles) to maintain the pH of the aqueous solution above 7 (pH was monitored with neutral litmus paper). The cold solution was mantained at 5 °C for 4h and 2d at room temperature. A hot (80 °C) solution (1L) of EDTA (14.6g, 0.25moles) was added to the reaction mixture, stirred for 1h and cooled to 10 °C. The precipitate collected by vacuum filtration was washed with water and 30mL of 95% ethanol. The powder, containing the product was purified by first adding the powder to $1 \times 10^{-1}$M hydrochloric acid (300mL) and filtering off the sediment. The filtrate was slowly neutralized with 0.01N sodium hydroxide and the precipitate collected by gravity filtration. The latter was then air-dried to give 13g (57mmoles) (69.5% yield) of a white powder, mp 232-234 °C (lit.[33] mp 230-233 °C). $^1$H-NMR (DMSO-$d_6$) $\delta$ 1.36-190(6$H_a$,m); 3.20(2$H_b$,m); 4.20-4.53(1$H_c$,m); 7.16(1$H_d$,m, rotamer of amide -NH); 7.43(2$H_{e,e'}$,app. dd, $J_{ef}$=8Hz,$J_{ee'}$=2Hz); 7.63(1$H_f$,app. dd,$J_{fe}$=8Hz,$J_{fg}$=2Hz); 8.03(2$H_{f,f'}$,app dd,$J_{ge}$=8Hz,$J_{gf}$=2Hz); 9.50(1$H_h$,s)ppm. IR (Nujol) 3330, 3030, 1687, 1270, 735, 695cm$^{-1}$.

Ex. 62: S-(1-Phenyl-5-tetrazolyl) chlorothioformate (52)

52

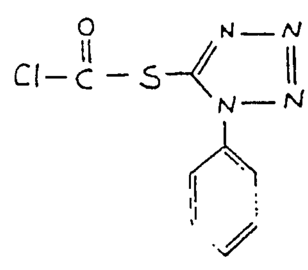

A 20% phosgene solution in toluene (14.4 ml, 25.2 mmole) was added to a mixture of triethylamine (2.58ml, 18.5 mmol) and 1-phenyl-1H-tetrazol-5-thiol (3.00g, 16.8 mmole) in THF (15ml) at 5 °C over 10 min. The reaction mixture was stirred for an hour at 5°C filtered. The filtrate obtained was evaporated and the residue washed thoroughly with ethyl ether. It was then recrystallized by dissolving in THF and precipitating with ethyl ether to give white crystal (2.92g, 72%) m.p. 112-114°C. IR (Film) 1718, 1645, 1590, 1490 and 1395 cm$^{-1}$. $^1$H-NMR ($CDCl_3$) $\delta$7.52 (s, aromatic H).

Ex. 63: S-(1-Phenyl-5-tetrazoyl)-N-[(N-BoC-L-prolyl)methyl]-N-isopropylthiocarbamate (53)

53

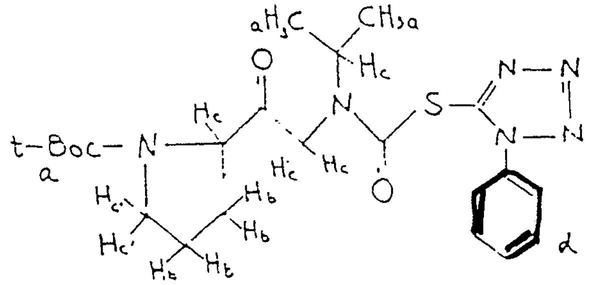

Compound 52 (1.30g, 5.40 mmole) was added to a solution of compound 20 (0.8g, 2.96 mmole) and triethylamine (0.6ml, 4.30 at 5°C. The mixture was stirred for 2h at 5°C. The reaction mixture was diluted with chloroform. The organic layer was washed with water, dried over $MgSO_4$ and evaporated to give an oil. The oil obtained was purified by column chromatography (silica gel, $CHCl_3$ 1% MeOH in $CHCl_3$ - 2% MeOH in $CHCl_3$) to give white crystals (0.6g, 42.8%). ¹H-NMR ($CCDCl_3$) δ1.2 (6Ha′ ,d), 1.4(9Ha,s), 1.95-(4Hb,m), 3.45 (4Hc′,t) 4.2 (4Ho,m), 7.55[5Hd,s). Anal. Calcd for $C_{22}H_{30}N_6O_4S$: C,55.69H,6.37, N,17.71; S,6.74. Found: C55.50; H, 6.43; N,17.66; S,6.68.

Ex. 64: S-(1-Phenyl-5-tetrazoyl)-N-prolylmethyl-N-isopropyl thiocarbamate hydrochloride 54

54

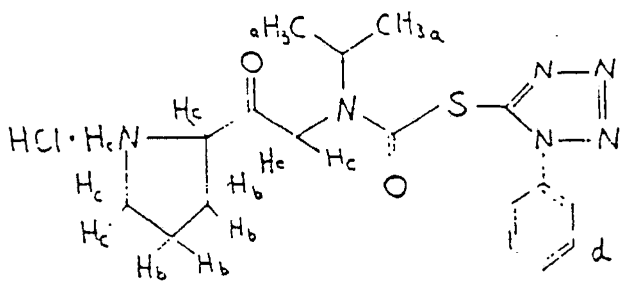

Hydrogen chloride gas was passed through a solution of 53 (0.6g) in ethyl acetate (5ml) at 5°C for 3 min. The solution was allowed to stand at 5°C for 30 min and then evaporated in vacuo. The residue wa triturated with ethyl ether to give white powder (0.24g, 46%). ¹HNMR δ1.2(6Ha,d), 2.0(4H6,m) 3.4-4.9-(7Hc,m), 7,5(5Hd,s).

Ex. 65: S-(1-Phenyl-5-tetrazoyl)-N-[Methoxysuccinyl-alanyl-($N_6$-carbonbenzoxyl )lysylprolylmethyl]-N-isopropyl thiocarbamate (PC5)

PC5

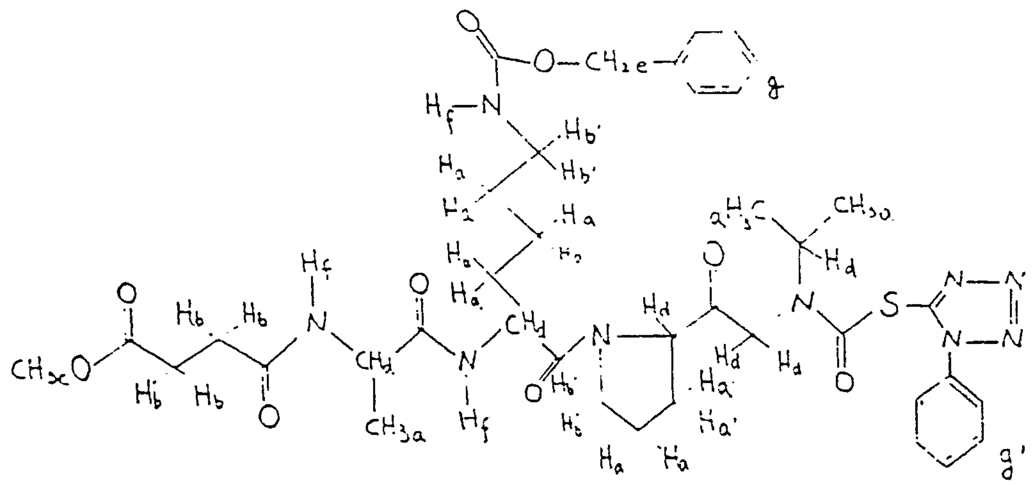

To a cooled solution (-20°C) of compound 7 (232mg, 0.498 mmole and N-methylmorpholine (0.064ml, 0.498 mmole) in THF (3ml), isobutyl chloroformate (0.06ml, 0.498 mmole) was added. The mixtur was stirred for 15 min at -15°C to -30°C. compound 54 (226mg, 0.55 mmole) was added as a solid followed by N-methyl morpholine (0.064m 0.55mmol). The reaction mixture was allowed to warm to room temperature and stirred overnight. The solution was diluted with methylene chloride, washed with 10% citric acid, brine and water. It was subsequently dried ($MgSO_4$) and the solvent evaporated. The residue was purified by column chromatography (silica gel, $CH_2Cl_2$ to 2% methanol in $CH_2Cl_2$). A white powder was obtained (200mg, 48.8%) m.p. 60-65°C $^1$H-NMR($CDCl_3$) δ1.35(15Ha,m), 1.95(4Ha′,m), 2.5(4Hb,d), 3.15(4Hb′ ,m), 3.62(3Hc,s), 3.7-4.9-(6Hd,m), 5.1(2He,s), 5.2-6.8(3Hf,m) 7.25(5Hg,s), 7.45(5Hg′,s). Anal. Cald for $C_{39}H_{51}N_9O_9S$. THF:C,57.77; H,6.65; N,14.10; S.3.57; found C,57.65; H,6.40; N,14.32; S,3.57.

Ex. 66: S-(1-Phenyl-5-tetrazoyl)-N-[methoxysuccinyl-Nδ-carbobenzoxyl) ornit alanyl prolyl methyl]-N-isopropyl thiocarbamate (PC6)

PC6

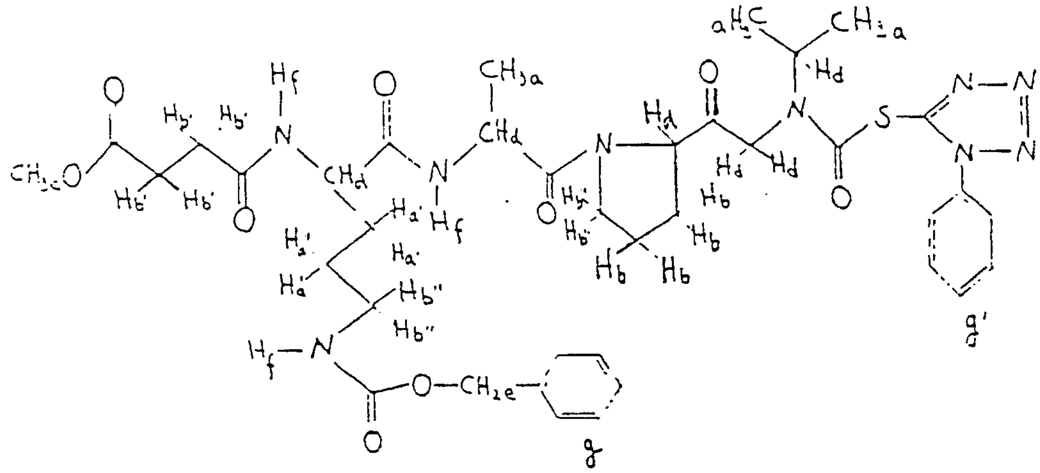

PC6 was prepared following a similar procedure as for the preparati of PC5 using compound 12 instead of 7. PC6 was obtained as a white powder (37.3%). m.p. 75°C. $^1$HNMR ($CDCl_3$) δ1.2 (9Ha, m), 1.6(4Ha,m 2.0(4Hb,m), 2.55(4Hb′ ,s); 2.9-3.2 (4H6″,m), 3.61(3Hc,s); 3.7-4.8(6Hd,m), 5.05(2He,s); 5.3-6.9(3Hf,m) 7.25-(5Hg,s], 7.5(5HG,S). Anal. calcd for $C_{38}H_{49}N_9O_9S$: C,56.50; H,6.11; N,15.60 S,3.96. Found: C,56.59; H,6.16; N,15.53; S,3.92.

Ex. 67: Elastase Enzyme Inhibitory Studies

These studies are conducted to show the activities of some of the compounds of this invention as inhibitors of various forms of the enzyme elastase. The results are shown in the following Tables.

## Inhibition of PPE and HLE by Novel Peptidyl Carbamates: Variations at $P_3$

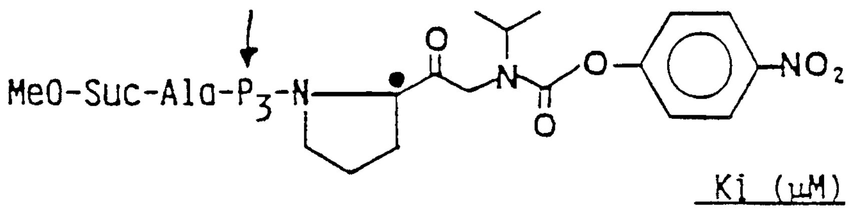

| Compound | $P_3$[a] | Isomer[b] | Ki (µM) PPE | Ki (µM) HLE |
|---|---|---|---|---|
| 5 (a,b) | N-ε-Cbz-LYs | a(D) | N.I.[c] | 11.30 |
| | | b(L) | 3.40 | 0.22 |
| 6 (a,b) | N-ε-Bz-Lys | a(D) | N.I. | 38.50 |
| | | b(L) | 3.80 | 0.31 |
| 7 (a,b) | N-δ-Cbz-Orn | a(D) | N.I. | 19.25 |
| | | b(L) | 8.65 | 1.05 |
| 8 (a,b) | N-δ-Bz-Orn | a(D) | N.I. | 100.00 |
| | | b(L) | 14.30 | 2.14 |

a. Cbz=$COOCH_2Ph$; Bz=COPh; Lys=lysine, Orn=ornithine
b. (L) and (D) refer to the configuration at the prolyl α-carbon
c. N.I. refers to no inhibition at I/E=100.

## Inhibition of PPE and HLE by Novel Peptidyl Carbamates Variations at $P_4$

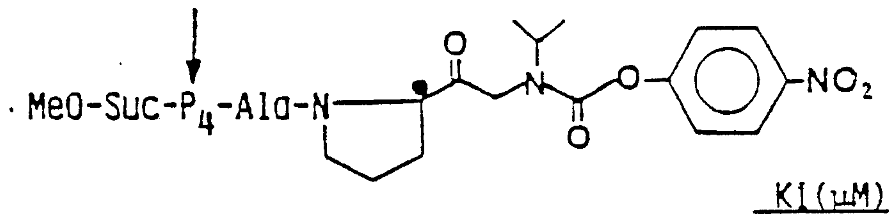

| Compound | $P_4$ [a] | Isomer [b] | Ki(μM) PPE | Ki(μM) HLE |
|---|---|---|---|---|
| 1(a,b) | N-ε-Cbz-Lys | a (L) | N.I. [c] | 0.47 |
| | | b (D) | N.I. | 7.63 |
| 2(a,b) | N-ε-Bz-Lys | a (L) | N.I. | 0.38 |
| | | b (D) | N.I. | 3.13 |
| 3(a,b) | N-δ-Cbz-Orn | a (L) | N.I. | 0.65 |
| | | b (D) | N.I. | 7.95 |
| 4(a,b) | N-δ-Bz-Orn | a (L) | N.I. | 0.19 |
| | | a (D) | N.I. | 17.70 |

a. Cbz=$COOCH_2Ph$; Bz=COPh; Lys=lysine; Orn=ornithine
b. (L) and (D) refer to the configuration at the prolyl α-carbon
c. N.I. refers to no inhibition at I/E=100

Another human leukocyte elastase inhibitor was disclosed in U.S. Application Serial No. 242,294 by DiGenis et. al. filed on September 9, 1988.

B. Elastase Inhibiting Polymers.

This invention in Application Serial No. 242,294 arose from a desire to improve on the biological half-life and/or potency of the elastase enzyme peptide inhibitors provided by the same inventors in U. S. Patent 4,643,991. The inventors unexpectedly discovered that if multiple units of the known peptide inhibitors were covalently bound to a flexible, linear polymer the product polymers had a surprisingly high biological half-life and/or potency with respect to the inhibition of the elastase enzyme.

The polymers provided herein have the formula

$P\text{-}(L\text{-}R)_q$

wherein

P is a polymer comprising at least one unit of the formula $(A_mB_n)$, wherein $(A_mB_n)$ is substantially nonbiodegradable and has an average molecular weight of about 1,000 to 500,000 daltons, m and n may be

the same or different and are about 5 to 3,000, and A and B may be the same or different and at least one of A and B is capable of covalently binding to one of L and R;
R is a compound selected from the group consisting of a compound C of the formula

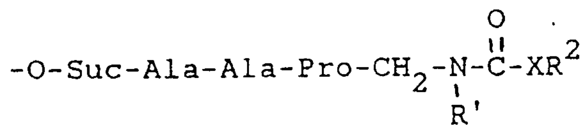

wherein
X is oxygen or sulfur;
R′ is selected from the group consisting of straight and secondary branch-chained ($C_1$-$C_4$) alkyl, ($C_2$-$C_3$) alkenyl, ($C_2$-$C_4$) alkynyl, ($C_3$-$C_6$) cycloalkyl and benzyl, and

$R^2$ is selected from the group consisting of substituted or unsubstituted phenyl, wherein the substituents are selected from the group consisting of nitro, pentafluoro, benzyl, $CH_2CF_2CF_2CF_3$, 1-lower alkyl tetrazolyl, 1-phenyltetrazolyl, 2-thioxo3-thiazolidinyl-, pyridyl and benzothiazolyl, provided that when $R^2$ is p-nitrophenyl R′ is other than tert-butyl, benzyl or cyclohexyl, and when X is sulfur $R^2$ is other than benzyl;
a compound D of the following general formula

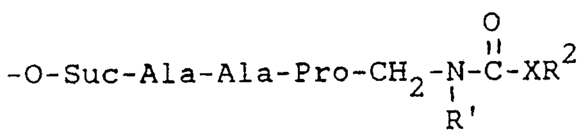

wherein
X is 0 or S,
$R^2$ is selected from the group consisting of phenyl, nitrophenyl, fluorophenyl, $-CH_2CF_2CF_2CF_3$, 1-lower alkyltetrazolyl, 1- phenyltetrazolyl, benzyl, 2-thioxo-3-thiazolidinyl, pyridyl and benzothiazolyl, and
R′ is selected from the group consisting of straight or secondary branch chained ($C_1$-$C_4$) alkyl, ($C_2$-$C_3$) alkenyl, ($C_2$-$C_4$) alkynyl, ($C_3$-$C_6$) cycloalkyl, and benzyl, provided that when $R^2$ is p- nitrophenyl R′ is other than tertiary-butyl, benzyl or cyclohexyl, and when X is sulfur $R^2$ is other than benzyl; and
a compound E of the formula

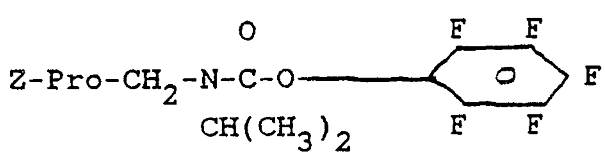

wherein
Z is MeO-Suc-Ala-Ala.
each said R being covalently bound to L or to one of A and B,
L is selected from the group consisting of a covalent bond and a linker group which is covalently bound to R and one of A and B; and
q is about 1 to m + n.

These polymers are suitable for the inhibition of the activity of the elastase enzyme both in vitro and in vivo. When they are utilized for inhibiting the elastase enzyme in vivo, solely pharmaceutically-acceptable polymers are to be utilized. These are known in the art and need not be specifically referred to herein.

When the polymers of this invention are utilized in the in vitro inhibition of the enzyme they need not be pharmaceutically- acceptable. Accordingly, a significantly larger number of polymers are ultimately suitable for the design of the inhibitory polymers of this invention for use in vitro.

In general the polymers suitable for use in this invention are water soluble polymers, and preferably polymers having a flexible backbone structure which are not easily biodegradable and which consequently have a prolonged biological half life. Even more preferred are polymers which are water soluble and substantially non-biodegradable but which also have a flexible polymer backbone. A high flexibility exhibited

by the polymer is helpful in increasing the accessibility of the polymer bound inhibitory molecule to the enzyme.

Suitable polymers for use in this invention are polymers containing amide bonds in the main chain. Particularly useful are derivatives of synthetic polyamino acids, examples of which include random copolymers of alpha hydroxy alkyl-D,L-aspartamide, e.g., poly alpha -[N-(2-hydroxyethyl)-D,L-aspartamide] in which a fraction of 2-hydroxyethyl side-chains is replaced by said appended reactive moiety.

Other examples of suitable polymers include polysaccharide derivatives, especially derivatives of dextran, cellulose, carboxymethyl cellulose, alginic acid and hyaluronic acid or combinations thereof or combinations with other polymers. Yet another example of suitable polymers with oxygen atoms in the main polymer chain are polyether polymers, examples of which include polyethyleneglycol (polyoxirane), divinylethermaleic acid copolymer (pyran copolymer, DIVEMA), and the like.

Examples of polymers with alpha-C-C- backbone suitable for use in this invention are copolymers prepared from mixtures of different types of monomers. One such group is a polymer formed by mixing one type of monomer which has reactive appended moieties and another type of monomer lacking such moieties. Particularly suitable are copolymers derived from hydrophilic vinylic and/or acrylic type monomers, examples of which include N-2-vinylpyrrolidone, 2-hydroxypropylmethacrylamide, 2-hydroxyethyl methacrylate and other hydrophilic esters and amides of arylic and methacrylic acid which are well known in the art. Suitable monomers containing appended reactive moieties for preparation of copolymers for use in this invention include, e.g., maleic acid anhydride and reactive esters of acrylic and methacrylic acid. Particularly suitable are, e.g., glycidyl acrylate, glycidylmethacrylate, p-nitrophenyl, N-hydroxysuccinimide, pentachlorophenyl or/and pentafluorophenyl esters of methacrylic and acrylic acids, wherein the alkoxy moiety of the reactive ester can be either bound directly to the carbonyl of methacrylic or acrylic acid or it can be bound via a spacer linker. Suitable spacer linkers for use in these types of polymers are generally known in the art. Examples of particularly suitable polymers include poly(N-vinylpyrrolidone), copoly-(N-vinylpyrrolidone-co-maleic acid anhydride), copoly-(N-vinylpyrrolidoneco-methacryloyl-N-Hydroxysuccinimide, copoly(N-(2-15 hydroxypropyl) methacrylamide-co-methacryloyl p-Nitrophenyl ester) and other copolymers formed by the monomers indicated above.

The linkers or spacers optionally incorporated in the polymer-bound inhibitors of the invention must contain at least two reactive groups. One of the reactive groups must be capable of covalently bonding to the appended moiety present in at least some of the monomer units contained in the polymer. The other reactive group must be capable of covalently bonding to a reactive group present in the free inhibitor molecule which is not involved in the binding to the active site of the enzyme. Suitable linkers are known in the art and need not be specifically described herein. One group of linkers which has been found suitable for use with this invention is that encompassing flexible backbone hydrocarbons containing at least two reactive groups. Suitable are reactive groups such as hydroxyl, sulfhydryl, amino, carboxyl, hydrazino and hydrazido, among others. However, other groups may also be utilized. The length of the linker or spacer may vary as desired for particular applications. Typically, ($C_2$-$C_{20}$) hydrocarbon linkers are utilized, preferably linear hydrocarbons. However, other types of molecules may also be incorporated herein.

A particularly suitable type of linker has been found to be those comprising ($C_1$-$C_{20}$) hydrocarbons having covalently bonded substituents to the first and last carbon atoms such as hydroxy amines. Other examples suitable for use in this invention are alpha, omega-diamines, alpha, omega-diamino alcohols and alpha, omega-diamino acids.

The novel substituted carbamate compound polymers, the pharmaceutical compositions containing them and the method for using these polymers are exemplified in the specific inhibition of porcine pancreatic elastase and human leukocyte elastase without affecting the similar serine dependent proteases, bovine pancreatic trypsin and chymotrypsin.

It is known from the art that proteases from polymorphonuclear leukocytes and macrophages, especially elastases (human leukocyte HL elastase and cathepsin G) appear to be responsible for chronic tissue destruction associated with inflammation, arthritis and emphysema. During infection or inflammation, the normal lung is protected from proteolytic digestion by the protease inhibitor, $alpha_1$-antitrypsin. This protective mechanism appears to be non-operative in individuals with an $alpha_1$-antitrypsin deficiency due to genetic or other causes. Synthetic elastase inhibitors capable of replacing $alpha_1$-antitrypsin are therefore useful in the treatment of pulmonary emphysema and related diseases.

According to the present invention, a class of known compounds containing carbamate functionality and oligopeptides which are active-site directed inhibitors of elastase in animals and humans have been found to exhibit an increased biological half-life and/or potency when multiple units thereof are bound to a substantially non-biodegradable polymer. Polymers with multiple-bound peptidyl carbamate chains therefore provide an opportunity to incorporate multiple inhibitory moieties into a single unit, thereby increasing the

efficiency of the transfer of the acrylating moiety to the active site of the enzyme. This, in turn, optimizes the affinity of the polymer inhibitor towards the enzyme in comparison with the low-molecular weight inhibitory peptides themselves.

The nature of the acylating moiety can be varied to optimize the duration of enzymatic inactivation as desired.

It is theorized that the mechanism of the invention takes advantage of the fact that carbamate esters react with proteases and esterases at the carbonyl carbon by losing the alkoxy portion thereof and transferring the carbamylating moiety to the active side of the enzyme. Deacylation then leads to the recovery of enzymatic activity.

Suitable carbamate compounds which are active in accordance with the above proposals as elastase inhibitors are various. These compounds are carbamates substituted by oligopeptides and may generally be described by the following general formula C

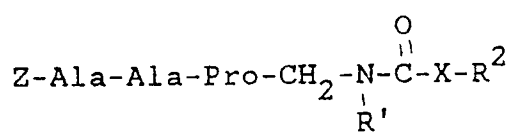

wherein
Z is selected from the group consisting of R″O-Suc-, wherein R″ is $(C_1-C_3)$ alkyl, $CF_3CO$- and.or a linker to a polymer.
X is oxygen or sulfur;
R′ is selected from the group consisting of straight and secondary branch-chained $(C_1-C_4)$ alkyl, $(C_2-C_3)$ alkenyl, $(C_2-C_4)$ alkynyl, $(C_3-C_6)$ cycloalkyl and benzyl, and
$R^2$ is selected from the group consisting of substituted or unsubstituted phenyl, wherein the substituents are selected from the group consisting of nitro, pentafluoro, benzyl, $CH_2CF_2CF_2CF_3$, 1-lower alkyl tetrazolyl, 1-phenyltetrazolyl, 2-thioxo3-thiazolidinyl-, pyridyl and benzothiazolyl, provided that when $R^2$ is p-nitrophenyl R′ is other than tert-butyl, benzyl or cyclohexyl, and when X is sulfur $R^2$ is other than benzyl.

In more preferred embodiments, the inhibitory peptides may be described by the following general formulas D or E

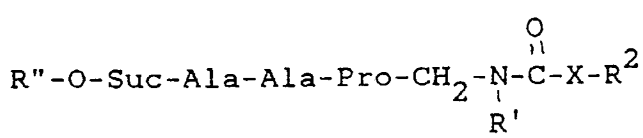

wherein
R′ is $(C_1-C_3)$ alkyl or a suitable linker to the polymer, X is oxygen or sulfur, and $R^2$ is selected from the group consisting of phenyl, fluorophenyl, nitrophenyl, 1-phenyltetrazolyl, 1-lower alkyl tetrazolyl, benzyl, 3-thiazolidinyl, pyridyl and benzothiazolyl, and R′ is straight or secondary branch chained $(C_2-C_4)$ alkyl, $(C_2-C_4)$ alkenyl, and $(C_2-C_4)$ alkynyl, provided that when $R^2$ is p-nitrophenyl R′ is other than tertiary-butyl, and when X is sulfur $R^2$ is other than benzyl; and

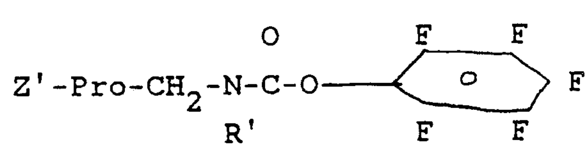

wherein
Z′ is selected from the group consisting of MeO-Suc-Ala-Ala, $CF_3CO$-Ala-Ala and a suitable linker connecting this compound with a polymer; and
R′ is as defined above but is preferably isopropyl.

Suitable linkers are known in the art for the particular type of atom groupings present herein.

In the inhibitory peptides suitable for use herein, the peptidyl carbamates are those wherein the amino portion contains the oligopeptide and the peptide portion is so chosen as to increase the specificity of the carbamate ester for elastase.

The peptides for the individual inhibitory compounds may be prepared by a sequential series of reactions beginning with L-proline protected on the ring nitrogen with ultimate coupling to the peptide. In the first step, an N-protected proline, e.g., N-t-Boc-L-proline, is reacted with diazomethane to obtain the diazoketone followed by treatment with HCL to obtain the chloromethyl ketone protected L-proline. The chloromethyl ketone thus obtained is reacted with the appropriate amine $H_2NR'$ to form the protected amine derivative. The amine is in turn reached with the appropriate chloroformate or thiochloroformate and deprotected by reaction with an acid such as HCl to provide the HCl salt. This compound is then coupled with Z-Ala-Ala by the mixed carbonic anhydride method to provide the compounds of the invention. The Z-Ala-Ala compounds are obtained by reaction of Ala-Ala with methyl succinic acid N-hydroxy succinimide ester when Z = R″O- Suc, e.g., MeOSuc. These Z-Ala-Ala intermediates may be prepared according to the following scheme:

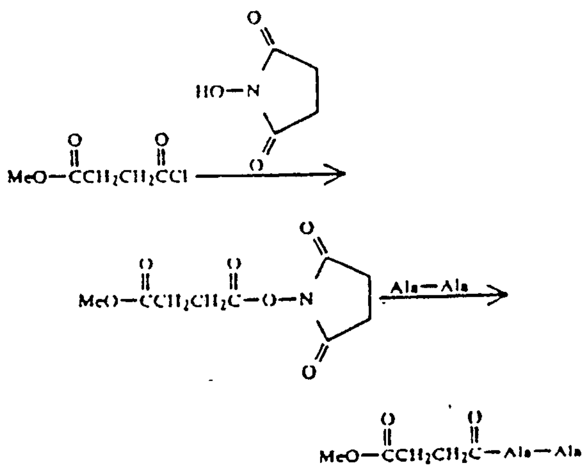


In conducting these reactions, the L-proline in the initial step is protected on the ring nitrogen by reaction with any suitable protective agent known to the art so that reaction will occur on the carboxylic acid portion of the molecule. Preferably, the nitrogen atom in the ring is protected with a known protective agent, such as t-BOC. For example, t-BOC-Pro is available commercially from Sigma Chemical Company, St. Louis, Missouri. The protected proline is reacted with diazomethane by the method of Penke et al. (B. Penke, J. Czombos, L. Balaspiri, J. Peters and K. Kovacs, Helv. Chim. Acta., 53:1057 (1970)). The resulting chloromethylketone is then reacted with the appropriate amine. This reaction is preferably conducted in a solvent solution, such as a lower alkyl alcohol, and preferably in the presence of an alkali metal iodide. The reactants are mixed under cool temperatures and then reacted at 50 to 750 °C to complete the reaction. The evolved HCl is neutralized, as with a sodium carbonate solution, and extracted. This intermediate is then reacted with the appropriate chloroformate or thiochloroformate and deprotected with hydrogen chloride to form the carbamate portion of the molecule. This molecule is then coupled with the peptide portion of the molecule to form the final product.

This reaction procedure may be illustrated as follows:

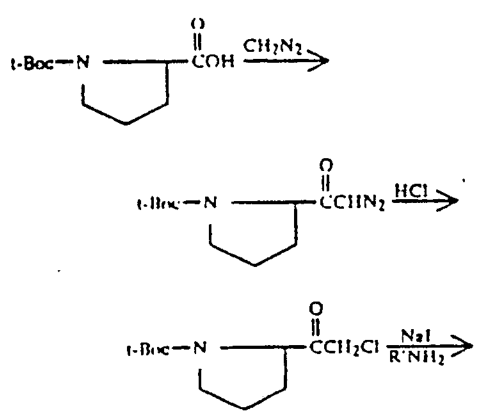

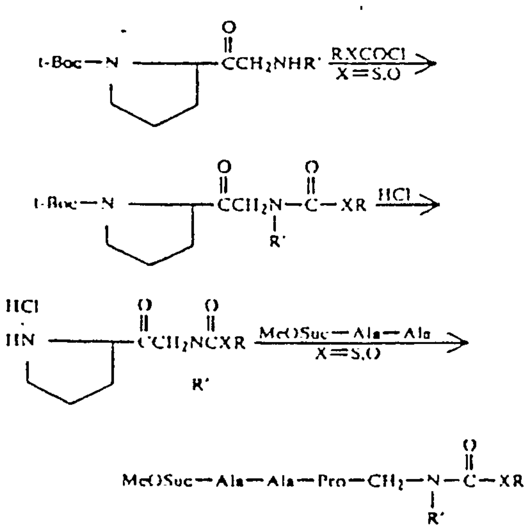


As pointed out above, the polymers of the invention may be employed as specific active site directed inhibitors of the enzyme elastase. For this purpose the polymers are preferably combined with a pharmaceutically acceptable carrier for the in vivo administration by injection or in the oral form.

Conventional adjuvants and carriers may be employed in combination with about 1 to 90 wt% of the active polymer. The polymers may be administered to animals or humans at dosage amounts of about 0.1 mg/kg to 300 mg/kg, preferably about 1 mg/kg to 30mg/kg, and still more preferably an average amount of about 12 mg/kg.

The following examples illustrate preferred embodiments of the invention but the invention is not considered to be limited thereto. In the examples and throughout this specification, parts are by weight unless otherwise indicated.

In synthesis of the compounds of the invention, melting points were determined on a Thomas-Hoover Unibelt apparatus and are uncorrected. ′H NMR spectra were obtained using a Varian EM-360 (60MHZ) or EM-390 (90MHZ) spectrometer. Infrared (IR) spectra were recorded on a Perkin-Elmer 567 spectrophotometer. Microanalyses were performed by Atlantic Microlab, Inc., Atlanta, Georgia or by Micro Analysis, Inc., Wilmington, Delaware.

Reactions were routinely followed by thin layer chromatography (TLC) using Whatman MK6F silica gel plates. Spots were detected by Spectrophotometry (254 nm), iodine or HBr-Ninhydrin spraying. Column chromatography was carried out using Silica Gel 60 (Merck, Darmstadt, Germany). All compounds were identified by spectral data and elemental analysis.

The loading of the inhibitors onto the polymers either directly or by means of a linker spacer is conducted via chemical reactions which are known in the art and need not be described here in detail. The degree of loading, i.e., density of the PC units along the polymer chain, can be varied in such a way that it is the most appropriate in accordance with the loading desired. This can be attained by varying the experimental conditions, e.g., the number of appended reactive moieties on the polymer chain, the number of spacer groups and/or the ratio of PC to the polymer in the reaction. In the examples appended hereafter specific reaction schemes are described but are by no means intending to be limiting of the invention.

Having now generally described this invention, the same will be better understood by reference to certain specific examples which are included herein for purposes of illustration only and are not intended to be limiting of the invention or any embodiment thereof, unless so specified.

## EXAMPLES

### EXAMPLE 68:

A peptidyl carbamate inhibitor (compound 65) suitable for the preparation of a polymer-bound inhibitor is prepared in accordance with the invention according to Scheme 7 hereinbelow.

Scheme 1

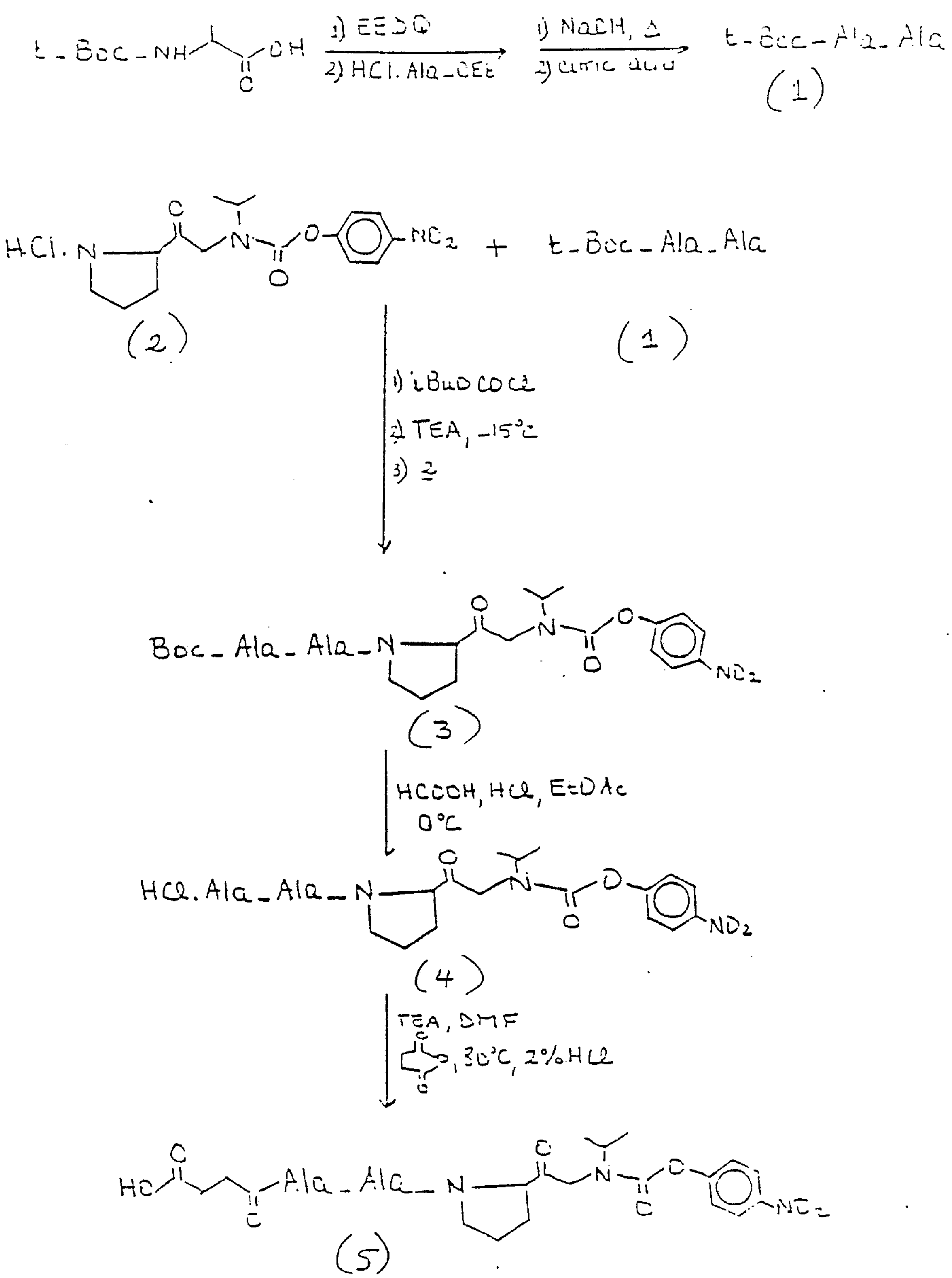


The individual steps of the procedure are described hereinbelow in greater detail.

Example 69: Synthesis of t-Boc-L-alanyl-L-alanine (Compound 61)

To a solution of t-Boc-L-alanine (5.9 g, 31.2 mmol) and L-alanine ethyl ester hydrochloride (4.8 g, 31.2 mmol) in $CH_2Cl_2$ MeOH (4:1) is added triethyl amine (4.3 ml, 31.2 mmol) with stirring at room temperature.

To this reaction mixture is added 1-ethoxycarbonyl-2-ethoxy-1,2-dihydroquinoline (EEDQ) (8.0 g, 32.3 mmol) and stirring is continued overnight.

The mixture is then extracted into $CH_2Cl_2$ (100 mml) and washed with 10% citric acid (50 ml x 3) and 5% $NaHCO_3$ (50 ml). The organic layer is dried ($Na_2SO_4$) and concentrated in vacuo.

The resulting oil is dissolved in EtOH (100 ml) and 30 ml of 1.0 N KOH are added and the mixture stirred at 35-40° C overnight. The evaporation of EtOH is followed by addition of citric acid (14 g in 50 ml $H_2O$)) to neutralize the excess KOH.

The reaction mixture is then extracted into EtOAC/tetrahydrofuran (THF) (150 ml x 2 of 1:1 mixture) and dried ($Na_2SO_4$). The solvent is then evaporated to give the product (1) which is purified by silica gel column chromatography ($CH_2Cl_2$ EtOAC 10:1) (12.1 g, 74%) mp. 89-91° C in accordance with Doyle, B.B.; Traub, W.; Lorenz, G.P.; Brown, E.R.; Blout, E.R., J. Mol. Biol., 51:47 (1970).

Example 70: Preparation of p-Nitrophenyl N-(t-Boc-L-alanyl-L-alanyl-L-prolylmethyl)-N-isopropyl carbamate (Compound 63)

To a solution of compound 1 (1.0 g, 3.9 mmol) in THF (30 ml) cooled to -30° C is added N-methylmorpholine (0.46 mL, 4.2 mmol) and the mixture is stirred for 5 min. Isobutyl chloroformate (0.55 ml, 4.2 mmol) in THF (2 ml) is added and stirring is continued for 10 min at -15° C.

To this reaction mixture is added a suspension of compound 2 (1.3 g, 3.5 mmol) and N-methyl morpholine (0.46 ml, 4.2 mmol) in acetonitrile (40 ml) at -40° C. Stirring is continued for 1 hr at room temperature.

The reaction mixture is then filtered and the filtrate extracted with $CHCl_3$, washed with 10% citric acid (3 x 10 ml), dried ($Na_2SO_4$) and the solvent evaporated to give an oil.

Saturation with ethyl acetate (EtOAC) gives the solid which is purified by silica gel column chromatography (6% $CH_3OH/CH_2Cl_2$) to give compound 3 (90%).

The characteristics of compound 3 are found to be mp:170-172° C

IR (K Br) $V_{max}$ 1730, 1650, 1520, 1345, 1190, 1155 cm -1;

NMR ($CDCl_3$) delta 8.20 (2H, d, J = 9Hz), 7.20 (2H, d, J = 9Hz), 4.1-4.5 (tH, m), 3.5-3.7 (3H, m), 2.0 (4H, m), 1.5 (9H, s), 1.7-1.6 (21H, m).

Example 71: Synthesis of p-nitrophenyl N-(L-alanyl-L-alanyl-L-1 - L - proplylmethyl-N- is opropyl carbamate hydrochloride (Compound 64)

Formic acid (1.25 ml) is added to a stirring solution of a compound 3 (0.7 g, 1.02 mmol) in EtOAC (7 ml ). Anhydrous HCl is then bubbled through the reaction mixture and the reaction is followed by thin layer chromatography (TLC). The solvent is evaporated and the formic acid is turned into an azeotropic mixture by addition of n-heptane. The resulting oil is used in the next step without further purification.

Example 72: Synthesis of p-nitrophenyl N-(succinyl-L-alanyl-L-alanyl-L-prolylmethyl) -N-isopropyl carbamate (Compound 65)

To a solution of compound 64 (1.5 g, 2.9 mmol) in DMF (20 ml) are added $Et_3N$ (0.5 mL, 3.6 mmol) and succinic anhydride (0.35 g, 3.6 mmol) and the mixture is stirred at 80° C for 1.5 hr. Diethyl ether (60 ml) is then added to the cooled mixture, the precipitated $Et_3N.HCl$ is filtered and the filtrate evaporated to give a pale yellow solid . The product is then triturated with 2% HCl, filtered and recrystallized from tetrahydrofuran (THF)/ether to give 1.6 g of compound 65 (PCl) (94%).

The characteristics of compound 65 are as follows.

mp: 185-186° C

IR (K Br) $V_{max}$ 3300 2700, 1780, 1750, 1651, 1560, 1200 $cm^{-1}$ NMR (DMSO $d_6$) delta 8.3 (2H, d, J=9 Hz), 1.06-1.6 (26H, m).

The anal. calculated for $C_{26}H_{35}N_9O_{10}$ 1/2 $H_2O$ is C, 53.43; H, 6.17; N, 11.94 and what is found is C, 53.34; H, 8.41; N, 11.93.

Example 73::

Peptidyl carbamate hemisuccinate prepared according to Example 68 (compound 65 of Scheme 7) is bound to the polymer carrier. The overall procedure is illustrated in Scheme 8 hereinbelow.

Scheme 2: Synthesis of Polymer-bound PC Inhibitor of Example 2

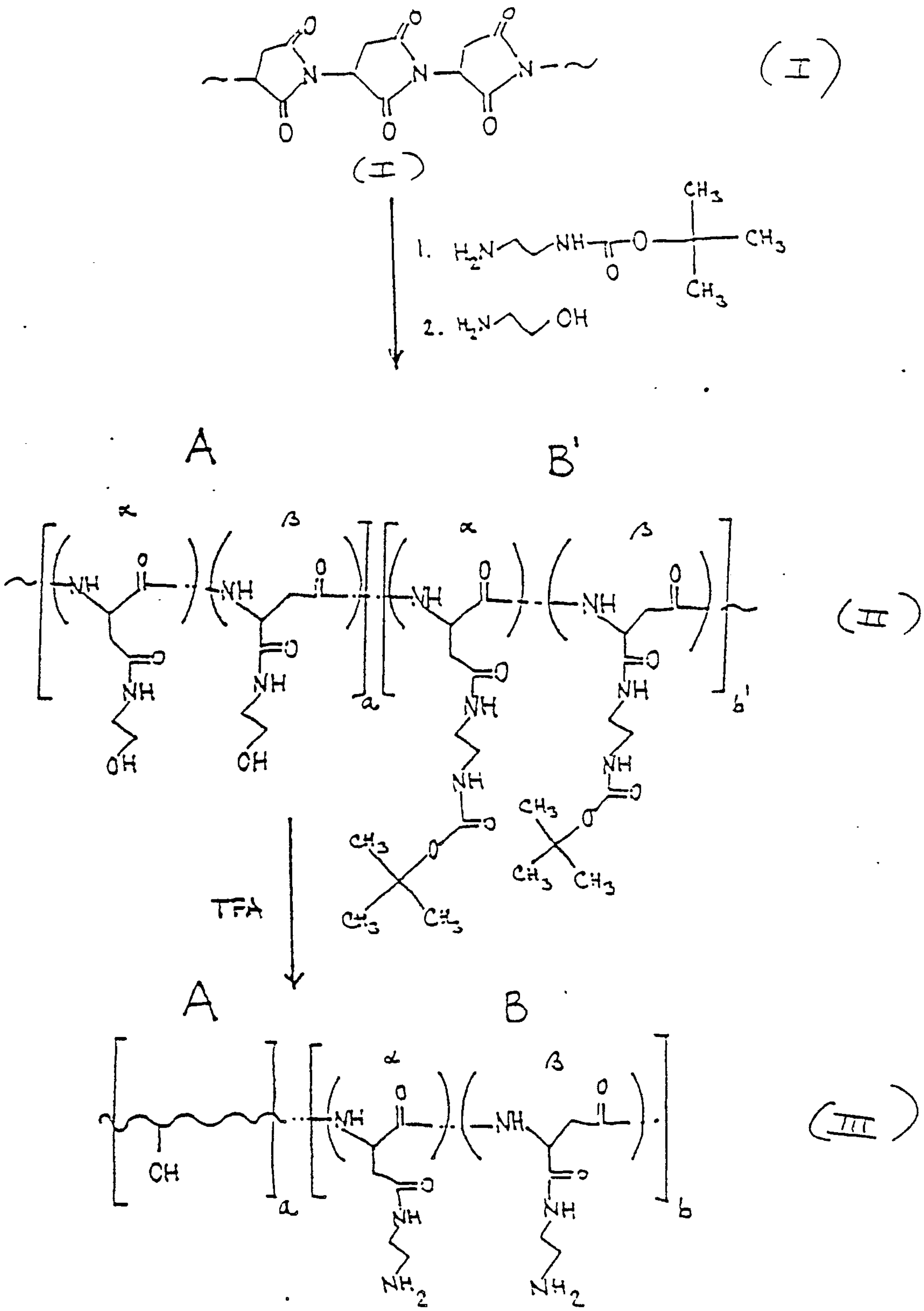

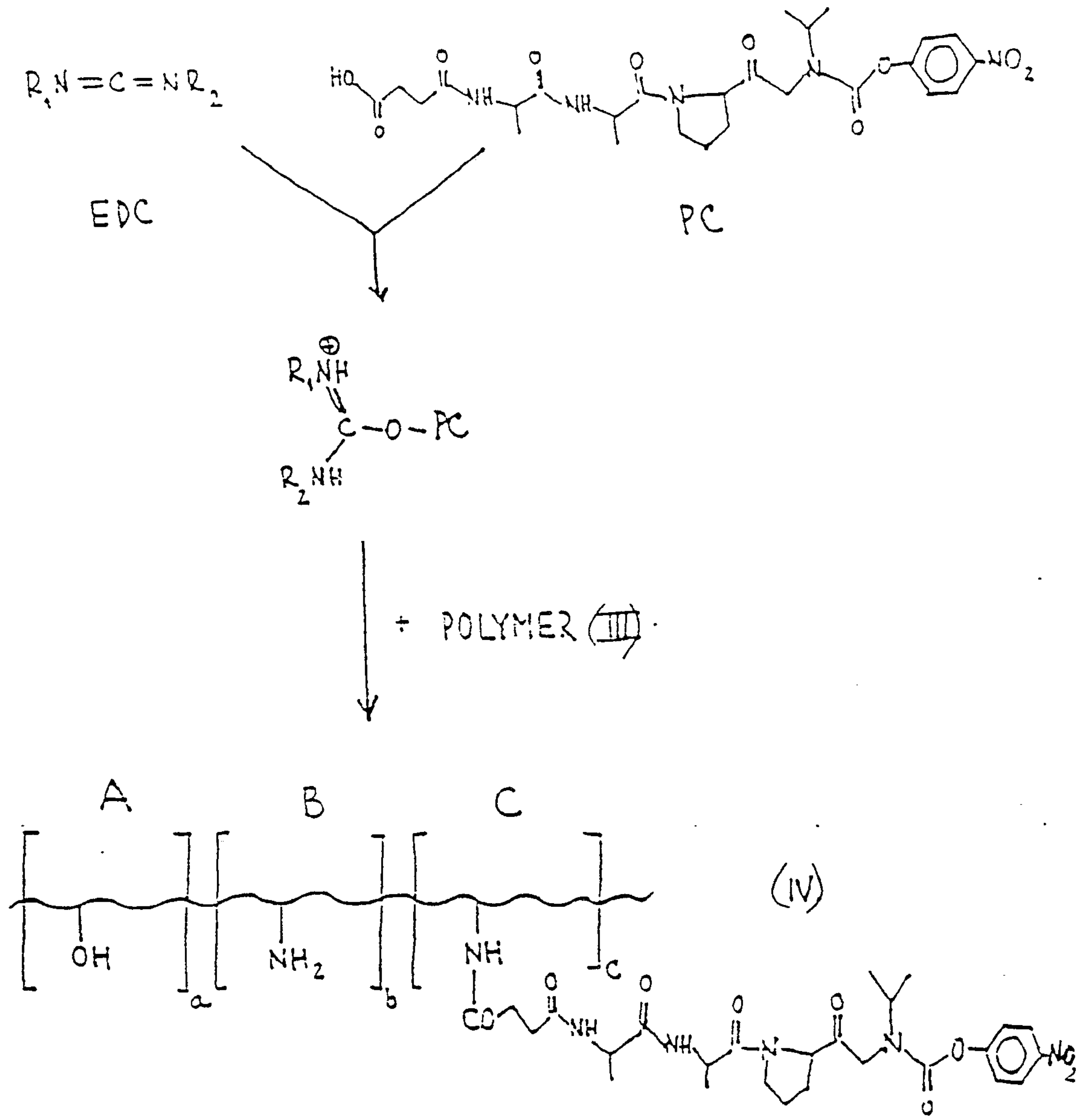


Example 74: Synthesis of a carrier polymer

Polysuccinimide (I) is prepared and fractioned accord ing to the method described by Vlasak, J., Rypacek, F., Drobnik, J., Saudek, V., J. Polymer Sci., Polymer Symp., 66:59-64 (1979).

10 g of polysuccinimide. (I) (the fraction with $M_w$ = 32,000) is dissolved in 50 ml of N,N′-dimethylformamide (D&F) and 2.80 g (0.01 mol) of mono-N-Boc1,2-diaminethane benzoate, and 0.8 ml (0.01 mol) of triethylamine are added.

The reaction mixture is left at room temperature for 4 days and then 11.0 ml (0.18 mol) of 2-aminoethanol are added and the reaction is continued for another 24 hours.

The mixture is then neutralized with acetic acid, dialyzed against water and the polymer isolated by freeze-drying.

Yield: 9.20 g PHEA(AE-BOC) (compound II of Scheme 2).

PHEA(AE-BOC) (Compound II) (8.50 g) is dissolved in 30 ml of trifluoroacetic acid. The solution is left for one hour at room temperature and then dialyzed against distilled water (Visking Dialysis Tubing, Serva).

The dialyzed solution is then concentrated to a volume of 30 ml by the ultrafiltration on an Amicon YM

10 membrane and diluted again with water up to 200 ml. The ultrafiltration is repeated in the same way 5 times. The polymer is isolated from the retentate by freeze-drying.

Yield 6.5 of PHEA(AE) (Compound (III) of Scheme 2).

Example 75: Binding of peptidylcarbamate-hemisuccinite to the polymer with aminoethyl spacer-chains (PHEA-AE).

Peptidyl-carbamate-hemisuccinate (compound 65 of (5) Scheme 1) (0.586 g, 0.001 mol) and N-ethyl-N′-(3-dimethylaminopropyl) carbodimide-hydrochloride (0.23 g, 0.0012 mol) are reacted in 4.0 ml of DMF for 45 minutes in an ice bath. Then the ice cold solution containing 2.20 g of polymer (III) (0.0012 mol of $-NH_2$) and 0.167 ml (0.0012 mol) of triethylamine in 12 ml of DMF is added thereto and the reaction mixture is stirred at 0-4 °C for 24 hours.

The polymer product is then dialyzed against a phosphate buffer pH 7.00 containing 0.15 M NaCl. The dialyzed polymer is further purified by gel permeation chromatography (GPC) on a Sephadex G25 F column (50 x 300 mm) in the same buffer and the collected polymer fractions are desalted by repeated ultrafiltration and dilution on Amicon YM 10 membrane. The polymer inhibitor is isolated by freeze-drying from water.

Yield: 1.35 g PHEA(AE-PC) (Compound IV of Scheme 8).

Example 76: Identification and characterization of the carrier polymer and the polymer-bound inhibitor

The molecular weight distribution analysis of all polymers is performed by size-exclusion chromatography (SEC) on a mixed-bed column (Sepharose CL-4B:Sephacryl S 200 SF : : Sephadex G-25 SF, 16 : 5: 3; 13 x 350 mm).

0.05 M phosphate buffer pH 7.50 containing 0.15 M NaCl is used as eluent.

The column is calibrated with standard samples of PHEA (Rypacek, F., Saudek, V., Pytela, J., Skarda, V., Drobnik, J., Makromol. Chem. Suppl. 9:129-135 (1985)).

The elution profiles are monitored by means of an 5 ISCO model 1840 Spectrophotometric detector. The values of molecular-weight averages, $M_w$ and $M_n$, and a cumulative molecular-weight distribution are calculated from the SEC data. These data are shown in Figure 2.

The contents of aminoethyl side chains in polymers III and IV are determined spectrophotometrically after the reaction of aminoethyl groups with 2,3,5trinitrobenzene sulphonic acid in accordance with Brown, H.H., Clin. Chem., 14:967, (1968). The data are shown in Table 4 hereinbelow.

Table 4

| Molecular Characteristics of the Polymer-Bound In-Inhibitor | | | | | |
|---|---|---|---|---|---|
| Polymer Structure | Composition (%) | | | Molecular weight averages | |
| | A | B | C | $M_w$ | $M_n$ |
| III | 91.2 | 8.8 | 0 | 31,600 | 21,000 |
| IV | 91.2 | 4.2 | 4.6 | 38,000 | 24,000 |
| The molecular-weight-equivalent per one PC unit: 4042, i.e., 0.247 umol PC/mg of the polymer-bound PC. | | | | | |

The content of peptidyl carbamate units in the polymer-bound inhibitor is determined from the absorption spectrum of the polymer-inhibitor (polymer IV) assuming the value of 9700 $mol^{-1}$ l.cm for molar absorptivity of the above PC inhibitor at 276 nm (the data are shown in Figure 3).

The time course of the binding reaction between the free PC and polymer III is followed by GPC. Typically, 10 ul samples of the reaction mixture are withdrawn at appropriate time intervals, diluted with

phosphate buffer (e.g., PBS) and applied onto a Sephadex G-25 SF (11x40 mm) column. The ratio between the polymer-bound PC inhibitor and the unbound low-molecular-weight PC inhibitor is determined from the areas under the respective peaks of the elution curve monitored as the optical density at 276 nm (the absorption maximum for the free PC). The results are shown in Figure 4 and 5.

Example 77

Poly-alpha, beta -(N-(2-hydroxyethyl)-D,L-aspartamide) copolymer with 6-aminohexyl spacer-chains (PHEA-AH) was prepared according to the overall procedure described in Example 2, but using mono-Nt-BOC-1,6-diaminohexane hydrochloride in place of mono-N-t-BOC-7 ,2diaminoethane. 172 mg of PHEA(AH) (0.1 mmol of -$NH_2$ groups) is reacted with 58.6 mg (0.1 mmol) of peptidylcarbamate hemisuccinate prepared in Example 1 (compound 5 of Scheme 1) and 23.0 mg (0.12 mmol of N-ethyl-N′-(3dimethylaminopropyl) carbodiimide (EDC) hydrochloride in presence of 0. 12 mmol of triethylamine in dimethylformiamide (2 ml) at temperature 0-4° C for 20 hours. The polymer product is dialised against water and further purified by GPC on a Sephadex G-25F column (Pharmacia) and the polymer inhibitor is isolated by freeze-drying from water.

Yield: 118 mg of PHEA (AH-PC). 68% of PC originally added to the reaction is bound to the polymer producing thus polymer-bound PC containing 6.2 mole % of PC side chains.

Example 78:

The copolymer of N-2-vinylpyrrolidone and 0methacryloyl-N-oxy-succinimide (p(VP-CO-MANSu)) containing 8 mole % methacryloyl-N-oxy-succinimide comonomer is prepared by the copolymerization of said comonomers in dioxane with azo-bis-iso-butyronitril as initiator. 1.46 g p(VP-CO-MANSu) copolymer (1 mmol of N-oxy-succinimide ester groups) is reacted with 0.51 g of mono-t-BOC-1,6-diaminohexane hydrochloride (2.0 mol) in presence of 2 mmol of triethylamine in DMF (10 ml) at temperature 50° C for 48 hours. The polymer product is then dialysed against distilled water, the solution is then concentrated to a volume about 5 ml and 5 ml of trifluoroacetic acid is added. The reaction mixture is left at ambient temperature for 4 hours then dialysed against distilled water and the polymer is further purified by GPC on a Sephadex G-25-F column. (Sephadex: Trademark of Pharmacia Uppsala, Sweden). The polymer poly (VP-CO-MA-AH), i.e. copolymer of N-2-vinylpyrrolidone and N-(6-aminohexyl) methyacrylamide is isolated by freeze drying from aqueous solution. The mole content of aminohexyl spacer-chains is determined to be 8.9 mole % from all monomer units of the copolymer.

149 mg of poly (VP-CO-MA-AH) (0.1 mmol of -$NH_2$ groups) is reacted with 58.6 mg (0.1 mmol) of peptidylCarbamate hemisuccinate (Compound 5 of Scheme 1) obtained in Example 1 using N-ethyl -N′ ( 3 dimethylaminopropyl) carbodiimide (EDC) hydrochloride as a coupling agent. Adopting the procedure analogous to that described in Example 2, 115 mg of the PC bound to the PVP type copolymer is obtained. The content of PC inhibitory moieties in the resulting polymerinhibitor is determined to be 4.8 mole % from all monomer units of the copolymer.

Example 79

1.62 g of dextran (MW 70,000; Pharmacia Uppsala, Sweden) is dissolved in 40 ml of 0.1 mol $1^{-2}$ borate buffer pH 8.00, 1.08 g of 1,2-epoxy-3-bromopropane is added and the mixture is vigorously stirred at 30° C for 4 hours. The reaction mixture is then extracted with ethylacetate, the aqueous layer is separated, 20 ml of concentrated ammonium hydroxide is added to it and the solution is stirred at ambient temperature for 24 hours. The solution is then neutralized, dialysed against distilled water and the resulting dextran derivative (Dextran-$NH_2$) is finally purified by GPC on a Sephadex G-25 column.

Yield 0.84g. Analysis shows 6.2 mole % of NH2 groups 15 per mole of anhydroglucose units.

58.6 mg (0. 1 nmol) of peptidyl-carbamate hemisuccinate inhibitor (compound 5 of Scheme 1) prepared according to Example 1 is reacted with 21 mg of EDC (0.11 mmol) in 1.0 ml of DMF at 0° C. After 60 minutes a solution of 260 mg of the above Dextran-$HN_2$ in 2 ml of 0.1 mol $1^{-1}$ borate buffer pH 9.00 is added and the mixture is stirred in an ice bath for another 16 hours. The reaction mixture is then diluted with 3 ml of 0.3 mol $1^{-1}$ NaCl and applied onto a Sephadex G-25 column. The dextran-bound PC inhibitor is isolated from the collected high-molecular-weight fraction by freeze-drying.

Yield: 210 mg, 3.2 mole % of PC inhibitory units per mole of anlydroglucose units. The inhibition of the elastase activity by the polymer-bound inhibitors prepared in accordance with the invention is evaluated according to the procedure described in detail hereinbelow.

Example 80: Enzyme Activity Inhibition Test

All enzyme assays are performed spectrophotometrically at 25 C using CARY 219 or 2200 Varian Spectrometers. The activity of PPE is measured using 5-Boc-L-alinine p-nitrophenyl ester (Boc-Ala-ONP), as the substrate and monitoring the absorbance at 347.5 nm (p-nitrophenol). The activity of HLE is measured using methoxy succinyl-L-analyl-L-alanyl L-prolyl-L-valine pnitroanilide (MeO-Suc-Ala-Ala-Pro-Val-NA), as the substrate and following the absorbance at 410 nm (pnitroaniline). Active inhibitors are tested against other serine dependent proteolytic enzymes such as tryps in and chymotryps in using their respective substrates, N-benzoyl-L-arginine ethyl ester, N-benzoyl-L-tyrosine ethyl ester and monitoring the absorbance at 253 and 256 respectively.

Screening Test (Time Course) Assays for PPE and HLE

Buffer:
for PPE 0.05 M sodium dihydrogen phosphate buffer, pH 6.5,
for HLE 0.1M HEPES (N-2 hydroxy ethyl piperazineN-2-ethanesulfonic acid) buffer pH 7.4 containing 0.5 M sodium chloride and 10% dimethyl sulfoxide.
Substrate:
for PPE, t-Boc-Ala-ONP (1.0 x $10^{-2}$M in methanol),
for HLE, MeO Suc-Ala-Ala-Pro-Val-NA (1.0 x $10^{-2}$ M in dimethyl sulfoxide).
Inhibitor: 2.0 x $10^{-3}$M in dimethyl sulfoxide
Enzyme:
for PPE: 1.5 mg in 5 ml of 0.05 M sodium phosphate buffer, pH 6.5.
for HLE: 1 mg in 2.4 ml of 0.05 M sodium acetate buffer, pH 5.5.
Procedure:

0.1 ml of the inhibitor and 0.1 ml of the substrate are added to 2.7 ml of sodium phosphate buffer in two quartz cuvettes. The cuvettes are thermally equilibrated in the spectrophotometer for two minutes and the absorbance balanced at 347.5 nm.

PPE (0.1 ml in buffer) is added to the sample cuvette, and 0.1 ml buffer is added to the reference cuvette. The mixture is shaken for twenty seconds and the increase in absorbance monitored for thirty minutes.

Control experiment:

0.1 ml dimethyl sulfoxide are added to both cuvettes instead of inhibitor solution.

Methods of Enzymatic Assay

Example 81: Steady state kinetics for determination of $K_i$ of polymer-bound PC inhibitor and free PC inhibitor

Reagents:
Buffer:
0.1 M HEPES (N-2 hydroxy ethyl piperazine-N-2ethanesulfonic acid) buffer pH 7.5 containing .05 M NaCl and 10% dimethyl sulfoxide.
Substrate: MeO-Suc-Ala-Ala-Pro-Val-NA, (1.27, 8.47, 4.23) x $10^{-3}$M in dimethyl sulfoxide.
Enzyme:
for HLE: 0.27 mg in 4.2 ml of 0.05 M sodium acetate buffer, pH 5.5.
Inhibitor:

Polymer-bound inhibitor (2.09, 1.05, 0.42, 0.21) x $10^{-5}$M in 0.05M potassium dihydrogen phosphate buffer, pH 6.5.
Free inhibitor (6.98, 3.49, 1.74, 0.70) x $10^{-3}$M in dimethyl sulfoxide.
Procedure:

33 ml of substrate, 33 micro of inhibitor and 33 ul of dimethylsulfoxide (or 33 micro of HEPES buffer for the free PC inhibitor experiments) are added ta 1.9 ml of HEPES buffer in each of two quartz cuvettes. The curvettes are thermally equilibrated in the spectrophotometer at 25° C for two minutes, and the absorbance is balanced at 410 nm.

The enzyme (33 micro) is added to the sample cuvette and 0.05M sodium acetate buffer (33 micro) added to the reference cuvette. After shaking the mixture for fifteen seconds, the increase in absorbance is monitored for three minutes at 410 nm.

Controls:

Controls are run by adding 33 micro of 0.05M potassium dihydrogen phosphate buffer pH 6.5 in place of polymer-bound PC inhibitor (04 33 micro of dimethylsulfoxide in place of free PC inhibitor).

The Ki values are determined from Dixon plots and slope replots of Lineweaver-Burke plots. The Ki for polymer bound free PC inhibitor is 8.0 x $10^{-7}$M and the $K_i$ for the free PC inhibitor is 0.4-1.0 x $10^{-5}$M.

Example 82: Preincubation method (Percent Enzymatic Activity Remaining) (for Ki Determination)

Regents:
Buffer:
0.IM Hepes buffer pH 7.5 containing 0.05M sodium chloride and 10% dimethyl sulfoxide, Substrate:
MeO-Suc-Ala-Ala-Pro-Val-NA, 1.26 x $10^{-2}$M in dimethyl sulfoxide.
Inhibitor:

Polymer bound free PC inhibitor (4.56, 1.82, 0.91, 0.45) x $10^{-5}$M in O.IM Hepes buffer pH 7.5 containing 0.05M NaCl and 10% dimethyl sulfoxide.
Enzyme:
2.1 x $10^{-6}$M in 0.05 M sodium acetate buffer pH 5.5. Procedure:
33 micro of the inhibitor and 33 ul of the enzyme are added to 1.9 ml of the O.1M Hepes buffer in a quartz cuvette and mixed. The reference cuvette contains 33 micro of the inhibitor, 33 micro of 0.05 M sodium acetate buffer and 1.90 ml of 0.1M Hepes buffer.

The cuvettes are thermally equilibrated in a spectrophotometer for 2 minutes and the absorbance balanced at 410 nm. At the end of a predetermined incubation period (2. 5 to 20 minutes), 33 micro of substrate is added to both the reference and sample cuvettes and the mixtures are shaken for 15 seconds. The reaction is then monitored for 3 minutes and the release of p-niroaniline recorded at 410 nm.

Control:

Control is conducted as above, except that 33 micro of 0.1 M Hepes buffer is used instead of inhibitor, and is considered as 100% activity.

Ki values are obtained from a reciprocal plot of $k_{obs}$ (pseudo first-order rate constant for inhibition) vs. inhibitor concentration.

Example 83: Determination of Presence or Absence of Tight Binding for Polymer-bound free PC 10 Inhibitor

Reagents:
Buffer:
0.1 M Hepes buffer containing 0.05 M NaCl and 20% dimethyl sulfoxide pH 7.5.
Substrate:
MeO-Suc-Ala-Ala-Pro-Val-NA, 4.23 x $10^{-3}$M in dimethyl sulfoxide.

Inhibitor:
Polymer bound or free PC inhibitor (5.58, 2.79, 20 2.15, 1.86, 1.40, 0.933, 0.698, 0.349, 0.209, 0.105) x $10^{-4}$M in 0.1 M Hepes buffer containing 0.05 M NaCl and 10% dimethyl sulfoxide pH 7.5.
Enzyme:
2.25 x $10^{-6}$M in 0.5M sodium acetate buffer pH 5.5. Procedure:
33 micro of the substrate and 33 micro of the inhibitor are added to 1.9 ml of the 0.1M Hepes buffer in each of two quartz cuvettes, thermally equilibrated for 2 minutes at 25° C, and absorbance balanced at 410 nm. The enzyme (33 micro) is added to the sample cuvette and the mixture shaken for 15 seconds. The release of pnitroaniline is followed at 410 nm for 3 minutes. 0.05M sodium acetate buffer (33 micro) is added to the reference cuvette in place of the enzyme.

Control Experiment:

Conducted as above, but 33 micro of the O.1M Hepes buffer is added in place of the inhibitor.

A velocity versus inhibitor concentration plot is used to demonstrate the presence of tight binding between polymer bound PCI and the enzyme.

The data obtained according to the above procedures show that the elastase inhibitory capacity (EIC) of the free peptidyl carbamate inhibitor is not only retained, but improved upon its binding to a water-soluble polymer.

The dissociation constant of the enzyme-inhibitor complex (Ki) can be used as an index of inhibitory potency. When this is done, it is found that upon binding the PC inhibitor to the polymer an at least 700 fold decrease in the $K_i$ value is observed indicating improved potency. Figure 1 provides comparison of the inhibitory activity of the free PC inhibitor (PC-I) polymer-bound-PC inhibitor (PHEA-(AE)-PC) (P-PCII) and a natural inhibitor of elastase, i.e. alpha-lproteinase inhibitor (alpha$_1$-PI). Even if the polymerbound PC inhibitor contains such a low content of PC units as 1.6 mole % in this case it is at least as active as alpha$_1$-PI.

The polymer PHEA has been shown to lack toxicity (Neri, P.; Antoni, G.; Benvenute, F.; Cocola, F. and Gazzei, G., J. Med. Chem., 16:893-897 (1973). In fact, it is not possible to determine its LD50 in mice and rats owing to its extreme tolerability over a period of 40 days. Daily i.v. injections of PHEA at doses 10 fold greater than those likely to be used in man do not induce any significant changes in total weight gain or organ weight. Moreover, no adverse effect of PHEA on the biosynthetic mechanism of the serum proteins or of blood cells has been observed (Neri et al, supra).

Additionally, PHEA has been shown to lack antigenecity. When PHEA is injected in rabbits and guinea pigs according to a wide number of immunization patterns no evidence of an immune response to it is found. (Neri et al, supra).

C. Method of Using the Disclosed Human Leukocyte Elastase Inhibitors.

The method of treatment disclosed in this invention arose from a desire to improve over prior art methods for treating ocular scarring or fibroblast proliferation, particularly when it occurs in the cornea of the eye.

The inventors have found for the first time that if an HLE inhibitory agent is applied to a subject's eye it has an inhibitory effect on the proliferation and growth of fibroblasts or scar tissue. For the first time the inventors have shown that HLE inhibitors have an ophthalmological application.

Thus, the present invention relies on the therapeutic application of HLE inhibitory agents to the corneal area of the eye to prevent and/or reduce corneal scarring or fibroblast proliferation as well as new vascularization. These conditions have been found to be associated with a variety of pathological states of the eye such as infections, burns and mechanical and chemical injury, among others.

The inhibitory agents utilized in the present methods may be applied to the eye topically, intraocularly, by injection or through a contact lens. Preferred is topical. By means of example the agents may be applied as a solution in a solvent which is not detrimental to the functions of the eye. Typically, an aqueous solution of the HLE inhibitory agent may be utilized. However, given that some HLE inhibitory agents are cleared very rapidly from the eye, they may also be bound to a water soluble or hydrophilic polymer to ensure a more prolonged residence in the desired area. The aqueous solution may also contain other ingredients as is known in the art.

This invention thus provides a method of reducing corneal scarring or fibroblast proliferation which comprises applying to an area of a subject's eye afflicted with the condition a corneal scar-reducing or fibroblast proliferation-reducing amount of an HLE inhibitory agent under conditions and for a period of time

effective to attain the desired effect.

Any HLE inhibitory agent may be utilized when practicing the method of the invention. Typically, many HLE inhibitory agents are known in the art as discussed above and need not be further described herein. By means of example, inhibitory agents which are suitable for use with this invention are those described in U.S. Patent 4,643,991 to Digenis et al. and the peptidyl carbanate inhibitors disclosed hereinbefore. However, other HLE inhibitory agents may also be utilized for practicing the present method.

As already indicated above, the HLE inhibitory agents may be bound to a hydrophilic or water-soluble polymer in order to extend their residence in the area to which they are applied. The HLE inhibitory agents may be covalently bound to any hydrophilic and/or water-soluble polymer which does not detrimentally affect the function of the eye. Many such polymers are known in the art and discussed hereinabove. The polymer-bound HLE inhibitory agent described above are suitable for practicing this invention. Also suitable, however, are other polymer-bound HLE inhibitory agents which can be prepared by chemically binding known HLE inhibitory agents to somewhat hydrophilic and/or watersoluble polymers as an artisan would know, and is for example described supra.

In general, the polymers suitable for use in this invention are water soluble polymers, and preferably, polymers having a flexible backbone structure which are not easily biodegradable and which consequently have a prolonged biological half life. Even more preferred are polymers which are water soluble and substantially non-biodegradable but which also have a flexible polymer backbone. A high flexibility exhibited by the polymer is helpful in increasing the accessibility of the polymer bound inhibitory molecule to the enzyme.

Suitable polymers for use in this invention are polymers containing amide bonds in the main chain. Particularly useful are derivatives of synthetic polyaminoacids, examples of which include random copolymers of alpha, beta hydroxy alkyl-D,L-aspartamide, e.g., poly alpha, beta-(N-(2-hydroxyethyl)-D,L-aspartamide) in which a fraction of 2-hydroxyethyl side-chains is replaced by appended reactive moieties having HLE inhibitory activity.

Other examples of suitable polymers include polysaccharide derivatives, especially derivatives of dextran, cellulose, carboxymethyl cellulose, alginic acid and hyaluronic acid, combinations thereof or combinations with other polymers. Yet another example of suitable polymers with oxygen atoms in the main polymer chain are polyether polymers, examples of which include polyethyleneglycol (polyoxirane), divinylethermaleic acid copolymer (pyran copolymer, DIVEMA), and the like. Examples of polymers with a-C-C- backbone suitable for use in this invention are copolymers prepared from mixtures of different types of monomers. One such group is a polymer formed by mixing one type of monomer which has active appended moieties and another type of monomer lacking such moieties. Particularly suitable are copolymers derived from hydrophilic vinylic and/or acrylic type monomers. Examples include N-2-vinylpyrrolidone, 2-hydroxzypropylmethacrylamide, 2- hydroxyethylmethacrylate and other hydrophilic esters and amides of acrylic and methacrylic acid which are well known in the art. Suitable monomers containing appended reactive moieties for preparation of copolymers for use in this invention include, e.g., maleic acid anhydride and reactive esters of acrylic and methacrylic acid. Particularly suitable are, e.g., glycidyl acrylate, glycidyl methacrylate, pnitrophenyl, N-hydroxysuccinimide, pentachlorophenyl or/and pentafluorophenyl esters of methacrylic and acrylic acids, wherein the alkoxy moiety of the reactive ester can be either bound directly to the carbonyl of methacrylic or acrylic acid or it can be bound via a spacer or linker.

Suitable spacers or linkers for use in these types of polymers are generally known in the art. Examples of particularly suitable polymers include poly(N-vinyl-pyrrolidone), co-poly-(N-vinylpyrrolidone-co-maleic acid anhydride), copoly-(N-vinylpyrrolidone co-methacryloyl-N-hydroxysuccinimide, co-poly(N-(2-hydroxypropyl) methacrylamide-co-methacryloyl p-nitrophenyl4 ester) and other copolymers formed by the monomers indicated above.

The polymers utilized for practicing the present invention must be pharmaceuticallyacceptable polymers. These are known in the art as such.

Where the HLE inhibitory agents are attached to the polymer by means of a linker or spacer the linkers or spacers must contain at least two reactive groups. One of the reactive groups must be capable of covalently bonding to the appended moiety present in at least some of the monomer units contained in the polymer. The other reactive group must be capable of covalently bonding to a reactive group present in the free HLE inhibitory agent molecule which is not involved in the binding to the active site of the enzyme. Suitable linkers are known in the art and need not be specifically described herein. One group of linkers which has been found suitable for use with this invention is that encompassing flexible backbone hydrocarbons containing at least two reactive groups. Suitable are reactive groups such as hydroxyl, sulfhydryl, amino, carboxyl, hydrazino and hydrozido, among others. However, other groups may also be

utilized. The length of the linker or spacer may vary as desired for particular applications. Typically, ($C_2$-$C_{20}$)hydrocarbon linkers are utilized, preferably linear hydrocarbons. However, other types of molecules may also be incorporated herein.

Particularly suitable types of linker or spacer has been found to be those comprising ($C_1$-$C_{20}$) hydrocarbons having covalently bonded substituents to the first and last carbon atoms such as hydroxylamines. Other examples suitable for use in this invention are alpha, omega -diamines, alpha, omega -diamino alcohols and alpha, omega -diamino acids. By means of example, polymers with multiple-bound HLE inhibitory agents provide an opportunity to incorporate several inhibitory moieties into a single unit, thereby increasing the efficiency of the transfer of the inhibitory agent to the active site of the enzyme. This, in turn, optimizes the affinity of the polymer-bound inhibitor towards the enzyme when compared with the low-molecular weight HLE inhibitory agent itself.

The HLE inhibitory agents are prepared as is taught by the art. By means of example, a group of derivatized ologopeptides having HLE inhibitory activity may be prepared as described in U.S. Patent 4,643,991 to Digenis et al and supra.

The loading of the HLE inhibitory agents onto the polymers either alone or by means of a linker or spacer is conducted by chemical reactions which are known in the art and need not be described here in detail. The degree of loading, i.e., density of the HLE inhibitory units along the polymeric chain can be varied in a way such that it is most appropriate in accordance with the loading desired. This can be attained by varying the experimental conditions, e.g., the number of appended HLE active moieties on the polymeric chain, the number of spacer groups and/or the ratio of HLE inhibitory groups to the polymer in the reaction. Examples of these are shown supra. However, other means can also be utilized for adjusting the density of the inhibitory units along the polymeric chain as is known in the art.

In a preferred embodiment of the invention, the method is practiced subsequent to an ocular operation, and the corneal scarring or fibroblast proliferation is therefore post-operative.

In another particular embodiment, the method of the invention is applied to corneal scarring or fibroblast proliferation which is associated with ocular conjunctivitis or other ocular infections or biological damage.

In another embodiment of the invention the method is applied to corneal scarring or fibroblast proliferation associated with corneal burning or other heat-associated damage of the eye.

In still another embodiment, the method of the invention is applied to corneal scarring or fibroblast proliferation which is associated with mechanical injury or chemical injury.

The method of the invention may be practiced by applying the HLE inhibitory agent topically, intraocularly, by injection or through a contact lens. Preferred is topical application.

Typically, the HLE inhibitory agent is applied as a composition comprising an about 0.001 - 99.9 wt.% aqueous solution of the agent, preferably about 0.0199 wt.% solution of the agent, and more preferably about 0.1 - 90 wt.% solution thereof. Most preferably, the inhibitory agent is applied in accordance with the present invention as a composition comprising an aqueous solution containing about 1 - 10 wt.% of the agent. Other amounts however may also be utilized. The composition may also contain other ingredients known as useful in ocular treatments. These are additives which are known in the art.

In still another embodiment of the invention the above method may be applied as a preventative method to a subject who is susceptible to the ocular condition.

The HLE inhibitory agent may be applied to the eye of a subject at varied intervals of time. Typically, the interval of time will depend on the residence time of the inhibitory agent in the eye. Thus, polymer-bound agents are "longer acting" and need not be reapplied for longer periods of time than free agents as is known in the art. Typically, polymer-bound HLE inhibitory agents may be applied every about 24 hours to seven days, and in some instances even longer periods of time. The free HLE inhibitory agents may be reapplied every about one hour to 24 hours. However, other intervals are utilized as suitable for specific HLE inhibitory agents and ocular conditions.

Also part of this invention is a method of reducing neovascularization of corneal scar tissue or fibroblast proliferation comprising applying to an area of a subject's eye afflicted with the condition a neovascularization-inhibitory amount of a human leukocyte elastase (HLE) inhibitory agent under conditions and for a period of time effective to attain the desired effect.

Typically, the application is conducted for a few days to up to a few months, and sometimes for longer periods of time as needed to attain the desired result.

As in the case of the prior method the HLE inhibitory agent may be selected from the group consisting of free and water-soluble polymer-bound HLE inhibitory agents described above and/or known in the art.

The method described herein may also be practiced by applying the inhibitory agent to corneal scarring or fibroblast proliferation associated with post-operative conditions. Thus, the agent may be applied immediately after surgery, or as soon as ocular bleeding stops.

In another embodiment the corneal scarring or fibroblast proliferation to which the method is applied is associated with ocular conjunctivitis or other ocular infections or biological damage. In this case, the corneal scarring is produced by injury associated with microorganisms lodged in the ocular cavity and/or tissues. The ocular composition may in such cases additionally contain an antibiotic or other drug for the purpose of fighting the infection as well.

In another embodiment, the method the invention may be applied to corneal scarring or fibroblast proliferation which is associated with corneal burning or heat-coagulation injuries.

In still another embodiment the method may be practiced on corneal scarring or fibroblast proliferation which is associated with mechanical injury of the eye, e.g., mechanical injury of the ocular cornea.

In still another embodiment the corneal scarring or fibroblast proliferation the method is applied to is associated with chemical injury. This typically occurs by exposure of the eye, and particularly the cornea, to chemical products which have a detrimental effect on ocular tissues.

In one embodiment of this method of the invention the HLE inhibitory agent may be applied topically, intraocularly, by injection or through a contact lens. Preferred is the topical application of an ocular composition, e.g., a solution of the agent. By means of example a composition comprising an aqueous solution of the compound may be applied to the eye as an about 0.001 - 99.9 wt.% solution thereof, preferably about 0.01 - 99 wt.%, and more preferably about 0.1 - 90 wt.%. A most preferred composition comprises a solution of the HLE inhibitory agent of about 1 - 10 wt.% thereof.

In still another embodiment, this method of the invention is a preventative method wherein the HLE inhibitory agent is applied to a subject susceptible to the condition prior to its occurrence.

Having now generally described this invention, the same will be better understood by reference to certain specific examples, which are included herein for purposes of illustration only and are not intended to be limiting of the invention or any embodiment thereof, unless so specified.

## EXAMPLES

### Example 84: Chemicals and Solutions

The following two representative compounds, KY-7-11A and KY-3-PC5, compounds A and B, respectively, were tested for use in the methods of the invention.

Table 5: Exemplary HLE Inhibitory Agents Utilized

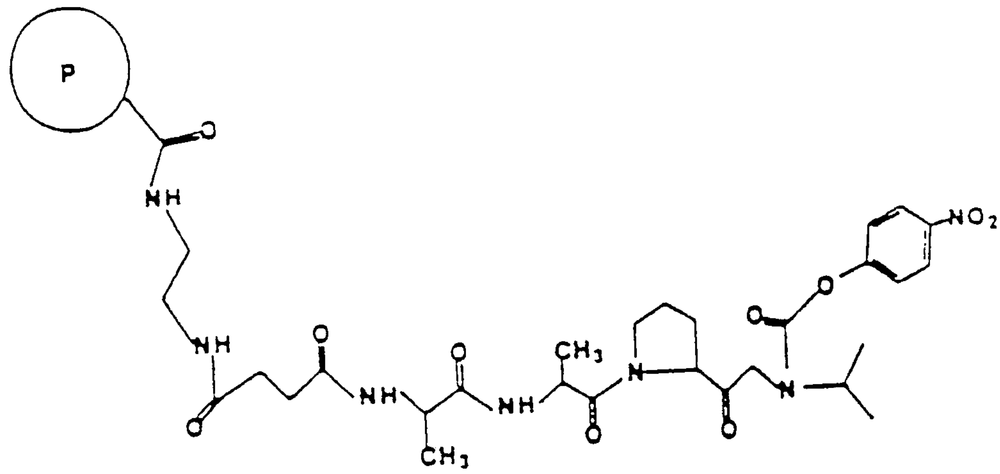

and

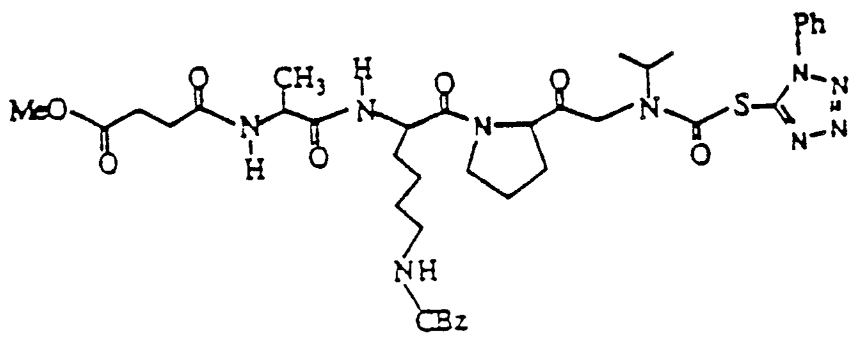

Compound 71 is a peptidyl carbamate elastase (HLE) inhibitor which is chemically bound to a non-biodegradable water soluble polymer with an average molecular weight of about 1,000 to 5000,000 daltons. This compound is described in US Patent Application Serial No. 07/242,294. Compound 72 is a peptidyl carbamate elastase (HLE) inhibitor and is representative of a large series of peptidyl carbamate HLE inhibitors described in U.S. Patent Application Serial No. 07/263,385.

Example 85: Solutions Tested

(1) Solution A: Control

This solution consists of normal saline (0.9% NaCl) and contains 1.4% w/v of polyvinyl alcohol. This solution was used as a control.

(2) Solution B:

This solution contains the active elastase inhibitory agent (Compound 71 or Compound 72) at a concentration of 10 micro g/mL. The inhibitory agent was dissolved in normal saline (0.9% NaCl) containing 1.4% w/v of polyvinyl alcohol.

(3) Solution C:

This solution contains the elastase inhibitory agent Compound 72 at a concentration of 50 micro g/mL. This inhibitory agent was dissolved in normal saline (0.9% NaCl) containing 1.4% w/v of polyvinyl alcohol.

All the above solutions were freshly made immediately prior to conducting the tests. The samples were then kept under refrigeration at 5° C.

Example 86: Animal Experiments

All surgery was performed by a Board-certified ophthalmologist (M.D.). Sedation and euthanasia were performed by a veterinarian (DVM) ophthalmologist (M.D. ) under aseptic conditions. Histopathology was conducted by an expert pathologist (M.D. ) at the University of Miami, School of Medicine, Miami, FL. and the Pathology Laboratory at the Ophthalmology Department at the University of Iowa, Iowa City, IA.

All samples were coded and all the above mentioned operators had no knowledge of the key to the code (blind test).

Example 87: General Animal Preparation

26 rabbits were tranquilized with acetylpromazine maleate, 16mg/kg intramuscularly. 2% lidocaine HC1 eye drops were instilled in each eye for corneal anesthesia. Once the corneas were anesthetized, an eyelid speculum was inserted and the lids were then separated.

The corneas of each eye were thermally coagulated with the tip of a battery-powered ophthalmic cautery. A burn measuring 3mm in diameter was placed near the superior limbus of each eye.

Two drops of solution A, B or C were instilled in designated treated eyes every 15 min for 6 hrs., then every 2 hrs. for 12 hrs. Following this, two eye drops of the same solutions were instilled four times a day. Daily eye examination was performed and photographs were taken.

Two weeks after treatment the rabbits were sacrificed by longer intracardiac injection of T-61 (50mg/kg). Subsequently, the rabbits' eyeballs were enucleated, the globes placed in Bounri's solution (10% formalin solution) and submitted for histopathology cell analysis.

Example 88: Histopathological Results

The eyes were fixed with 10% formalin solution and labeled right (R) and left (L), as appropriate sectioned and stained with H & E (hematoxylin & eosin stains). Histological sections were read by a pathologist without knowledge of the previously undertaken experimental procedure (blind test).

Table 6 below shows typical results obtained from a minimum of six (6) rabbits per treatment.

Table 6: Microscopic Description of Rabbit Eyes

| Treatment | Results |
|---|---|
| Untreated Solution A (normal saline & 1.4% w/v polyvinyl alcohol) | Eyes (the untreated ones) showed an area, 5-6 mm in extent, and involving the entire thickness of the cornea, of vascular and fibroblastic prolifera-tion with an intense inflammatory infiltrate consisting predominantly of eosinophils, with very few plasma cells and lymphocytes. In that area the surface epithelium was ulcerated. The deeper one half of the cornea showed almost a pure fibroblastic proliferation with collagent deposition. In most of the cases an intense stromal vascularization with central scarring and the presence of numerous polymorphonuclear leukocytes, were observed. |
| Treated with Solution B (10 micro g/ml of Compounds 71 or 72) | The reaction is not quite as intense as in the untreated specimen. Toward the periphery of the specimen, corneal vascularization and migration fibroblasts into the cornea, was was bserved. Centrally, there was a zone of superficial scarring, just beneath the corneal epithelium. Superficially, a zone of proliferating fibroblasts around the carotid collagen, was also noticed. |
| Treated with Solution C (50 micro g/ml Comp. B) | In contrast to the "untreated" eyes, the treated eyes (with Solution C) exhibited focal areas of subepithelial vascular proliferation of only 0.6-1.0mm in extent and involved only one third of the corneal thickness. This represents a protection against scarring, due to corneal burns of a tenfold magnitude. |

The above histopathologic results show that after application of the solutions (Solution A, B or C), severe fibrosis and neovascularization was still present in the control or untreated corneas while very little neovascularization or fibrosis could be detected in the experimental corneas (especially those treated with solution C). Accordingly, ophthalmic solutions of Compound 71 or Compound 72, which are representative of a larger group of HLE inhibitory agents, are proven effective in reducing fibrosis, neovascularization and inflammation in corneal wound-healing.

The invention now being fully described, it will be apparent to one of ordinary skill in the art that many changes and modifications can be made thereto without departing from the spirit or scope of the invention as set forth herein.

## Claims

1. A compound selected from the group consisting of a compound of the formula

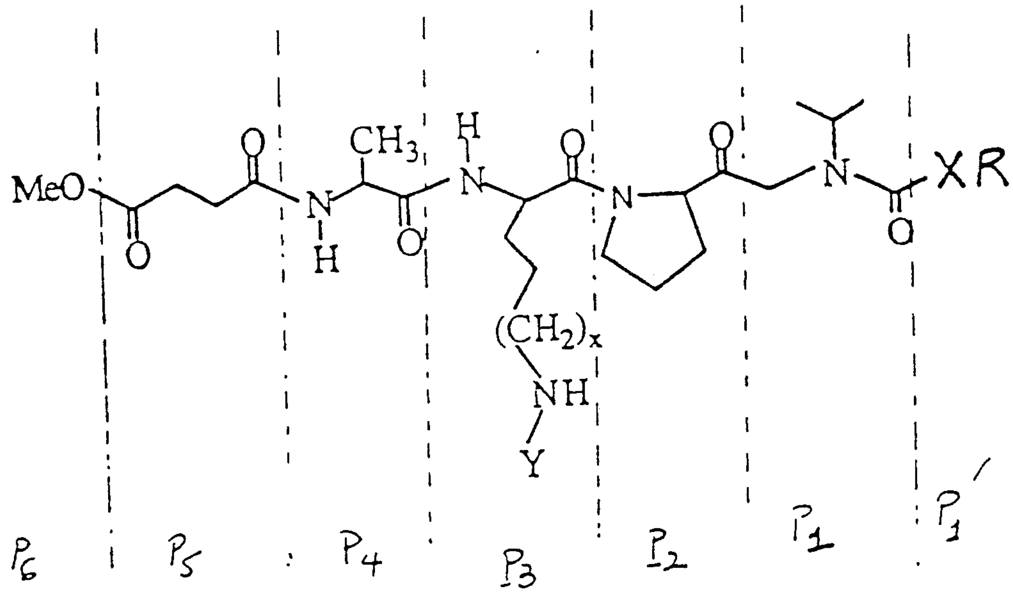

(I)

and a compound of the formula

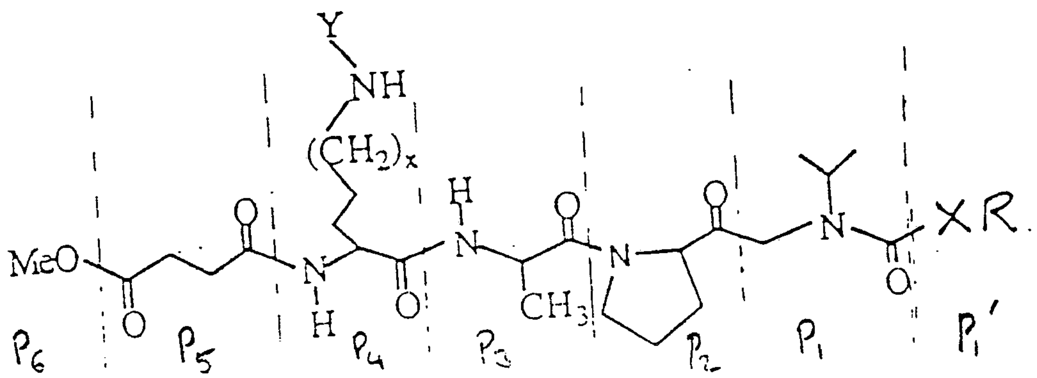

(II)

wherein
x is 1 or 2;
Y is carbobenzoxy or benzoyl; and
XR is

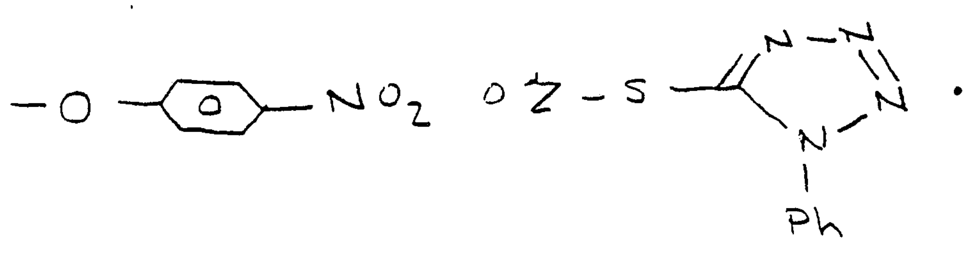

2. The compound of claim 1 having the formula

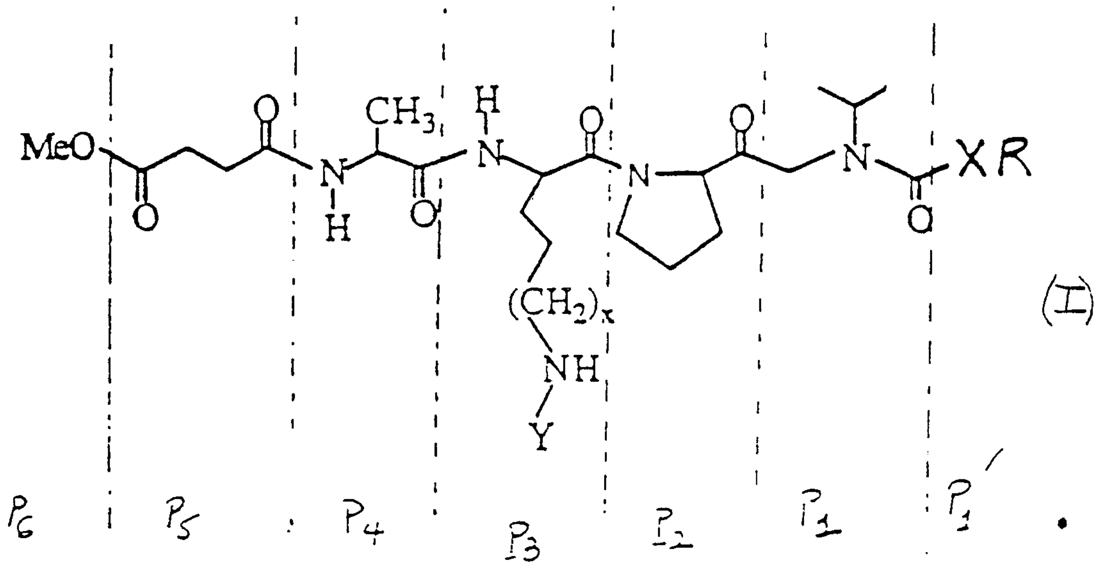

(I)

3. The compound of claim 1 having the formula

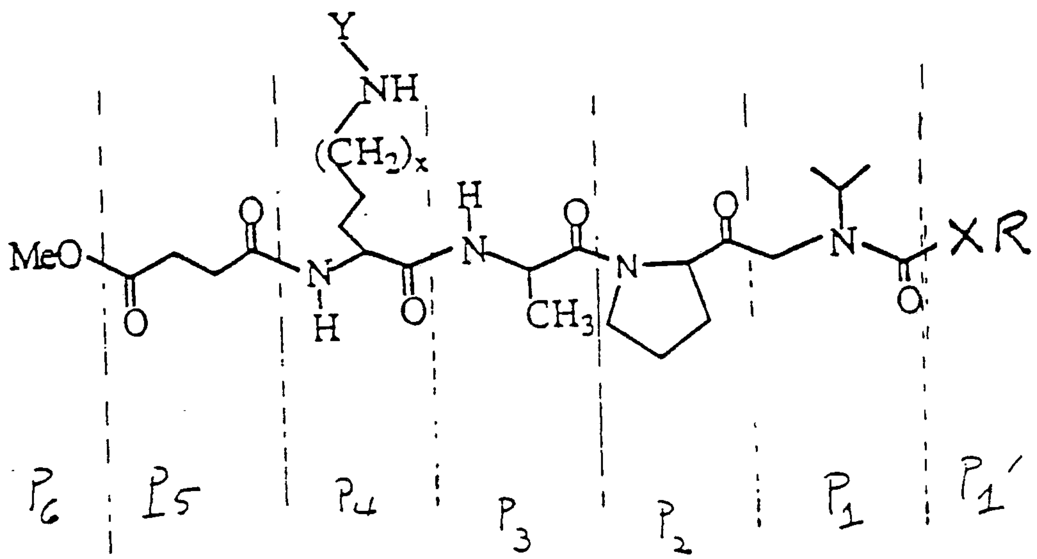

(II)

4. The compound of claim 1 being selected from the group consisting of

1. p-Nitrophenyl N-[(Methoxysuccinyl)-L-alanyl-L-alanyl-L-prolylmethyl]-N-isopropylcarbamate,
2. Methyl succinimide succinate
3. t-Butyl Methoxysuccinyl-L-alanine ester ,
4. Methoxysuccinyl-L-alanine,
5. $N_\alpha$-Methoxysuccinyl-L-alanyl-$N_\epsilon$-benzoyl-L-lysine,
6. $N_\alpha$-Methoxysuccinyl-L-alanyl-$N_\epsilon$-carbobenzoxy-L-lysine phenacyl ester,
7. $N_\alpha$-Methoxysuccinyl-L-alanyl-$N_\epsilon$-carbobenzoxy-L-lysine,
8. $N_\alpha$-Methoxysuccinyl-L-alanyl-$N_\delta$-carbobenzoxy-L-ornithine phenacyl ester,
9. $N_\alpha$-Methoxysuccinyl-L-alanyl-$N_\delta$-carbobenzoxy-L-ornithine,
10. $N_\alpha$-Methoxysuccinyl-$N_\delta$-carbobenzoxy-L-ornithine,
11. $N_\alpha$-Methoxysuccinyl-$N_\delta$-carbobenzoxy-L-ornithyl-L-alanine t-butyl ester,
12. $N_\alpha$-Methoxysuccinyl-$N_\delta$-carbobenzoyl-L-ornithyl-L-alanine,
13. $N\alpha$-Methoxysuccinyl-$N_\epsilon$--carbobenzoxy-L-lysine,
14. $N_\alpha$-Methoxysuccinyl-$N_\epsilon$-carbobenzoxy-L-lysyl-L-alanine t-butyl ester,
15. $N_\alpha$-Methoxysuccinyl-$N_\epsilon$-carbobenzoxy-L-lysyl-L-alanine,

16. $N_\alpha$-Methoxysuccinyl-$N_\epsilon$-benzoyl-L-lysine,
17. $N_\alpha$-Methoxysuccinyl-$N_\epsilon$-benzoyl-L-lysyl-L-alanine t-butyl ester,
18. $N_\alpha$-Methoxysuccinyl-$N_\epsilon$-benzoyl-L-lysyl-L-alanine,
19. N-Boc-L-prolyl chloromethyl ketone ,
20. N-[(N-Boc-L-prolyl)methyl]isopropylamine,
21. N[(N-Boc-L-prolyl)methyl]-N-isopropylcarbamate,
22. p-Nitrophenyl N-(L-prolylmethyl)-N-isopropyl-carbamate hydrochloride,
23. $N_\alpha$-Methoxysuccinyl-L-alanyl-$N_\epsilon$-carbobenzoxy-L-lysyl-D-proline phenacyl ester,
24. $N_\alpha$-Methoxysuccinyl-L-alanyl-$N_\epsilon$-carbobenzoxy-L-lysyl-L-proline phenacyl ester,
25. $N_\alpha$-Methoxysuccinyl-L-alanyl-$N_\epsilon$-carbobenzoxy-L-lysyl-D-proline,
26. $N_\alpha$-Methoxysuccinyl-L-alanyl-$N_\epsilon$-carbobenzoxy-L-lysyl-L-proline,
27. $N_\alpha$-Methoxysuccinyl-L-alanyl-$N_\epsilon$-carbobenzoxy-L-lysyl-D-prolyl chloromethyl ketone,
28. $N_\alpha$-Methoxysuccinyl-L-alanyl-$N_\epsilon$-carbobenzoxy-L-lysyl-L-prolyl chloromethyl ketone,
29. N-[Methoxysuccinyl-L-alanyl-($N_\epsilon$-carbobenzoxy)-L-lysyl-D prolylmethyl]-N-isopropylamine,
30. N-[Methoxysuccinyl-L-alanyl-($N_\epsilon$-carbobenzoxy)-L-lysyl-L-prolylmethyl]-N-isopropylamine,
31. $N_\alpha$-t-Boc-$N_\epsilon$-carbobenzoxy-L-lysine ,
32. $N_\alpha$-t-Boc-$N_\epsilon$-carbobenzoxy-L-lysine phenacyl ester,
33. $N_\alpha$-Carbobenzoxy-L-lysine phenacyl ester hydrochloride,
34. $N_\alpha$-t-Boc-$N_\delta$-carbobenzoyl-L-ornithine,
35. $N_\alpha$-t-Boc-$N_\delta$-carbobenzoyl-L-ornithine phenacyl ester,
36. $N_\delta$-Carbobenzoxy-L-ornithine phenacyl ester hydrochloride ,
37. N-t-Boc-D-proline,
38. N-t-Boc-L-proline,
39. N-t-Boc-D-proline phenacyl ester,
40. N-t-Boc-L-proline phenacyl ester,
41. D-Proline phenacyl ester hydrochloride,
42. L-Proline phenacyl ester hydrochloride,
43. $N_\epsilon$-Benzoyl-L-lysine,

44a. p-Nitrophenyl N-[Methoxysuccinyl-($N_\epsilon$-carbobenzoxy)-L-lysyl-L-alanyl-L-prolylmethyl]-N-isopropylcarbamate,

44b. p-Nitrophenyl N-[Methoxysuccinyl-($N_\epsilon$-carbobnezoxy)-L-lysyl-L-alanyl-D-prolylmethyl]-N-isopropylcarbamate,

45a. p-Nitrophenyl N-[Methoxysuccinyl-($N_\epsilon$-benzoyl)-L-lysyl-L-alanyl-L-prolylmethyl]-N-isopropylcarbamate,

45b. p-Nitrophenyl N-[Methoxysuccinyl-($N_\epsilon$-benzoyl)-L-lysyl-L-alanyl-D-prolylmethyl]-N-isopropylcarbamate,

46a. p-Nitrophenyl N-[Methoxysuccinyl-($N_\delta$-carbobenzoxy)-L-ornithyl-L-alanyl-L-prolylmethyl]-N-isopropylcarbamate,

46b. p-Nitrophenyl-($N_\delta$-carbobenzoxy)-L-ornithyl-L-alanyl-D-prolylmethyl]-N-isopropylcarbamate,

47a. p-Nitrophenyl N-[Methoxysuccinyl-($N_\delta$-benzoyl)-L-ornithyl-L-alanyl-L-prolylmethyl]-N-isopropylcarbamate ,

47b. p-Nitrophenyl N-[Methoxysuccinyl-($N_\delta$-benzoyl)-L-ornithyl-L-alanyl-D-prolylmethyl]-N-isopropylcarbamate,

48a. p-Nitrophenyl N-[Methoxysuccinyl-L-alanyl-($N_\epsilon$-carbobenzoxy)-L-lysyl-L-prolylmethyl]-N-isopropylcarbamate,

48b. p-Nitrophenyl N-[Methoxysuccinyl-L-alanyl-($N_\epsilon$-carbobenzoxy)-L lysyl-D-prolylmethyl]-N-isopropylcarbamate ,

49a. p-Nitrophenyl N-[Methoxysuccinyl-L-alanyl-($N_\epsilon$-benzoyl)-L-lysyl-L-prolylmethyl]-N-isopropylcarbamate,

49b. p-Nitrophenyl N-[Methoxysuccinyl-L-alanyl-($N_\epsilon$-benzoyl)-L-lysyl-D-prolylmethyl]-N-isopropylcarbamate,

50a. p-Nitrophenyl N-[Methoxysuccinyl-L-alanyl-($N_\delta$-carbobenzoxy)-L-ornithyl-L-prolylmethyl]-N-isopropylcarbamate,

50b. p-Nitrophenyl N-[Methoxysuccinyl-L-alanyl-($N_\delta$-carbobenzoxy)-L-ornithyl-D-prolylmethyl]-N-isopropylcarbamate ,

51a. p-Nitrophenyl N-[Methoxysuccinyl-L-alanyl-($N_\delta$-benzoyl)-L-ornithyl-L-prolylmethyl]-N-isopropylcarbamate,

51b. p-Nitrophenyl N-[Methoxysuccinyl-L-alanyl-($N_\delta$-benzoyl)-L-ornithyl-D-prolylmethyl]-N-isopropyl-

carbamate.

52. S- (1-phenyl-5-tetrazol) chloroformate.

53. S-(1-phenyl-5-tetrazoyl)-N-[(N-Boc-L-prolyl)methyl]-N-isopropyl-thiocarbamate.

54. S-(1-phenyl-5-tetrazoyl-N-prolymethyl)-N-isopropyl-thio carbamate hydrochloride.

PC5. S-(1-phenyl-5-tetrazoyl)-N-[methoxysuccinyl-alanyl-($N_\epsilon$-Carbobenzoxyl) lysyl prolyl methyl]-N-isopropyl-thio carbamate. and

PC5. S-(1-phenyl-5-tetrazoyl)-N-[methoxysuccinyl-(N-carbobenzoyl)ornithylalanyl(prolylmethyl)-N-isopropylthio carbamate.

5. The compound of claim 1 selected from the group consisting of

1. p-Nitrophenyl N-[(Methoxysuccinyl)-L-alanyl-L-alanyl-L-prolylmethyl]-N-isopropylcarbamate.
2. Methyl succinimide succinate
3. t-Butyl Methoxysuccinyl-L-alanine ester .
4. Methoxysuccinyl-L-alanine.
5. $N_\alpha$-Methoxysuccinyl-L-alanyl-$N_\epsilon$-benzoyl-L-lysine.
6. $N_\alpha$-Methoxysuccinyl-L-alanyl-$N_\epsilon$-carbobenzoxy-L-lysine phenacyl ester.
7. $N_\alpha$-Methoxysuccinyl-L-alanyl-$N_\epsilon$-carbobenzoxy-L-lysine.
8. $N_\alpha$-Methoxysuccinyl-L-alanyl-$N_\delta$-carbobenzoxy-L-ornithine phenacyl ester.
9. $N_\alpha$-Methoxysuccinyl-L-alanyl-$N_\delta$-carbobenzoxy-L-ornithine.
10. $N_\alpha$-Methoxysuccinyl-$N_\delta$-carbobenzoxy-L-ornithine.
11. $N_\alpha$-Methoxysuccinyl-$N_\delta$-carbobenzoxy-L-ornithyl-L-alanine t-butyl ester.
12. $N_\alpha$-Methoxysuccinyl-$N_\delta$-carbobenzoyl-L-ornithyl-L-alanine.
13. $N_\alpha$-Methoxysuccinyl-$N_\epsilon$-carbobenzoxy-L-lysine.
14. $N_\alpha$-Methoxysuccinyl-$N_\epsilon$-carbobenzoxy-L-lysyl-L-alanine t-butyl ester.
15. $N_\alpha$-Methoxysuccinyl-$N_\epsilon$-carbobenzoxy-L-lysyl-L-alanine.
16. $N_\alpha$-Methoxysuccinyl-$N_\epsilon$-benzoyl-L-lysine.
17. $N_\alpha$-Methoxysuccinyl-$N_\epsilon$-benzoyl-L-lysyl-L-alanine t-butyl ester.
18. $N_\alpha$-Methoxysuccinyl-$N_\epsilon$-benzoyl-L-lysyl-L-alanine.
19. N-Boc-L-prolyl chloromethyl ketone .
20. N-[(N-Boc-L-prolyl)methyl]isopropylamine.
21. N[(N-Boc-L-prolyl)methyl]-N-isopropylcarbamate.
22. p-Nitrophenyl N-(L-prolylmethyl)-N-isopropyl-carbamate hydrochloride.
23. $N_\alpha$-Methoxysuccinyl-L-alanyl-$N_\epsilon$-carbobenzoxy-L-lysyl-D-proline phenacyl ester.
24. $N_\alpha$-Methoxysuccinyl-L-alanyl-$N_\epsilon$-carbobenzoxy-L-lysyl-L-proline phenacyl ester.
25. $N_\alpha$-Methoxysuccinyl-L-alanyl-$N_\epsilon$-carbobenzoxy-L-lysyl-D-proline.
26. $N_\alpha$-Methoxysuccinyl-L-alanyl-$N_\epsilon$-carbobenzoxy-L-lysyl-L-proline. and
27. $N_\alpha$-Methoxysuccinyl-L-alanyl-$N_\epsilon$-carbobenzoxy-L-lysyl-D-prolyl chloromethyl ketone.

6. The compound of claim 1 selected from the group consisting of

28. $N_\alpha$-Methoxysuccinyl-L-alanyl-$N_\epsilon$-carbobenzoxy-L-lysyl-L-prolyl chloromethyl ketone.
29. N-[Methoxysuccinyl-L-alanyl-($N_\epsilon$-carbobenzoxy)-L-lysyl-D prolylmethyl]-N-isopropylamine.
30. N-[Methoxysuccinyl-L-alanyl-($N_\epsilon$-carbobenzoxy)-L-lysyl-L-prolylmethyl]-N-isopropylamine.
31. $N_\alpha$-t-Bcc-$N_\epsilon$-carbobenzoxy-L-lysine .
32. $N_\alpha$-t-Boc-$N_\epsilon$-carbobenzoxy-L-lysine phenacyl ester.
33. $N_\epsilon$-Carbobenzoxy-L-lysine phenacyl ester hydrochloride.
34. $N_\alpha$-t-Boc-$N_\delta$-carbobenzoyl-L-ornithine.
35. $N_\alpha$-t-Boc-$N_\delta$-carbobenzoxy-L-ornithine phenacyl ester.
36. $N_\delta$-Carbobenzoxy-L-ornithine phenacyl ester hydrochloride .
37. N-t-Boc-D-proline.
38. N-t-Boc-L-proline.
39. N-t-Boc-L-proline phenacyl ester.
40. N-t-Boc-L-proline phenacyl ester.
41. D-Proline phenacyl ester hydrochloride.
42. L-Proline phenacyl ester hydrochloride.
43. $N_\epsilon$-Benzoyl-L-lysine.

44a. p-Nitrophenyl N-[Methoxysuccinyl-($N_\epsilon$-carbobenzoxy)-L-lysyl-L-alanyl-L-prolylmethyl]-N-isopropylcarbamate.

44b. p-Nitrophenyl N-[Methoxysuccinyl-($N_\epsilon$-carbobenzoxy)-L-lysyl-L-alanyl-D-prolylmethyl]-N-isopropylcarbamate.

45a. p-Nitrophenyl N-[Methoxysuccinyl-($N_\epsilon$-benzoyl)-L-lysyl-L-alanyl-L-prolylmethyl]-N-isopropylcarbamate,

45b. p-Nitrophenyl N-[Methoxysuccinyl-($N_\epsilon$-benzoyl)-L-lysyl-L-alanyl-D-prolylmethyl]-N-isopropylcarbamate,

46a. p-Nitrophenyl N-[Methoxysuccinyl-($N_\delta$-carbobenzoxy)-L-ornithyl-L-alanyl-L-prolylmethyl]-N-isopropylcarbamate,

46b. p-Nitrophenyl-($N_\delta$-carbobenzoxy)-L-ornithyl-L-alanyl-D-prolylmethyl]-N-isopropylcarbamate,

47a. p-Nitrophenyl N-[Methoxysuccinyl-($N_\delta$-benzoyl)-L-ornithyl-L-alanyl-L-prolylmethyl]-N-isopropylcarbamate ,

47b. p-Nitrophenyl N-[Methoxysuccinyl-($N_\delta$-benzoyl)-L-ornithyl-L-alanyl-D-prolylmethyl]-N-isopropylcarbamate, and

48a. p-Nitrophenyl N-[Methoxysuccinyl-L-alanyl-($N_\epsilon$-carbobenzoxy)-L-lysyl-L-prolylmethyl]-N-isopropylcarbamate.

7. The compound of claim 1 selected from the group consisting of

48b. p-Nitrophenyl N-[Methoxysuccinyl-L-alanyl-($N_\epsilon$-carbobenzoxy)-L lysyl-D-prolylmethyl]-N-isopropylcarbamate ,

49a. p-Nitrophenyl N-[Methoxysuccinyl-L-alanyl-($N_\epsilon$-benzoyl)-L-lysyl-L-prolylmethyl]-N-isopropylcarbamate,

49b. p-Nitrophenyl N-[Methoxysuccinyl-L-alanyl-($N_\epsilon$-benzoyl)-L-lysyl-D-prolylmethyl]-N-isopropylcarbamate,

50a. p-Nitrophenyl N-[Methoxysuccinyl-L-alanyl-($N_\delta$-carbobenzoxy)-L-ornithyl-L-prolylmethyl]-N-isopropylcarbamate,

50b. p-Nitrophenyl N-[Methoxysuccinyl-L-alanyl-($N_\delta$-carbobenzoxy)-L-ornithyl-D-prolylmethyl]-N-isopropylcarbamate ,

51a. p-Nitrophenyl N-[Methoxysuccinyl-L-alanyl-($N_\delta$-benzoyl)-L-ornithyl-L-prolylmethyl]-N-isopropylcarbamate,

51b. p-Nitrophenyl N-[Methoxysuccinyl-L-alanyl-($N_\delta$-benzoyl)-L-ornithyl-D-prolylmethyl]-N-isopropylcarbamate,

52. S- (1-phenyl-5-tetrazol) chloroformate,

53. S-(1-phenyl-5-tetrazoyl)-N-[(N-Boc-L-prolyl)methyl]-N-isopropyl-thiocarbamate,

54. S-(1-phenyl-5-tetrazoyl-N-prolylmethyl)-N-isopropyl-thio carbamate hydrochloride,

PC5. S-(1-phenyl-5-tetrazoyl)-N-[methoxysuccinyl-alanyl-($N_\epsilon$-Carbobenzoxyl) lysyl prolyl methyl]-N-isopropyl-thio carbamate, and

PC6. S-(1-phenyl-5-tetrazoyl)-N-[methoxysuccinyl-(N-carbobenzoyl)ornithylalanyl(prolylmethyl)-N-isopropylthio carbamate.

8. The compound of claim 1 having the formula

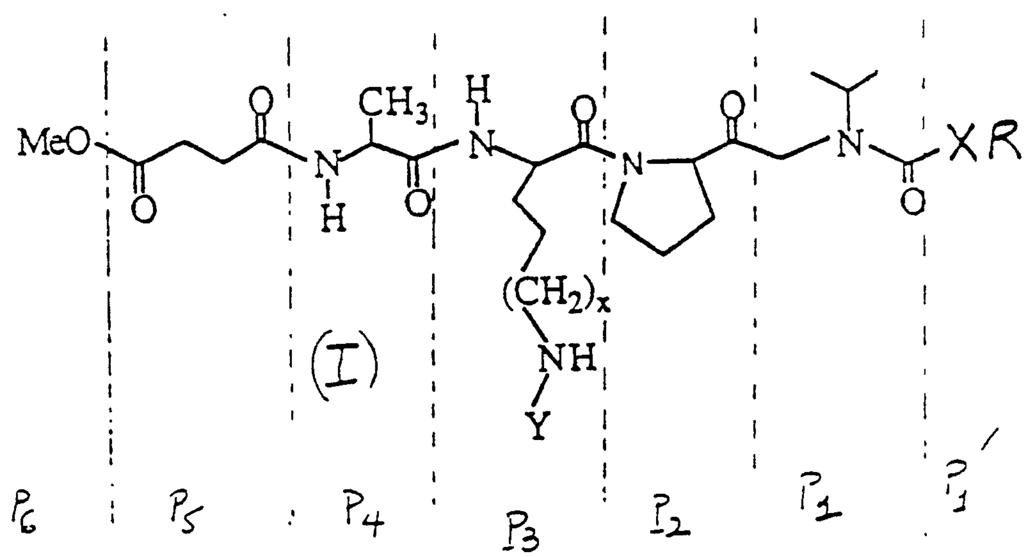

(I)

wherein

x is 1 or 2; Y is carbobenzoxy; and

XR is

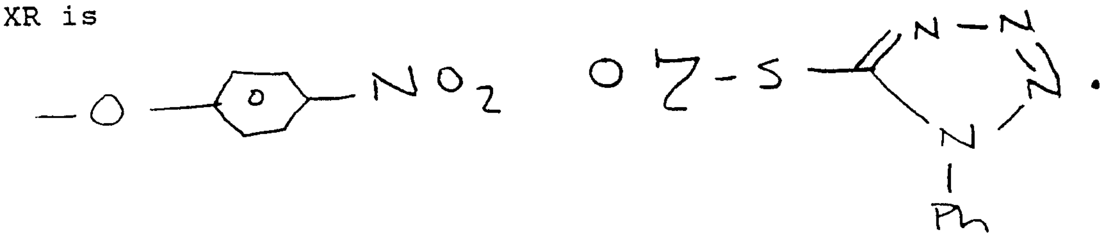

9. The compound of claim 1 having the formula

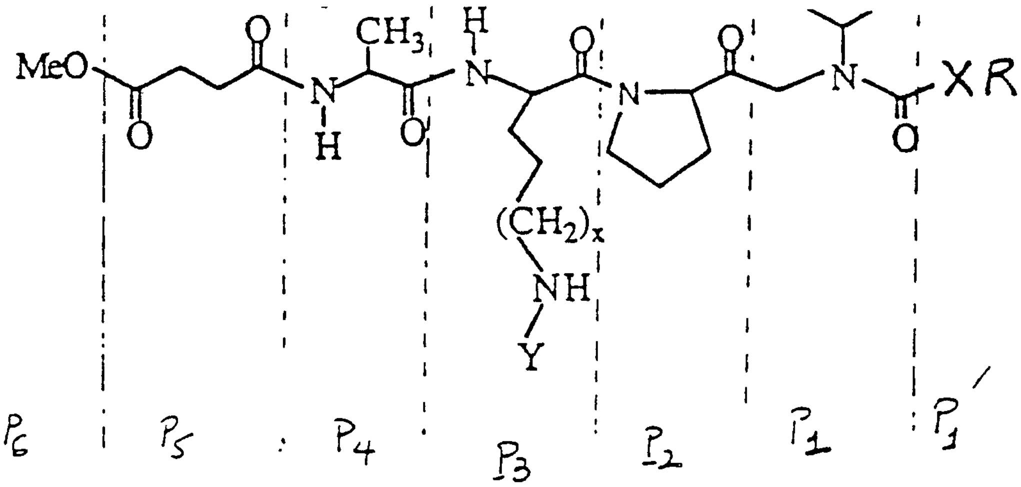

(I)

wherein
x is 1 or 2;
Y is benzoyl, and
XR is

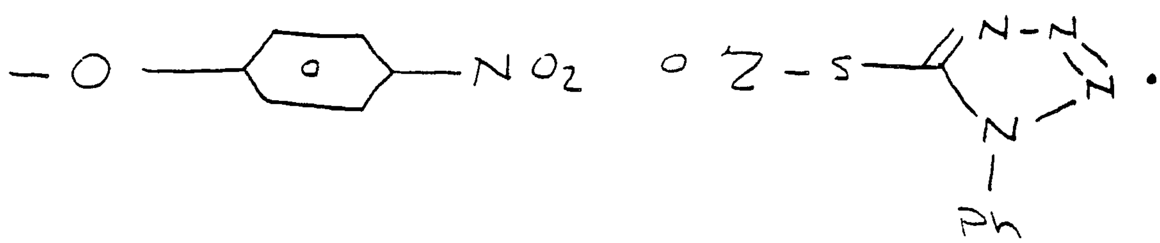

10. The compound of claim 1 having the formula

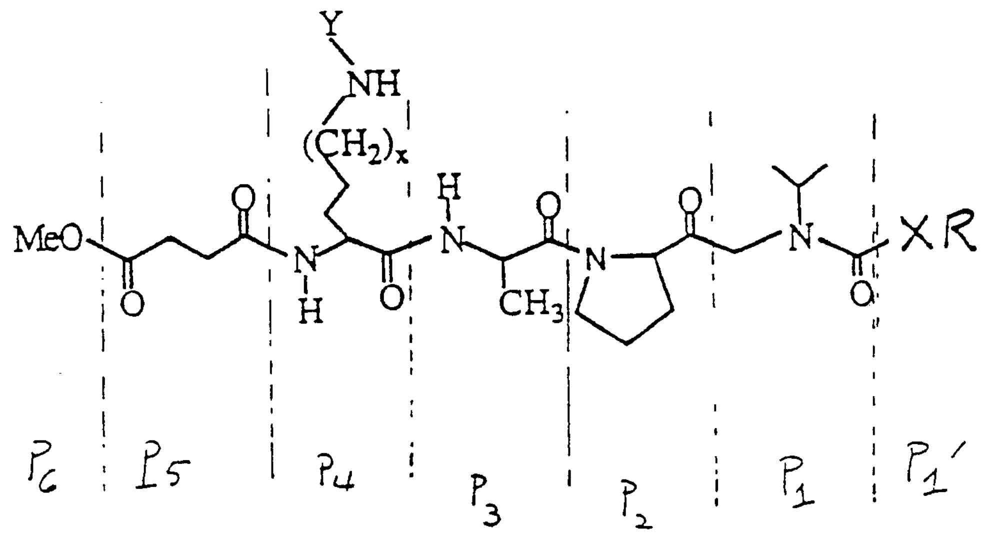

(II)

wherein
x is 1 or 2;
Y is carbobenzoxy; and
XR is

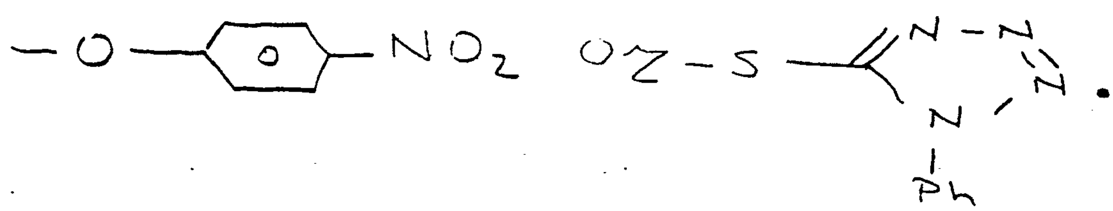

11. The compound of claim 1 having the formula

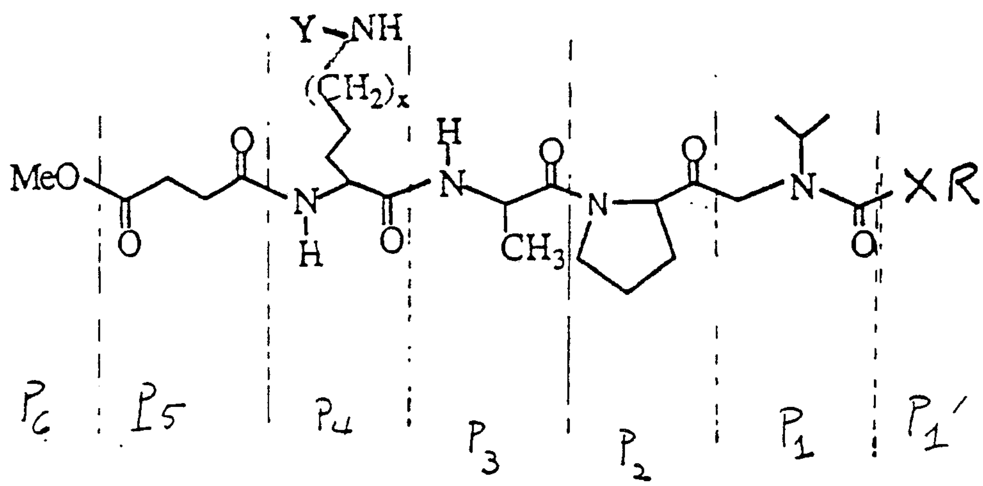

(II)

wherein
x is 1 or 2;
Y is benzoyl; and
XR is

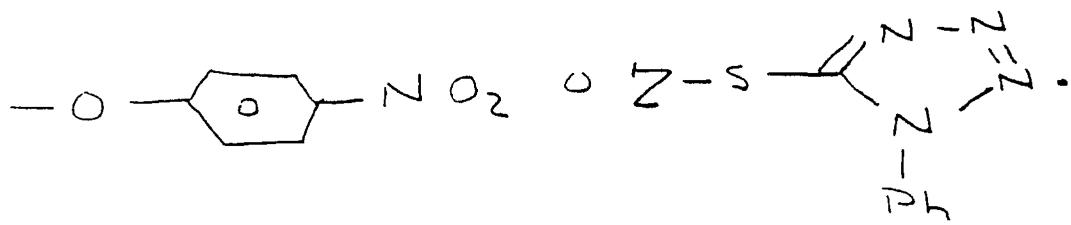

12. The compound of claim 1 having the formula

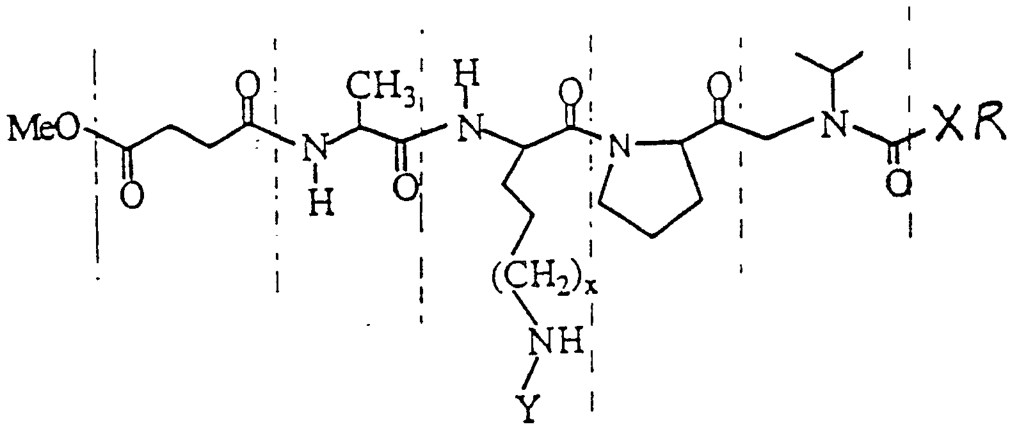 (I)

wherein
x = 1 or 2;
Y is carbobenzoxy; and
XR is

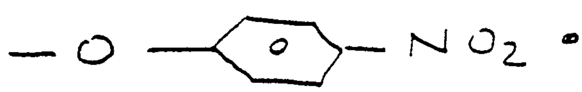

13. The compound of Claim 1 having the formula

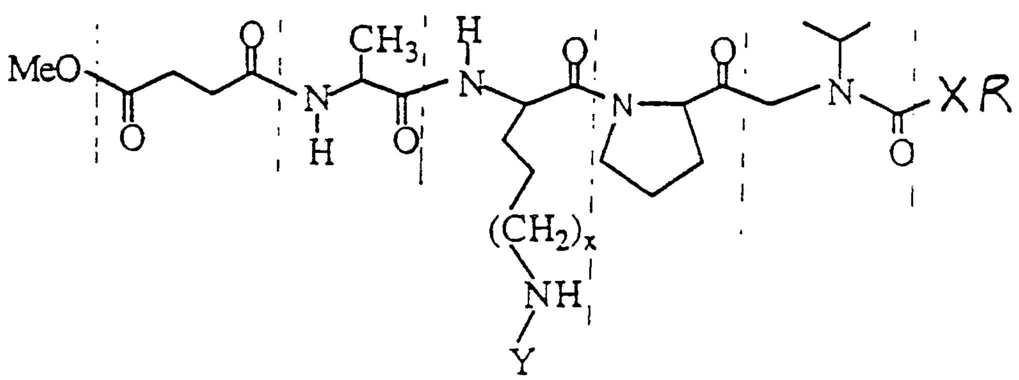 (I)

wherein
x = 1 or 2;
Y is carbobenzosy or benzoyl, and
XR is

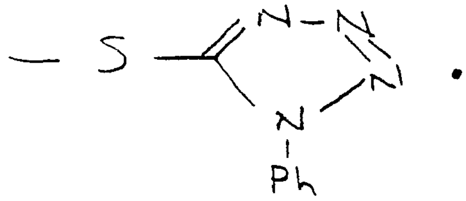

14. The compound of claim 1 having the formula

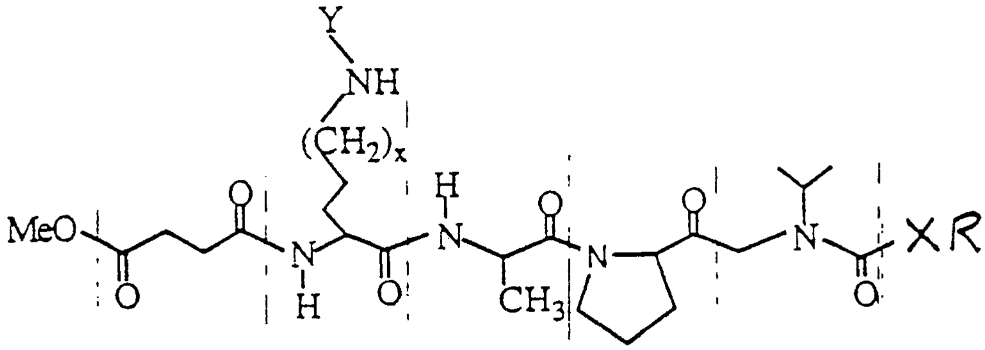

(II)

wherein
x = 1 or 2;
Y is carbobenzoxy or benzoyl, and
XR is

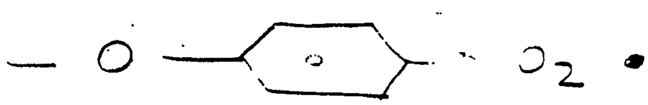

15. The compound of claim 1 having the formula

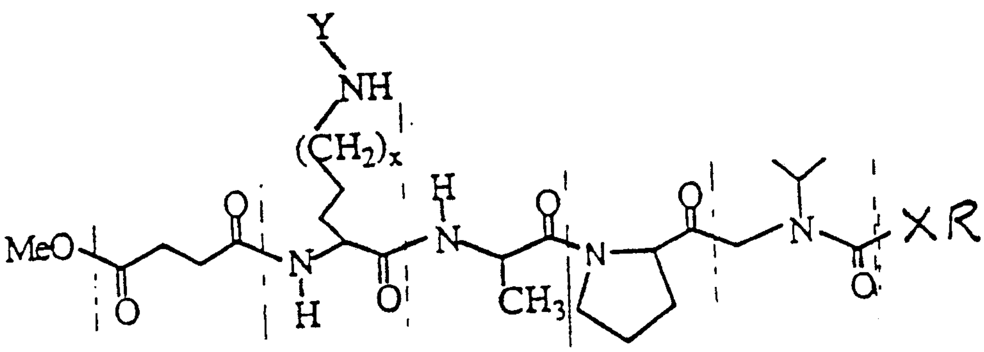

(II)

wherein
x is 1 or 2;
Y is carbobenzoxy or benzoyl, and
XR is

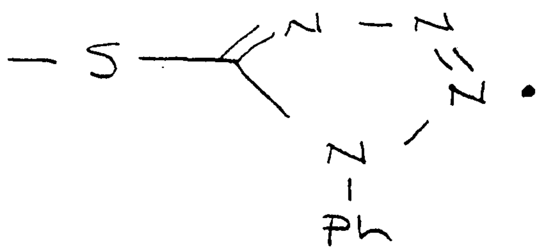

16. An enzyme elastase inhibitory composition, comprising an enzyme elastase inhibitory amount of the compound of claim 1; and a carrier.

17. The composition of claim 16, wherein the carrier is a pharmaceutically-acceptable carrier.

18. A method of selectively inhibiting the enzyme elastase in an animal or a human in need of such treatment comprising administering to said animal or human an enzyme elastase inhibiting amount of the compound of claim 1.

19. A method of selectively inhibiting the enzyme elastase in an animal or a human in need of such treatment comprising administering to said animal or human an enzyme elastase inhibiting amount of the composition of claim 18.

20. The compound of claim 1 being selected from the group consisting of

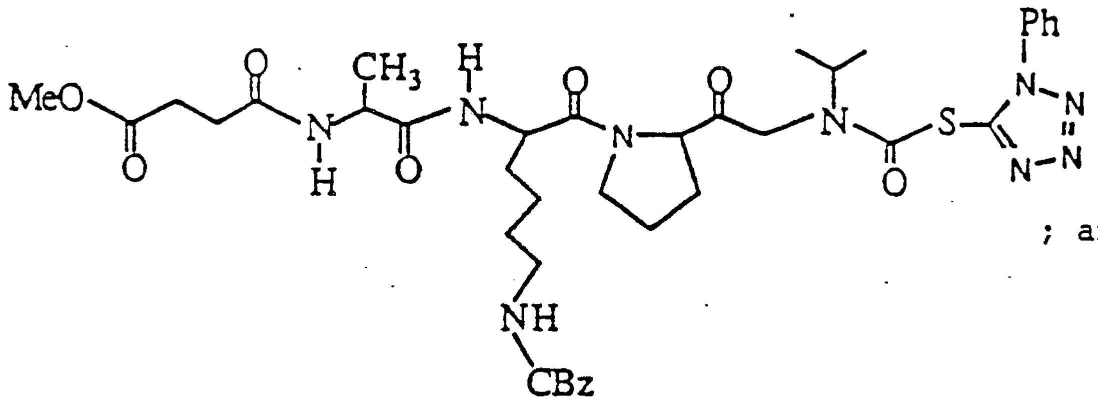

; and

**PC5**

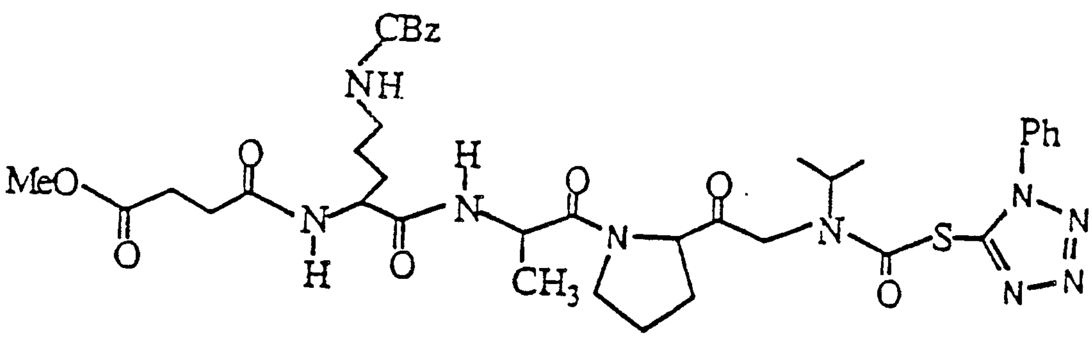

**PC6**

21. A method of reducing corneal scarring or fibroblast proliferation comprising applying to an area of a subject's eye afflicted with the condition a corneal scar- or fibroblast proliferation-reducing amount of an HLE inhibitory agent under conditions and for a period of time effective to attain the effect.

22. The method of claim 21, wherein the HLE inhibitory agent is selected from the group consisting of free and hydrophilic and water-soluble pharmaceutically-acceptable polymer-bound HLE inhibitory agents.

23. The method of claim 21, wherein the corneal scarring or fibroblast proliferation is post-operative.

24. The method of claim 21, wherein the corneal scarring or fibroblast proliferation is associated with ocular infection.

25. The method of claim 21, wherein the corneal scarring or fibroblast proliferation is associated with corneal burning.

26. The method of claim 21, wherein the corneal scarring or fibroblast proliferation is associated with mechanical injury.

27. The method of claim 21, wherein the corneal scarring or fibroblast proliferation is associated with chemical injury.

28. The method of claim 21, wherein the inhibitory agent is applied topically.

29. The method of claim 21, wherein the inhibitory agent is applied as an about 0.001 to 99.9% aqueous solution.

30. The method of claim 21, being a preventative method applied to a subject susceptible to the condition.

31. A method of reducing neovascularization of corneal scar tissue comprising applying to an area of a subject's eye afflicted with the condition a neovascularization-inhibitory amount of an HLE inhibitory agent under conditions and for a period of time effective to attain the desired effect.

32. The method of claim 31, wherein the HLE inhibitory agent is selected from the group consisting of free and hydrophilic and water-soluble pharmaceutically-acceptable polymer-bound HLE inhibitory agents.

33. The method of claim 31, wherein the corneal scarring or fibroblast proliferation is post-operative.

34. The method of claim 31, wherein the corneal scarring or fibroblast proliferation is associated with ocular infection.

35. The method of claim 31, wherein the corneal scarring or fibroblast proliferation is associated with corneal burning.

36. The method of claim 31, wherein the corneal scarring or fibroblast proliferation is associated with mechanical injury.

37. The method of claim 31, wherein the corneal scarring or fibroblast proliferation is associated with chemical injury.

38. The method of claim 31, wherein the inhibitory agent is applied topically.

39. The method of claim 31, wherein the inhibitory agent is applied as an about 0.001 to 99.9% aqueous solution.

40. The method of claim 11, being a preventative method applied to a subject susceptible to the condition.

FIGURE 1

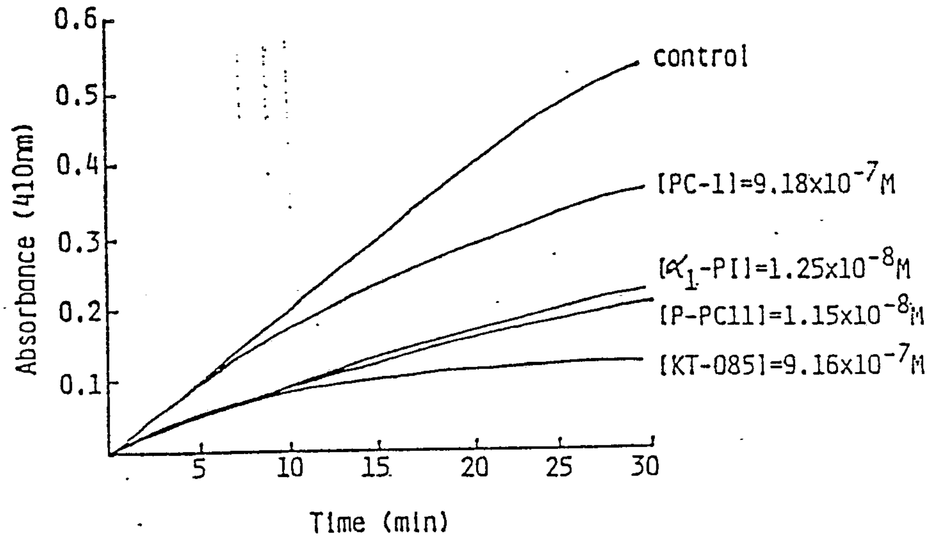
0.6
0.5
0.4
0.3
0.2
0.1
Absorbance (410nm)
5
10
15
20
25
30
Time (min)
control
[PC-1]=9.18x10-7M
[α1-PI]=1.25x10-8M
[P-PC11]=1.15x10-8M
[KT-085]=9.16x10-7M

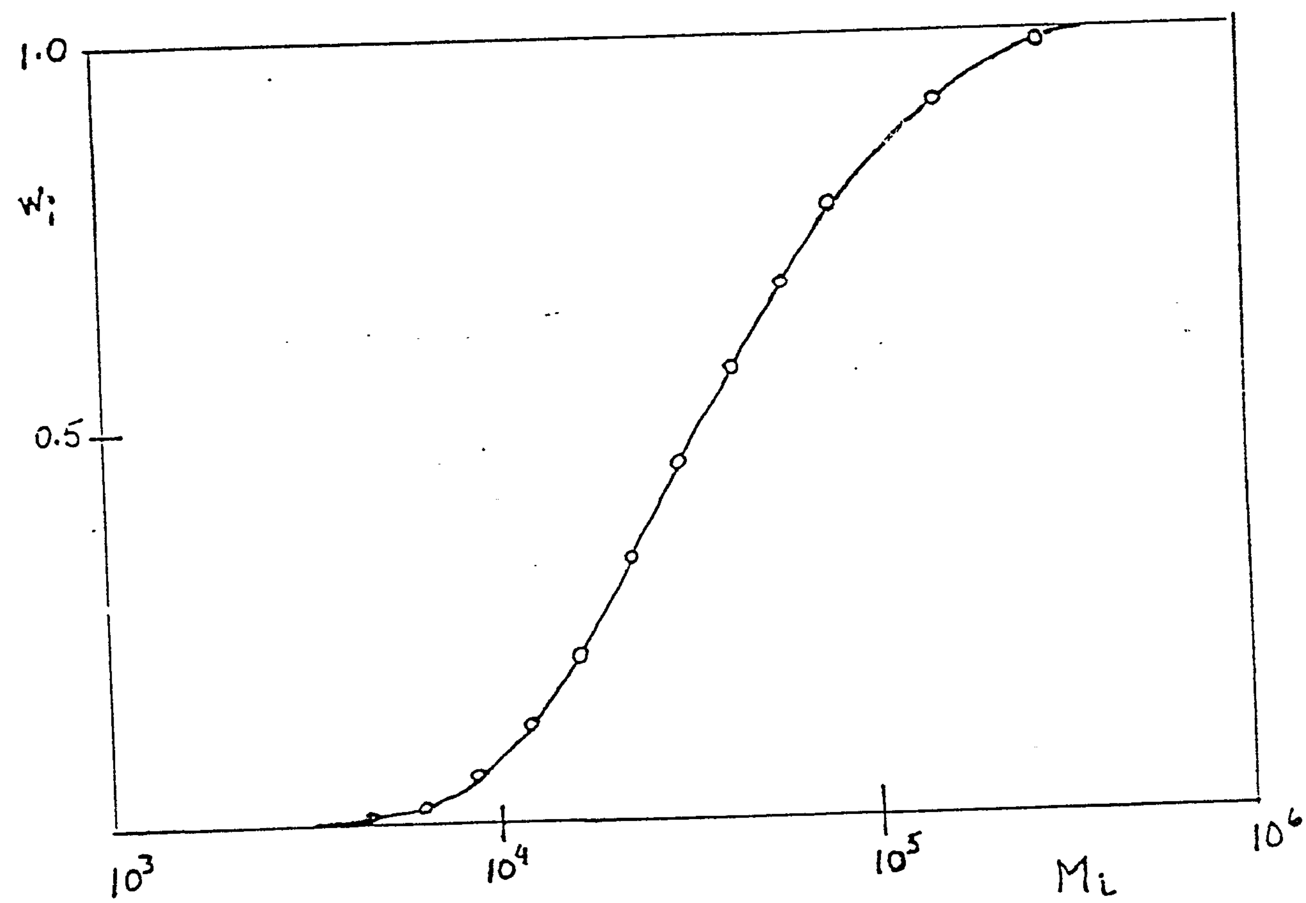


FIG. 2

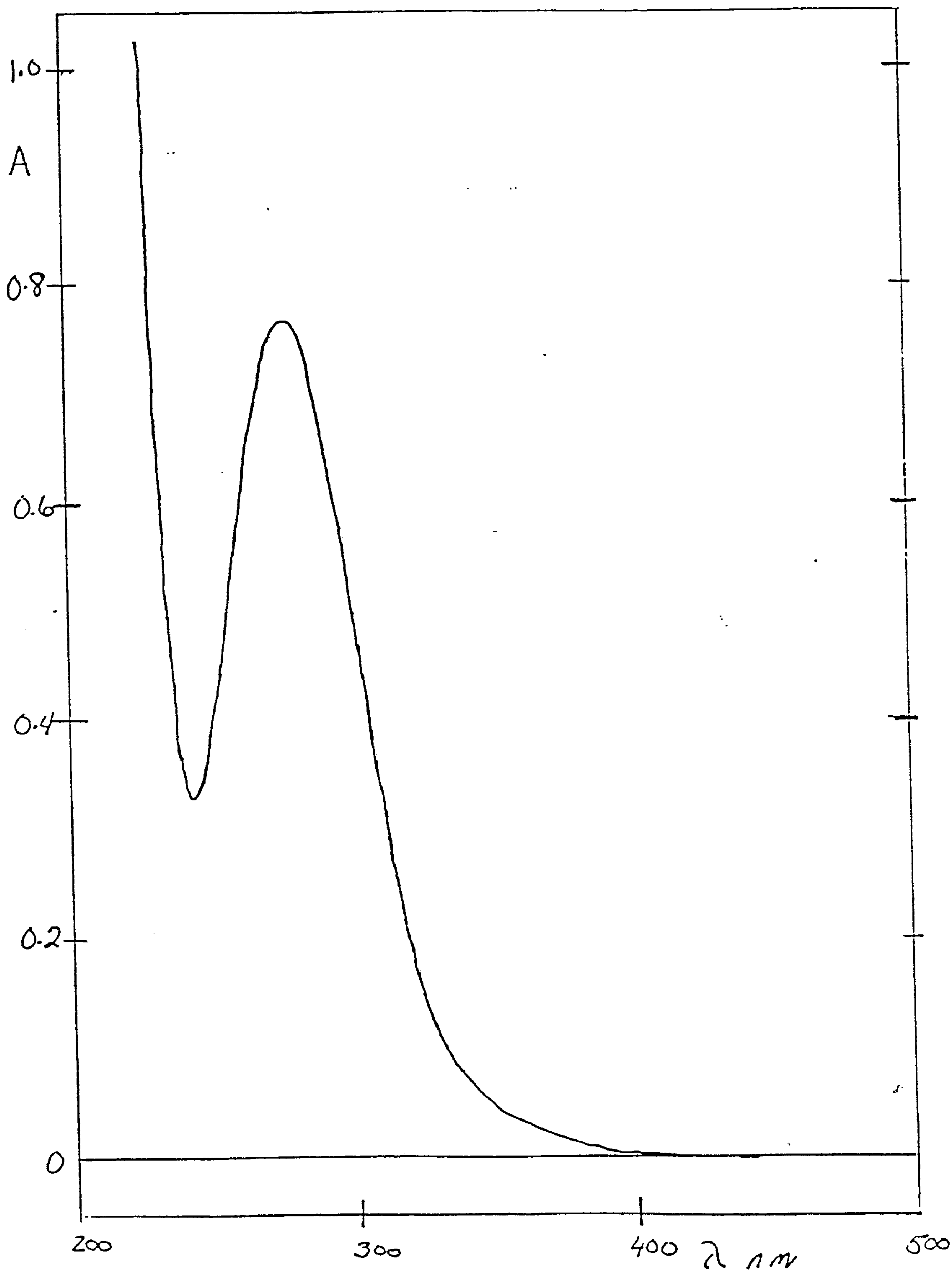


FIG. 3

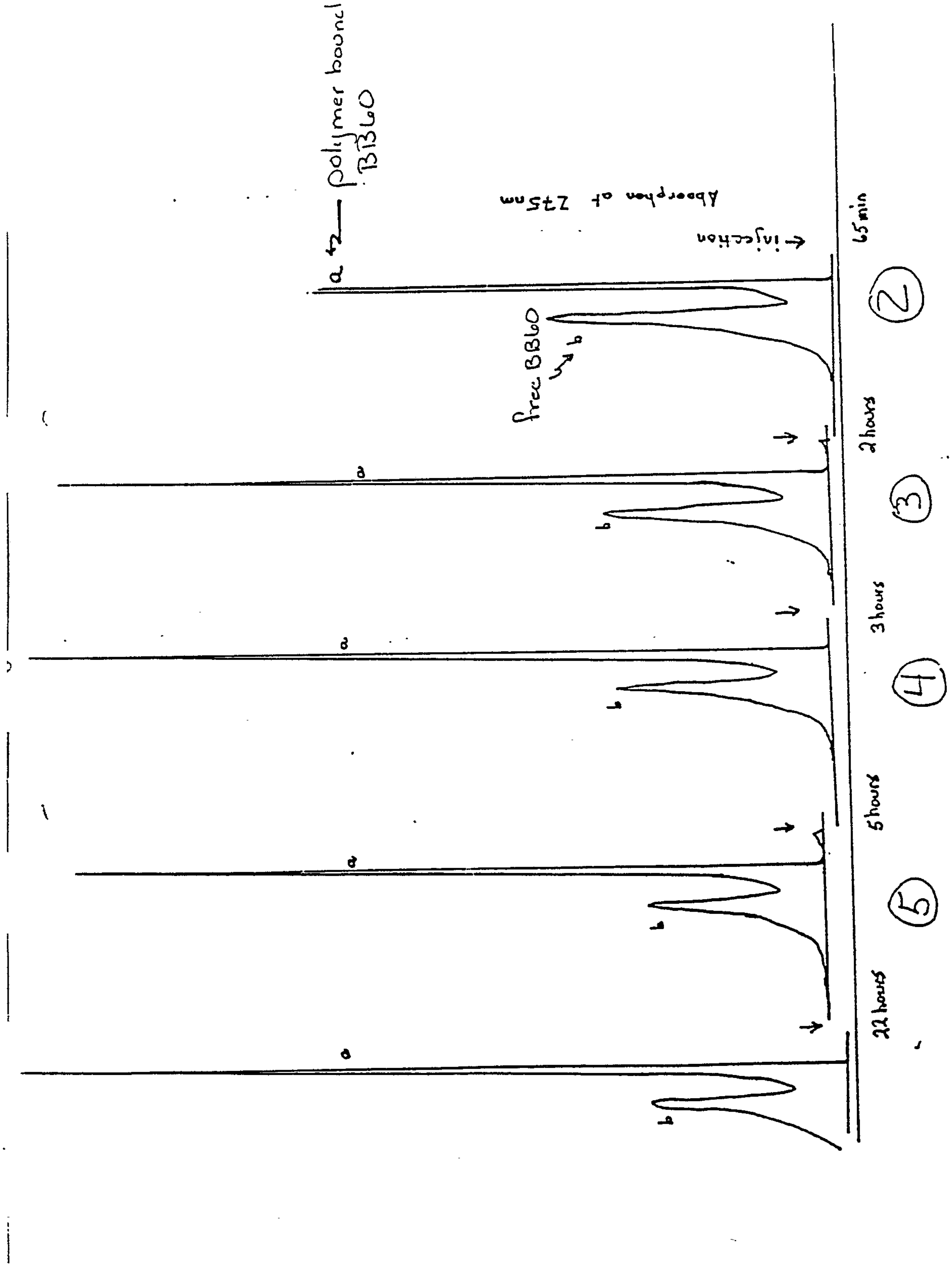

polymer bound BB60
a
Absorption at 275nm
← injection
65 min
free BB60
b
2 hours
3 hours
5 hours
22 hours
2
3
4
5


FIG. 4

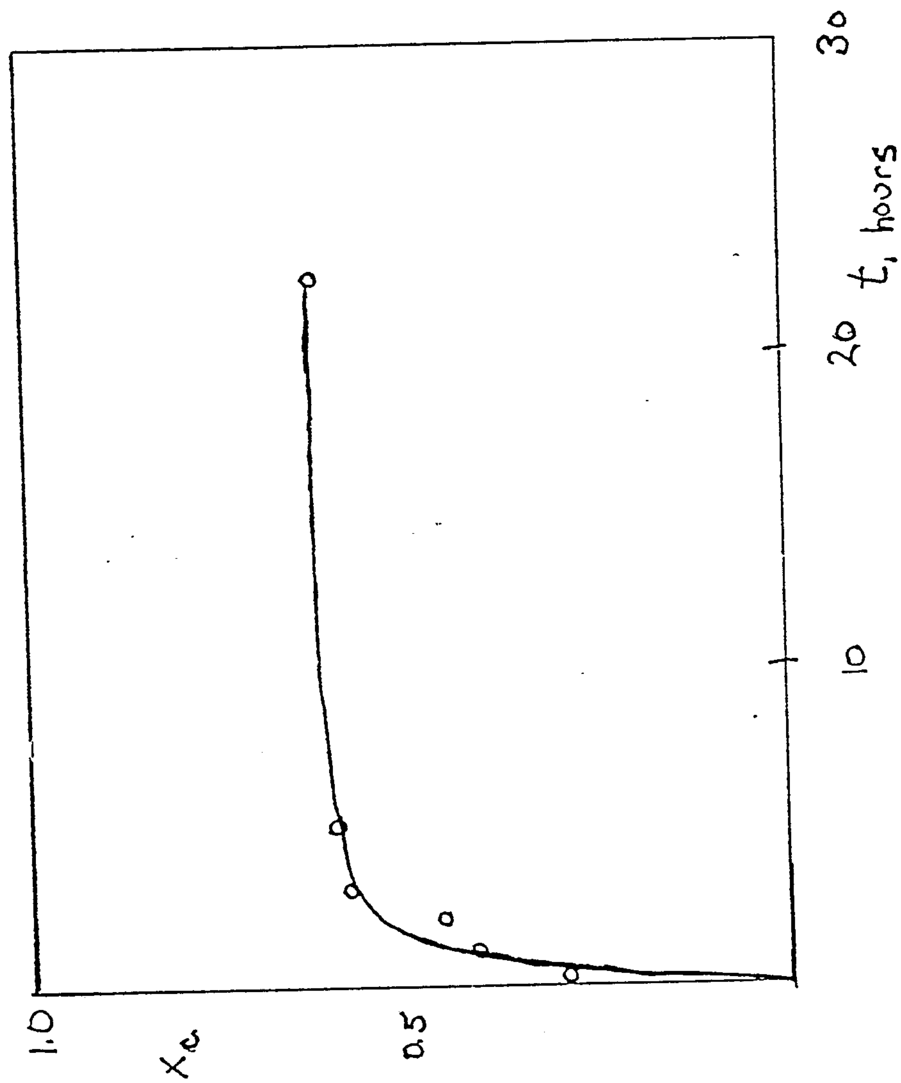


FIG. 5

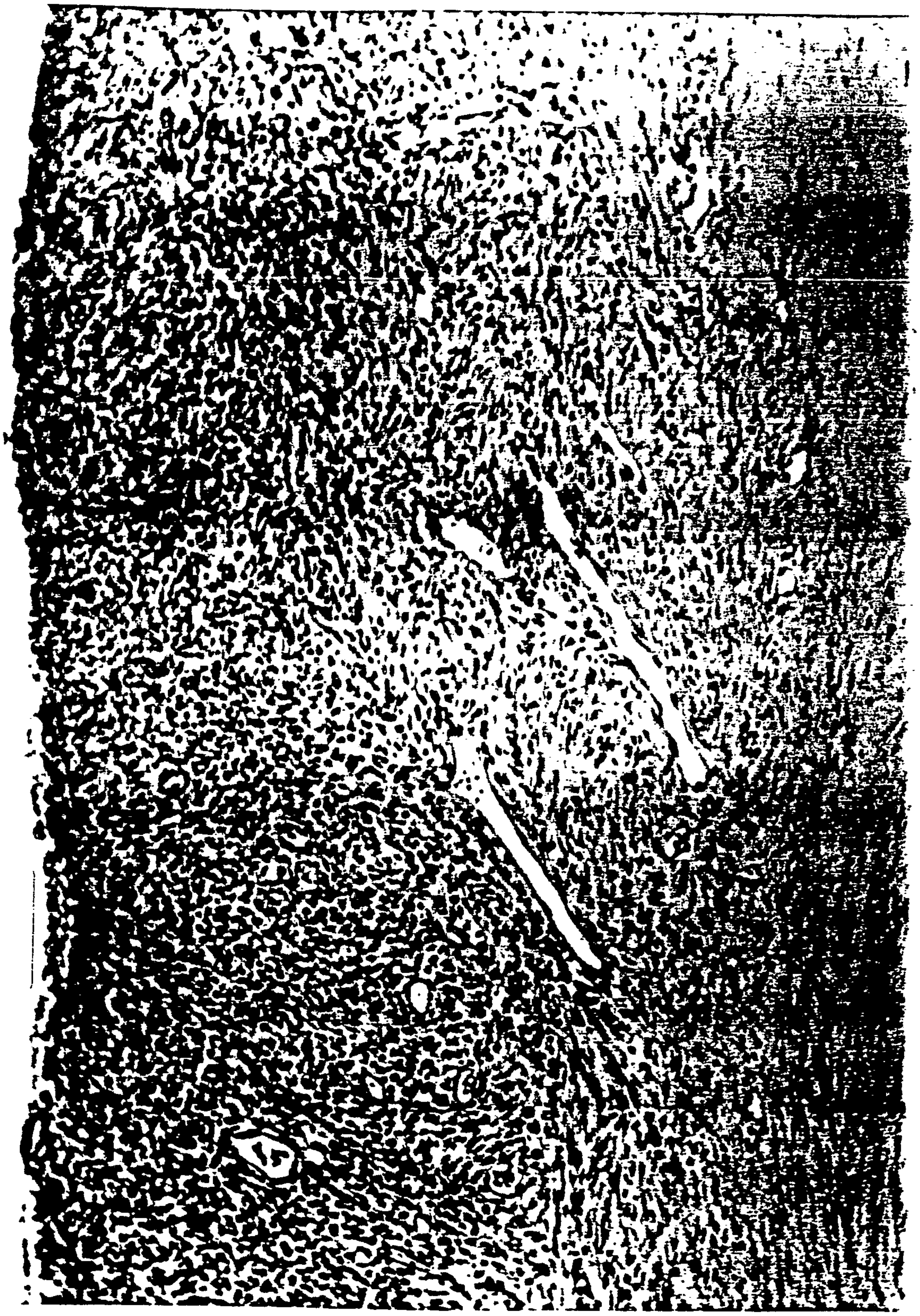

FIG. 6

FIG. 7

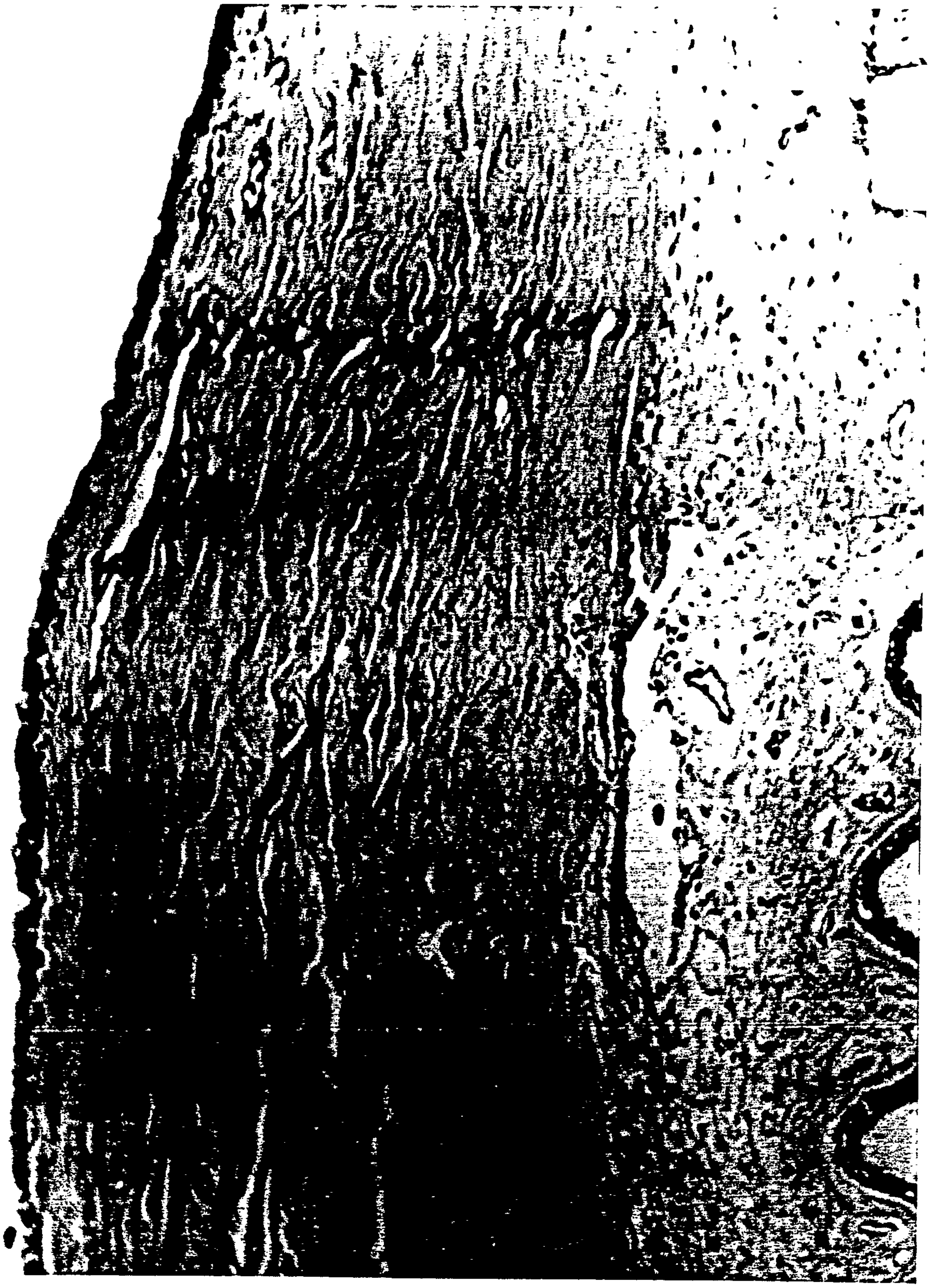

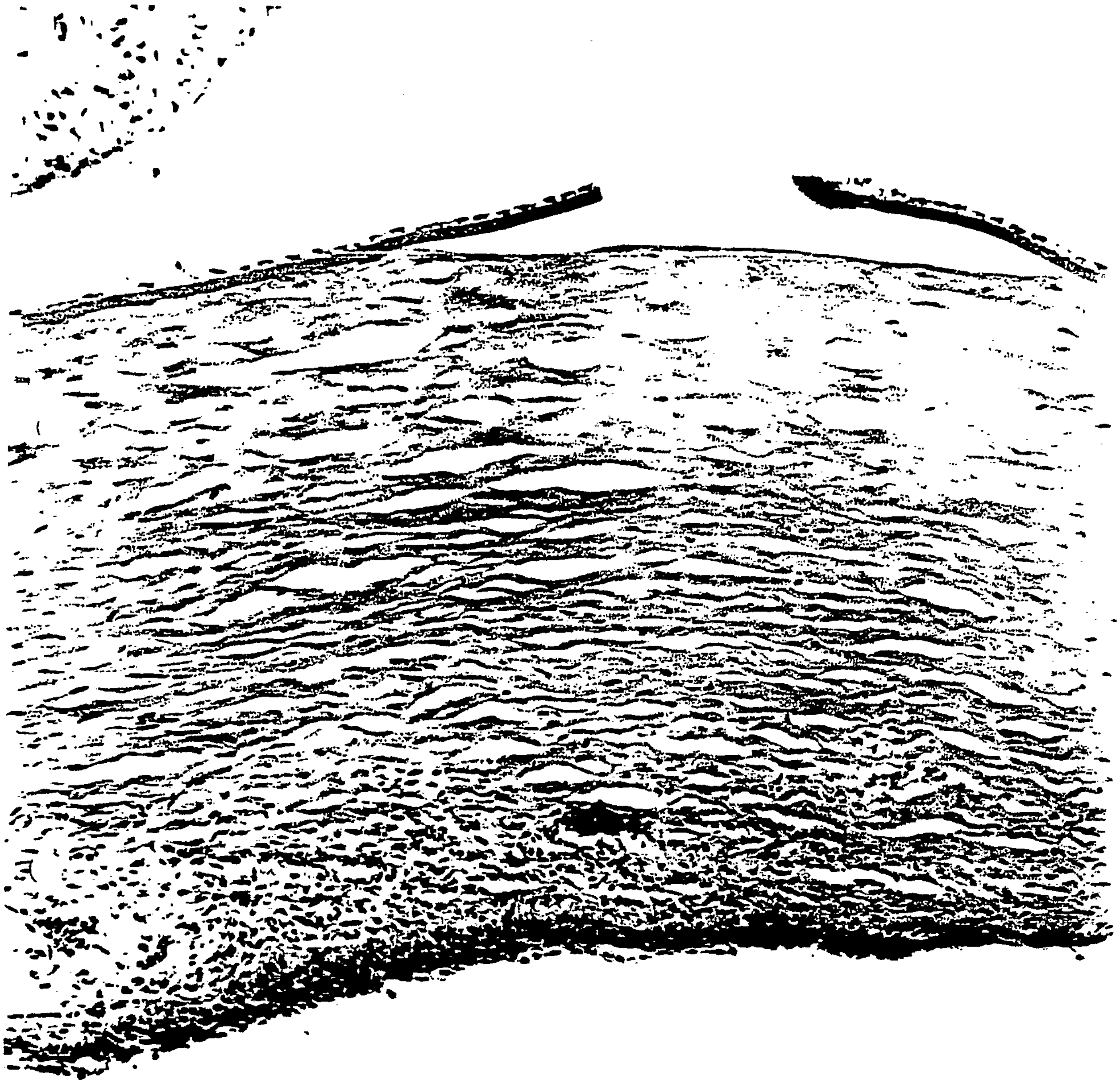

FIG. 8

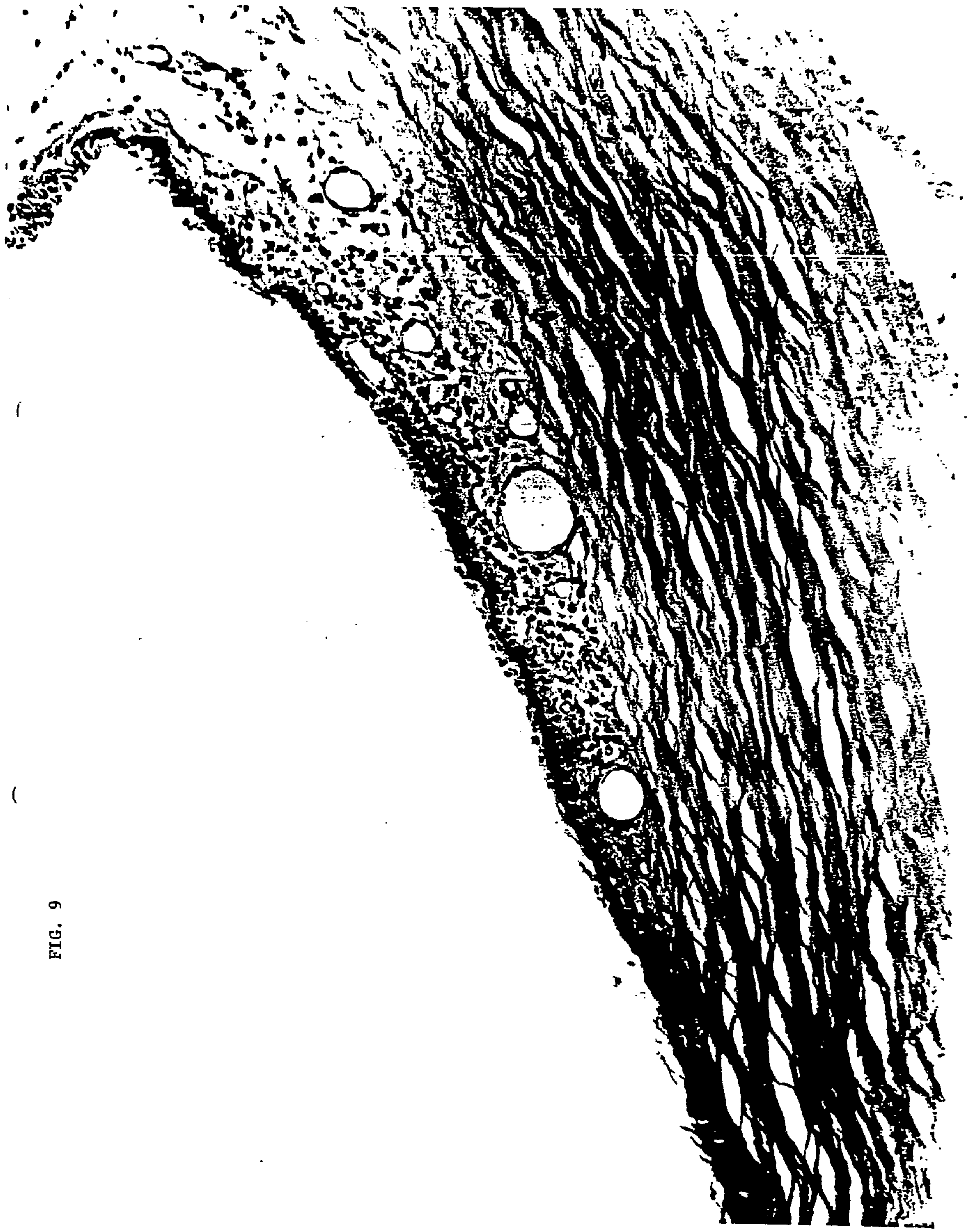

FIG. 9

FIG. 10

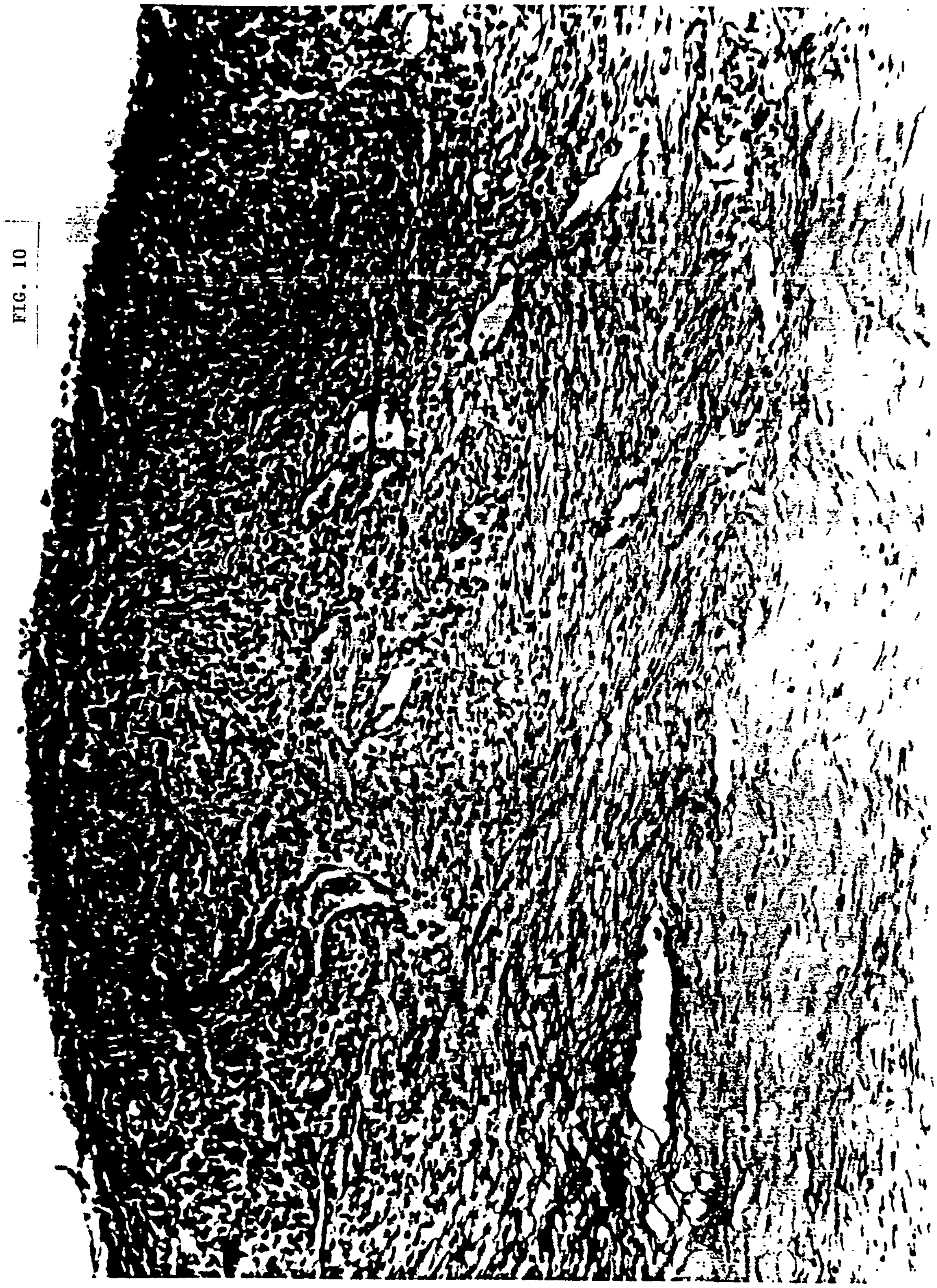

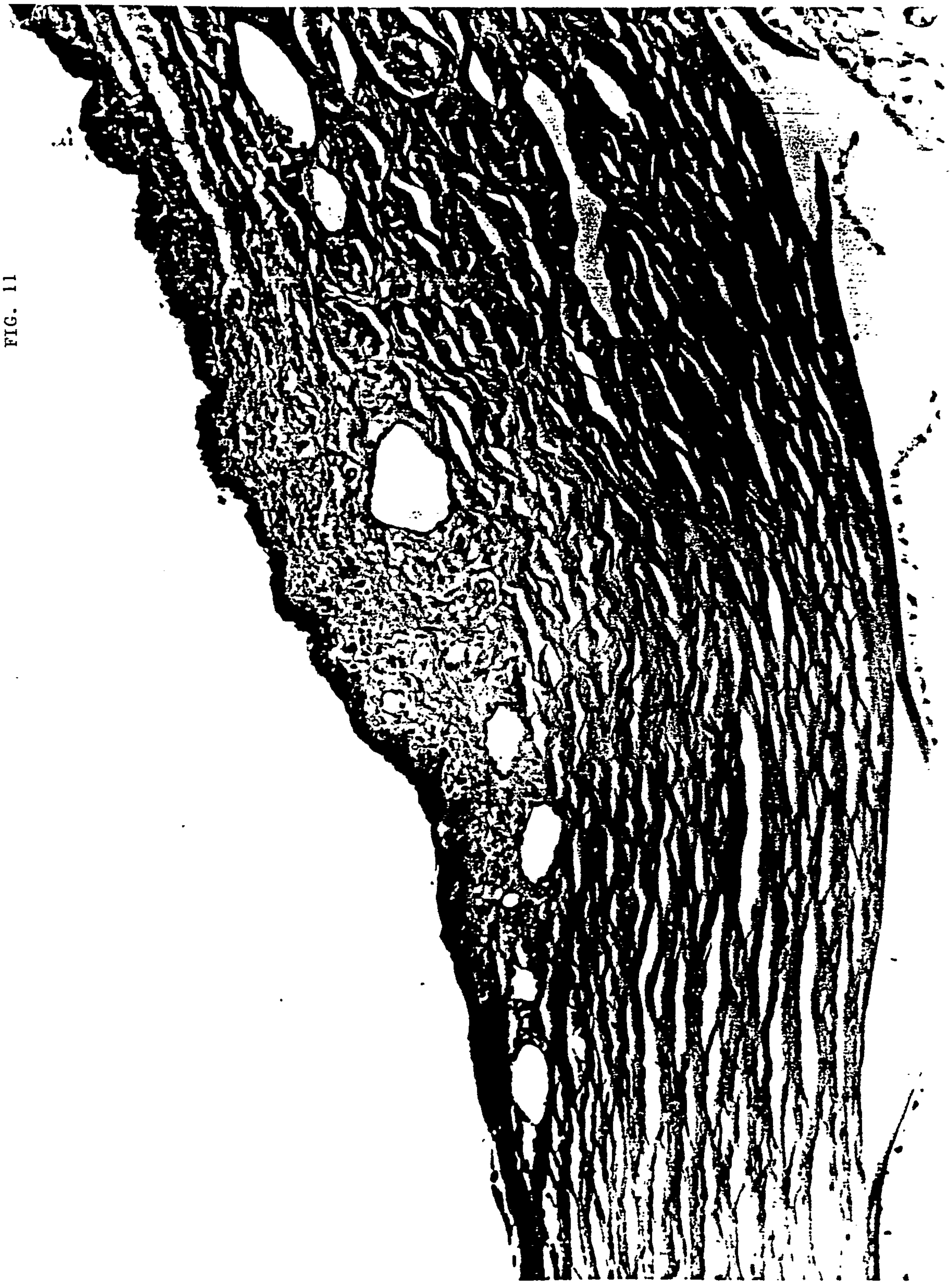

FIG. 11

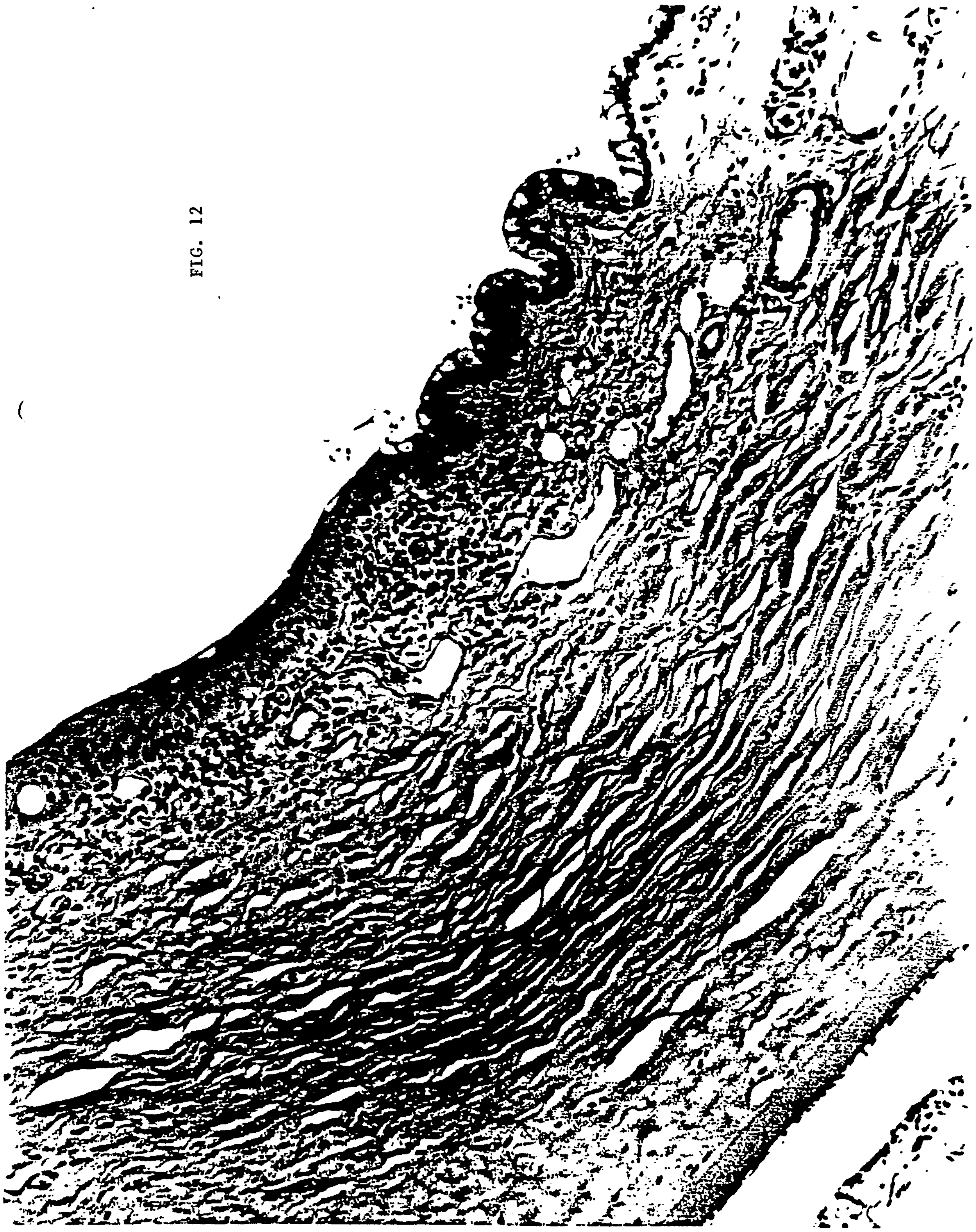

FIG. 12

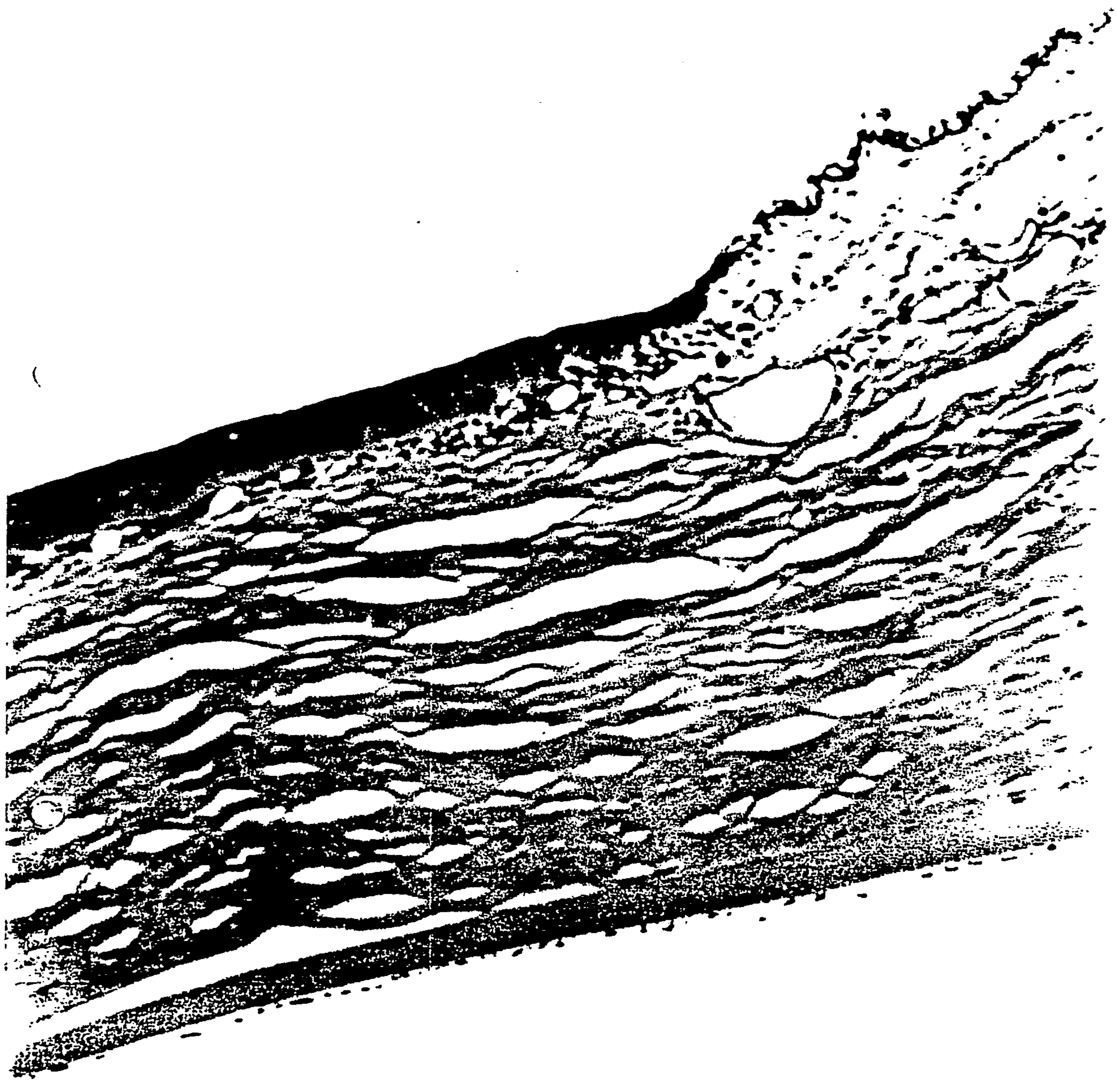

FIG. 13